# EUROPEAN PATENT APPLICATION

(11) **EP 2 695 944 A2**
(43) Date of publication of application: **12.02.2014**
(21) Application number: 13186068.6
(22) Date of filing: 10.08.2009
(51) Int. Cl.: C12N 15/82, C07K 14/415

(54) **Transgenic plants with increased yield**

(30) Priority: 20.08.2008 US 90308 P; 21.08.2008 US 90625 P; 21.08.2008 US 90669 P
(62) Divisional of application: 09781662.3
(71) Applicant: BASF Plant Science GmbH, 67056 Ludwigshafen (DE)
(72) Inventor: McKersie, Bryan, Raleigh, NC 27617 (US); Bruce, Wesley, Raleigh, NC 27613 (US)
(74) Representative: Oswald, Oliver

(57) **Abstract**

Polynucleotides are disclosed which are capable of enhancing yield of a plant transformed to contain such polynucleotides. Also provided are methods of using such polynucleotides and transgenic plants and agricultural products, including seeds, containing such polynucleotides as transgenes.

## Description

This application claims the priority benefit of U.S. provisional patent application serial number 61/090,308, filed August 20, 2008; U.S. provisional patent application serial number 61/090,625, filed August 21, 2008; and U.S. provisional patent application serial number 61/090,669, filed August 21, 2008. The entire contents of each of the above-referenced applications is incorporated herein by reference.

### FIELD OF THE INVENTION

This invention relates generally to transgenic plants which overexpress isolated polynucleotides that encode polypeptides active in lipid metabolism, in specific plant tissues and organelles, thereby improving yield of said plants.

### BACKGROUND OF THE INVENTION

Population increases and climate change have brought the possibility of global food, feed, and fuel shortages into sharp focus in recent years. Agriculture consumes 70% of water used by people, at a time when rainfall in many parts of the world is declining. In addition, as land use shifts from farms to cities and suburbs, fewer hectares of arable land are available to grow agricultural crops. Agricultural biotechnology has attempted to meet humanity' s growing needs through genetic modifications of plants that could increase crop yield, for example, by conferring better tolerance to abiotic stress responses or by increasing biomass.

Crop yield is defined herein as the number of bushels of relevant agricultural product (such as grain, forage, or seed) harvested per acre. Crop yield is impacted by abiotic stresses, such as drought, heat, salinity, and cold stress, and by the size (biomass) of the plant. Traditional plant breeding strategies are relatively slow and have in general not been successful in conferring increased tolerance to abiotic stresses. Grain yield improvements by conventional breeding have nearly reached a plateau in maize. The harvest index, i.e., the ratio of yield biomass to the total cumulative biomass at harvest, in maize has remained essentially unchanged during selective breeding for grain yield over the last hundred years. Accordingly, recent yield improvements that have occurred in maize are the result of the increased total biomass production per unit land area. This increased total biomass has been achieved by increasing planting density, which has led to adaptive phenotypic alterations, such as a reduction in leaf angle, which may reduce shading of lower leaves, and tassel size, which may increase harvest index.

When soil water is depleted or if water is not available during periods of drought, crop yields are restricted. Plant water deficit develops if transpiration from leaves exceeds the supply of water from the roots. The available water supply is related to the amount of water held in the soil and the ability of the plant to reach that water with its root system. Transpiration of water from leaves is linked to the fixation of carbon dioxide by photosynthesis through the stomata. The two processes are positively correlated so that high carbon dioxide influx through photosynthesis is closely linked to water loss by transpiration. As water transpires from the leaf, leaf water potential is reduced and the stomata tend to close in a hydraulic process limiting the amount of photosynthesis. Since crop yield is dependent on the fixation of carbon dioxide in photosynthesis, water uptake and transpiration are contributing factors to crop yield. Plants which are able to use less water to fix the same amount of carbon dioxide or which are able to function normally at a lower water potential have the potential to conduct more photosynthesis and thereby to produce more biomass and economic yield in many agricultural systems.

Agricultural biotechnologists have used assays in model plant systems, greenhouse studies of crop plants, and field trials in their efforts to develop transgenic plants that exhibit increased yield, either through increases in abiotic stress tolerance or through increased biomass. For example, water use efficiency (WUE), is a parameter often correlated with drought tolerance. Studies of a plant' s response to desiccation, osmotic shock, and temperature extremes are also employed to determine the plant' s tolerance or resistance to abiotic stresses.

An increase in biomass at low water availability may be due to relatively improved efficiency of growth or reduced water consumption. In selecting traits for improving crops, a decrease in water use, without a change in growth would have particular merit in an irrigated agricultural system where the water input costs were high. An increase in growth without a corresponding jump in water use would have applicability to all agricultural systems. In many agricultural systems where water supply is not limiting, an increase in growth, even if it came at the expense of an increase in water use also increases yield.

Agricultural biotechnologists also use measurements of other parameters that indicate the potential impact of a transgene on crop yield. For forage crops like alfalfa, silage corn, and hay, the plant biomass correlates with the total yield. For grain crops, however, other parameters have been used to estimate yield, such as plant size, as measured by total plant dry weight, above-ground dry weight, above-ground fresh weight, leaf area, stem volume, plant height, rosette diameter, leaf length, root length, root mass, tiller number, and leaf number. Plant size at an early developmental stage will typically correlate with plant size later in development. A larger plant with a greater leaf area can typically absorb more light and carbon dioxide than a smaller plant and therefore will likely gain a greater weight during the same period. There is a strong genetic component to plant size and growth rate, and so for a range of diverse genotypes plant size under one environmental condition is likely to correlate with size under another. In this way a standard environment is used to approximate the diverse and dynamic environments encountered at different locations and times by crops in the field.

Harvest index is relatively stable under many environmental conditions, and so a robust correlation between plant size and grain yield is possible. Plant size and grain yield are intrinsically linked, because the majority of grain biomass is dependent on current or stored photosynthetic productivity by the leaves and stem of the plant. As with abiotic stress tolerance, measurements of plant size in early development, under standardized conditions in a growth chamber or greenhouse, are standard practices to measure potential yield advantages conferred by the presence of a transgene.

Plant membranes contain diverse molecular species and the composition of these membranes change in response to environmental cues during acclimation processes and as a consequence of cellular injury from environmental stress. Plant membranes are generally considered to be a primary site of injury following exposure to low temperature stress and various forms of oxidative stress such as occur during water deprivation. This degradation may involve the action of specific hydrolytic enzymes or may be the consequence of oxidative reactions mediated by free radicals. In the case of oxidative free radical reactions, degradation may occur at the unsaturated double bonds of the fatty acid acyl chain or at the ester bond linking the fatty acid acyl chain to the glycerol backbone of the phospholipid. This degradation if sufficiently severe will promote cell death, but in more moderate circumstances, degradation products are components of cell signaling mechanisms that promote an acclimation response. During the acclimation process, plants adapt and develop greater tolerance of environmental stress. Coincidently, plants generally alter both the quantity of membrane components in each cell and the composition of those membranes. These alterations in composition are coincident with increased tolerance of the whole plant. Common changes include changes in fatty acid unsaturation and phospholipid head groups. In addition, genetic differences among plants contribute to both differences in membrane composition, stress signaling mechanisms and the whole plant' s ability to tolerate stress. Thus plant membranes are considered to be a central site for perceiving, tolerating and responding to environmental stress.

Phospholipids are the major structural components of biological membranes and also serve as important -signaling molecules. Phospholipids are commonly synthesized in the endoplasmic reticulum and transported to other membranes, but phospholipids can be synthesized in other cell compartments, including the mitochondria and chloroplast. Phospholipases hydrolyze phospholipids, and in plants, three classes of phospholipases have been reported including phospholipase A (PLA), phospholipase C (PLC), and phospholipase D (PLD). PLCs hydrolyze phosphotidylinositol 4,5-bisphosphate (PIP2) to inositol 1,4,5-triphosphate and diacylglycerol, which are components of the inositol signaling pathway.

As set forth above, plants can acclimate to environmental stress through moderate increases in the activity of enzymes that alter fatty acid oxidation. Most eukaryotic cells have two fatty-acid beta-oxidation systems, one in mitochondria and the other in peroxisomes. The Escherichia coli gene B2341 encodes a bifunctional anaerobic fatty acid oxidation complex protein associated with both enoyl-CoA hydratase and 3-hydroxyacyl-CoA epimerase activity. WO 2006/069610 and WO 2007/087815 disclose metabolic changes in plants transformed with the E. coli gene B2341 (SEQ ID NO:21).

Many enzymes exist as proenzymes or zymogens that require activation by a non-protein molecule, or cofactor, in order to exhibit full activity. Cofactors may be loosely categorized as coenzymes, prosthetic groups, or metal activators. A coenzyme is a small, heat-stable organic molecule that readily dissociates from a proenzyme that functions as a carrier of chemical groups between enzymes. Prosthetic groups are firmly bound to the proenzyme and form a permanent part of the protein structure.

The vitamin riboflavin is a component of the coenzymes flavin adenine dinucleotide (FAD) and flavin mononucleotide (FMN), which are required in the enzymatic oxidation of carbohydrates and other electron transport reactions critical for plants in their response to environmental stress conditions. Biosynthesis of riboflavin requires GTP and ribulose-5-phosphate (R5P) as precursors. The microbial riboflavin biosynthesis genes RibA-RibF have been cloned and biochemically characterized.

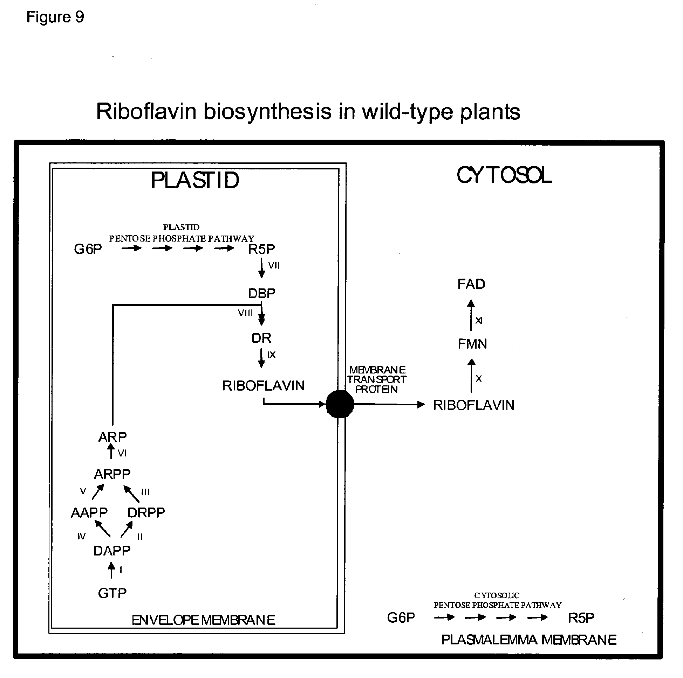

Homologs of RibA, RibB and RibE have been cloned from plants, and based on sequence analysis, the subcellular localization of the plant proteins has been deduced. Proteins that function in the plastid have a typical amino acid domain at the N terminus of the protein that acts as a targeting sequence to direct the protein into the plastid. The plant enzymes involved in riboflavin synthesis contain this plastid targeting sequence, whereas those that encode proteins involved in the biosynthesis of FAD from riboflavin do not. Therefore, the synthesis of the coenzymes FMN and FAD in plants is believed to occur sequentially in the plastid and the cytosol compartments. In wild type plants, the conversion of GTP to 5-amino-6-ribitylamino-2,4 (1 H,3H)-pyrimidinedione (ARP) occurs by several enzymatic reactions that are localized in plastids. The plastid compartment contains a pentose phosphate pathway which forms R5P. Within the plastid, R5P is metabolized to 3,4-dihydroxy-2-butanone 4-phosphate (DBP) which is combined by the enzymatic action of 6,7-dimethyl-8-ribityllumazine synthase (RibH) to form 6,7-dimethyl-8-ribityllumazine (DR). DR is converted to riboflavin by the enzyme riboflavin synthase in the plastid and then transported to the cytosol via an unknown mechanism. In the cytosol, riboflavin is phosphorylated to FMN and converted to FAD. Figure 9 depicts the compartmentalized riboflavin and FAD biosynthetic pathway in wild type plants.

Enzyme I of Figure 9 is GTP cyclohydrolase II (EC 3.4.5.25), or RibA, which catalyzes the first step in riboflavin synthesis. RibA is sometimes found as a bifunctional enzyme with 3,4-dihydroxy-2-butanone 4-phosphate synthase (DHBP_synthase) activity in addition to GTP cyclohydrolase II activity.

Enzyme VIII of Figure 9 is RibH (also known as RibE, riboflavin synthase subunit beta, and lumazine synthase). U.S. Pat. Nos. 6,146,866 and 6,323,013 disclose the cloning of lumazine synthase from spinach, tobacco, Arabidopsis, and Magnaporthe grisea. The chloroplast targeting sequences of the spinach, tobacco, and Arabidopsis RibH polypeptides are identified in U.S. Pat. Nos. 6,146,866 and 6,323,013.

A second class of cofactors is the vitamin group known as vitamin B6. Three compounds belong to the vitamin group: pyridoxal, pyridoxine, and pyridoxamine, all of which are widely distributed in animals and plants, especially in cereal grains. Pyridoxal and pyridoxamine also occur in nature as their phosphate derivatives, pyridoxal 5'-phosphate (PLP) and pyridoxamine 5'-phosphate (PMP), which are the coenzyme forms of the vitamin. PLP participates in catalysis of several important reactions of amino acid metabolism, such as transamination, decarboxylation, and racemization.

De novo synthesis of PLP occurs only in bacteria, fungi, and plants. In Escherichia coli, de novo synthesis occurs through condensation of 4-phosphohydroxy-L-threonine and deoxyxyulose 5-phosphate to form pyridoxine 5'-phosphate (PNP). The condensation reaction is catalyzed by the concerted action of the PdxA and PdxJ enzymes. In the E. coli de novo pathway, PNP is then oxidized by the PdxH oxidase to form PLP. Recently a different de novo PLP biosynthetic pathway has been identified which is independent of deoxyxyulose 5-phosphate. In this pathway, PLP is synthesized from ribose 5-phosphate or R5P and either glyceraldehyde 3-phosphate or dihydroxyacetone phosphate, via the products of two genes designated PDX1 and PDX2, which show no homology to any of the E. coli PLP synthetic genes. The PDX1 and PDX2 gene products are predicted to function as a glutamine amidotransferase, with PDX2 as the glutaminase domain and PDX1 as the acceptor/synthase domain. The pdx1 gene product of the filamentous fungus Cercospora nicotianae is highly homologous to a conserved gene family designated SOR1, which is widespread in archeabacteria, eubacteria, plants, and fungi. The two pathways of PLP de novo synthesis are autoexclusive, that is, organisms have the genes for one or the other pathway, but not both.

PLP may also be synthesized by a second pathway, designated a salvage pathway, through which pyridoxal (PL), pyridoxine (PN), and pyridoxamine (PM) taken up from the cell' s growth medium. The salvage pathway is present in addition to the de novo synthetic pathway in E. coli, and is the only means by which mammalian cells can make PLP. In the E. coli salvage pathway, PL, PN, and PM are first phosphorylated by kinases to form PLP, pyridoxine 5'-phosphate (PNP), and PMP, respectively. PNP and PMP are oxidized by the PdxH oxidase referenced above. The pdxK gene encodes a PN/PUPM kinase, and the pdxY gene encodes a PL kinase, and the products of both genes share a number of conserved motifs with the PfkB superfamily of carbohydrate kinases. Homologs of the PdxK and PdxY kinases have been identified from humans, Trypanosoma brucei, Haemophilus influenzae, Caenorhabditis elegans, Rattus norvegicus, Saccharomyces cerevisiae, and Salmonella typhimurium.

Although some genes that are involved in stress responses, water use, and/or biomass in plants have been characterized, to date, success at developing transgenic crop plants with improved yield has been limited, and no such plants have been commercialized. There is a need, therefore, to identify additional genes that have the capacity to increase yield of crop plants.

### SUMMARY OF THE INVENTION

The present inventors have discovered that there are three critical components that must be optimized to achieve improvement in plant yield through transgenic expression of certain polypeptides When targeted under the regulatory elements as described herein, the polynucleotides and polypeptides set forth in Table 1 are capable of improving yield of transgenic plants

**Table 1**

| Gene Name | Organism | Polynucleotide SEQ ID NO: | Amino acid SEQ ID NO: |
|---|---|---|---|
| YBR029C | S. cerevisiae | 1 | 2 |
| BN04MC30805 | Brassica napus | 3 | 4 |
| ZM06MC30283 | Zea mays | 5 | 6 |
| YKL192C | S. cerevisiae | 7 | 8 |
| BN1004MS43616414 | B. napus | 9 | 10 |
| GM06MC07589 | G. max | 11 | 12 |
| HA1004MS66693619 | Helianthus annuus | 13 | 14 |
| YDR018C | S. cerevisiae | 15 | 16 |
| GM06MC27072 | G. max | 17 | 18 |
| ZM06MC04863 | Z. mays | 19 | 20 |
| B2341 | E. coli | 21 | 22 |
| ZM06MC04303 | Z. mays | 23 | 24 |
| ZM06MC15742 | Z. mays | 25 | 26 |
| B0452 | E. coli | 27 | 28 |
| BN42634969 | B. napus | 29 | 30 |
| BNP5302_30 | B. napus | 31 | 32 |
| GMsae90f11 | G. max | 33 | 34 |
| YNL202W | S. cerevisiae | 35 | 36 |
| HA66688442 | Helianthus annuus | 37 | 38 |
| YKL140W | S. cerevisiae | 39 | 40 |
| SLL1023 | Synechocystis sp. PCC 6803 | 41 | 42 |
| SLR0252 | Synechocystis sp. | 43 | 44 |
| b3803 | E. coli | 45 | 46 |
| BN51286476 | B. napus | 47 | 48 |
| GM59791864 | G. max | 49 | 50 |
| ZMBFb0243J04 | Z. mays | 51 | 52 |
| b3209 | E. coli | 53 | 54 |
| GMss34d01 | G. max | 55 | 56 |
| HA03MC1392 | H. annuus | 57 | 58 |
| b2578 | E. coli | 59 | 60 |
| b2682 | E. coli | 61 | 62 |
| b3285 | E. coli | 63 | 64 |
| b1938 | E. coli | 65 | 66 |
| SLL1894 | S. sp. PCC 6803 | 67 | 68 |
| GM08000037 | G. max | 69 | 70 |
| SLL1282 | S. sp. PCC 6803 | 71 | 72 |
| GM06MC29296 | G. max | 73 | 74 |
| ZM07MC00430 | Z. mays | 75 | 76 |
| ZM07MC23187 | Z. mays | 77 | 78 |
| b1636 | E.coli | 79 | 80 |
| pdxH | E.coli | 81 | 82 |
| ECpdxK | E.coli | 83 | 84 |
| TBpdxK | T rypanosoma brucei | 85 | 86 |
| CEpdxK | Caenorhabditis elegans | 87 | 88 |
| STyfei | Salmonella typhimurium | 89 | 90 |
| Hlyfei | Haemophilus. influenzae | 91 | 92 |
| Yn8fp | S. cerevisiae | 93 | 94 |
| SLR1779 | S. sp. PCC 6803 | 95 | 96 |
| pdxJ | E. coli | 97 | 98 |
| pdx1.1 | A. thaliana | 99 | 100 |
| pdx1.3 | A. thaliana | 101 | 102 |
| CNpdx1 | Cercospora nicotianae | 103 | 104 |
| SCpdx1 | S. cerevisiae | 105 | 106 |
| BSpdx1 | Bacillus subtilis | 107 | 108 |
| OSpdx1 | Oryza sativa | 109 | 110 |
| HBpdx1 | Hevea brasiliensis | 111 | 112 |
| SLpdx1 | Stellaria longipes | 113 | 114 |
| BNpdx1 | B. napus | 115 | 116 |
| PPpdx1 | Physcomitrella patens | 117 | 118 |
| SPpdx1 | Schizosaccharomyce s pombe | 119 | 120 |

In one embodiment, the invention provides a transgenic plant transformed with an expression cassette comprising, in operative association, an isolated polynucleotide encoding a promoter capable of enhancing gene expression in roots and shoots; an isolated polynucleotide encoding a subcellular targeting peptide; and an isolated polynucleotide encoding a full-length phosphatidate cytidylyltransferase polypeptide, wherein the transgenic plant demonstrates increased yield as compared to a wild type plant of the same variety which does not comprise the expression cassette.

In another embodiment, the invention provides a transgenic plant transformed with an expression cassette comprising in operative association, an isolated polynucleotide encoding a promoter capable of enhancing expression in leaves; an isolated polynucleotide encoding a mitochondrial transit peptide; and an isolated polynucleotide encoding a full-length acyl-carrier protein, wherein the transgenic plant demonstrates increased yield as compared to a wild type plant of the same variety which does not comprise the expression cassette.

In another embodiment, the invention provides a transgenic plant transformed with an expression cassette comprising, in operative association, isolated polynucleotide encoding a promoter; an isolated polynucleotide encoding a subcellular targeting peptide; and an isolated polynucleotide encoding a full-length acyltransferase polypeptide; wherein the transgenic plant demonstrates increased yield as compared to a wild type plant of the same variety which does not comprise the expression cassette.

In another embodiment, the invention provides a method of increasing yield of a plant by transforming a wild-type plant with an expression cassette comprising, in operative association, an isolated polynucleotide encoding a promoter capable of enhancing gene expression in leaves; an isolated polynucleotide encoding a mitochondrial transit peptide; and an isolated polynucleotide encoding a full-length bifunctional anaerobic fatty acid oxidation complex polypeptide, regenerating transgenic plants from the transformed plant cell, and selecting higher-yielding plants from the transgenic plants.

In another embodiment, the invention provides a method of increasing yield of a plant by transforming a wild-type plant with an expression cassette comprising, in operative association, an isolated polynucleotide encoding a promoter capable of enhancing gene expression in leaves; an isolated polynucleotide encoding a subcellular targeting peptide; and an isolated polynucleotide encoding a full-length acyl-CoA thioesterase polypeptide; wherein the transgenic plant demonstrates increased yield as compared to a wild type plant of the same variety which does not comprise the expression cassette.

In another embodiment, the invention provides a method of increasing yield of a plant by transforming a wild-type plant with an expression cassette comprising, in operative association, an isolated polynucleotide encoding a promoter; an isolated polynucleotide encoding a subcellular targeting peptide; and an isolated polynucleotide encoding a full-length sterol esterase polypeptide; wherein the transgenic plant demonstrates increased yield as compared to a wild type plant of the same variety which does not comprise the expression cassette.

In another embodiment, the invention provides a method of increasing yield of a plant by transforming a wild-type plant with an expression cassette comprising, in operative association, an isolated polynucleotide encoding a promoter capable of enhancing gene expression in leaves; an isolated polynucleotide encoding a mitochondrial targeting peptide; and an isolated polynucleotide encoding a full-length 2,4-dienoyl-CoA reductase polypeptide; wherein the transgenic plant demonstrates increased yield as compared to a wild type plant of the same variety which does not comprise the expression cassette.

In another embodiment, the invention provides a method of increasing yield of a plant by transforming a wild-type plant with an expression cassette comprising, in operative association, an isolated polynucleotide encoding a promoter; an isolated polynucleotide encoding a plastid transit peptide; and an isolated polynucleotide encoding a full-length succinate-CoA ligase polypeptide; wherein the transgenic plant demonstrates increased yield as compared to a wild type plant of the same variety which does not comprise the expression cassette.

In another embodiment, the invention provides a method of increasing yield of a plant by transforming a wild-type plant with an expression cassette comprising, in operative association, an isolated polynucleotide encoding a promoter and an isolated polynucleotide encoding a full-length cobalt-precorrin-6A reductase polypeptide; wherein the transgenic plant demonstrates increased yield as compared to a wild type plant of the same variety which does not comprise the expression cassette.

In another embodiment, the invention provides a transgenic plant transformed with an expression cassette comprising, in operative association, an isolated polynucleotide encoding a promoter and an isolated polynucleotide encoding a full-length polypeptide having uroporphyrin-III C-methyltransferase activity and a HemX signature sequence; wherein the transgenic plant demonstrates increased yield as compared to a wild type plant of the same variety which does not comprise the expression cassette.

In another embodiment, the invention provides a transgenic plant transformed with an expression cassette comprising in operative association, an isolated polynucleotide encoding a promoter capable of enhancing gene expression in roots and shoots and an isolated polynucleotide encoding a full-length polypeptide having isoprenoid biosynthesis activity and a DJ-1_Pfp1 signature sequence; wherein the transgenic plant demonstrates increased yield as compared to a wild type plant of the same variety which does not comprise the expression cassette.

In another embodiment, the invention provides a transgenic plant transformed with an expression cassette comprising, in operative association, an isolated polynucleotide encoding a promoter capable of enhancing gene expression in roots and shoots; and an isolated polynucleotide encoding a a full-length polypeptide having LysE type translocator activity and a LysE signature sequence comprising amino acids 14 to 195 of SEQ ID NO:60; wherein the transgenic plant demonstrates increased yield as compared to a wild type plant of the same variety which does not comprise the expression cassette.

In another embodiment, the invention provides a transgenic plant transformed with an expression cassette comprising, in operative association, an isolated polynucleotide encoding a promoter capable of enhancing gene expression in roots and shoots and an isolated polynucleotide encoding a full-length polypeptide having LIV-E family branched-chain amino acid transport activity and an AzIC signature sequence; wherein the transgenic plant demonstrates increased yield as compared to a wild type plant of the same variety which does not comprise the expression cassette.

In another embodiment, the invention provides a transgenic plant transformed with an expression cassette comprising, in operative association, an isolated polynucleotide encoding a promoter capable of enhancing gene expression in roots and shoots; and an isolated polynucleotide encoding a truncated DNA-binding polypeptide having a sequence comprising amino acids 1 to 102 of SEQ ID NO:64; wherein the transgenic plant demonstrates increased yield as compared to a wild type plant of the same variety which does not comprise the expression cassette.

In another embodiment, the invention provides a transgenic plant transformed with an expression cassette comprising, in operative association, an isolated polynucleotide encoding a promoter capable of enhancing gene expression in roots and shoots and an isolated polynucleotide encoding a full-length polypeptide having a first YscJ_FliF signature sequence and a second YscJ_FliF signature sequence; wherein the transgenic plant demonstrates increased yield as compared to a wild type plant of the same variety which does not comprise the expression cassette.

In another embodiment, the invention provides a transgenic plant transformed with an expression cassette comprising, in operative association, an isolated polynucleotide encoding a promoter; and an isolated polynucleotide encoding a full length GTP cyclohydrolase II polypeptide which does not comprise a subcellular targeting peptide; wherein the transgenic plant demonstrates increased yield as compared to a wild type plant of the same variety that does not comprise the expression cassette.

In another embodiment, the invention provides a transgenic plant transformed with an expression cassette comprising, in operative association, an isolated polynucleotide encoding a promoter and an isolated polynucleotide encoding a full length lumazine synthase polypeptide which does not comprise a subcellular targeting peptide; wherein the transgenic plant demonstrates increased yield as compared to a wild type plant of the same variety that does not comprise the expression cassette.

In another embodiment, the invention provides a transgenic plant transformed with an expression cassette comprising, in operative association, an isolated polynucleotide encoding a promoter and an isolated polynucleotide encoding a full length polypeptide capable of enhancing pyridoxal 5'-phosphate synthesis; wherein the transgenic plant demonstrates increased yield as compared to a wild type plant of the same variety that does not comprise the expression cassette. In this embodiment, the expression cassette may further comprise a polynucleotide encoding a mitochondrial or plastid transit peptide.

In a further embodiment, the invention provides a seed produced by the transgenic plants described above, wherein the seed is true breeding for a transgene comprising the expression vectors described above. Plants derived from the seed of the invention demonstrate increased tolerance to an environmental stress, and/or increased plant growth, and/or increased yield, under normal or stress conditions as compared to a wild type variety of the plant.

In a still another aspect, the invention concerns products produced by or from the transgenic plants of the invention, their plant parts, or their seeds, such as a foodstuff, feedstuff, food supplement, feed supplement, fiber, cosmetic or pharmaceutical.

The invention further provides certain isolated polynucleotides identified in Table 1, and certain isolated polypeptides identified in Table 1. The invention is also embodied in recombinant vector comprising an isolated polynucleotide of the invention.

In yet another embodiment, the invention concerns a method of producing the aforesaid transgenic plant, wherein the method comprises transforming a plant cell with an expression vector comprising an isolated polynucleotide of the invention, and generating from the plant cell a transgenic plant that expresses the polypeptide encoded by the polynucleotide. Expression of the polypeptide in the plant results in increased tolerance to an environmental stress, and/or growth, and/or yield under normal and/or stress conditions as compared to a wild type variety of the plant.

In still another embodiment, the invention provides a method of increasing a plant' s tolerance to an environmental stress, and/or growth, and/or yield. The method comprises the steps of transforming a plant cell with an expression cassette comprising an isolated polynucleotide of Table 1, and generating a transgenic plant from the plant cell, wherein the transgenic plant comprises the polynucleotide.

### BRIEF DESCRIPTION OF THE DRAWINGS

Figure 1 shows an alignment of the amino acid sequences of full-length phosphatidate cytidylyltransferase polypeptide designated YBR029C (SEQ ID NO:2), BN04MC30805 (SEQ ID NO:4) and ZM06MC30283 (SEQ ID NO:6) The alignment was generated using Align X of Vector NTI Advance 10.3.0.

Figure 2 shows an alignment of the amino acid sequences of the acyl-carrier proteins designated YKL192C (SEQ ID NO:8), BN1004MS43616414 (SEQ ID NO:10), GM06MC07589(SEQ ID NO:12), and HA1004MS66693619 (SEQ ID NO:14) . The alignment was generated using Align X of Vector NTI Advance 10.3.0.

Figure 3 shows an alignment of the amino acid sequences of the acyltransferases designated: YDR018C (SEQ ID NO:16), GM06MC27072 (SEQ ID NO:18) and ZM06MC04863 (SEQ ID NO:20). The alignment was generated using Align X of Vector NTI Advance 10.3.0.

Figure 4 shows an alignment of the amino acid sequences encoding bifunctional anaerobic fatty acid oxidation complex polypeptides designated b2341 (SEQ ID NO:22), ZM06MC04303 (SEQ ID NO:24) and ZM06MC15742 (SEQ ID NO:26). The alignment was generated using Align X of Vector NTI Advance 10.3.0.

Figure 5 shows an alignment of the amino acid sequences encoding acyl-CoA thioesterase polypeptides designated B0452 (SEQ ID NO:28), BN42634969 (SEQ ID NO:30), BNP5302_30 (SEQ ID NO:32), and GMsae90f11 (SEQ ID NO:34). The alignment was generated using Align X of Vector NTI Advance 10.3.0.

Figure 6 shows an alignment of the amino acid sequences encoding 2,4-dienoyl-CoA reductase polypeptides designated YNL202W (SEQ ID NO:36) and HA66688442 (SEQ ID NO:38). The alignment was generated using Align X of Vector NTI Advance 10.3.0.

Figure 7 shows an alignment of the amino acid sequences of uroporphyrin-III C-methyltransferases designated b3803 (SEQ ID NO:46), BN51286476 (SEQ ID NO:48), GM59791864 (SEQ ID NO:50), and ZMBFb0243J04 (SEQ ID NO:52). The alignment was generated using Align X of Vector NTI Advance 10.3.0.

Figure 8 shows an alignment of the amino acid sequences of the isoprenoid biosynthesis proteins designated b3209 (SEQ ID NO:54), GMss34d01 (SEQ ID NO:56), and HA03MC1392 (SEQ ID NO:58). The alignment was generated using Align X of Vector NTI Advance 10.3.0.

Figure 9 shows a flow diagram of the riboflavin/FAD biosynthesis pathway in wild type plants. Enzyme designations are as follows: Enzyme I is GTP cyclohydrolase II (RibA); Enzyme II is 2,5-diamino-6-ribosylamino-4 (3H)-pyrimidinone 50-phosphate deaminase; Enzyme III is 5-amino-6-ribosylamino-2,4 (1 H,3H)-pyrimidinedione 50-phosphate reductase; Enzyme IV is 2,5-diamino-6-ribosylamino-4 (3H)-pyrimidinone 50-phosphate reductase; Enzyme V is 2,5-diamino-6-ribitylamino-4 (3H)-pyrimidinedione 50-phosphate deaminase; Enzyme VI is a hypothetical phosphatase; Enzyme VII is 3,4-dihydroxy-2-butanone-4-phosphate synthase; Enzyme VIII is 6,7-dimethyl-8-ribityllumazine synthase (RibH); Enzyme IX is riboflavin synthase; Enzyme X is riboflavin kinase; and Enzyme XI is FAD synthetase. Intermediates in the biosynthesis of riboflavin and FAD are GTP; DAPP (2,5-diamino-6-ribosylamino-4 (3H)-pyrimidinone 50-phosphate); AAPP (5-amino-6-ribosylamino-2,4 (1H,3H)-pyrimidinedione 50-phosphate); DRPP (2,5-diamino-6-ribitylamino-4 (3H)-pyrimidinedione 50-phosphate); ARPP (5-amino-6-ribitylamino-2,4 (1 H,3H)-pyrimidinedione 50-phosphate); ARP; G6P (Glucose-6-phosphate); riboflavin; with R5P, DBP, DR, FMN, and FAD as set forth above.

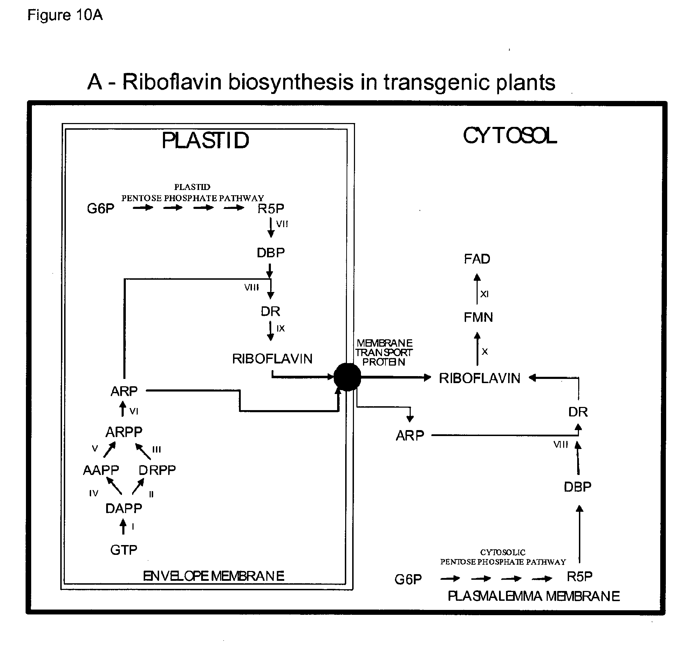

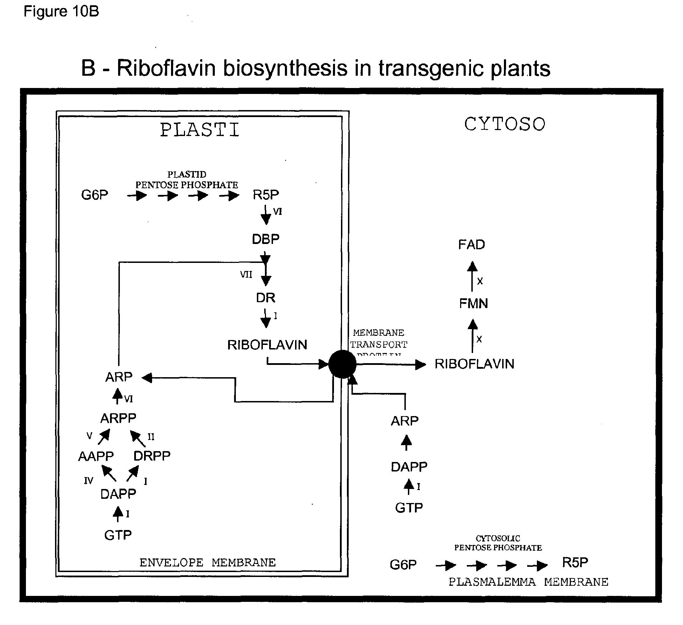

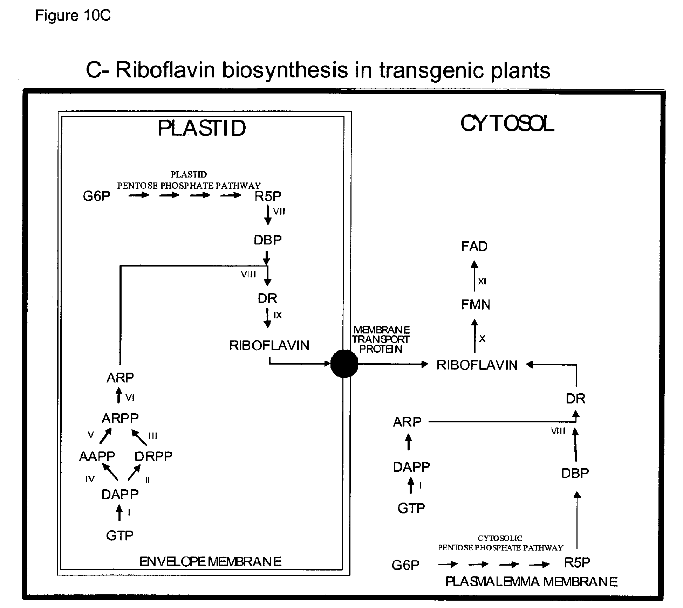

Figure 10A shows a flow diagram of the proposed riboflavin/FAD biosynthesis pathway in the transgenic plants of the invention. Abbreviations are as set forth in Figure 9. Figure 10A shows the pathway with RibH targeted to the cytosol. Figure 10B shows the pathway with RibA targeted to the cytosol; Figure 10C shows the pathway with both RibH and RibA targeted to the cytosol.

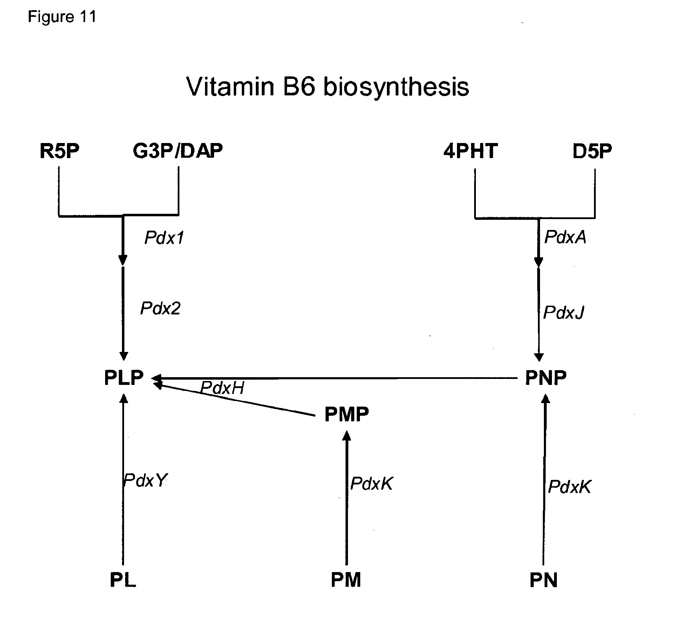

Figure 11 shows a flow diagram of vitamin B6 synthesis as it relates to the present invention. Abbreviations: DAP: dihydroxyacetone-P; G3P: glyceraldehyde-3P; with PL, PLP, PM, PN, PNP, and R5P as set forth above.

Figure 12 shows an alignment of the amino acid sequences of GTP cyclohydrolase II designated SLL1894 (SEQ ID NO:68) and Gm018000037 (SEQ ID NO:70), The alignment was generated using Align X of Vector NTI Advance 10.3.0. Amino acids 1 to 49 of SEQ ID NO:70 correspond to the subcellular targeting sequence.

Figure 13 shows an alignment of the amino acid sequences of the lumazine synthase polypeptides designated SLL1282 (SEQ ID NO:72), GM06MC29296 (SEQ ID NO:74), ZM07MC00430 (SEQ ID NO:76) and ZM07MC23187 (SEQ ID NO:78). The alignment was generated using Align X of Vector NTI. Advance 10.3.0. Subcellular targeting sequences correspond to amino acids 1 to 53 of SEQ ID NO:74; amino acids 1 to 56 of SEQ ID NO:76; and amino acids 1 to 80 of SEQ ID NO:78.

### DETAILED DESCRIPTION OF THE PREFERRED EMBODIMENTS

Throughout this application, various publications are referenced. The disclosures of all of these publications and those references cited within those publications in their entireties are hereby incorporated by reference into this application in order to more fully describe the state of the art to which this invention pertains. The terminology used herein is for the purpose of describing specific embodiments only and is not intended to be limiting. As used herein, " a" or " an" can mean one or more, depending upon the context in which it is used. Thus, for example, reference to " a cell" can mean that at least one cell can be used.

In one embodiment, the invention provides a transgenic plant that overexpresses an isolated polynucleotide identified in Table 1 in the subcellular compartment and tissue indicated herein. The transgenic plant of the invention demonstrates an improved yield as compared to a wild type variety of the plant. As used herein, the term "improved yield" means any improvement in the yield of any measured plant product, such as grain, fruit or fiber. In accordance with the invention, changes in different phenotypic traits may improve yield. For example, and without limitation, parameters such as floral organ development, root initiation, root biomass, seed number, seed weight, harvest index, tolerance to abiotic environmental stress, reduction of nutrient, e.g., nitrogen or phosphorus, input requirement, leaf formation, phototropism, apical dominance, and fruit development, are suitable measurements of improved yield. Any increase in yield is an improved yield in accordance with the invention. For example, the improvement in yield can comprise a 0.1%, 0.5%, 1 %, 3%, 5%, 10%, 15%, 20%, 30%, 40%, 50%, 60%, 70%, 80%, 90% or greater increase in any measured parameter. For example, an increase in the bu/acre yield of soybeans or corn derived from a crop comprising plants which are transgenic for the nucleotides and polypeptides of Table 1, as compared with the bu/acre yield from untreated soybeans or corn cultivated under the same conditions, is an improved yield in accordance with the invention.

As defined herein, a " transgenic plant" is a plant that has been altered using recombinant DNA technology to contain an isolated nucleic acid which would otherwise not be present in the plant. As used herein, the term " plant" includes a whole plant, plant cells, and plant parts. Plant parts include, but are not limited to, stems, roots, ovules, stamens, leaves, embryos, meristematic regions, callus tissue, gametophytes, sporophytes, pollen, microspores, and the like. The transgenic plant of the invention may be male sterile or male fertile, and may further include transgenes other than those that comprise the isolated polynucleotides described herein.

As used herein, the term " variety" refers to a group of plants within a species that share constant characteristics that separate them from the typical form and from other possible varieties within that species. While possessing at least one distinctive trait, a variety is also characterized by some variation between individuals within the variety, based primarily on the Mendelian segregation of traits among the progeny of succeeding generations. A variety is considered " true breeding" for a particular trait if it is genetically homozygous for that trait to the extent that, when the true-breeding variety is self-pollinated, a significant amount of independent segregation of the trait among the progeny is not observed. In the present invention, the trait arises from the transgenic expression of one or more isolated polynucleotides introduced into a plant variety. As also used herein, the term " wild type variety" refers to a group of plants that are analyzed for comparative purposes as a control plant, wherein the wild type variety plant is identical to the transgenic plant (plant transformed with an isolated polynucleotide in accordance with the invention) with the exception that the wild type variety plant has not been transformed with an isolated polynucleotide of the invention. The term " wild type" as used herein refers to a plant cell, seed, plant component, plant tissue, plant organ, or whole plant that has not been genetically modified with an isolated polynucleotide in accordance with the invention.

The term " control plant" as used herein refers to a plant cell, an explant, seed, plant component, plant tissue, plant organ, or whole plant used to compare against transgenic or genetically modified plant for the purpose of identifying an enhanced phenotype or a desirable trait in the transgenic or genetically modified plant. A " control plant" may in some cases be a transgenic plant line that comprises an empty vector or marker gene, but does not contain the recombinant polynucleotide of interest that is present in the transgenic or genetically modified plant being evaluated. A control plant may be a plant of the same line or variety as the transgenic or genetically modified plant being tested, or it may be another line or variety, such as a plant known to have a specific phenotype, characteristic, or known genotype. A suitable control plant would include a genetically unaltered or non-transgenic plant of the parental line used to generate a transgenic plant herein.

As defined herein, the term " nucleic acid" and " polynucleotide" are interchangeable and refer to RNA or DNA that is linear or branched, single or double stranded, or a hybrid thereof. The term also encompasses RNA/DNA hybrids. An " isolated" nucleic acid molecule is one that is substantially separated from other nucleic acid molecules which are present in the natural source of the nucleic acid (i.e., sequences encoding other polypeptides). For example, a cloned nucleic acid is considered isolated. A nucleic acid is also considered isolated if it has been altered by human intervention, or placed in a locus or location that is not its natural site, or if it is introduced into a cell by transformation. Moreover, an isolated nucleic acid molecule, such as a cDNA molecule, can be free from some of the other cellular material with which it is naturally associated, or culture medium when produced by recombinant techniques, or chemical precursors or other chemicals when chemically synthesized. While it may optionally encompass untranslated sequence located at both the 3' and 5' ends of the coding region of a gene, it may be preferable to remove the sequences which naturally flank the coding region in its naturally occurring replicon.

As used herein, the term " environmental stress" refers to a sub-optimal condition associated with salinity, drought, nitrogen, temperature, metal, chemical, pathogenic, or oxidative stresses, or any combination thereof. As used herein, , the term " drought" refers to an environmental condition where the amount of water available to support plant growth or development is less than optimal. As used herein, the term " fresh weight" refers to everything in the plant including water. As used herein, the term " dry weight" refers to everything in the plant other than water, and includes, for example, carbohydrates, proteins, oils, and mineral nutrients.

Any plant species may be transformed to create a transgenic plant in accordance with the invention. The transgenic plant of the invention may be a dicotyledonous plant or a monocotyledonous plant. For example and without limitation, transgenic plants of the invention may be derived from any of the following diclotyledonous plant families: Leguminosae, including plants such as pea, alfalfa and soybean; Umbelliferae, including plants such as carrot and celery; Solanaceae, including the plants such as tomato, potato, aubergine, tobacco, and pepper; Cruciferae, particularly the genus Brassica, which includes plant such as oilseed rape, beet, cabbage, cauliflower and broccoli); and A. thaliana; Compositae, which includes plants such as lettuce; Malvaceae, which includes cotton; Fabaceae, which includes plants such as peanut, and the like. Transgenic plants of the invention may be derived from monocotyledonous plants, such as, for example, wheat, barley, sorghum, millet, rye, triticale, maize, rice, oats and sugarcane. Transgenic plants of the invention are also embodied as trees such as apple, pear, quince, plum, cherry, peach, nectarine, apricot, papaya, mango, and other woody species including coniferous and deciduous trees such as poplar, pine, sequoia, cedar, oak, and the like. Especially preferred are A. thaliana, Nicotiana tabacum, rice, oilseed rape, canola, soybean, corn (maize), cotton, and wheat.

### A. Phosphatidate Cytidylyltransferase

In one embodiment, the invention provides a provides a transgenic plant transformed with an expression cassette comprising, in operative association, an isolated polynucleotide encoding a promoter capable of enhancing gene expression in roots and shoots; an isolated polynucleotide encoding a subcellular targeting peptide; and an isolated polynucleotide encoding a full-length phosphatidate cytidylyltransferase polypeptide, wherein the transgenic plant demonstrates increased yield as compared to a wild type plant of the same variety which does not comprise the expression cassette. In accordance with the invention, when the promoter is capable of enhancing expression in roots or shoots, the subcellular targeting peptide is a plastid transit peptide.

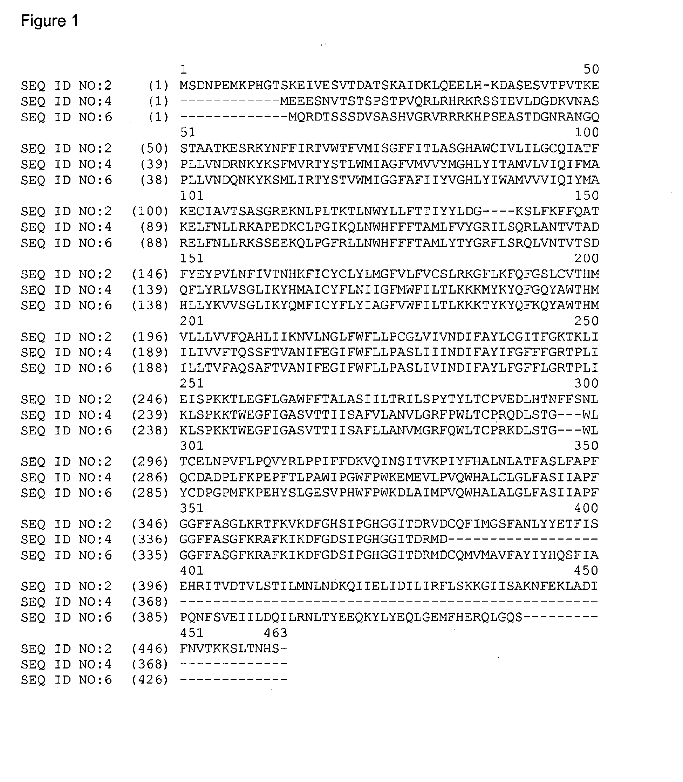

As indicated in Table 2 below, when the S. cerevisiae gene product YBR029C (SEQ ID NO:2) is targeted to the chloroplast and the gene' s transcriptional expression is driven by the Super promoter, transgenic plants demonstrate improved response to water-limiting conditions. Moreover, Table 3 indicates that under well-watered conditions, plants expressing YBR029C which is targeted to the plastid or to mitochondria were larger than control plants. Gene YBR029C encodes a phosphatidate cytidylyltransferase (EC 2.7.7.41), also known as CDP-diacylglycerol synthase (CDS), which catalyzes the synthesis of CDP-diacylglycerol from CTP and phosphatidate. CDS is a membrane-bound protein, with eight predicted membrane spanning regions in potato and Arabidopsis. Phosphatidate cytidylyltransferases are characterized, in part, by a distinctive signature sequence of " S - x - [LIVMF] - K - R - x(4) - K - D - x - [GSA] - x(2) - [LIF] - [PGS] - x - H - G - G - [LIVMF] - x - D - R - [LIVMFT] - D" where amino acid positions within square brackets can be any of the designated residues, and unbracketed amino acid positions can only be that specific amino acid residue. Such conserved signature sequences are exemplified in the phosphatidate cytidylyltransferase proteins set forth in Figure 1.

The transgenic plant of this embodiment may comprise any polynucleotide encoding phosphatidate cytidylyltransferase. Preferably, the transgenic plant of this embodiment comprises a polynucleotide encoding a polypeptide having phosphatidate cytidylyltransferase activity, wherein the polypeptide comprises a phosphatidate cytidylyltransferase signature sequence selected from the group consisting of amino acids 351 to 377 of SEQ ID NO:2, amino acids 341 to 367 of SEQ ID NO:4, or amino acids 340 to 366 of SEQ ID NO:6. More preferably, the transgenic plant of this embodiment comprises a polynucleotide encoding phosphatidate cytidylyltransferase domain having a sequence comprising amino acids 65 to 377 of SEQ ID NO:2, amino acids 54 to 367 of SEQ ID NO:4, or amino acids 53 to 366 of SEQ ID NO:6. Most preferably, the transgenic plant of this embodiment comprises a polynucleotide encoding phosphatidate cytidylyltransferase polypeptide comprising amino acids 1 to 457 of SEQ ID NO:2, amino acids 1 to 367 of SEQ ID NO:4, or amino acids 1 to 425 of SEQ ID NO:6.

### B. Acyl Carrier Protein

In another embodiment, the invention provides a transgenic plant transformed with an expression cassette comprising in operative association, an isolated polynucleotide encoding a promoter capable of enhancing expression in leaves; an isolated polynucleotide encoding a mitochondrial transit peptide; and an isolated polynucleotide encoding an acyl carrier protein; wherein the transgenic plant demonstrates increased yield as compared to a wild type plant of the same variety which does not comprise the expression cassette.

As shown in Table 4 below, when the S. cerevisiae gene product YKL192C (SEQ ID NO:8) is targeted to the mitochondria under the control of the USP promoter (SEQ ID NO:123), transgenic plants demonstrate improved response to water-limiting conditions. Gene YKL192C encodes an acyl-carrier protein (ACP), containing a phosphopantetheine binding domain (PF00550). Acyl carrier proteins catalyze a condensation reaction to form peptide bonds in non-ribosomal protein biosynthesis. Acyl carrier protein is a universal and highly conserved carrier of acyl groups in fatty acid biosynthesis. The amino-terminal region of the ACP proteins is well defined and consists of alpha four helices arranged in a right-handed bundle held together by inter-helical hydrophobic interactions.

Phosphopantetheine (or pantetheine 4' phosphate) is the prosthetic group of ACP in some multienzyme complexes, where it serves as a 'swinging arm' for the attachment of activated fatty acid and amino acid groups. Phosphopantetheine binding domains are characterized, in part, by the presence of the distinctive phosphopantetheine attachment site signature sequence, " [DEQGSTALMKRH]-[LIVMFYSTAC] - [GNQ] - [LIVMFYAG] - [DNEKHS] - S - [LIVMST] - {PCFY}-[STAGCPQLIVMF] - [LIVMATN] - [DENQGTAKRHLM] - [LIVMWSTA] - [LIVGSTACR]-{LPIY} - {VY} - [LIVMFA]" where amino acid positions within square brackets can be any of the designated residues, amino acid positions within curly brackets can be any amino acid residue except the one(s) listed and unbracketed amino acid positions can only be that specific amino acid residue. The phosphopantetheine moiety is attached to serine residue indicated in bold italic in the above signature sequence. This serine residue is present in the amino terminus of helix II, a domain of the protein referred to as the recognition helix and which is responsible for the interaction of ACPs with the enzymes of type II fatty acid synthesis.

Preferably, the transgenic plant of this embodiment comprises a polynucleotide encoding a full-length ACP comprising a phosphopantetheine binding site signature sequence selected from the group consisting of amino acids 77 to 92 of SEQ ID NO:8, amino acids 73 to 88 of SEQ ID NO:10, amino acids 75 to 90 of SEQ ID NO:12, or amino acids 84 to 99 of SEQ ID NO:14. More preferably, the transgenic plant of this embodiment comprises a polynucleotide encoding an acyl-carrier protein comprising an acyl carrier protein domain selected from the group consisting of amino acids 49 to 106 of SEQ ID NO:8, amino acids 49 to 102 of SEQ ID NO:10, amino acids 51 to 104 of SEQ ID NO:12, amino acids 60 to 113 of SEQ ID NO:14. Most preferably, the transgenic plant of this embodiment comprises a polynucleotide encoding an acyl-carrier protein comprising amino acids 1 to 125 of SEQ ID NO:8, amino acids 1 to 117 of SEQ ID NO:10, amino acids 1 to 128 of SEQ ID NO:12, or amino acids 1 to 119 of SEQ ID NO:14.

### C. Acyltransferase

In another embodiment, the invention provides a transgenic plant transformed with an expression cassette comprising, in operative association, isolated polynucleotide encoding a promoter; an isolated polynucleotide encoding a subcellular targeting peptide; and an isolated polynucleotide encoding an acyltransferase polypeptide; wherein the transgenic plant demonstrates increased yield as compared to a wild type plant of the same variety which does not comprise the expression cassette.

As set forth in Table 5 below, when the S. cerevisiae acyltransferase gene product YDR018C (SEQ ID NO:16) is targeted to the chloroplast or mitochondria under the control of the Super promoter or the USP promoter (SEQ ID NO: 123), transgenic plants demonstrate improved response to water-limiting conditions. Moreover, Table 6 indicates that under well-watered conditions, plants expressing YDR018C under control of the USP promoter (SEQ ID NO:123), and targeted to the mitochondria, were larger than control plants.

The transgenic plant of this embodiment may comprise any polynucleotide encoding an acyltransferase polypeptide. Preferably, the transgenic plant of this embodiment comprises a polynucleotide encoding an acyltransferase polypeptide comprising an acyltransferase domain selected from the group consisting of amino acids 108 to 272 of SEQ ID NO:16, amino acids 80 to 222 of SEQ ID NO:18, or amino acids 116 to 258 of SEQ ID NO:20. More preferably, the transgenic plant of this embodiment comprises a polynucleotide encoding an acyltransferase having a sequence comprising amino acids 1 to 396 of SEQ ID NO:16, amino acids 1 to 384 of SEQ ID NO:18, or amino acids 1 to 332 of SEQ ID NO:20.

### D. Bifunctional anaerobic fatty acid oxidation complex polypeptide

In another embodiment, the invention provides a method of increasing yield of a plant species by transforming a wild type cell of said species with with an expression cassette comprising, in operative association, an isolated polynucleotide encoding a promoter capable of enhancing expression in leaves; an isolated polynucleotide encoding a mitochondrial transit peptide; and an isolated polynucleotide encoding a bifunctional anaerobic fatty acid oxidation complex polypeptide In a second step, transgenic plantlets are regenerated from the transformed plant cell. In a third step, the transgenic plantlets are subjected to a yield-related assay, and higher-yielding plants are selected from the regenerated transgenic plants.

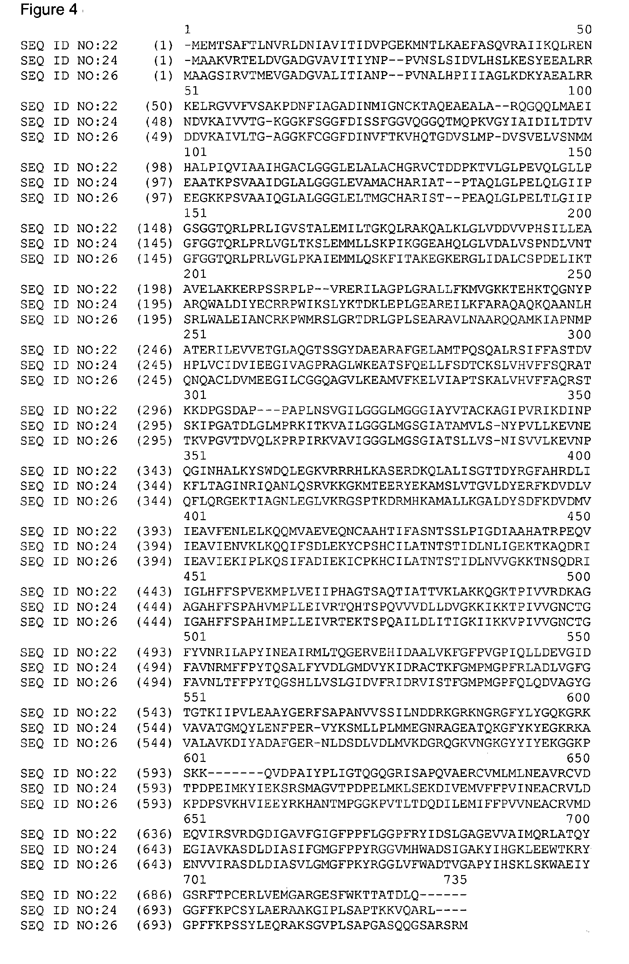

As shown in Tables 7 and 8 below, when transcription of the E. coli gene B2341 (SEQ ID NO:21) is under control of the USP promoter (SEQ ID NO:123) and the gene product (SEQ ID NO:22) is targeted to the mitochondria, transgenic plants tend to be larger and darker green than control plants. Gene B2341 encodes a protein that comprises three domains: a domain characteristic of the ECH or enoyl-CoA hydratase/isomerase family (PF00378); a C-terminal domain characteristic 3HCDH or 3-hydroxyacyl-CoA dehydrogenase (PF00725); and a 3HCDH_N,3-hydroxyacyl-CoA dehydrogenase, NAD binding domain (PF02737). The 3HCDH domain is characterized, in part, by the presence of the signature sequence, " [DNES] - x(2)-[GA] - F - [LIVMFYA] - x - [NT] - R - x(3) - [PA] - [LIVMFY] - [LIVMFYST] - x(5,6)-[LIVMFYCT] - [LIVMFYEAH] - x(2) - [GVE]" , where amino acid positions within square brackets can be any of the designated residues, and unbracketed amino acid positions can only be that specific amino acid residue. All the sequences shown in Figure 4 exhibit this characteristic signature sequence, the only deviation within this group is the presence of a leucine residue at position 498 in ZM06MC15742 (SEQ ID NO:26) instead of the canonical arginine residue.

The method of this embodiment may employ any polynucleotide encoding a bifunctional anaerobic fatty acid oxidation complex polypeptide comprising an ECH domain, a 3HCDH-C terminal domain and a 3HCHD-NAD binding domain. Preferably, the method of this embodiment employs a polynucleotide encoding a bifunctional anaerobic fatty acid oxidation complex polypeptide comprising three domains: a) an ECH domain selected from the group consisting of amino acids 17 to 190 of SEQ ID NO:22; amino acids 17 to 187 SEQ ID NO:24 and amino acids 18 to 187 SEQ ID NO:26; b) a 3HCDH domain selected from the group consisting of amino acids 489 to 513 of SEQ ID NO:22, amino acids 490 to 514 of SEQ ID NO:24, amino acids 490 to 514 of SEQ ID NO:26; and c) a 3HCDH-N domain selected from the group consisting of amino acids 310 to 490 of SEQ ID NO:22; amino acids 312 to 491 of SEQ ID NO:24 and amino acids 312 to 491 SEQ ID NO:26 More preferably, the method of this embodiment employs a polynucleotide encoding a bifunctional anaerobic fatty acid oxidation complex polypeptide having a sequence comprising amino acids 1 to 714 of SEQ ID NO:22, amino acids 1 to 723 of SEQ ID NO:24, or amino acids 1 to 727 of SEQ ID NO:26.

The invention is also embodied in a transgenic plant comprising a polynucleotide encoding a bifunctional anaerobic fatty acid oxidation complex polypeptide comprising a) an ECH domain selected from the group consisting of amino acids 17 to 190 of SEQ ID NO:22; amino acids 17 to 187 SEQ ID NO:24 and amino acids 18 to 187 SEQ ID NO:26; b) a 3HCDH domain selected from the group consisting of amino acids 489 to 513 of SEQ ID NO:22, amino acids 490 to 514 of SEQ ID NO:24, amino acids 490 to 514 of SEQ ID NO:26; and c) a 3HCDH-N domain selected from the group consisting of amino acids 310 to 490 of SEQ ID NO:22; amino acids 312 to 491 of SEQ ID NO:24 and amino acids 312 to 491 SEQ ID NO:26 More preferably, the transgenic plant of this embodiment comprises a polynucleotide encoding a bifunctional anaerobic fatty acid oxidation complex polypeptide having a sequence comprising amino acids 1 to 714 of SEQ ID NO:22, amino acids 1 to 723 of SEQ ID NO:24, or amino acids 1 to 727 of SEQ ID NO:26.

### E. Acyl-CoA thioesterase

In another embodiment, the invention provides a transgenic plant transformed with an expression cassette comprising, in operative association, an isolated polynucleotide encoding a promoter capable of enhancing gene expression in leaves; an isolated polynucleotide encoding a mitochondrial transit peptide; and an isolated polynucleotide encoding a full-length acyl-CoA thioesterase polypeptide; wherein the transgenic plant demonstrates increased yield as compared to a wild type plant of the same variety which does not comprise the expression cassette.

As shown in Table 9 below, when transcription of the E. coli gene b0452 is targeted to mitochondria under the control of the USP promoter, transgenic plants were larger than control plants, both under well-watered and drought conditions. Gene B0452 (SEQ ID NO:27) encodes an acyl-CoA thioesterase (EC 3.1.2.2). Acyl-CoA thioesterases (ACH) are a group of enzymes that catalyze the hydrolysis of acyl-CoAs to the free fatty acid and coenzyme A (CoASH), providing the potential to regulate intracellular levels of acyl-CoAs, free fatty acids and CoASH. This enzyme displays high levels of activity on medium- and long chain acyl CoAs. Two families of ACHs have been identified in A. thaliana. One family, consisting of AtACH1 and AtACH2, appears to be peroxisomal, as they have type-1 peroxisomal targeting sequences. The other family, consisting of AtACH4 and AtACH5, resides in the endoplasmic reticulum.

The transgenic plant of this embodiment may comprise any polynucleotide encoding an acyl-CoA thioesterase. Preferably, the transgenic plant of this embodiment comprises a polynucleotide encoding a full-length polypeptide having acyl-CoA thioesterase activity, wherein the polypeptide comprises a domain selected from the group consisting of amino acids 107 to 184 of SEQ ID NO:28, amino acids 54 to 138 of SEQ ID NO:30, amino acids 127 to 211 of SEQ ID NO:32, and amino acids 3 to 87 of SEQ ID NO:34. More preferably, the transgenic plant of this embodiment comprises a polynucleotide encoding an acyl-CoA thioesterase having a sequence comprising amino acids 1 to 286 of SEQ ID NO:28, amino acids 1 to 248 of SEQ ID NO:30, amino acids 1 to 212 of SEQ ID NO:32, or amino acids 1 to 197 of SEQ ID NO:34.

### F. 2,4-dienoyl-CoA reductase

In another embodiment, the invention provides a transgenic plant transformed with an expression cassette comprising, in operative association, an isolated polynucleotide encoding a promoter capable of enhancing expression in leaves; an isolated polynucleotide encoding a mitochondrial transit peptide; and an isolated polynucleotide encoding a full-length 2,4-dienoyl-CoA reductase polypeptide; wherein the transgenic plant demonstrates increased yield as compared to a wild type plant of the same variety which does not comprise the expression cassette.

As shown in Table 10 below, when transcription of the S. cerevisiae gene YNL202W (SEQ ID NO:35) is targeted to mitochondria under control of the USP promoter, under cycling drought conditions, transgenic plants were larger than control plants. YNL202W is a 2,4-dienoyl-CoA reductase (EC 1.3.1.34, DECR), an auxiliary enzyme of beta-oxidation. DECR participates in the degradation of unsaturated fatty enoyl-CoA esters having double bonds in both even- and odd-numbered positions in peroxisome. It catalyzes the NADP-dependent reduction of 2,4-dienoyl-CoA to yield trans-3-enoyl-CoA.

The transgenic plant of this embodiment may comprise any polynucleotide encoding a 2,4-dienoyl-CoA reductase. Preferably, the transgenic plant of this embodiment comprises a polynucleotide encoding a full-length polypeptide having 2,4-dienoyl-CoA reductase activity, wherein the polypeptide comprises a domain selected from the group consisting of amino acids 107 to 270 of SEQ ID NO:36 and amino acids 54 to 227 of SEQ ID NO:38. More preferably, the transgenic plant of this embodiment comprises a polynucleotide encoding a 2,4-dienoyl-CoA reductase having a sequence comprising amino acids 1 to 296 of SEQ ID NO:36 or amino acids 1 to 264 of SEQ ID NO:38.

### G. Sterol esterase

In another embodiment, the invention provides a transgenic plant transformed with an expression cassette comprising, in operative association, an isolated polynucleotide encoding a promoter; an isolated polynucleotide encoding a subcellular transit peptide; and an isolated polynucleotide encoding a full-length sterol esterase; wherein the transgenic plant demonstrates increased yield as compared to a wild type plant of the same variety which does not comprise the expression cassette.

As shown in Table 11 below, when transcription of the S. cerevisiae gene YKL140W (SEQ ID NO:39) is targeted to plastids under control of the PcUbi promoter, transgenic plants were larger than control plants under well-watered conditions. Table 11 also shows that when YKL140W transcription was targeted to mitochondria under control of the USP promoter, transgenic plants were larger than control plants under cycling drought conditions. YKL140W encodes a sterol esterase (EC 3.1.1.13). In yeast, sterol esterase mediates the hydrolysis of stearyl esters and is required for mobilization of stearyl ester, thereby playing a central role in lipid metabolism. This enzyme may have weak lipase activity toward triglycerides under some conditions.

The transgenic plant of this embodiment may comprise any polynucleotide encoding a sterol esterase. Preferably, the transgenic plant of this embodiment comprises a polynucleotide encoding a full-length polypeptide having sterol esterase activity, wherein the polypeptide has a sequence comprising amino acids 1 to 548 of SEQ ID NO:40.

### H. Succinate-CoA Ligase

In another embodiment, the invention provides a transgenic plant transformed with an expression cassette comprising, in operative association, an isolated polynucleotide encoding a promoter; an isolated polynucleotide encoding a chloroplast transit peptide; and an isolated polynucleotide encoding a full-length succinate-CoA ligase polypeptide; wherein the transgenic plant demonstrates increased yield as compared to a wild type plant of the same variety which does not comprise the expression cassette.

As shown in Table 12 below, when transcription of the Synechocystis gene SLL1023 (SEQ ID NO:41) is targeted to plastids under control of the PcUbi promoter, transgenic plansts are larger than control plants, both under cycling drought and well-watered conditions. SLL1023 is a succinate-CoA ligase (EC 6.2.1.5). Succinate-CoA ligase catalyzes the reaction of GTP + succinate + CoA = GDP + phosphate + succinyl-CoA. This reaction is part of the TCA cycle for sugar metabolism.

The transgenic plant of this embodiment may comprise any polynucleotide encoding a succinate-CoA ligase. Preferably, the transgenic plant of this embodiment comprises a polynucleotide encoding a succinate-CoA ligase having a sequence comprising amino acids 1 to 401 of SEQ ID NO:42.

### I. Cobalt-precorrin-6A reductase

In another embodiment, the invention provides a transgenic plant transformed with an expression cassette comprising, in operative association, an isolated polynucleotide encoding a promoter and an isolated polynucleotide encoding a full-length cobalt-precorrin-6A reductase polypeptide; wherein the transgenic plant demonstrates increased yield as compared to a wild type plant of the same variety which does not comprise the expression cassette.

As shown in Table 13 below, when the Synechocystis gene SLR0252 (SEQ ID NO:43) is expressed under control of the PcUbi promoter, transgenic plants were larger than control plants under cycling drought conditions. SLR0252 is a cobalt-precorrin-6A reductase (EC 1.3.1.54). Cobalt-precorrin-6A reductase catalyzes the reduction of the macrocycle of cobalt-precorrin-6X into cobalt-precorrin-6Y. Cobalt-precorrin-6Y is a co-factor of many enzymes.

The transgenic plant of this embodiment may comprise any polynucleotide encoding a cobalt-precorrin-6A reductase. Preferably, the transgenic plant of this embodiment comprises a polynucleotide encoding a full-length polypeptide encoding a cobalt-precorrin-6A reductase having a sequence comprising amino acids 1 to 261 of SEQ ID NO:44.

### J. Uroporphyrin-III C-methyltransferase

In another embodiment, the invention provides a transgenic plant transformed with an expression cassette comprising, in operative association, an isolated polynucleotide encoding a promoter capable of enhancing expression in leaves, and an isolated polynucleotide encoding a full-length uroporphyrin-III C-methyltransferase; wherein the transgenic plant demonstrates increased yield as compared to a wild type plant of the same variety which does not comprise the expression cassette. Table 14 below shows that when the E. coli gene b3803 (SEQ ID NO:45) is expressed under control of the USP promoter, transgenic plants were larger than control plants under well-watered growth conditions. The b3803 gene encodes a uroporphyrin-III C-methyltransferase, also known as S-adenosyl-L-methionine-dependent Uroporphyrinogen-III C-methyltransferase (SUMT), an enzyme involved in the biosynthesis of siroheme and cobalamin (vitamin B12). SUMT (EC 2.1.1.107) is a branchpoint enzyme that plays a key role in the biosynthesis of modified tetrapyrroles. By catalyzing the transformation of uroporphyrinogen III into precorrin-2, SUMT controls the flux to compounds such as vitamin B12 and siroheme, an important co-factor of nitrate reductase and sulfite reductase enzymes. In plants, uroporphyrinogen III enters the pathway that leads to chlorophyll synthesis.

The transgenic plant of this embodiment may preferably comprise any polynucleotide encoding a uroporphyrin-III C-methyltransferase. More preferably, the transgenic plant of this embodiment comprises a polynucleotide encoding a full-length polypeptide having uroporphyrin-III C-methyltransferase activity, wherein the polypeptide comprises a domain comprising amino acids 101 to 356 of SEQ ID NO:46; amino acids 97 to 353 of SEQ ID NO:48; amino acids 91 to 346 of SEQ ID NO:50; or amino acids 100 to 355 of SEQ ID NO:52. Most preferably, the transgenic plant of this embodiment comprises a polynucleotide encoding a uroporphyrin-III C-methyltransferase having a sequence comprising amino acids 1 to 393 of SEQ ID NO:46, amino acids 1 to 368 of SEQ ID NO:48; amino acids 1 to 363 of SEQ ID NO:50, or amino acids 1 to 379 of SEQ ID NO:52.

### K. Isoprenoid biosynthesis protein

In another embodiment, the invention provides a transgenic plant transformed with an expression cassette comprising, in operative association, an isolated polynucleotide encoding a promoter capable of enhancing gene expression in roots and shoots and an isolated polynucleotide encoding a full-length polypeptide having isoprenoid biosynthesis activity; wherein the transgenic plant demonstrates increased yield as compared to a wild type plant of the same variety which does not comprise the expression cassette.

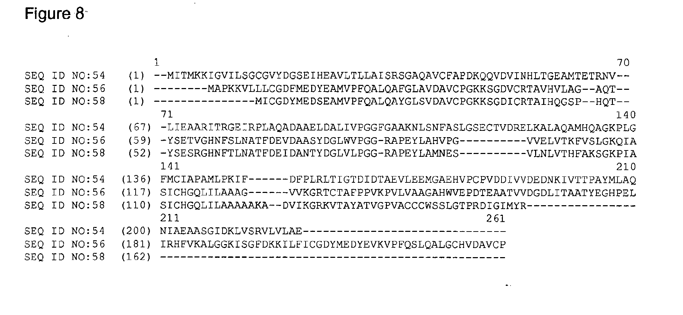

Table 15 below shows that when the E. coli gene b3209 (SEQ ID NO:53) is expressed under control of the Super promoter, transgenic plants are larger than control plants under well-watered growth conditions. While the specific function of the b3209 protein is not known, it has been shown to increase lycopene production in E. coli when over-expressed. Lycopene is an important component of the photosystem and a potent anti-oxidant that protects cells from oxidative damage. In plant cells it is also converted to other carotenoids and precursors for plant hormones. Isoprenoid biosynthesis proteins are characterized, in part, by the presence of a DJ-1_Pfp1 signature sequence. Such signature sequences are exemplified in the isoprenoid biosynthesis proteins set forth in Figure 8.

The transgenic plant of this embodiment may comprise any polynucleotide encoding an isoprenoid biosynthesis protein. Preferably, the transgenic plant of this embodiment comprises a polynucleotide encoding a full-length polypeptide having isoprenoid biosynthesis activity, wherein the polypeptide comprises a DJ-1_Pfp1 signature sequence selected from the group consisting of amino acids 46 to 208 of SEQ ID NO:54; amino acids 32 to 189 of SEQ ID NO:56; and amino acids 25 to 151 of SEQ ID NO:58. Most preferably, the transgenic plant of this embodiment comprises a polynucleotide encoding an isoprenoid biosynthesis protein having a sequence comprising amino acids 1 to 220 of SEQ ID NO:54, amino acids 1 to 231 of SEQ ID NO:56, or amino acids 1 to 161 of SEQ ID NO:58.

### L. LysE type translocator

In another embodiment, the invention provides a transgenic plant transformed with an expression cassette comprising, in operative association, an isolated polynucleotide encoding a promoter capable of enhancing gene expression in roots and shoots and an isolated polynucleotide encoding a full-length polypeptide having LysE type translocator activity; wherein the transgenic plant demonstrates increased yield as compared to a wild type plant of the same variety which does not comprise the expression cassette.

As shown in Table 16 below, when the E. coli gene b2578 (SEQ ID NO:59) is expressed under control of the Super promoter, transgenic plants were larger than control plants under well-watered conditions. The b2578 gene encodes a LysE type translocator protein, a membrane protein with six predicted transmembrane domains, which is involved in maintaining intercellular levels of L-lysine by exporting excess L-lysine from the cell. These proteins are characterized, in part, by the presence of a LysE signature sequence.

The transgenic plant of this embodiment may comprise any polynucleotide encoding a LysE type translocator. Preferably, the transgenic plant of this embodiment comprises a polynucleotide encoding a full-length polypeptide having LysE type translocator activity, wherein the polypeptide comprises a LysE signature sequence comprising amino acids 14 to 195 of SEQ ID NO:60. Most preferably, the transgenic plant of this embodiment comprises a polynucleotide encoding a LysE type translocator having a sequence comprising amino acids 1 to 195 of SEQ ID NO:60.

### M. Branched-chain amino acid transporter

In another embodiment, the invention provides a transgenic plant transformed with an expression cassette comprising, in operative association, an isolated polynucleotide encoding a promoter capable of enhancing gene expression in roots and shoots; and an isolated polynucleotide encoding a full-length polypeptide having LIV-E family branched-chain amino acid transport activity; wherein the transgenic plant demonstrates increased yield as compared to a wild type plant of the same variety which does not comprise the expression cassette.

As shown in Table 17 below, when the E. coli gene b2682 (SEQ ID NO:61) is expressed under control of the Super promoter, transgenic plants were larger than control plants under well-watered growth conditions. The b2682 gene encodes a LIV-E family branched-chain amino acid transport protein, a membrane protein with five predicted transmembrane domains involved in the export of L-valine. These proteins are characterized, in part, by the presence of a AzIC signature sequence.

The transgenic plant of this embodiment may comprise any polynucleotide encoding a branched-chain amino acid transporter. Preferably, the transgenic plant of this embodiment comprises a polynucleotide encoding a full-length polypeptide having LIV-E family branched-chain amino acid transport activity, wherein the polypeptide comprises a AzIC signature sequence comprising amino acids 23 to 167 of SEQ ID NO:62. Most preferably, the transgenic plant of this embodiment comprises a polynucleotide encoding a branched-chain amino acid transporter having a sequence comprising amino acids 1 to 245 of SEQ ID NO:62.

### N. DNA-binding protein

In another embodiment, the invention provides a transgenic plant transformed with an expression cassette comprising, in operative association, an isolated polynucleotide encoding a promoter capable of enhancing gene expression in roots and shoots; and an isolated polynucleotide encoding a truncated DNA-binding polypeptide; wherein the transgenic plant demonstrates increased yield as compared to a wild type plant of the same variety which does not comprise the expression cassette.

As shown in Table 18 below, when the E. coli gene b3285 (SEQ ID NO:63) is expressed under control of the Super promoter, transgenic plants are larger than control plants under well-watered growth conditions. The b3285 gene encodes a truncated DNA-binding protein comprising the C-terminal portion of the E. coli SMF protein (public database accession number YP_026211), and may facilitate the access of DNA-modifying proteins to genomic DNA. Such DNA-binding proteins are characterized, in part, by the presence of an SMF signature sequence.

The transgenic plant of this embodiment may comprise any polynucleotide homologous to the polynucleotide encoding the truncated DNA-binding protein of SEQ ID NO:64. Preferably, the transgenic plant of this embodiment comprises a polynucleotide encoding a DNA-binding protein having a sequence comprising amino acids 1 to 102 of SEQ ID NO:64.

### O. YscJ/FliF protein

In another embodiment, the invention provides a transgenic plant transformed with an expression cassette comprising, in operative association, an isolated polynucleotide encoding a promoter capable of enhancing gene expression in roots and shoots; and an isolated polynucleotide encoding a full-length YscJ/Flif family polypeptide; wherein the transgenic plant demonstrates increased yield as compared to a wild type plant of the same variety which does not comprise the expression cassette.

As shown in Table 19 below, when the E. coli gene b1938 (SEQ ID NO:65), a YscJ/FliF family protein, is expressed under control of the Super promoter, transgenic plnats are larger than control plants. The YscJ/Flif family proteins stabilize membrane proteins and complexes such as transporters, which provide cells with important compounds for cell growth. YscJ/FliF family proteins are characterized, in part, by the presence of a YscJ_FliF signature sequence.

The transgenic plant of this embodiment may comprise any polynucleotide encoding an YscJ/FliF family protein. Preferably, the transgenic plant of this embodiment comprises a polynucleotide encoding a full-length polypeptide having YscJ/FliF activity, wherein the polypeptide comprises a first YscJ/FliF signature sequence comprising amino acids 17 to 225 of SEQ ID NO:66 and a secondYscJ/FliF signature sequence comprising amino acids 250 to 429 of SEQ ID NO:66. Most preferably, the transgenic plant of this embodiment comprises a polynucleotide encoding a YscJ/FliF family protein having a sequence comprising amino acids 1 to 552 of SEQ ID NO:66.

### P. Riboflavin Biosynthetic Genes

In another embodiment, the invention provides a transgenic plant transformed with an expression cassette comprising, in operative association, a promoter and an isolated polynucleotide encoding a full length GTP cyclohydrolase II polypeptide which does not comprise a subcellular targeting sequence; wherein the transgenic plant demonstrates increased yield as compared to a wild type plant of the same variety that does not comprise the expression cassette.

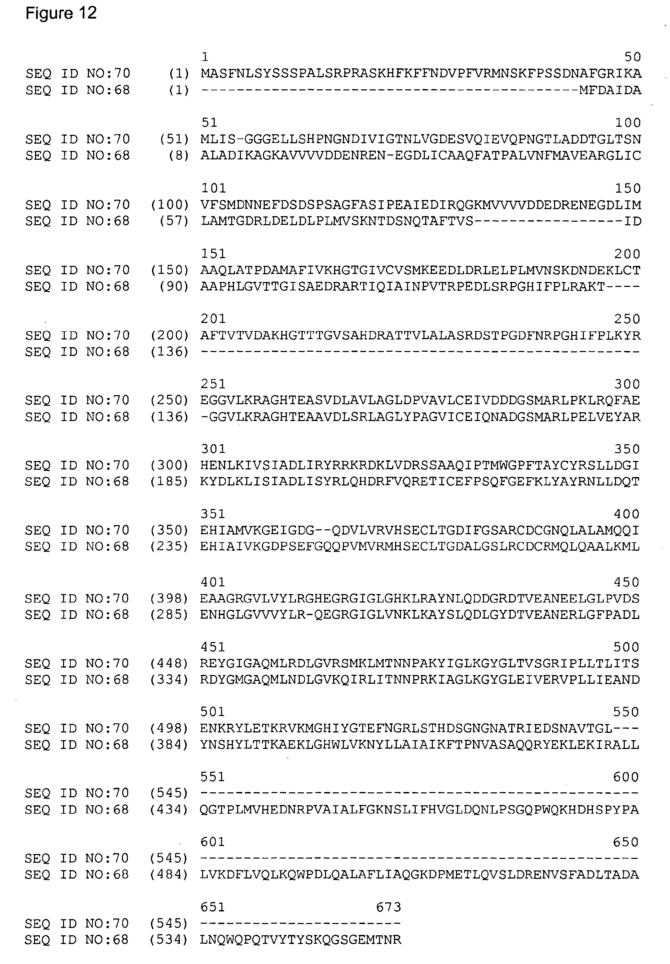

As shown in Table 20 below, when the Synechocystis gene SLL1894 (SEQ ID NO:67), a GTP cyclohydrolase II, was expressed under control of the PcUbi promoter with no subcellular targeting, transgenic plants were larger than control plants under water limited conditions. GTP cyclohydrase II enzymes are characterized, in part, by the presence of a DHBP_synthase signature sequence in the N-terminus and a GTP cyclohydrolase II signature sequence in the C-terminus. Such signature sequences are exemplified in the GTP cyclohydrolase II proteins set forth in Figure 12.

The expression cassette employed in the transgenic plant of this embodiment may comprise any polynucleotide encoding a full-length polypeptide having GTP cyclohydrolase II activity which does not comprise a subcellular targeting peptide. Preferably, the GTP cyclohydrolase II polypeptide comprises a DHBP synthase domain selected from the groups consisting of amino acids 5 to 202 of SEQ ID NO:68 and amino acids 120 to 317 of SEQ ID NO:70 and a GTP cyclohydrolase II domain selected from the group consisting of amino acids 207 to 377 of SEQ ID NO:68 and amino acids 322 to 491 of SEQ ID NO:70. In accordance with the invention, when the the G. max GTP cyclohydrolase II set forth in SEQ ID NO:70 is employed in the expression cassette, polynucleotide sequence encoding the subcellular targeting peptide (amino acids 1 to 49 of SEQ ID NO:70) is deleted. More preferably, the transgenic plant of this embodiment comprises a polynucleotide encoding a GTP cyclohydrolase II polypeptide having a sequence comprising amino acids 1 to 556 of SEQ ID NO:68 or amino acids 50 to 544 of SEQ ID NO:70.

In another embodiment, the invention provides a transgenic plant transformed with an expression cassette comprising, in operative association, an isolated polynucleotide encoding a promoter and an isolated polynucleotide encoding a lumazine synthase polypeptide which does not comprise a plastid targeting sequence; wherein the transgenic plant demonstrates increased yield as compared to a wild type plant of the same variety which does not comprise the expression cassette.

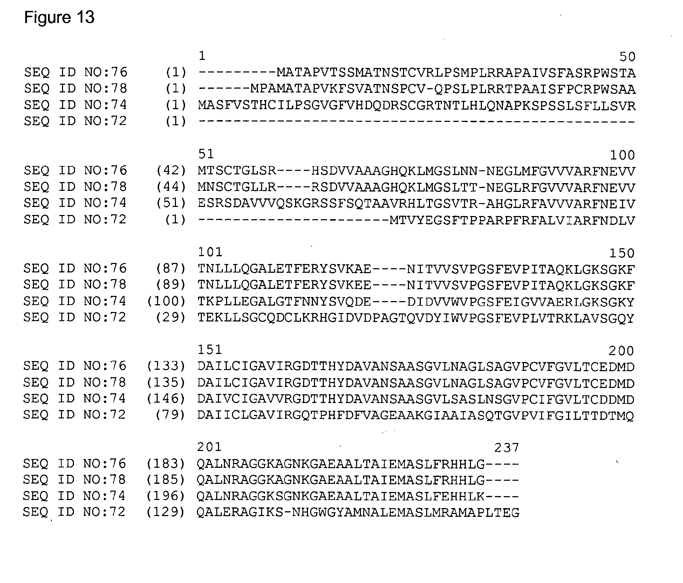

As shown in Tables 21 and 22 below, when the Synechocystis gene SLL1282 (SEQ ID NO:71), a lumazine synthase, is expressed under control of the PcUbi promoter, transgenic plants demonstrate increased yield as compared to a wild type plant of the same variety which does not comprise the SLL1282 gene. Lumazine synthase enzymes are characterized, in part, by the presence of a DMRL_synthase signature sequence. Such signature sequences are exemplified in the lumazine synthase proteins set forth in Figure 13.

The expression cassette employed in the transgenic plant of this embodiment may comprise any polynucleotide encoding a lumazine synthase polypeptide which does not comprise a subcellular targeting peptide. Preferably, the transgenic plant of this embodiment comprises a polynucleotide encoding a full-length polypeptide having lumazine synthase activity, wherein the polypeptide comprises a DMRL_synthase signature sequence selected from the group consisting of amino acids 12 to 158 of SEQ ID NO:72; amino acids 83 to 226 of SEQ ID NO:74; amino acids 70 to 213 of SEQ ID NO:76, amino acids 70 to 215 of SEQ ID NO:78. In accordance with the invention, when any of the plant lumazine synthases set forth in SEQ ID NOs:100, 76, or 78 is employed in the expression cassette, the polynucleotide sequences encoding the subcellular targeting peptide (amino acids 1 to 53 of SEQ ID NO:74; amino acids 1 to 56 of SEQ ID NO:76, and amino acids 1 to 80 of SEQ ID NO:78) are deleted. More preferably, the transgenic plant of this embodiment comprises a polynucleotide encoding a lumazine synthase comprising amino acids 1 to 164 of SEQ ID NO:72, amino acids 54 to 228 of SEQ ID NO:74, amino acids 57 to 217 of SEQ ID NO:76, or amino acids 81 to 217 of SEQ ID NO:78.

### Q. Vitamin B6 Biosynthetic Genes

In another embodiment, the invention provides a transgenic plant transformed with an expression cassette comprising, in operative association, an isolated polynucleotide encoding a promoter capable of enhancing gene expression in roots and shoots and an isolated polynucleotide encoding a full length polypeptide which is capable of enhancing PLP synthesis. In this embodiment, the expression cassette may optionally further comprise an isolated polynucleotide encoding a mitochondrial or plastid transit peptide. Polynucleotides comprising any gene of the vitamin B6 synthetic pathway are suitable for use in this embodiment of the invention. For example, the vitamin B6 synthetic gene may be pdxY (e.g., SEQ ID NO:79), pdxH (e.g., SEQ ID NO:81), pdxK (e.g., SEQ ID NOs:83, 85, and 87), yfei (e.g., SEQ ID NOs: 89 and 91), Yn8fp (SEQ ID NO:93); pdxJ (e.g., SEQ ID NOs: 95 and 97), pdx1/ sor1 (e.g., SEQ IS NOs: 99, 101, 103, 105, 107, 109, 111, 113, 115, 117, or 119).

When the transgenic plant comprises the vitamin B6 biosynthetic gene PdxY, the promoter is preferably a root- or shoot-specific promoter and the expression cassette further comprises a polynucleotide encoding a plastid transit peptide. As shown in Tables 23 and 24 below, when the E. coli gene b1636 (SEQ ID NO:79), designated pdxY, is targeted to the plastid under control of the Super promoter, transgenic plants were larger than control plants under well watered and water-limited conditions.

The PdxY gene encodes a PL kinase which comprises a phosphomethyl-pyrimidine kinase domain. The transgenic plant of this embodiment may comprise any polynucleotide encoding a PL kinase. Preferably, the transgenic plant of this embodiment comprises a polynucleotide encoding a full-length polypeptide having PL kinase activity, wherein the polypeptide comprises a phosphomethyl-pyrimidine kinase signature sequence such as amino acids 61 to 259 of SEQ ID NO:80. More preferably, the transgenic plant of this embodiment comprises a polynucleotide encoding a PL kinase having a sequence comprising amino acids 1 to 287 of SEQ ID NO:80.

As shown in Table 25 below, when the Synechocystis gene SLL1779 (SEQ ID NO:95), designated pdxJ, is expressed under control of the PcUbi promoter, with or without subcellular targeting, transgenic plants are larger than control plants under well-watered conditions. Table 26 shows that when SLL1779 (SEQ ID NO:95) is targeted to mitochondria under control of the PcUbi promoter, transgenic plants are larger than control plants when tested under water-limited conditions. Accordingly, when the transgenic plant comprises the PdxJ gene, the expression cassette may optionally further comprise a polynucleotide encoding a mitochondrial or plastid transit peptide. As set forth above, the PdxJ enzyme acts in a concerted manner with the PdxA enzyme to form PNP. Amino acids 2 to 218 of SEQ ID NO:96 represent a signature sequence of the PdxJ gene from Synechocystis sp PCC6803. Preferably, the PdxJ gene employed in the expression cassette of this embodiment encodes a polypeptide amino acids 1 to 221 of SEQ ID NO:96 or amino acids 1 to 243 of SEQ ID NO:98.

The invention further provides a seed which is true breeding for the expression cassettes (also referred to herein as " transgenes") described herein, wherein transgenic plants grown from said seed demonstrate increased yield as compared to a wild type variety of the plant. The invention also provides a product produced by or from the transgenic plants expressing the polynucleotide, their plant parts, or their seeds. The product can be obtained using various methods well known in the art. As used herein, the word " product" includes, but not limited to, a foodstuff, feedstuff, a food supplement, feed supplement, fiber, cosmetic or pharmaceutical. Foodstuffs are regarded as compositions used for nutrition or for supplementing nutrition. Animal feedstuffs and animal feed supplements, in particular, are regarded as foodstuffs. The invention further provides an agricultural product produced by any of the transgenic plants, plant parts, and plant seeds. Agricultural products include, but are not limited to, plant extracts, proteins, amino acids, carbohydrates, fats, oils, polymers, vitamins, and the like.

The invention also provides an isolated polynucleotide which has a sequence selected from the group consisting of SEQ ID NO:3; SEQ ID NO:5; SEQ ID NO:9; SEQ ID NO:11; SEQ ID NO:13; SEQ ID NO:17; SEQ ID NO:19; SEQ ID NO:23; SEQ ID NO:25; SEQ ID NO:29; SEQ ID NO:31; SEQ ID NO:33; SEQ ID NO:37; SEQ ID NO:45; SEQ ID NO:53; SEQ ID NO:59; SEQ ID NO:61; SEQ ID NO:63; SEQ ID NO:65; SEQ ID NO:69; SEQ ID NO:73; SEQ ID NO:75; and SEQ ID NO:77. Also encompassed by the isolated polynucleotide of the invention is an isolated polynucleotide encoding a polypeptide having an amino acid sequence selected from the group consisting of SEQ ID NO:4; SEQ ID NO:6; SEQ ID NO:10; SEQ ID NO:12; SEQ ID NO:14; SEQ ID NO:18; SEQ ID NO:20; SEQ ID NO:24; SEQ ID NO:26; SEQ ID NO:30; SEQ ID NO:32; SEQ ID NO:34; SEQ ID NO:38; SEQ ID NO:54; SEQ ID NO:60; SEQ ID NO:62; SEQ ID NO:64; SEQ ID NO:70; SEQ ID NO:74; SEQ ID NO:76; and SEQ ID NO:78. A polynucleotide of the invention can be isolated using standard molecular biology techniques and the sequence information provided herein, for example, using an automated DNA synthesizer.

The isolated polynucleotides of the invention include homologs of the polynucleotides of Table 1. " Homologs" are defined herein as two nucleic acids or polypeptides that have similar, or substantially identical, nucleotide or amino acid sequences, respectively. Homologs include allelic variants, analogs, and orthologs, as defined below. As used herein, the term " analogs" refers to two nucleic acids that have the same or similar function, but that have evolved separately in unrelated organisms. As used herein, the term " orthologs" refers to two nucleic acids from different species, but that have evolved from a common ancestral gene by speciation. The term homolog further encompasses nucleic acid molecules that differ from one of the nucleotide sequences shown in Table 1 due to degeneracy of the genetic code and thus encode the same polypeptide.

To determine the percent sequence identity of two amino acid sequences (e.g., one of the polypeptide sequences of Table 1 and a homolog thereof), the sequences are aligned for optimal comparison purposes (e.g., gaps can be introduced in the sequence of one polypeptide for optimal alignment with the other polypeptide or nucleic acid). The amino acid residues at corresponding amino acid positions are then compared. When a position in one sequence is occupied by the same amino acid residue as the corresponding position in the other sequence then the molecules are identical at that position. The same type of comparison can be made between two nucleic acid sequences.

Preferably, the isolated amino acid homologs, analogs, and orthologs of the polypeptides of the present invention are at least about 50-60%, preferably at least about 60-70%, and more preferably at least about 70-75%, 75-80%, 80-85%, 85-90%, or 90-95%, and most preferably at least about 96%, 97%, 98%, 99%, or more identical to an entire amino acid sequence identified in Table 1. In another preferred embodiment, an isolated nucleic acid homolog of the invention comprises a nucleotide sequence which is at least about 40-60%, preferably at least about 60-70%, more preferably at least about 70-75%, 75-80%, 80-85%, 85-90%, or 90-95%, and even more preferably at least about 95%, 96%, 97%, 98%, 99%, or more identical to a nucleotide sequence shown in Table 1.

For the purposes of the invention, the percent sequence identity between two nucleic acid or polypeptide sequences is determined using Align 2.0 (Myers and Miller, CABIOS (1989) 4:11-17) with all parameters set to the default settings or the Vector NTI Advance 10.3.0 (PC) software package (Invitrogen, 1600 Faraday Ave., Carlsbad, CA92008). For percent identity calculated with Vector NTI, a gap opening penalty of 15 and a gap extension penalty of 6.66 are used for determining the percent identity of two nucleic acids. A gap opening penalty of 10 and a gap extension penalty of 0.1 are used for determining the percent identity of two polypeptides. All other parameters are set at the default settings. For purposes of a multiple alignment (Clustal W algorithm), the gap opening penalty is 10, and the gap extension penalty is 0.05 with blosum62 matrix. It is to be understood that for the purposes of determining sequence identity when comparing a DNA sequence to an RNA sequence, a thymidine nucleotide is equivalent to a uracil nucleotide.

Nucleic acid molecules corresponding to homologs, analogs, and orthologs of the polypeptides listed in Table 1 can be isolated based on their identity to said polypeptides, using the polynucleotides encoding the respective polypeptides or primers based thereon, as hybridization probes according to standard hybridization techniques under stringent hybridization conditions. As used herein with regard to hybridization for DNA to a DNA blot, the term " stringent conditions" refers to hybridization overnight at 60°C in 10X Denhart' s solution, 6X SSC, 0.5% SDS, and 100 µg/ml denatured salmon sperm DNA. Blots are washed sequentially at 62°C for 30 minutes each time in 3X SSC/0.1% SDS, followed by 1X SSC/0.1% SDS, and finally 0.1X SSC/0.1% SDS. As also used herein, in a preferred embodiment, the phrase " stringent conditions" refers to hybridization in a 6X SSC solution at 65°C. In another embodiment, " highly stringent conditions" refers to hybridization overnight at 65°C in 10X Denhart' s solution, 6X SSC, 0.5% SDS and 100 µg/ml denatured salmon sperm DNA. Blots are washed sequentially at 65°C for 30 minutes each time in 3X SSC/0.1 % SDS, followed by 1X SSC/0.1% SDS, and finally 0.1X SSC/0.1% SDS. Methods for performing nucleic acid hybridizations are well known in the art.

The isolated polynucleotides employed in the invention may be optimized, that is, genetically engineered to increase its expression in a given plant or animal. To provide plant optimized nucleic acids, the DNA sequence of the gene can be modified to: 1) comprise codons preferred by highly expressed plant genes; 2) comprise an A+T content in nucleotide base composition to that substantially found in plants; 3) form a plant initiation sequence; 4) to eliminate sequences that cause destabilization, inappropriate polyadenylation, degradation and termination of RNA, or that form secondary structure hairpins or RNA splice sites; or 5) elimination of antisense open reading frames. Increased expression of nucleic acids in plants can be achieved by utilizing the distribution frequency of codon usage in plants in general or in a particular plant. Methods for optimizing nucleic acid expression in plants can be found in EPA 0359472; EPA 0385962; PCT Application No. WO 91/16432; U.S. Patent No. 5,380,831; U.S. Patent No. 5,436,391; Perlack et al., 1991, Proc. Natl. Acad. Sci. USA 88:3324-3328; and Murray et al., 1989, Nucleic Acids Res. 17:477-498.

The invention further provides a recombinant expression vector which comprises an expression cassette selected from the group consisting of a) an expression cassette comprising, in operative association, an isolated polynucleotide encoding a promoter, an isolated polynucleotide encoding a subcellular targeting peptide, and an isolated polynucleotide encoding a full-length phosphatidate cytidylyltransferase polypeptide; b) an expression cassette comprising, in operative association, an isolated polynucleotide encoding a promoter capable of enhancing expression in leaves, an isolated polynucleotide encoding a mitochondrial transit peptide, and an isolated polynucleotide encoding an acyl-carrier protein; and c) an expression cassette comprising, in operative association, an isolated polynucleotide encoding a promoter, an isolated polynucleotide encoding a subcellular targeting peptide; and an isolated polynucleotide encoding an acyltransferase polypeptide.

In another embodiment, the recombinant expression vector of the invention comprises an isolated polynucleotide having a sequence selected from the group consisting of SEQ ID NO:4; SEQ ID NO:6; SEQ ID NO:10; SEQ ID NO:12; SEQ ID NO:14; SEQ ID NO:18; SEQ ID NO:20; SEQ ID NO:24; SEQ ID NO:26; SEQ ID NO:30; SEQ ID NO:32; SEQ ID NO:34; SEQ ID NO:38; SEQ ID NO:54; SEQ ID NO:60; SEQ ID NO:62; SEQ ID NO:64; SEQ ID NO:70; SEQ ID NO:74; SEQ ID NO:76; and SEQ ID NO:78. In addition, the recombinant expression vector of the invention comprises an isolated polynucleotide encoding a polypeptide having an amino acid sequence selected from the group consisting SEQ ID NO:4; SEQ ID NO:6; SEQ ID NO:10; SEQ ID NO:12; SEQ ID NO:14; SEQ ID NO:18; SEQ ID NO:20; SEQ ID NO:24; SEQ ID NO:26; SEQ ID NO:30; SEQ ID NO:32; SEQ ID NO:34; SEQ ID NO:38; SEQ ID NO:54; SEQ ID NO:60; SEQ ID NO:62; SEQ ID NO:64; SEQ ID NO:70; SEQ ID NO:74; SEQ ID NO:76; and SEQ ID NO:78.

The recombinant expression vector of the invention may also include one or more regulatory sequences, selected on the basis of the host cells to be used for expression, which is in operative association with the isolated polynucleotide to be expressed. As used herein with respect to a recombinant expression vector, " in operative association" or " operatively linked" means that the polynucleotide of interest is linked to the regulatory sequence(s) in a manner which allows for expression of the polynucleotide when the vector is introduced into the host cell (e.g., in a bacterial or plant host cell). The term " regulatory sequence" is intended to include promoters, enhancers, and other expression control elements (e.g., polyadenylation signals).

As set forth above, certain embodiments of the invention employ promoters that are capable of enhancing gene expression in leaves. In some embodiments, the promoter is a leaf-specific promoter. Any leaf-specific promoter may be employed in these embodiments of the invention. Many such promoters are known, for example, the USP promoter from Vicia faba (SEQ ID NO:123 or SEQ ID NO:124, Baeumlein et al. (1991) Mol. Gen. Genet. 225, 459-67), promoters of light-inducible genes such as ribulose-1.5-bisphosphate carboxylase (rbcS promoters), promoters of genes encoding chlorophyll a/b-binding proteins (Cab), Rubisco activase, B-subunit of chloroplast glyceraldehyde 3-phosphate dehydrogenase from A. thaliana, (Kwon et al. (1994) Plant Physiol. 105,357-67) and other leaf specific promoters such as those identified in Aleman, I. (2001) Isolation and characterization of leaf-specific promoters from alfalfa (Medicago sativa), Masters thesis, New Mexico State University, Los Cruces, NM, and the likea constitutive promoter. Constitutive promoters are active under most conditions. Examples of constitutive promoters suitable for use in these embodiments include the parsley ubiquitin promoter from Petroselinum crispum described in WO 2003/102198 (SEQ ID NO:121); the CaMV 19S and 35S promoters, the sX CaMV 35S promoter, the Sep1 promoter, the rice actin promoter, the Arabidopsis actin promoter, the maize ubiquitin promoter, pEmu, the figwort mosaic virus 35S promoter, the Smas promoter, the super promoter (U.S. Patent No. 5, 955,646), the GRP1-8 promoter, the cinnamyl alcohol dehydrogenase promoter (U.S. Patent No. 5,683,439), promoters from the T-DNA of Agrobacterium, such as mannopine synthase, nopaline synthase, and octopine synthase, the small subunit of ribulose biphosphate carboxylase (ssuRUBISCO) promoter, and the like.

In other embodiments of the invention, a root or shoot specific promoter is employed. For example, the Super promoter (SEQ ID NO:122) provides high level expression in both root and shoots (Ni et al. (1995) Plant J. 7: 661-676). Other root specific promoters include, without limitation, the TobRB7 promoter (Yamamoto et al. (1991) Plant Cell 3, 371-382), the roID promoter (Leach et al. (1991) Plant Science 79, 69-76); CaMV 35S Domain A (Benfey et al. (1989) Science 244, 174-181), and the like.

In accordance with the invention, a chloroplast transit sequence refers to a nucleotide sequence that encodes a chloroplast transit peptide. Chloroplast targeting sequences are known in the art and include the chloroplast small subunit of ribulose1,5-bisphosphate carboxylase (Rubisco) (de Castro Silva Filho et al. (1996) Plant Mol. Biol. 30:769-780; Schnell et al. (1991) J. Biol. Chem. 266(5):3335-3342); 5-(enolpyruvyl)shikimate-3-phosphate synthase (EPSPS) (Archer et al. (1990) J. Bioenerg. Biomemb. 22(6):789-810); tryptophan synthase (Zhao et al. (1995) J. Biol. Chem. 270(11):6081-6087); plastocyanin (Lawrence et al. (1997) J. Biol. Chem. 272(33):20357-20363); chorismate synthase (Schmidt et al. (1993) J. Biol. Chem. 268(36):27447-27457); ferredoxin NADP+ oxidoreductase (Jansen et al. (1988) Curr. Genetics 13:517-522) (SEQ ID NO:13); nitrite reductase (Back et al (1988) MGG 212:20-26) and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

As defined herein, a mitochondrial transit sequence refers to a nucleotide sequence that encodes a mitochondrial presequence and directs the protein to mitochondria. Examples of mitochondrial presequences include groups consisting of ATPase subunits, ATP synthase subunits, Rieske-FeS protein, Hsp60, malate dehydrogenase, citrate synthase, aconitase, isocitrate dehydrogenase, pyruvate dehydrogenase, malic enzyme, glycine decarboxylase, serine hydroxymethyl transferase, isovaleryl-CoA dehydrogenase and superoxide dismutase. Such transit peptides are known in the art. See, for example, Von Heijne et al. (1991) Plant Mol. Biol. Rep. 9:104-126; Clark et al. (1989) J. Biol. Chem. 264:17544-17550; Romer et al. (1993) Biochem. Biophys. Res. Commun. 196:1414-1421; Faivre-Nitschke et al (2001) Eur J Biochem 268 1332- 1339 and Shah et al. (1986) Science 233: 478-481.

In a preferred embodiment of the present invention, the polynucleotides listed in Table 1 are expressed in plant cells from higher plants (e.g., the spermatophytes, such as crop plants). A polynucleotide may be " introduced" into a plant cell by any means, including transfection, transformation or transduction, electroporation, particle bombardment, agroinfection, and the like. Suitable methods for transforming or transfecting plant cells are disclosed, for example, using particle bombardment as set forth in U.S. Pat. Nos. 4,945,050; 5,036,006; 5,100,792; 5,302,523; 5,464,765; 5,120,657; 6,084,154; and the like. More preferably, the transgenic corn seed of the invention may be made using Agrobacterium transformation, as described in U.S. Pat. Nos. 5,591,616; 5,731,179; 5,981,840; 5,990,387; 6,162,965; 6,420,630, U.S. patent application publication number 2002/0104132, and the like. Transformation of soybean can be performed using for example any of the techniques described in European Patent No. EP 0424047, U.S. Patent No. 5,322,783, European Patent No.EP 0397 687, U.S. Patent No. 5,376,543, or U.S. Patent No. 5,169,770. A specific example of wheat transformation can be found in PCT Application No. WO 93/07256. Cotton may be transformed using methods disclosed in U.S. Pat. Nos. 5,004,863; 5,159,135; 5,846,797, and the like. Rice may be transformed using methods disclosed in U.S. Pat. Nos. 4,666,844; 5,350,688; 6,153,813; 6,333,449; 6,288,312; 6,365,807; 6,329,571, and the like. Canola may be transformed, for example, using methods such as those disclosed in U.S. Pat. Nos.5,188,958; 5,463,174; 5,750,871; EP1566443; WO02/00900; and the like. Other plant transformation methods are disclosed, for example, in U.S. Pat. Nos. 5,932,782; 6,153,811; 6,140,553; 5,969,213; 6,020,539, and the like. Any plant transformation method suitable for inserting a transgene into a particular plant may be used in accordance with the invention.

According to the present invention, the introduced polynucleotide may be maintained in the plant cell stably if it is incorporated into a non-chromosomal autonomous replicon or integrated into the plant chromosomes. Alternatively, the introduced polynucleotide may be present on an extra-chromosomal non-replicating vector and may be transiently expressed or transiently active.

The invention is also embodied in a method of producing a transgenic plant comprising at least one polynucleotide listed in Table 1, wherein expression of the polynucleotide in the plant results in the plant' s increased growth and/or yield under normal or water-limited conditions and/or increased tolerance to an environmental stress as compared to a wild type variety of the plant comprising the steps of: (a) introducing into a plant cell an expression cassette described above, (b) regenerating a transgenic plant from the transformed plant cell; and selecting higher-yielding plants from the regenerated plant sells. The plant cell may be, but is not limited to, a protoplast, gamete producing cell, and a cell that regenerates into a whole plant. As used herein, the term " transgenic" refers to any plant, plant cell, callus, plant tissue, or plant part, that contains the expression cassette described above. In accordance with the invention, the expression casette is stably integrated into a chromosome or stable extra-chromosomal element, so that it is passed on to successive generations.

The effect of the genetic modification on plant growth and/or yield and/or stress tolerance can be assessed by growing the modified plant under normal and/or less than suitable conditions and then analyzing the growth characteristics and/or metabolism of the plant. Such analytical techniques are well known to one skilled in the art, and include measurements of dry weight, wet weight, seed weight, seed number, polypeptide synthesis, carbohydrate synthesis, synthesis, evapotranspiration rates, general plant and/or crop yield, flowering, reproduction, seed setting, root growth, respiration rates, photosynthesis rates, metabolite composition, and the like.

The invention is further illustrated by the following examples, which are not to be construed in any way as imposing limitations upon the scope thereof.

### EXAMPLE 1

### Characterization of Genes

YBR029C (SEQ ID NO:1), YKL192C (SEQ ID NO:7), YDR018C (SEQ ID NO:15), B2341 (SEQ ID NO:21), B0452 (SEQ ID NO:27), YNL202W (SEQ ID NO:35), YKL140W (SEQ ID NO:39), SLL1023 (SEQ ID NO:41), SLR0252 (SEQ ID NO:43), b3803 (SEQ ID NO:45), b3209 (SEQ ID NO:53), b2578 (SEQ ID NO:59), b2682 (SEQ ID NO:61), b3285 (SEQ ID NO:63), b1938 (SEQ ID NO:65), SLL1894 (SEQ ID NO: 67), SLL1282 (SEQ ID NO:5), b1636 (SEQ ID NO:79) and SLR1779 (SEQ ID NO:95) were cloned using standard recombinant techniques. The functionality of each lead gene was predicted by comparing the amino acid sequence of the gene with other genes of known functionality. Homolog cDNAs were isolated from proprietary libraries of the respective species using known methods. Sequences were processed and annotated using bioinformatics analyses..

The YBR029C (SEQ ID NO:2) from S. cerevisiae encodes a phosphatidate cytidylyltransferase. The DNA sequence of this gene was blasted against proprietary databases of canola and maize cDNAs at an e value of e-10 (Altschul et al., 1997, Nucleic Acids Res. 25: 3389-3402). One homolog from canola and one homolog from maize were identified. The amino acid relatedness of these sequences is indicated in the alignments shown in Figure 1.

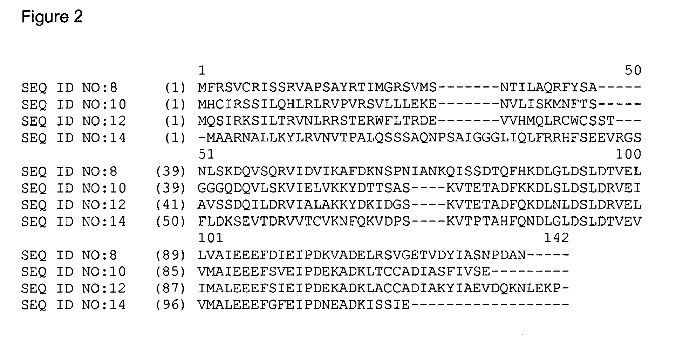

The YKL192C (SEQ ID NO:8) from S. cerevisiae encodes an acyl-carrier protein. The DNA sequence of this gene was blasted against proprietary databases of plant cDNAs at an e value of e-10 (Altschul et al., 1997, Nucleic Acids Res. 25: 3389-3402). One homolog each from canola, soybean, and sunflower were identified. The amino acid relatedness of these sequences is indicated in the alignments shown in Figure 2.

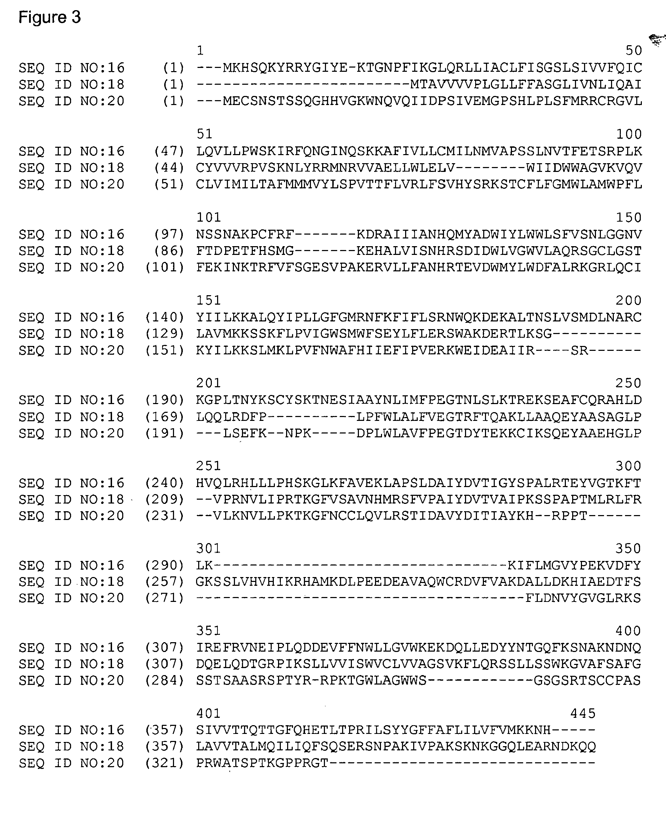

The of YDR018C (SEQ ID NO:16) from S. cerevisiae encodes an acyltransferase protein. The DNA sequence of this gene was blasted against proprietary databases of soybean and maize cDNAs at an e value of e-10 (Altschul et al., 1997, Nucleic Acids Res. 25: 3389-3402). One homolog from soybean and one from maize were identified. The amino acid relatedness of these sequences is shown in Figure 3.

The B2341 (SEQ ID NO:21) gene from E. coli encodes a bifunctional anaerobic fatty acid oxidation complex polypeptide. The DNA sequence of this gene was blasted against a proprietary maize cDNA database at an e value of e-10 (Altschul et al., 1997, Nucleic Acids Res. 25: 3389-3402). Two homologs from maize were identified. The amino acid relatedness of these sequences is shown in Figure 4.

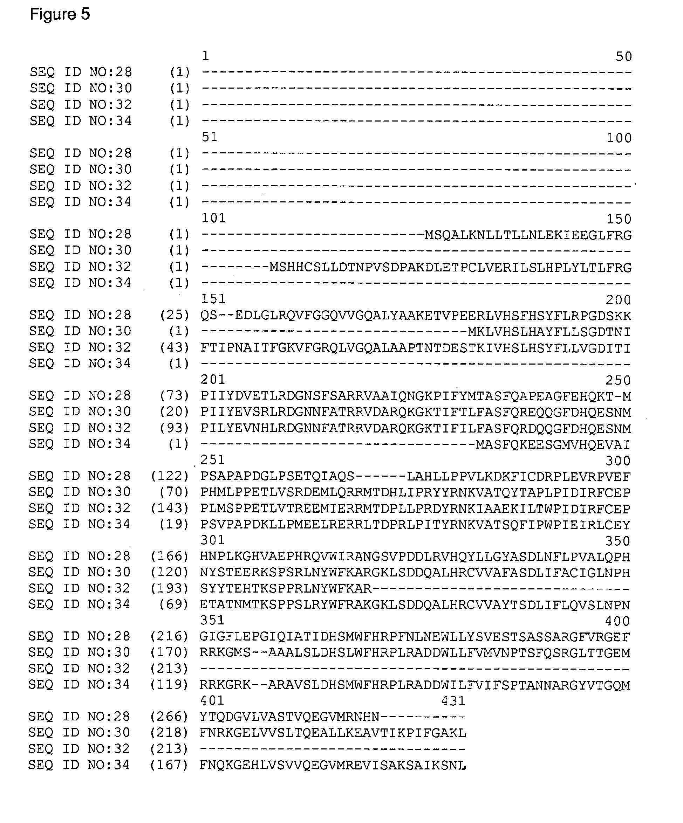

The B0452 (SEQ ID NO:28) from E. coli encodes an acyl-CoA thioesterase protein. The DNA sequence of this gene was blasted against proprietary databases of plant cDNAs at an e value of e-1 0 (Altschul et al., 1997, Nucleic Acids Res. 25: 3389-3402). Two homologs from canola and one from soybean were identified. The amino acid relatedness of these sequences is shown in Figure 5.

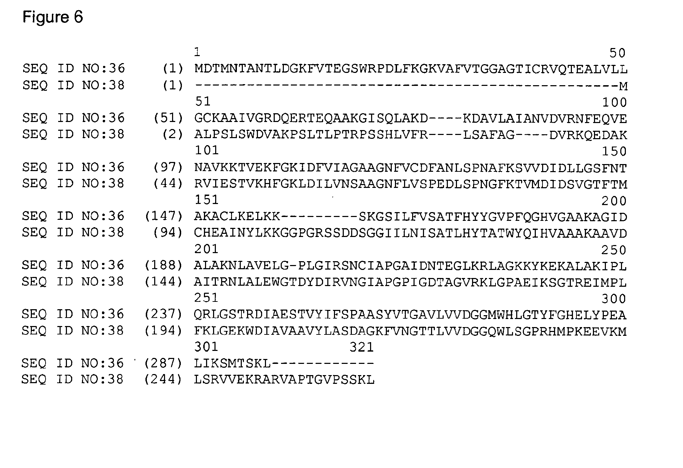

YNL202W (SEQ ID NO:36) from S. cerevisiae encodes a 2,4-dienoyl-CoA reductase protein. The DNA sequence of this gene was blasted against proprietary databases of plant cDNAs at an e value of e-10 (Altschul et al., 1997, Nucleic Acids Res. 25: 3389-3402). One homolog from sunflower were identified. The amino acid relatedness of these sequences is shown in Figure 6.

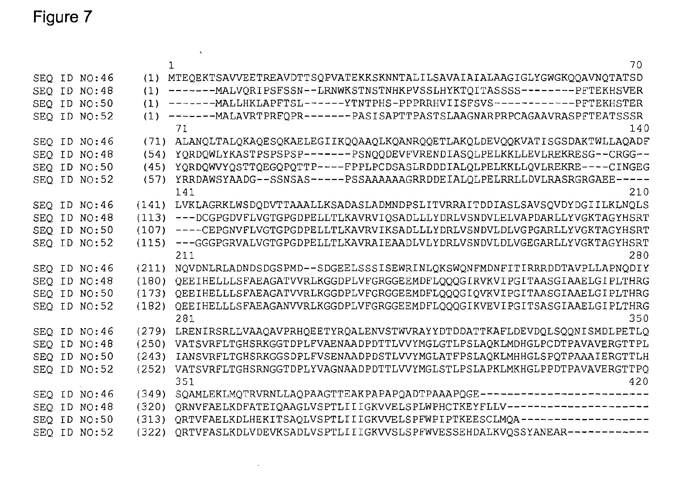

The b3803 gene from E. coli encodes a uroporphyrin-III C-methyltransferase. The full-length amino acid sequence of the functional homologs of b3803 from Table 11 were blasted against proprietary databases of cDNAs at an e value of e-10 (Altschul et al., 1997, Nucleic Acids Res. 25: 3389-3402). One homolog from Brassica napus, one homolog from Glycine max, and one homolog from Zea mays were identified. Homolog cDNAs were isolated from proprietary libraries of the respective species using known methods. Sequences were processed and annotated using bioinformatics analyses. The amino acid relatedness of these sequences is shown in Figure 7.

The b3209 gene from E. coli encodes an isoprenoid biosynthesis protein. The full-length amino acid sequence of functional homologs of b3209 from Table 15 were blasted against proprietary databases of cDNAs at an e value of e-10 (Altschul et al., 1997, Nucleic Acids Res. 25: 3389-3402). One homolog from Glycine max and one homolog from Helianthus annuus were identified. The amino acid relatedness of these sequences is shown in Figure 8.

The SLL1894 (SEQ ID NO:67) gene from Synechocystis sp. PCC 6803 encodes a GTP cyclohydrase II. The full-length DNA sequence of SLL1894 was blasted against a proprietary databases of soybean cDNAs at an e value of e-10 (Altschul et al., 1997, Nucleic Acids Res. 25: 3389-3402) and one homolog from soybean was identified. The amino acid relatedness of these sequences is shown in Figure 12.

The SLL1282 gene (SEQ ID NO:71) from Synechocystis sp. PCC 6803 encodes a lumazine synthase. The full-length DNA sequence of this gene was blasted against proprietary databases of soybean and maize cDNAs at an e value of e-10 (Altschul et al., 1997, Nucleic Acids Res. 25: 3389-3402). One homolog from soybean and two homologs from maize were identified. The amino acid relatedness of these sequences is shown in Figure 13.

### EXAMPLE 2

### Overexpression of Lead Genes in Plants

Each of the genes described in Example 1 was ligated into an expression cassette using known methods. Four different promoters were used to control expression of the transgenes in Arabidopsis: the parsley ubiquitin promoter (SEQ ID NO:121) designated " PCUbi" in Tables 2 to 26 ; the super promoter (SEQ ID NO:122) designated " Super" in Tables 2 to 26; the USP promoter from Vicia faba (SEQ ID NO:124), designated " USP" in Tables 2 to 26 was used for expression of genes from prokaryotes or SEQ ID NO:123 was used for expression of genes from S. cerevisiae). For selective targeting of the polypeptides, the mitochondrial transit peptide from an A. thaliana gene encoding mitochondrial isovaleryl-CoA dehydrogenase designated " Mit" in Tables 2 to 26, SEQ ID NO:126 was used for expression of genes from prokaryotes or SEQ ID NO:128 was used for expression of genes from S. cerevisiae. In addition, for selective targeting of polypeptides to the chloroplast, the transit peptide of an Spinacia oleracea gene encoding ferredoxin nitrite reductase designated " Chlor" in Tables 2 to 26; SEQ ID NO:130 was used.

The Arabidopsis ecotype C24 was transformed with constructs containing the lead genes described in Example 1 using known methods. Seeds from T2 transformed plants were pooled on the basis of the promoter driving the expression, gene source species and type of targeting (chloroplastic, mitochondrial and cytoplasmic). The seed pools were used in the primary screens for biomass under well watered and water limited growth conditions. Hits from pools in the primary screen were selected, molecular analysis performed and seed collected. The collected seeds were then used for analysis in secondary screens where a larger number of individuals for each transgenic event were analyzed. If plants from a construct were identified in the secondary screen as having increased biomass compared to the controls, it passed to the tertiary screen. In this screen, over 100 plants from all transgenic events for that construct were measured under well watered and drought growth conditions. The data from the transgenic plants were compared to wild type Arabidopsis plants or to plants grown from a pool of randomly selected transgenic Arabidopsis seeds using standard statistical procedures.

Plants that were grown under well watered conditions were watered to soil saturation twice a week. Images of the transgenic plants were taken at 17 and 21 days using a commercial imaging system. Alternatively, plants were grown under water limited growth conditions by watering to soil saturation infrequently which allowed the soil to dry between watering treatments. In these experiments, water was given on days 0, 8, and 19 after sowing. Images of the transgenic plants were taken at 20 and 27 days using a commercial imaging system.

Image analysis software was used to compare the images of the transgenic and control plants grown in the same experiment. The images were used to determine the relative size or biomass of the plants as pixels and the color of the plants as the ratio of dark green to total area. The latter ratio, termed the health index, was a measure of the relative amount of chlorophyll in the leaves and therefore the relative amount of leaf senescence or yellowing and was recorded at day 27 only. Variation exists among transgenic plants that contain the various lead genes, due to different sites of DNA insertion and other factors that impact the level or pattern of gene expression.

Tables 2 to 26 show the comparison of measurements of the Arabidopsis plants. Percent change indicates the measurement of the transgenic relative to the control plants as a percentage of the control non-transgenic plants; p value is the statistical significance of the difference between transgenic and control plants based on a T-test comparison of all independent events where NS indicates not significant at the 5% level of probabilty; " No. of events" indicates the total number of independent transgenic events tested in the experiment; " No. of positive events" indicates the total number of independent transgenic events that were larger than the control in the experiment; " No. of negative events" indicates the total number of independent transgenic events that were smaller than the control in the experiment. NS indicates not significant at the 5% level of probability.

### A. Phosphatidate Cytidylyltransferase

The phosphatidate cytidylyltransferase designated as YBR029C (SEQ ID NO:2) was expressed in Arabidopsis using three constructs: in one construct YBR029C expression was controlled by the PCUbi promoter (SEQ ID NO: 121) and targeted to chloroplasts; in another construct, YBR029C expression was controlled by the Super promoter (SEQ ID NO:122) and targeted to chloroplasts; and in the third construct YBR029C expression was controlled by the USP promoter (SEQ ID NO:123) and targeted to the mitochondria. Table 2 sets forth biomass and health index data obtained from the Arabidopsis plants transformed with these constructs and tested under water-limiting conditions.

**Table 2**

| Gene | Promoter | Targeting | Measurement | % Change | p-Value | No. of Events | No of Positive Events | No. of Negative Events |
|---|---|---|---|---|---|---|---|---|
| YBR029C | PCUbi | Chlor | Biomass at day 20 | -16.7 | 0.0000 | 6 | 1 | 5 |
| YBR029C | PCUbi | Chlor | Biomass at day 27 | -17.2 | 0.0000 | 6 | 0 | 6 |
| YBR029C | PCUbi | Chlor | Health index | -9.5 | 0.0056 | 6 | 1 | 5 |
| YBR029C | Super | Chlor | Biomass at day 20 | 8.2 | 0.0340 | 6 | 4 | 2 |
| YBR029C | Super | Chlor | Biomass at day 27 | 19.4 | 0.0000 | 6 | 5 | 1 |
| YBR029C | Super | Chlor | Health index | 0.6 | NS | 6 | 4 | 2 |
| YBR029C | USP | Mit | Biomass at day 20 | -10.1 | 0.0040 | 8 | 2 | 6 |
| YBR029C | USP | Mit | Biomass at day 27 | -9.5 | 0.0034 | 8 | 2 | 6 |
| YBR029C | USP | Mit | Health index | -1.8 | NS | 8 | 2 | 6 |

Table 2 shows that, under water limiting conditions, Arabidopsis plants expressing the YBR029C (SEQ ID NO:1) gene under control of the Super promoter with targeting of the protein product to the chloroplast and grown were significantly larger than the control plants. Table 2 also shows that the majority of independent Super promoter/chloroplast-targeted transgenic events were larger than the controls. Table 2 also shows that Arabidopsis plants grown under water limiting conditions and expressing the YBR029C gene under control of either the USP promoter with targeting of the protein product to mitochondria or the PCUbi promoter with targeting of the protein product to the chloroplast were significantly smaller than the control plants that did not express YBR029C. Table 2 also shows that the majority of independent transgenic events were smaller than the controls.

Table 3 sets forth biomass and health index data obtained from the Arabidopsis plants transformed with these constructs and tested under well watered conditions.

**Table 3**

| Gene | Promoter | Targeting | Measurement | % Change | p-Value | No. of Events | No of Positive Events | No. of Negative Events |
|---|---|---|---|---|---|---|---|---|
| YBR029C | PCUbi | Chlor | Biomass at day 17 | 9.5 | 0.0001 | 6 | 5 | 1 |
| YBR029C | PCUbi | Chlor | Biomass at day 21 | 5.5 | 0.0066 | 6 | 5 | 1 |
| YBR029C | PCUbi | Chlor | Health index | -9.6 | 0.0052 | 6 | 0 | 6 |
| YBR029C | Super | Chlor | Biomass at day 17 | 5.2 | 0.050 | 6 | 4 | 2 |
| YBR029C | Super | Chlor | Biomass at day 21 | 6.7 | 0.0035 | 6 | 4 | 2 |
| YBR029C | Super | Chlor | Health index | -10.7 | 0.0012 | 6 | 0 | 6 |
| YBR029C | USP | Chlor | Biomass at day 17 | 6.2 | 0.0378 | 6 | 5 | 1 |
| YBR029C | USP | Chlor | Biomass at day 21 | 6.2 | 0.0174 | 6 | 5 | 1 |
| YBR029C | USP | Chlor | Health index | -2.7 | NS | 6 | 2 | 4 |
| YBR029C | USP | Mit | Biomass at day 17 | 14.7 | 0.0000 | 8 | 7 | 1 |
| YBR029C | USP | Mit | Biomass at day 21 | 9.6 | 0.0000 | 8 | 8 | 0 |
| YBR029C | USP | Mit | Health index | 1.2 | NS | 8 | 4 | 4 |

Table 3 shows that, when grown under well-watered conditions, Arabidopsis plants expressing YBR029C were larger than the control plants that did not express YBR029C. The increase in biomass occurred with the PCUbi, Super and Ubi promoters and the effect of the transgene was observed in constructs where the YBR029C protein product (SEQ ID NO:2) was targeted to the mitochondria or the chloroplast.

### B. Acyl-Carrier Protein

The acyl-carrier protein designated as YKL192C (SEQ ID NO:8) was expressed in Arabidopsis using a construct wherein the acyl-carrier protein expression was controlled by the USP promoter (SEQ ID NO: 123) and targeted to the mitochondria. Table 4 sets forth biomass and health index data obtained from the Arabidopsis plants transformed with these constructs and tested under water-limiting conditions.

**Table 4**

| Gene | Promoter | Targeting | Measurement | % Change | p-Value | No. of Events | No of Positive Events | No. of Negative Events |
|---|---|---|---|---|---|---|---|---|
| YKL192C | USP | Mit | Biomass at day 20 | 29.8 | 0.0000 | 5 | 5 | 0 |
| YKL192C | USP | Mit | Biomass at day 27 | 16.6 | 0.0000 | 5 | 4 | 1 |
| YKL192C | USP | Mit | Health index | 14.2 | 0.0000 | 5 | 4 | 1 |

Table 4 shows that Arabidopsis plants expressing the YKL192C (SEQ ID NO:7) gene under control of the USP promoter with targeting to the mitochondria that were grown under water limiting conditions were significantly larger than the control plants that did not express YKL192C. Table 4 also shows that the majority of independent transgenic events were larger and healthier than the controls.

### C. Acyltransferase

The acyltransferase designated as YDR018C (SEQ ID NO: 16) was expressed in Arabidopsis using two different constructs: in one construct, YDR018C (SEQ ID NO:15) gene expression was controlled by the Super promoter (SEQ ID NO:122) and the YDR018C protein product was targeted to the choloroplast, and in the second construct, YDR018C expression was controlled by the USP promoter (SEQ ID NO:123) and targeted to the mitochondria. Table 5 sets forth biomass and health index data obtained from the Arabidopsis plants transformed with these constructs and tested under water-limiting conditions.

**Table 5**

| Gene | Promoter | Targeting | Measurement | % Change | p-Value | No. of Events | No of Positive Events | No. of Negative Events |
|---|---|---|---|---|---|---|---|---|
| YDR018C | Super | Chlor | Biomass at day 20 | 6.8 | 0.0486 | 6 | 5 | 1 |
| YDR018C | Super | Chlor | Biomass at day 27 | 21.1 | 0.0000 | 6 | 5 | 1 |
| YDR018C | Super | Chlor | Health index | 3.0 | NS | 6 | 4 | 2 |
| YDR018C | USP | Mit | Biomass at day 20 | 11.5 | 0.0240 | 6 | 4 | 2 |
| YDR018C | USP | Mit | Biomass at day 27 | 7.1 | NS | 6 | 4 | 2 |
| YDR018C | USP | Mit | Health index | 1.7 | NS | 6 | 4 | 2 |

Table 5 shows that Arabidopsis plants expressing YDR018C tended to be significantly larger than the control plants that did not express YDR018C when they were grown under water limiting conditions. Table 5 also shows that the majority of independent transgenic events were larger than the controls.

Table 6 sets forth biomass and health index data obtained from the Arabidopsis plants transformed with these constructs and tested under well watered conditions.

**Table 6**

| Gene | Promoter | Targeting | Measurement | % Change | p-Value | No. of Events | No of Positive Events | No. of Negative Events |
|---|---|---|---|---|---|---|---|---|
| YDR018C | Super | Chlor | Biomass at day 17 | -0.2 | NS | 6 | 3 | 3 |
| YDR018C | Super | Chlor | Biomass at day 21 | 1.7 | NS | 6 | 3 | 3 |
| YDR018C | Super | Chlor | Health index | -7.2 | 0.0375 | 6 | 0 | 6 |
| YDR018C | USP | Mit | Biomass at day 17 | 20.9 | 0.0000 | 6 | 6 | 0 |
| YDR018C | USP | Mit | Biomass at day 21 | 20.3 | 0.0000 | 6 | 5 | 1 |
| YDR018C | USP | Mit | Health index | 2.4 | NS | 6 | 3 | 3 |

Table 6 shows that Arabidopsis plants expressing YDR018C under control of the USP promoter with targeting to mitochondria were larger than the control plants that did not express YDR018C when grown under well watered conditions. However, when the YDR018C gene was expressed under control of the Super promoter and the protein product was targeted to the chloroplast, the plants were not significantly larger than control plants that did not express YDR018C, when the plants were grown under well watered conditions.

### D. Bifunctional anaerobic fatty acid oxidation complex polypeptide

The bifunctional anaerobic fatty acid oxidation complex polypeptide designated as B2341 (SEQ ID NO:22) was expressed in Arabidopsis using a construct wherein B2341 expression was controlled by the USP promoter (SEQ ID NO:124) and the protein product was targeted to the mitochondria. Table 7 sets forth biomass and health index data obtained from the Arabidopsis plants transformed with these constructs and tested under water-limiting conditions.

**Table 7**

| Gene | Promoter | Targeting | Measurement | % Change | p-Value | No. of Events | No of Positive Events | No. of Negative Events |
|---|---|---|---|---|---|---|---|---|
| b2341 | USP | Mit | Biomass at day 20 | 24.2 | 0.0000 | 6 | 5 | 1 |
| b2341 | USP | Mit | Biomass at day 27 | 15.9 | 0.0009 | 6 | 5 | 1 |
| b2341 | USP | Mit | Health index | 10.6 | 0.0007 | 6 | 5 | 1 |

Table 7 shows that Arabidopsis plants expressing the B2341 gene (SEQ ID NO:21) were significantly larger than the control plants that did not express B2341, when grown under water limiting conditions. Table 7 also shows that the majority of independent transgenic events were larger than the controls.

Table 8 sets forth biomass and health index data obtained from Arabidopsis plants transformed with these constructs and tested under well watered conditions.

**Table 8**

| Gene | Promoter | Targeting | Measurement | % Change | p-Value | No. of Events | No of Positive Events | No. of Negative Events |
|---|---|---|---|---|---|---|---|---|
| b2341 | USP | Mit | Biomass at day 20 | 8.5 | 0.0067 | 6 | 6 | 0 |
| b2341 | USP | Mit | Biomass at day 27 | 1.6 | NS | 6 | 5 | 1 |
| b2341 | USP | Mit | Health index | 14.2 | 0.0100 | 6 | 5 | 1 |

Table 8 shows that the majority of independent transgenic events expressing B2341 were larger than the control plants that did not express B2341 when grown under well-watered conditions. Table 8 also shows that the plants expressing B2341 were significantly darker green than the controls.

### E. Acyl-CoA thioesterase

B0452 (SEQ ID NO:27) was expressed in Arabidopsis under control of the USP promoter (SEQ ID NO:124) and the protein product (SEQ ID NO:28) was targeted to the mitochondria. Table 9 sets forth biomass and health index data obtained from the Arabidopsis plants transformed with this construct and tested under cycling drought (CD) or well-watered (WW) conditions.

**Table 9**

| Assay Type | Gene | Promoter | Targeting | Measurement | % Change | p-Value | No. of Events | Positive Events | Negative Events |
|---|---|---|---|---|---|---|---|---|---|
| CD | B0452 | USP | Mit | Day 20 | 36.56 | 0.0000 | 4 | 4 | 0 |
| CD | B0452 | USP | Mit | Day 27 | 21.03 | 0.0000 | 4 | 4 | 0 |
| CD | B0452 | USP | Mit | Health Index | 15.14 | 0.0002 | 4 | 4 | 0 |
| WW | B0452 | USP | Mit | Day 17 | 30.57 | 0.0000 | 5 | 5 | 0 |
| WW | B0452 | USP | Mit | Day 21 | 15.39 | 0.0000 | 5 | 5 | 0 |
| WW | B0452 | USP | Mit | Health Index | 2.59 | NS | 5 | 3 | 2 |

Table 9 shows that transgenic plants expressing the B0452 gene under the control of the USP promoter with targeting to the mitochondria were significantly larger under either well-watered or drought conditions than the control plants that did not express the B0452 gene. The difference was even more striking at earlier stages, indicating a positive effect on seedling vigor. In these experiments, all of the independent transgenic events were larger than the controls in both the cycling drought and well-watered environments. The growth advantage of the transgenic plants was even more prominent under cycling drought conditions. The transgenic plants expressing the B0452 gene stayed significantly healthier than the wild-type control under the cycling drought conditions.

### F. 2,4-dienoyl-CoA reductase

YNL202W (SEQ ID NO:35) was expressed in Arabidopsis using two constructs, one of which transcription was under control of the USP promoter (SEQ ID NO:123) and the protein product YNL202W (SEQ ID NO:36) was targeted to the mitochondria, and the other construct in which transcriptional expression was under control of the PCUbi promoter (SEQ ID NO:121) and the protein product was targeted to the plastids, respectively. Table 10 sets forth biomass and health index data obtained from the Arabidopsis plants transformed with these constructs and tested under cycling drought (CD) and well-watered (WW) conditions.

**Table 10**

| Assay Type | Gene | Promoter | Targeting | Measurement | % Change | p-Value | No. of Events | Positive Events | Negative Events |
|---|---|---|---|---|---|---|---|---|---|
| CD | YNL202W | PCUbi | Chlor | Day 20 | -0.77 | 0.8773 | 6 | 3 | 3 |
| CD | YNL202W | PCUbi | Chlor | Day 27 | -13.24 | 0.0646 | 6 | 0 | 6 |
| CD | YNL202W | PCUbi | Chlor | Health Index | -0.78 | 0.8045 | 6 | 3 | 3 |
| CD | YNL202W | USP | Mit | Day 20 | 58.29 | 0.0000 | 6 | 6 | 0 |
| CD | YNL202W | USP | Mit | Day 27 | 27.33 | 0.0000 | 6 | 6 | 0 |
| CD | YNL202W | USP | Mit | Health Index | 0.36 | 0.9046 | 6 | 2 | 4 |
| WW1 | YNL202W | USP | Mit | Day 17 | 33.64 | 0.0000 | 8 | 8 | 0 |
| WW1 | YNL202W | USP | Mit | Day 21 | 25.52 | 0.0000 | 8 | 8 | 0 |
| WW1 | YNL202W | USP | Mit | Health Index | 0.16 | 0.9592 | 8 | 5 | 3 |
| WW2 | YNL202W | USP | Mit | Day 17 | 31.51 | 0.0000 | 6 | 6 | 0 |
| WW2 | YNL202W | USP | Mit | Day 21 | 21.82 | 0.0000 | 6 | 6 | 0 |
| WW2 | YNL202W | USP | Mit | Health Index | 12.62 | 0.0002 | 6 | 5 | 1 |

Table 10 shows that transgenic plants expressing the YNL202W gene under control of the USP promoter with targeting to the mitochondria were significantly larger under cycling drought conditions and in two experiments (WW1 and WW2) under well-watered conditions than the control plants that did not express the YNL202W gene. In these experiments, all the independent transgenic events with mitochondria targeting were larger than the controls in both the cycling drought and well-watered environments. The transgenic plants expressing the YNL202W gene under control of the USP promoter with targeting to the mitochondria were also significantly healthier in one experiment under well-watered conditions than the control plants that did not express the YNL202W gene.

Table 10 shows that transgenic plants expressing the YNL202W gene under control of the PCUbi promoter with targeting to the plastids were smaller but not significantly different compared to the control plants that did not express the YNL202W gene, under cycling drought conditions.

### G. Sterol esterase

YKL140W (SEQ ID NO:39) was expressed in Arabidopsis using three different constructs: in one construct, expression was controlled by the PCUbi promoter (SEQ ID NO:121) and and the protein product (SEQ ID NO:40) was targeted to the mitochondria, expression in the second construct was also under the control of the PCUbi promoter, but the protein product was targeted to plastids; and expression in the third construct was controlled by the USP promoter (SEQ ID NO: 123) with the protein product being targeted to plastids. Table 11 sets forth biomass and health index data obtained from the Arabidopsis plants transformed with these constructs and tested under cycling drought or well-watered conditions.

**Table 11**

| Assay Type | Gene | Promoter | Targeting | Measurement | % Change | p-Value | No. of Events | Positive Events | Negative Events |
|---|---|---|---|---|---|---|---|---|---|
| CD | YKL140W | USP | Mit | Day 20 | 13.86 | 0.0003 | 6 | 6 | 0 |
| CD | YKL140W | USP | Mit | Day 27 | -2.03 | 0.5931 | 6 | 3 | 3 |
| CD | YKL140W | USP | Mit | Health Index | 5.65 | 0.0969 | 6 | 5 | 1 |
| CD | YKL140W | USP | Chlor | Day 20 | -37.76 | 0.0000 | 5 | 0 | 5 |
| CD | YKL140W | USP | Chlor | Day 27 | -21.06 | 0.0000 | 5 | 0 | 5 |
| CD | YKL140W | USP | Chlor | Health Index | -10.81 | 0.0001 | 5 | 0 | 5 |
| WW | YKL140W | PCUbi | Chlor | Day 17 | 42.97 | 0.0000 | 5 | 5 | 0 |
| WW | YKL140W | PCUbi | Chlor | Day 21 | 26.82 | 0.0000 | 5 | 5 | 0 |
| WW | YKL140W | PCUbi | Chlor | Health Index | -1.09 | 0.7119 | 5 | 2 | 3 |

Table 11 shows that transgenic plants expressing the YKL140W gene under control of the PCUbi promoter with targeting to the plastids were significantly larger under well-watered conditions than the control plants that did not express the YKL140W gene. In these experiments, all of the independent transgenic events with plastid targeting were larger than the controls in the cycling drought environment. Transgenic plants expressing the YKL140W gene under control of the USP promoter with targeting to the mitochondria were also significantly larger under cycling drought conditions than the control wild-type plants. Table 11 shows that transgenic plants expressing the YKL140W gene under control of the USP promoter with targeting to the plastid were significantly smaller under drought conditions than the control plants that did not express the YKL140W gene. Additionally, these transgenic plants had lower health index scores relative to the control in water-limited conditions.

### H. Succinate-CoA ligase

The Synechocystis gene SLL1023 (SEQ ID NO:41) was expressed in Arabidopsis under control of the PCUbi promoter (SEQ ID NO:121) and targeted to the plastids. Table 12 sets forth biomass and health index data obtained from Arabidopsis plants transformed with this construct and tested under cycling drought and well-watered conditions.

**Table 12**

| Assay Type | Gene | Promoter | Targeting | Measurement | % Change | p-Value | No. of Events | Positive Events | Negative Events |
|---|---|---|---|---|---|---|---|---|---|
| CD | SLL1023 | PCUbi | Chlor | Day 20 | 43.02 | 0.0000 | 6 | 6 | 0 |
| CD | SLL1023 | PCUbi | Chlor | Day 27 | 37.78 | 0.0000 | 6 | 6 | 0 |
| CD | SLL1023 | PCUbi | Chlor | Health Index | 0.61 | 0.8357 | 6 | 3 | 3 |
| WW | SLL1023 | PCUbi | Chlor | Day 17 | 24.08 | 0.0000 | 6 | 6 | 0 |
| WW | SLL1023 | PCUbi | Chlor | Day 21 | 16.79 | 0.0000 | 6 | 6 | 0 |
| WW | SLL1023 | PCUbi | Chlor | Health Index | 9.33 | 0.0038 | 6 | 5 | 1 |

Table 12 shows that transgenic plants expressing the SLL1023 gene under control of the PCUbi promoter with targeting to the plastids were significantly larger under cycling drought and well-watered conditions than the control plants that did not express the SLL1023 gene. In these experiments, all the independent transgenic events with plastid targeting were larger than the controls under both cycling drought and well-watered conditions. The transgenic plants expressing the SLL1023 gene under control of the PCUbi promoter with targeting to the plastids were also significantly healthier under well-watered conditions than the control plants. The presence of the SLL1023 protein in the plastids promoted plant growth under both well-watered and drought conditions.

### I. Cobalt-precorrin-6A reductase

The Synechocystis gene SLR0252 (SEQ ID NO:43) was expressed in Arabidopsis under control of the PCUbi promoter (SEQ ID NO:121). Table 13 sets forth biomass and health index data obtained from the Arabidopsis plants transformed with this construct and tested under cycling drought conditions.

**Table 13**

| Assay Type | Gene | Promoter | Targeting | Measurement | % Change | p-Value | No. of Events | Positive Events | Negative Events |
|---|---|---|---|---|---|---|---|---|---|
| CD | SLR0252 | PCUbi | None | Day 20 | 20.20 | 0.0000 | 5 | 5 | 0 |
| CD | SLR0252 | PCUbi | None | Day 27 | 16.30 | 0.0001 | 5 | 5 | 0 |
| CD | SLR0252 | PCUbi | None | Health Index | -4.07 | 0.0076 | 5 | 0 | 5 |

Table 13 shows that transgenic plants expressing the SLR0252 gene under control of the PCUbi promoter were significantly larger under cycling drought conditions than the control plants that did not express the SLR0252 gene. In these experiments, all the independent transgenic events were larger than the controls in the cycling drought environment. As evidenced by the observation that the transgenic plants were larger than the control under cycling drought conditions, the presence of the SLR0252 protein in the cytoplasm enabled the transgenic plant to growth better under water-limited conditions.

### J. Uroporphyrin-III C-methyltransferase

The E. coli gene designated b3803 (SEQ ID NO:45), encoding a uroporphyrin-III C-methyltransferase, was expressed in Arabidopsis using two different constructs: constructs controlled by the USP promoter (SEQ ID NO:124) and constructs controlled by the super promoter (SEQ ID NO:122). Table 14 sets forth biomass data obtained from Arabidopsis plants transformed with these constructs and tested under well-watered conditions.

**Table 14**

| Gene | Promoter | Measurement | Control type | % Change | p-Value | No. of Events | No of Positive Events | No. of Negative Events |
|---|---|---|---|---|---|---|---|---|
| b3803 | USP | Biomass at day 20 | wt | 15.31 | 0.0000 | 6 | 6 | 0 |
| b3803 | Super | Biomass at day 20 | wt | -1.87 | 0.5346 | 6 | 3 | 3 |
| b3803 | Super | Biomass at day 27 | wt | -1.25 | 0.6151 | 6 | 3 | 3 |
| b3803 | USP | Biomass at day 27 | wt | 14.86 | 0.0000 | 6 | 6 | 0 |
| b3803 | Super | Biomass at day 20 | superpool | 8.89 | 0.0139 | 6 | 5 | 1 |
| b3803 | Super | Biomass at day 27 | superpool | 2.22 | 0.4364 | 6 | 3 | 3 |

Table 14 shows that Arabidopsis plants expressing b3803 under control of the USP promoter that were grown under well-watered conditions were significantly larger than the control plants that did not express b3803. Table 14 also shows that the majority of independent transgenic events were larger than the controls.

### K. Isoprenoid biosynthesis protein

The gene designated b3209 (SEQ ID NO:53), endcoding an isoprenoid biosynthesis protein, was expressed in Arabidopsis using a construct controlled by the super promoter (SEQ ID NO:122). Table 15 sets forth biomass data obtained from Arabidopsis plants transformed with these constructs and tested under well-watered conditions.

**Table 15**

| Gene | Promo ter | Measurement | Control type | % Change | p-Value | No. of Events | No of Positive Events | No. of Negative Events |
|---|---|---|---|---|---|---|---|---|
| b3209 | Super | Biomass at day 20 | wt | 17.71 | 0.000 0 | 7 | 7 | 0 |
| b3209 | Super | Biomass at day 27 | wt | 14.47 | 0.000 0 | 7 | 7 | 0 |
| b3209 | Super | Biomass at day 20 | superpool | 29.46 | 0.000 0 | 7 | 7 | 0 |
| b3209 | Super | Biomass at day 27 | superpool | 22.10 | 0.000 0 | 7 | 7 | 0 |

Table 15 shows that Arabidopsis plants expressing b3209 under control of the super promoter that were grown under well-watered conditions were significantly larger than the control plants that did not express b3209. Table 15 also shows that the majority of independent transgenic events were larger than the controls.

### L. LysE type translocator

The E. coli gene designated b2578 (SEQ ID NO:59), encoding a LysE type translocator, was expressed in Arabidopsis using a construct controlled by the super promoter (SEQ ID NO:122). Table 16 sets forth biomass and health index data obtained from Arabidopsis plants transformed with these constructs and tested under well-watered conditions.

**Table 16**

| Gene | Promoter | Measurement | Control type | % Change | p-Value | No. of Events | No of Positive Events | No. of Negative Events |
|---|---|---|---|---|---|---|---|---|
| b2578 | Super | Health index | wt | -6.74 | 0.0221 | 7 | 1 | 6 |
| b2578 | Super | Biomass at day 20 | wt | 7.66 | 0.0352 | 7 | 5 | 2 |
| b2578 | Super | Biomass at day 27 | wt | 8.22 | 0.0061 | 7 | 6 | 1 |
| b2578 | Super | Health index | superpool | 8.17 | 0.0200 | 7 | 6 | 1 |
| b2578 | Super | Biomass at day 20 | superpool | 18.41 | 0.0000 | 7 | 6 | 1 |
| b2578 | Super | Biomass at day 27 | superpool | 15.44 | 0.0000 | 7 | 6 | 1 |

Table 16 shows that Arabidopsis plants expressing b2578 that were grown under well-watered conditions were significantly larger than the control plants that did not express b2578. Table 16 also shows that the majority of independent transgenic events were larger than the controls.

### M. Branched-chain amino acid transporter

The gene designated b2682 (SEQ ID NO:61), encoding a branched-chain amino acid transporter, was expressed in Arabidopsis using a construct controlled by the super promoter (SEQ ID NO:122). Table 17 sets forth biomass and health index data obtained from the Arabidopsis plants transformed with these constructs and tested under well-watered conditions.

**Table 17**

| Gene | Promoter | Measurement | Control type | % Change | p-Value | No. of Events | No of Positive Events | No. of Negative Events |
|---|---|---|---|---|---|---|---|---|
| b2682 | Super | Health index | wt | -0.95 | 0.7523 | 7 | 3 | 4 |
| b2682 | Super | Biomass at day 20 | wt | 16.28 | 0.0000 | 7 | 7 | 0 |
| b2682 | Super | Biomass at day 27 | wt | 7.49 | 0.0073 | 7 | 7 | 0 |
| b2682 | Super | Health index | superpool | 14.89 | 0.0000 | 7 | 6 | 1 |
| b2682 | Super | Biomass at day 20 | superpool | 27.89 | 0.0000 | 7 | 7 | 0 |
| b2682 | Super | Biomass at day 27 | superpool | 14.66 | 0.0000 | 7 | 7 | 0 |

Table 17 shows that Arabidopsis plants expressing b2682 under control of the super promoter that were grown under well-watered conditions were significantly larger than the control plants that did not express b2682. Table 17 also shows that the majority of independent transgenic events were larger than the controls.

### N. DNA-binding protein

The gene designated b3285 (SEQ ID NO:63), encoding a DNA-binding protein, was expressed in Arabidopsis using a construct controlled by the super promoter (SEQ ID NO:122). Table 18 sets forth biomass and health index data obtained from Arabidopsis plants transformed with these constructs and tested under well-watered conditions.

**Table 18**

| Gene | Promoter | Measurement | Control type | % Change | p-Value | No. of Events | No of Positive Events | No. of Negative Events |
|---|---|---|---|---|---|---|---|---|
| b3285 | Super | Health index | wt | -6.46 | 0.0351 | 7 | 2 | 5 |
| b3285 | Super | Biomass at day 20 | wt | 16.84 | 0.0000 | 7 | 6 | 1 |
| b3285 | Super | Biomass at day 27 | wt | 14.14 | 0.0000 | 7 | 7 | 0 |
| b3285 | Super | Health index | superpool | 8.49 | 0.0200 | 7 | 5 | 2 |
| b3285 | Super | Biomass at day 20 | superpool | 28.51 | 0.0000 | 7 | 7 | 0 |
| b3285 | Super | Biomass at day 27 | superpool | 21.75 | 0.0000 | 7 | 7 | 0 |

Table 18 shows that Arabidopsis plants expressing b3285 under control of the super promoter that were grown under well-watered conditions were significantly larger than the control plants that did not express b3285. Table 18 also shows that the majority of independent transgenic events were larger than the controls.

### O. YscJ/FliF protein

The gene designated b1938 (SEQ ID NO:65), encoding a YscJ/FliF protein, was expressed in Arabidopsis using a construct controlled by the super promoter (SEQ ID NO:122). Table 19 sets forth biomass and health index data obtained from the Arabidopsis plants transformed with these constructs and tested under well-watered conditions.

**Table 19**

| Gene | Promoter | Measurement | Control type | % Change | p-Value | No. of Events | No of Positive Events | No. of Negative Events |
|---|---|---|---|---|---|---|---|---|
| b1938 | Super | Biomass at day 20 | wt | 5.64 | 0.1074 | 7 | 5 | 2 |
| b1938 | Super | Biomass at day 27 | wt | 7.41 | 0.0123 | 7 | 5 | 2 |
| b1938 | Super | Biomass at day 20 | superpool | 16.18 | 0.0000 | 7 | 6 | 1 |
| b1938 | Super | Biomass at day 27 | superpool | 14.57 | 0.0000 | 7 | 5 | 2 |

Table 19 shows that Arabidopsis plants expressing b1938 under control of the super promoter that were grown under well-watered conditions were significantly larger than the control plants that did not express b1938. Table 19 also shows that the majority of independent transgenic events were larger than the controls.

### P. Riboflavin Biosynthetic Genes

The Synechocystis gene designated SLL1894 (SEQ ID NO:67) encoding GTP cyclohydrolase II was expressed in Arabidopsis using three different constructs controlled by the parsley ubiquitin promoter (SEQ ID NO: 121): constructs with no subcellular targeting, constructs targeted to the chloroplast, and constructs targeted to mitochondria. Table 20 sets forth biomass and health index data obtained from Arabidopsis plants transformed with SLL1894 under control of the parsley ubiquitin promoter with and without subcellular targeting, and tested under water limited conditions.

**Table 20**

| Gene | Promoter | Targeting | Measurement | % Change | p-Value | No. of Events | No of Positive Events | No. of Negative Events |
|---|---|---|---|---|---|---|---|---|
| Sll1894 | PCUbi | None | Biomass at 20 days | 12.2 | 0.0016 | 7 | 6 | 1 |
| sll1894 | PCUbi | None | Biomass at 27 days | 7.8 | 0.0499 | 7 | 5 | 2 |
| sll1894 | PCUbi | None | Health Index | 9.4 | 0.0092 | 7 | 5 | 2 |
| sll1894 | PCUbi | Mito | Biomass at 20 days | -2.1 | NS | 6 | 3 | 3 |
| sll1894 | PCUbi | Mito | Biomass at 27 days | -21.8 | 0.0002 | 6 | 0 | 6 |
| sll1894 | PCUbi | Mito | Health Index | 2.5 | NS | 6 | 5 | 1 |
| sll1894 | PCUbi | Chlor | Biomass at 20 days | -1.3 | NS | 6 | 3 | 3 |
| sll1894 | PCUbi | Chlor | Biomass at 27 days | -0.9 | NS | 6 | 4 | 2 |
| sll1894 | PCUbi | Chlor | Health Index | 7.1 | 0.0327 | 6 | 5 | 1 |

Transgenic plants expressing the SLL1894 gene with no subcellular targeting were significantly larger under water limited conditions than the control plants that did not express the SLL1894 gene. In addition, the transgenic plants were darker green in color than the controls under water limited conditions as shown by the increased health index. In these experiments, the majority of the independent transgenic events were larger than the controls in the water limited environment.

Transgenic plants expressing the SLL1894 gene with subcellular targeting to the mitochondria were significantly smaller under water limited conditions than the control plants that did not express the SLL1894 gene. Transgenic plants expressing the SLL1894 gene with subcellular targeting to the plastid were similar in size under water limited conditions to the control plants that did not express the SLL1894 gene, but had a larger health index.

The Synechocystis gene designated gene SLL1282 (SEQ ID NO:71) encoding lumazine synthase was expressed in Arabidopsis using a construct controlled by the parsley ubiquitin promoter (SEQ ID NO:121) with no subcellular targeting. Table 21 sets forth biomass and health index data obtained from Arabidopsis plants transformed with this construct and tested under well-watered conditions.

**Table 21**

| Gene | Promoter | Targeting | Measurement | % Change | p-Value | No. of Events | No of Positive Events | No. of Negative Events |
|---|---|---|---|---|---|---|---|---|
| sll12 82 | PCUbi | None | Biomass at 17 days | 2.2 | NS | 6 | 3 | 3 |
| sll12 82 | PCUbi | None | Biomass at 21 days | -0.6 | NS | 6 | 2 | 4 |
| sII12 82 | PCUbi | None | Health Index | -1.5 | NS | 6 | 3 | 3 |

The growth of the Arabidopsis plants expressing the SLL1282 gene controlled by the PCUbi promoter and with no subcellular targeting was similar to control plants under well watered conditions.

Table 22 sets forth biomass and health index data obtained from the Arabidopsis plants transformed with SLL1282 under control of the parsley ubiquitin promoter, with and without subcellular targeting, and tested under water limited conditions.

**Table 22**

| Gene | Promoter | Targeting | Measurement | % Change | p-Value | No. of Events | No of Positive Events | No. of Negative Events |
|---|---|---|---|---|---|---|---|---|
| sll12 82 | PCUbi | None | Biomass at 20 days | 27.5 | 0.0000 | 6 | 6 | 0 |
| sll12 82 | PCUbi | None | Biomass at 27 days | 34.5 | 0.0000 | 6 | 6 | 0 |
| sll12 82 | PCUbi | None | Health Index | 9.5 | 0.0003 | 6 | 6 | 0 |
| sll12 82 | PCUbi | Mito | Biomass at 20 days | 8.7 | 0.0166 | 7 | 4 | 3 |
| sll12 82 | PCUbi | Mito | Biomass at 27 days | 10.7 | 0.0029 | 7 | 5 | 2 |
| sll12 82 | PCUbi | Mito | Health Index | -3.1 | NS | 7 | 3 | 4 |
| sll12 82 | PCUbi | Chlor | Biomass at 20 days | -16.9 | 0.0000 | 6 | 1 | 5 |
| sll12 82 | PCUbi | Chlor | Biomass at 27 days | -4.6 | NS | 6 | 1 | 5 |
| sll12 82 | PCUbi | Chlor | Health Index | -6.8 | 0.0044 | 6 | 1 | 5 |

Arabidopsis plants that were grown under water limited conditions were significantly larger than the control plants that did not express the SLL1282 gene at two measuring times, if the protein did not contain a subcellular targeting sequence. If a mitochondrial targeting sequence was included, the gene was less effective, but did provide some improvement relative to the control. Targeting the protein to the plastid resulted in reduced growth. In these experiments, all independent transgenic events with no subcellular targeting were larger than the controls in the water limited environment.

### Q. Vitamin B6 Biosynthetic Genes

The E. coli gene designated b1636 (SEQ ID NO:79) encoding the pyridoxal kinase PdxY was expressed in Arabidopsis using a construct controlled by the Super promoter (SEQ ID NO:122) targeted to the chloroplast. Table 23 sets forth biomass and health index data obtained from Arabidopsis plants transformed with these constructs and tested under well-watered conditions.

**Table 23**

| Gene | Promoter | Targeting | Measurement | % Change | p-Value | No. of Events | No of Positive Events | No. of Negative Events |
|---|---|---|---|---|---|---|---|---|
| b1636 | super | Chlor | Biomass at 17 days | 10.7 | 0.0001 | 6 | 5 | 1 |
| b1636 | super | Chlor | Biomass at 21 days | 10.0 | 0.0000 | 6 | 5 | 1 |
| b1636 | super | Chlor | Health Index | -10.7 | 0.0009 | 6 | 1 | 5 |

Arabidopsis plants that were grown under well watered conditions were significantly larger than the control plants that did not express the b1636 gene at two measuring times. In these experiments, the majority of the independent transgenic events were larger than the controls in the well watered environment.

Table 24 sets forth biomass and health index data obtained from the Arabidopsis plants transformed with b1636 controlled by the super promoter targeted to the chloroplast and tested under water limited conditions.

**Table 24**

| Gene | Promoter | Targeting | Measurement | % Change | p-Value | No. of Events | No of Positive Events | No. of Negative Events |
|---|---|---|---|---|---|---|---|---|
| b163 6 | super | Chlor | Biomass at 20 days | 8.9 | 0.0243 | 6 | 4 | 2 |
| b163 6 | super | Chlor | Biomass at 27 days | 21.9 | 0.0000 | 6 | 3 | 3 |
| b163 6 | super | Chlor | Health Index | 1.6 | NS | 6 | 4 | 2 |

Arabidopsis plants that were grown under water limited conditions were significantly larger than the control plants that did not express the b1636 gene at two measuring times. In these experiments, three or four of the six independent transgenic events were larger than the controls in the water limited environment.

The Synechocystis gene designated SLR1779 (SEQ ID NO:95) encoding the pyridoxal phosphate biosynthetic protein PdxJ was expressed in Arabidopsis using three different constructs controlled by the parsley ubiquitin promoter (SEQ ID NO:121): constructs with no subcellular targeting, constructs targeted to the chloroplast, and constructs targeted to mitochondria. Table 25 sets forth biomass and health index data obtained from the Arabidopsis plants transformed with these constructs and tested under well-watered conditions.

**Table 25**

| Gene | Promoter | Targeting | Measurement | % Change | p-Value | No. of Events | No of Positive Events | No. of Negative Events |
|---|---|---|---|---|---|---|---|---|
| slr1779 | PCUbi | None | Biomass at 17 days | 23.8 | 0.0000 | 8 | 8 | 0 |
| slr1779 | PCUbi | None | Biomass at 21 days | 20.6 | 0.0000 | 8 | 8 | 0 |
| slr1779 | PCUbi | None | Health Index | -1.1 | NS | 8 | 4 | 4 |
| slr1779 | PCUbi | Mito | Biomass at 17 days | 18.8 | 0.0000 | 6 | 6 | 0 |
| slr1779 | PCUbi | Mito | Biomass at 21 days | 7.6 | 0.0008 | 6 | 6 | 0 |
| slr1779 | PCUbi | Mito | Health Index | 8.7 | 0.0104 | 6 | 6 | 0 |
| slr1779 | PCUbi | Chlor | Biomass at 17 days | 27.6 | 0.0000 | 6 | 6 | 0 |
| slr1779 | PCUbi | Chlor | Biomass at 21 days | 15.1 | 0.0000 | 6 | 6 | 0 |
| slr1779 | PCUbi | Chlor | Health Index | -1.0 | NS | 6 | 3 | 3 |

Table 26 sets forth biomass and health index data of the Arabidopsis plants transformed with the SLR1779 gene controlled by the parsley ubiquitin promoter with subcellular targeting and tested under water limited conditions.

**Table 26**

| Gene | Promoter | Targeting | Measurement | % Change | p-Value | No. of Events | No of Positive Events | No. of Negative Events |
|---|---|---|---|---|---|---|---|---|
| slr17 79 | PCUbi | Mito | Biomass at 20 days | -6.5 | 0.005 8 | 6 | 4 | 2 |
| slr17 79 | PCUbi | Mito | Biomass at 27 days | -4.7 | 0.030 2 | 6 | 3 | 3 |
| slr17 79 | PCUbi | Mito | Health Index | -0.4 | NS | 6 | 3 | 3 |
| slr17 79 | PCUbi | Chlor | Biomass at 20 days | 16.6 | 0.000 0 | 6 | 5 | 1 |
| slr17 | PCUbi | Chlor | Biomass at | 8.5 | 0.000 | 6 | 4 | 2 |
| 79 | | | 27 days | | 2 | | | |
| slr1779 | PCUbi | Chlor | Health Index | 13.2 | 0.0000 | 6 | 5 | 1 |

Transgenic plants expressing the SLR1779 gene were larger under well watered conditions than the control plants that did not express the SLR1779 gene. This effect was observed with all three constructs that had no subcellular targeting or with subcellular targeting either to the mitochondria or to the plastid.

Transgenic plants expressing the SLR1779 gene with subcellular targeting to the plastid were significantly larger under water limited conditions than the control plants that did not express the SLR1779 gene. In addition, the transgenic plants were darker green in color than the controls under water limited conditions as shown by the increased health index. In these experiments, the majority of the independent transgenic events were larger than the controls in the water limited environment.

Transgenic plants expressing the SLR1779 gene with subcellular targeting to the mitochondria were significantly smaller under water limited conditions than the control plants that did not express the SLR1779 gene.

## Claims

1. A method of increasing a plant' s tolerance to an environmental stress, and/or growth, and/or yield wherein the method comprises the steps of
i. transforming a plant cell with an expression cassette comprising
a) an isolated polynucleotide encoding a promoter capable of enhancing expression in leaves;
b) an isolated polynucleotide encoding a mitochondrial transit peptide; and
c) an isolated polynucleotide encoding a full-length bifunctional anaerobic fatty acid oxidation complex polypeptide, wherein the bifunctional anaerobic fatty acid oxidation complex polypeptide comprises a) an ECH domain selected from the group consisting of amino acids 17 to 190 of SEQ ID NO:22; amino acids 17 to 187 SEQ ID NO:24 and amino acids 18 to 187 SEQ ID NO:26; b) a 3HCDH domain selected from the group consisting of amino acids 489 to 20 513 of SEQ ID NO:22, amino acids 490 to 514 of SEQ ID NO:24, amino acids 490 to 514 of SEQ ID NO:26; and c) a 3HCDH -N domain selected from the group consisting of amino acids 310 to 490 of SEQ ID NO:22; amino acids 312 to 491 of SEQ ID NO:24 and amino acids 312 to 491 SEQ ID NO:26;
or
d) an isolated polynucleotide encoding a promoter capable of enhancing expression in leaves;
e) an isolated polynucleotide encoding a mitochondrial transit peptide; and
f) an isolated polynucleotide encoding a full-length an acyltransferase polypeptide, wherein the acyltransferase polypeptide comprises an acyltransferase domain selected from the group consisting of amino 20 acids 108 to 272 of SEQ ID NO:16, amino acids 80 to 222 of SEQ ID NO:18, or amino acids 116 to 258 of SEQ ID NO:20;
ii. and generating a transgenic plant from the plant cell, wherein the transgenic plant comprises the polynucleotide of c) or f).

2. A method of producing a transgenic plant having enhanced yield as compared to a wild type plant of the same variety, the method comprising the steps of:
a) transforming a plant cell with an expression vector comprising, in operative association,
i) an isolated polynucleotide encoding a promoter capable of enhancing expression in leaves;
ii) an isolated polynucleotide encoding a mitochondrial transit peptide; and
iii) an isolated polynucleotide encoding a full-length bifunctional anaerobic fatty acid oxidation complex polypeptide, wherein the bifunctional anaerobic fatty acid oxidation complex polypeptide comprises a) an ECH domain selected from the group consisting of amino acids 17 to 190 of SEQ ID NO:22; amino acids 17 to 187 SEQ ID NO:24 and amino acids 18 to 187 SEQ ID NO:26; b) a 3HCDH domain selected from the group consisting of amino acids 489 to 20 513 of SEQ ID NO:22, amino acids 490 to 514 of SEQ ID NO:24, amino acids 490 to 514 of SEQ ID NO:26; and c) a 3HCDH -N domain selected from the group consisting of amino acids 310 to 490 of SEQ ID NO:22; amino acids 312 to 491 of SEQ ID NO:24 and amino acids 312 to 491 SEQ ID NO:26;;
or
iv) an isolated polynucleotide encoding a promoter capable of enhancing expression in leaves;
v) an isolated polynucleotide encoding a mitochondrial transit peptide; and
vi) an isolated polynucleotide encoding a full-length an acyltransferase polypeptide, wherein the acyltransferase polypeptide comprises an acyltransferase domain selected from the group consisting of amino 20 acids 108 to 272 of SEQ ID NO:16, amino acids 80 to 222 of SEQ ID NO:18, or amino acids 116 to 258 of SEQ ID NO:20;
b) regenerating transgenic plants from the transformed plant cell; and
c) selecting higher-yielding plants from the regenerated transgenic plants.

3. A transgenic plant transformed with an expression cassette comprising, in operative association,
a) an isolated polynucleotide encoding a promoter capable of enhancing expression in leaves;
b) an isolated polynucleotide encoding a mitochondrial transit peptide; and
c) an isolated polynucleotide encoding a full-length bifunctional anaerobic fatty acid oxidation complex polypeptide, wherein the bifunctional anaerobic fatty acid oxidation complex polypeptide comprises a) an ECH domain selected from the group consisting of amino acids 17 to 190 of SEQ ID NO:22; amino acids 17 to 187 SEQ ID NO:24 and amino acids 18 to 187 SEQ ID NO:26; b) a 3HCDH domain selected from the group consisting of amino acids 489 to 20 513 of SEQ ID NO:22, amino acids 490 to 514 of SEQ ID NO:24, amino acids 490 to 514 of SEQ ID NO:26; and c) a 3HCDH -N domain selected from the group consisting of amino acids 310 to 490 of SEQ ID NO:22; amino acids 312 to 491 of SEQ ID NO:24 and amino acids 312 to 491 SEQ ID NO:26;;
or
d) an isolated polynucleotide encoding a promoter capable of enhancing expression in leaves;
e) an isolated polynucleotide encoding a mitochondrial transit peptide; and
f) an isolated polynucleotide encoding a full-length an acyltransferase polypeptide, wherein the acyltransferase polypeptide comprises an acyltransferase domain selected from the group consisting of amino 20 acids 108 to 272 of SEQ ID NO:16, amino acids 80 to 222 of SEQ ID NO:18, or amino acids 116 to 258 of SEQ ID NO:20;
wherein the transgenic plant demonstrates increased yield as compared to a wild type plant of the same variety which does not comprise the expression cassette.

4. A seed which is true breeding for a transgene comprising, in operative association,
a) an isolated polynucleotide encoding a promoter capable of enhancing expression in leaves;
b) an isolated polynucleotide encoding a mitochondrial transit peptide; and
c) an isolated polynucleotide encoding a full-length bifunctional anaerobic fatty acid oxidation complex polypeptide, wherein the bifunctional anaerobic fatty acid oxidation complex polypeptide comprises a) an ECH domain selected from the group consisting of amino acids 17 to 190 of SEQ ID NO:22; amino acids 17 to 187 SEQ ID NO:24 and amino acids 18 to 187 SEQ ID NO:26; b) a 3HCDH domain selected from the group consisting of amino acids 489 to 20 513 of SEQ ID NO:22, amino acids 490 to 514 of SEQ ID NO:24, amino acids 490 to 514 of SEQ ID NO:26; and c) a 3HCDH -N domain selected from the group consisting of amino acids 310 to 490 of SEQ ID NO:22; amino acids 312 to 491 of SEQ ID NO:24 and amino acids 312 to 491 SEQ ID NO:26;
or
d) an isolated polynucleotide encoding a promoter capable of enhancing expression in leaves;
e) an isolated polynucleotide encoding a mitochondrial transit peptide; and
f) an isolated polynucleotide encoding a full-length an acyltransferase polypeptide, wherein the acyltransferase polypeptide comprises an acyltransferase domain selected from the group consisting of amino 20 acids 108 to 272 of SEQ ID NO:16, amino acids 80 to 222 of SEQ ID NO:18, or amino acids 116 to 258 of SEQ ID NO:20;
wherein a transgenic plant grown from said seed demonstrates increased yield as compared to a wild type plant of the same variety which does not comprise the expression cassette.

5. The method of claim 1 or 2 or the transgenic plant of claim 3 or the seed of claim 4 or,
wherein
i. the bifunctional anaerobic fatty acid oxidation complex polypeptide comprises amino acids 1 to 714 of SEQ ID NO:22, amino acids 1 to 723 of SEQ ID NO:24, or amino acids 1 to 727 of SEQ ID NO:26;
or
ii. the acyltransferase polypeptide comprises amino acids 1 to 396 of SEQ ID NO:16, amino acids 1 to 384 of SEQ ID NO:18, or amino acids 1 to 332 of SEQ ID NO:20.

6. The method of claim 1 or 2 or 5, or the transgenic plant of claim 3 or 5, or the seed of claim 4 or 5, wherein the plant is a dicotyledonous plant or a monocotyledonous plant.

7. The method of claim 1 or 2 or 5, or the transgenic plant of claim 3 or 5, or the seed of claim 4 or 5, further defined as a species selected from the group consisting of Arabidopsis thaliana, Nicotiana tabacum, rice, oilseed rape, canola, soybean, corn (maize), cotton and wheat.

8. A plant expression cassette comprising a polynucleotide encoding a full-length polypeptide of the sequence of SEQ ID NO: 16, 18, 20, 24, or 26 or homologs thereof.

9. A recombinant expression vector comprising the expression cassette of claim 8.

10. A plant cell comprising the recombinant vector of claim 9 or the expression cassette of claim 8.

11. A bacterial host cell comprising the vector of claim 9.

12. Use of a polynucleotide encoding a full-length polypeptide as defined in claim 1, 5 or 6, or an expression cassette of claim 8 or an expression vector of claim 9, or a host cell transformed with said vector comprising said expression cassette or a host cell comprising said polynucleotide to increase yield of a plant as compared to a wild type plant of the same variety or for the production of a plant with increased yield as compared to a wild type plant of the same variety.


SEQUENCE LISTING

<110>  McKersie, Bryan
       Bruce, Wesley

<120>  Transgenic Plants With Increased Yield

<130>  61142

<150>  USSN 61/090,308
<151>  2008-08-20

<150>  USSN 61/090,625
<151>  2008-08-21

<150>  USSN 61/090,669
<151>  2008-08-21

<160>  130

<170>  PatentIn version 3.4

<210>  1
<211>  1374
<212>  DNA
<213>  Saccharomyces cerevisiae


<220>
<221>  CDS
<222>  (1)..(1374)
<223>  phosphatidate cytidylyltransferase (YBR029C)

<400>  1
atg tct gac aac cct gag atg aaa cca cat ggt acg agc aag gag att        48
Met Ser Asp Asn Pro Glu Met Lys Pro His Gly Thr Ser Lys Glu Ile
1               5                   10                  15

gtg gag tcg gtt act gac gcc acc tca aag gcg att gat aaa ttg caa        96
Val Glu Ser Val Thr Asp Ala Thr Ser Lys Ala Ile Asp Lys Leu Gln
            20                  25                  30

gaa gaa ctc cac aag gac gcc agc gaa tcc gtc acg ccg gtg acc aag       144
Glu Glu Leu His Lys Asp Ala Ser Glu Ser Val Thr Pro Val Thr Lys
        35                  40                  45

gaa agc act gct gct aca aag gaa agc agg aaa tac aac ttt ttc att       192
Glu Ser Thr Ala Ala Thr Lys Glu Ser Arg Lys Tyr Asn Phe Phe Ile
    50                  55                  60

aga aca gtt tgg acg ttt gtt atg atc agt ggt ttc ttc atc acc tta       240
Arg Thr Val Trp Thr Phe Val Met Ile Ser Gly Phe Phe Ile Thr Leu
65                  70                  75                  80

gca tcg ggt cat gca tgg tgt ata gtg ctg att ttg ggc tgc caa att       288
Ala Ser Gly His Ala Trp Cys Ile Val Leu Ile Leu Gly Cys Gln Ile
                85                  90                  95

gct act ttt aaa gag tgt att gcc gta aca agt gca tct ggt cgc gaa       336
Ala Thr Phe Lys Glu Cys Ile Ala Val Thr Ser Ala Ser Gly Arg Glu
            100                 105                 110

aag aat ttg cca ttg aca aag acg ttg aac tgg tac ctt ctc ttc acc       384
Lys Asn Leu Pro Leu Thr Lys Thr Leu Asn Trp Tyr Leu Leu Phe Thr
        115                 120                 125

act atc tat tac tta gat ggg aaa tca ctc ttc aag ttt ttc caa gct       432
Thr Ile Tyr Tyr Leu Asp Gly Lys Ser Leu Phe Lys Phe Phe Gln Ala
    130                 135                 140

act ttt tac gag tat cct gta ttg aac ttt atc gta aca aat cat aag 480
Thr Phe Tyr Glu Tyr Pro Val Leu Asn Phe Ile Val Thr Asn His Lys
145                 150                 155                 160

ttt atc tgc tat tgt ctc tat ctt atg gga ttt gtt ctg ttc gtt tgt 528
Phe Ile Cys Tyr Cys Leu Tyr Leu Met Gly Phe Val Leu Phe Val Cys
                165                 170                 175

agt tta aga aag gga ttt ttg aaa ttc cag ttc gga tca ttg tgc gtt 576
Ser Leu Arg Lys Gly Phe Leu Lys Phe Gln Phe Gly Ser Leu Cys Val
            180                 185                 190

act cat atg gtt ctt ctt ctg gta gta ttt cag gct cat ttg atc atc 624
Thr His Met Val Leu Leu Leu Val Val Phe Gln Ala His Leu Ile Ile
        195                 200                 205

aaa aac gtg ctc aat ggg cta ttc tgg ttc tta tta cca tgc ggg ttg 672
Lys Asn Val Leu Asn Gly Leu Phe Trp Phe Leu Leu Pro Cys Gly Leu
    210                 215                 220

gta att gtt aat gat atc ttc gcc tac ctg tgc ggc att aca ttc ggt 720
Val Ile Val Asn Asp Ile Phe Ala Tyr Leu Cys Gly Ile Thr Phe Gly
225                 230                 235                 240

aag act aaa cta ata gaa atc tcg ccc aag aaa act tta gaa ggt ttc 768
Lys Thr Lys Leu Ile Glu Ile Ser Pro Lys Lys Thr Leu Glu Gly Phe
                245                 250                 255

ctt ggt gcc tgg ttt ttc act gct cta gct agt att ata tta aca agg 816
Leu Gly Ala Trp Phe Phe Thr Ala Leu Ala Ser Ile Ile Leu Thr Arg
            260                 265                 270

att ctg agc cct tat act tac ttg acg tgc cct gtt gaa gat ctc cat 864
Ile Leu Ser Pro Tyr Thr Tyr Leu Thr Cys Pro Val Glu Asp Leu His
        275                 280                 285

acc aat ttt ttc tct aat ttg aca tgt gaa cta aac cca gtt ttc ctt 912
Thr Asn Phe Phe Ser Asn Leu Thr Cys Glu Leu Asn Pro Val Phe Leu
    290                 295                 300

cca caa gtt tac aga ctt cca ccc atc ttt ttc gat aaa gtt caa atc 960
Pro Gln Val Tyr Arg Leu Pro Pro Ile Phe Phe Asp Lys Val Gln Ile
305                 310                 315                 320

aat tca att aca gta aaa cca att tat ttc cat gct tta aac tta gct 1008
Asn Ser Ile Thr Val Lys Pro Ile Tyr Phe His Ala Leu Asn Leu Ala
                325                 330                 335

acc ttt gca tca tta ttt gcg cca ttt gga ggc ttt ttc gca tct ggt 1056
Thr Phe Ala Ser Leu Phe Ala Pro Phe Gly Gly Phe Phe Ala Ser Gly
            340                 345                 350

cta aag aga act ttt aaa gtt aaa gac ttt ggc cac tcc atc cca ggc 1104
Leu Lys Arg Thr Phe Lys Val Lys Asp Phe Gly His Ser Ile Pro Gly
        355                 360                 365

cat ggt ggt atc aca gac agg gtt gat tgc caa ttc ata atg ggt tct 1152
His Gly Gly Ile Thr Asp Arg Val Asp Cys Gln Phe Ile Met Gly Ser
    370                 375                 380

ttt gcc aat ttg tac tat gaa aca ttc atc agc gaa cac aga ata aca 1200
Phe Ala Asn Leu Tyr Tyr Glu Thr Phe Ile Ser Glu His Arg Ile Thr
385                 390                 395                 400

gta gat aca gtt tta tcc act att tta atg aac ctg aac gac aag cag 1248
Val Asp Thr Val Leu Ser Thr Ile Leu Met Asn Leu Asn Asp Lys Gln
                405                 410                 415

att ata gaa ttg att gat atc ttg att aga ttc cta tct aaa aaa ggt      1296
Ile Ile Glu Leu Ile Asp Ile Leu Ile Arg Phe Leu Ser Lys Lys Gly
            420                 425                 430

ata ata tca gcg aag aat ttt gaa aag ttg gct gac atc ttt aat gtg      1344
Ile Ile Ser Ala Lys Asn Phe Glu Lys Leu Ala Asp Ile Phe Asn Val
        435                 440                 445

acc aag aaa tca ttg acc aat cac tct tga                              1374
Thr Lys Lys Ser Leu Thr Asn His Ser
    450                 455

<210>  2
<211>  457
<212>  PRT
<213>  Saccharomyces cerevisiae

<400>  2

Met Ser Asp Asn Pro Glu Met Lys Pro His Gly Thr Ser Lys Glu Ile
1               5                   10                  15

Val Glu Ser Val Thr Asp Ala Thr Ser Lys Ala Ile Asp Lys Leu Gln
            20                  25                  30

Glu Glu Leu His Lys Asp Ala Ser Glu Ser Val Thr Pro Val Thr Lys
        35                  40                  45

Glu Ser Thr Ala Ala Thr Lys Glu Ser Arg Lys Tyr Asn Phe Phe Ile
    50                  55                  60

Arg Thr Val Trp Thr Phe Val Met Ile Ser Gly Phe Phe Ile Thr Leu
65                  70                  75                  80

Ala Ser Gly His Ala Trp Cys Ile Val Leu Ile Leu Gly Cys Gln Ile
                85                  90                  95

Ala Thr Phe Lys Glu Cys Ile Ala Val Thr Ser Ala Ser Gly Arg Glu
            100                 105                 110

Lys Asn Leu Pro Leu Thr Lys Thr Leu Asn Trp Tyr Leu Leu Phe Thr
        115                 120                 125

Thr Ile Tyr Tyr Leu Asp Gly Lys Ser Leu Phe Lys Phe Phe Gln Ala
    130                 135                 140

Thr Phe Tyr Glu Tyr Pro Val Leu Asn Phe Ile Val Thr Asn His Lys
145                 150                 155                 160

Phe Ile Cys Tyr Cys Leu Tyr Leu Met Gly Phe Val Leu Phe Val Cys
                165                 170                 175

Ser Leu Arg Lys Gly Phe Leu Lys Phe Gln Phe Gly Ser Leu Cys Val
            180                 185                 190

Thr His Met Val Leu Leu Leu Val Val Phe Gln Ala His Leu Ile Ile

195                 200                 205

Lys Asn Val Leu Asn Gly Leu Phe Trp Phe Leu Leu Pro Cys Gly Leu
    210                 215                 220

Val Ile Val Asn Asp Ile Phe Ala Tyr Leu Cys Gly Ile Thr Phe Gly
225                 230                 235                 240

Lys Thr Lys Leu Ile Glu Ile Ser Pro Lys Lys Thr Leu Glu Gly Phe
                245                 250                 255

Leu Gly Ala Trp Phe Phe Thr Ala Leu Ala Ser Ile Ile Leu Thr Arg
            260                 265                 270

Ile Leu Ser Pro Tyr Thr Tyr Leu Thr Cys Pro Val Glu Asp Leu His
        275                 280                 285

Thr Asn Phe Phe Ser Asn Leu Thr Cys Glu Leu Asn Pro Val Phe Leu
    290                 295                 300

Pro Gln Val Tyr Arg Leu Pro Pro Ile Phe Phe Asp Lys Val Gln Ile
305                 310                 315                 320

Asn Ser Ile Thr Val Lys Pro Ile Tyr Phe His Ala Leu Asn Leu Ala
                325                 330                 335

Thr Phe Ala Ser Leu Phe Ala Pro Phe Gly Gly Phe Phe Ala Ser Gly
            340                 345                 350

Leu Lys Arg Thr Phe Lys Val Lys Asp Phe Gly His Ser Ile Pro Gly
        355                 360                 365

His Gly Gly Ile Thr Asp Arg Val Asp Cys Gln Phe Ile Met Gly Ser
    370                 375                 380

Phe Ala Asn Leu Tyr Tyr Glu Thr Phe Ile Ser Glu His Arg Ile Thr
385                 390                 395                 400

Val Asp Thr Val Leu Ser Thr Ile Leu Met Asn Leu Asn Asp Lys Gln
                405                 410                 415

Ile Ile Glu Leu Ile Asp Ile Leu Ile Arg Phe Leu Ser Lys Lys Gly
            420                 425                 430

Ile Ile Ser Ala Lys Asn Phe Glu Lys Leu Ala Asp Ile Phe Asn Val
        435                 440                 445

Thr Lys Lys Ser Leu Thr Asn His Ser
    450                 455

<210>  3
<211>  1104

<212>  DNA
<213>  Brassica napus

<220>
<221>  CDS
<222>  (1)..(1104)
<223>  phosphatidate cytidylyltransferase (BN04MC30805)

<400>  3
atg gag gag gag agc aac gtg acc agc acc agc ccg agc acc ccg gtg      48
Met Glu Glu Glu Ser Asn Val Thr Ser Thr Ser Pro Ser Thr Pro Val
1               5                   10                  15

cag agg ctg agg cac agg aag agg agc agc acc gag gtg ctg gac ggc      96
Gln Arg Leu Arg His Arg Lys Arg Ser Ser Thr Glu Val Leu Asp Gly
            20                  25                  30

gac aag gtg aac gcc agc ccg ctg ctg gtg aac gac agg aac aag tac      144
Asp Lys Val Asn Ala Ser Pro Leu Leu Val Asn Asp Arg Asn Lys Tyr
        35                  40                  45

aag agc ttc atg gtg agg acc tac agc acc ctg tgg atg atc gcc ggc      192
Lys Ser Phe Met Val Arg Thr Tyr Ser Thr Leu Trp Met Ile Ala Gly
    50                  55                  60

ttc gtg atg gtg gtg tac atg ggc cac ctg tac atc acc gcc atg gtg      240
Phe Val Met Val Val Tyr Met Gly His Leu Tyr Ile Thr Ala Met Val
65                  70                  75                  80

ctg gtg atc cag atc ttc atg gcc aag gag ctg ttc aac ctg ctg agg      288
Leu Val Ile Gln Ile Phe Met Ala Lys Glu Leu Phe Asn Leu Leu Arg
                85                  90                  95

aag gcc ccg gag gac aag tgc ctg ccg ggc atc aag cag ctg aac tgg      336
Lys Ala Pro Glu Asp Lys Cys Leu Pro Gly Ile Lys Gln Leu Asn Trp
            100                 105                 110

cac ttc ttc ttc acc gcc atg ctg ttc gtg tac ggc agg atc ctg agc      384
His Phe Phe Phe Thr Ala Met Leu Phe Val Tyr Gly Arg Ile Leu Ser
        115                 120                 125

cag agg ctg gcc aac acc gtg acc gcc gac cag ttc ctg tac agg ctg      432
Gln Arg Leu Ala Asn Thr Val Thr Ala Asp Gln Phe Leu Tyr Arg Leu
    130                 135                 140

gtg agc ggc ctg atc aag tac cac atg gcc atc tgc tac ttc ctg aac      480
Val Ser Gly Leu Ile Lys Tyr His Met Ala Ile Cys Tyr Phe Leu Asn
145                 150                 155                 160

atc atc ggc ttc atg tgg ttc atc ctg acc ctg aag aag aag atg tac      528
Ile Ile Gly Phe Met Trp Phe Ile Leu Thr Leu Lys Lys Lys Met Tyr
                165                 170                 175

aag tac cag ttc ggc cag tac gcc tgg acc cac atg atc ctg atc gtg      576
Lys Tyr Gln Phe Gly Gln Tyr Ala Trp Thr His Met Ile Leu Ile Val
            180                 185                 190

gtg ttc acc cag agc agc ttc acc gtg gcc aac atc ttc gag ggc atc      624
Val Phe Thr Gln Ser Ser Phe Thr Val Ala Asn Ile Phe Glu Gly Ile
        195                 200                 205

ttc tgg ttc ctg ctg ccg gcc agc ctg atc atc atc aac gac atc ttc      672
Phe Trp Phe Leu Leu Pro Ala Ser Leu Ile Ile Ile Asn Asp Ile Phe
    210                 215                 220

gcc tac atc ttc ggc ttc ttc ttc ggc agg acc ccg ctg atc aag ctg      720
Ala Tyr Ile Phe Gly Phe Phe Phe Gly Arg Thr Pro Leu Ile Lys Leu
225                 230                 235                 240

agc ccg aag aag acc tgg gag ggc ttc atc ggc gcc agc gtg acc acc      768
Ser Pro Lys Lys Thr Trp Glu Gly Phe Ile Gly Ala Ser Val Thr Thr
                245                 250                 255

atc atc agc gcc ttc gtg ctg gcc aac gtg ctg ggc agg ttc ccg tgg      816
Ile Ile Ser Ala Phe Val Leu Ala Asn Val Leu Gly Arg Phe Pro Trp
            260                 265                 270

ctg acc tgc ccg agg cag gac ctg agc acc ggc tgg ctg cag tgc gac      864
Leu Thr Cys Pro Arg Gln Asp Leu Ser Thr Gly Trp Leu Gln Cys Asp
        275                 280                 285

gcc gac ccg ctg ttc aag ccg gag ccg ttc acc ctg ccg gcc tgg atc      912
Ala Asp Pro Leu Phe Lys Pro Glu Pro Phe Thr Leu Pro Ala Trp Ile
    290                 295                 300

ccg ggc tgg ttc ccg tgg aag gag atg gag gtg ctg ccg gtg cag tgg      960
Pro Gly Trp Phe Pro Trp Lys Glu Met Glu Val Leu Pro Val Gln Trp
305                 310                 315                 320

cac gcc ctg tgc ctg ggc ctg ttc gcc agc atc atc gcc ccg ttc ggc     1008
His Ala Leu Cys Leu Gly Leu Phe Ala Ser Ile Ile Ala Pro Phe Gly
                325                 330                 335

ggc ttc ttc gcc agc ggc ttc aag agg gcc ttc aag atc aag gac ttc     1056
Gly Phe Phe Ala Ser Gly Phe Lys Arg Ala Phe Lys Ile Lys Asp Phe
            340                 345                 350

ggc gac agc atc ccg ggc cac ggc ggc atc acc gac agg atg gac tga     1104
Gly Asp Ser Ile Pro Gly His Gly Gly Ile Thr Asp Arg Met Asp
        355                 360                 365


<210>  4
<211>  367
<212>  PRT
<213>  Brassica napus

<400>  4

Met Glu Glu Glu Ser Asn Val Thr Ser Thr Ser Pro Ser Thr Pro Val
1               5                   10                  15


Gln Arg Leu Arg His Arg Lys Arg Ser Ser Thr Glu Val Leu Asp Gly
            20                  25                  30


Asp Lys Val Asn Ala Ser Pro Leu Leu Val Asn Asp Arg Asn Lys Tyr
        35                  40                  45


Lys Ser Phe Met Val Arg Thr Tyr Ser Thr Leu Trp Met Ile Ala Gly
    50                  55                  60


Phe Val Met Val Val Tyr Met Gly His Leu Tyr Ile Thr Ala Met Val
65                  70                  75                  80


Leu Val Ile Gln Ile Phe Met Ala Lys Glu Leu Phe Asn Leu Leu Arg
                85                  90                  95


Lys Ala Pro Glu Asp Lys Cys Leu Pro Gly Ile Lys Gln Leu Asn Trp
            100                 105                 110

His Phe Phe Phe Thr Ala Met Leu Phe Val Tyr Gly Arg Ile Leu Ser
        115                 120                 125

Gln Arg Leu Ala Asn Thr Val Thr Ala Asp Gln Phe Leu Tyr Arg Leu
    130                 135                 140

Val Ser Gly Leu Ile Lys Tyr His Met Ala Ile Cys Tyr Phe Leu Asn
145                 150                 155                 160

Ile Ile Gly Phe Met Trp Phe Ile Leu Thr Leu Lys Lys Lys Met Tyr
                165                 170                 175

Lys Tyr Gln Phe Gly Gln Tyr Ala Trp Thr His Met Ile Leu Ile Val
            180                 185                 190

Val Phe Thr Gln Ser Ser Phe Thr Val Ala Asn Ile Phe Glu Gly Ile
        195                 200                 205

Phe Trp Phe Leu Leu Pro Ala Ser Leu Ile Ile Ile Asn Asp Ile Phe
    210                 215                 220

Ala Tyr Ile Phe Gly Phe Phe Phe Gly Arg Thr Pro Leu Ile Lys Leu
225                 230                 235                 240

Ser Pro Lys Lys Thr Trp Glu Gly Phe Ile Gly Ala Ser Val Thr Thr
                245                 250                 255

Ile Ile Ser Ala Phe Val Leu Ala Asn Val Leu Gly Arg Phe Pro Trp
            260                 265                 270

Leu Thr Cys Pro Arg Gln Asp Leu Ser Thr Gly Trp Leu Gln Cys Asp
        275                 280                 285

Ala Asp Pro Leu Phe Lys Pro Glu Pro Phe Thr Leu Pro Ala Trp Ile
    290                 295                 300

Pro Gly Trp Phe Pro Trp Lys Glu Met Glu Val Leu Pro Val Gln Trp
305                 310                 315                 320

His Ala Leu Cys Leu Gly Leu Phe Ala Ser Ile Ile Ala Pro Phe Gly
                325                 330                 335

Gly Phe Phe Ala Ser Gly Phe Lys Arg Ala Phe Lys Ile Lys Asp Phe
            340                 345                 350

Gly Asp Ser Ile Pro Gly His Gly Gly Ile Thr Asp Arg Met Asp
        355                 360                 365

<210>  5
<211>  1278
<212>  DNA
<213>  Zea mays

<220>
<221>  CDS
<222>  (1)..(1278)
<223>  phosphatidate cytidylyltransferase (ZM06MC30283)

<400>  5
atg cag agg gac acc agc agc agc gac gtg agc gcc agc cac gtg ggc      48
Met Gln Arg Asp Thr Ser Ser Ser Asp Val Ser Ala Ser His Val Gly
1               5                   10                  15

agg gtg agg agg agg aag cac ccg agc gag gcc agc acc gac ggc aac      96
Arg Val Arg Arg Arg Lys His Pro Ser Glu Ala Ser Thr Asp Gly Asn
            20                  25                  30

agg gcc aac ggc cag ccg ctg ctg gtg aac gac cag aac aag tac aag      144
Arg Ala Asn Gly Gln Pro Leu Leu Val Asn Asp Gln Asn Lys Tyr Lys
        35                  40                  45

agc atg ctg atc agg acc tac agc acc gtg tgg atg atc ggc ggc ttc      192
Ser Met Leu Ile Arg Thr Tyr Ser Thr Val Trp Met Ile Gly Gly Phe
    50                  55                  60

gcc ttc atc atc tac gtg ggc cac ctg tac atc tgg gcc atg gtg gtg      240
Ala Phe Ile Ile Tyr Val Gly His Leu Tyr Ile Trp Ala Met Val Val
65                  70                  75                  80

gtg atc cag atc tac atg gcc agg gag ctg ttc aac ctg ctg agg aag      288
Val Ile Gln Ile Tyr Met Ala Arg Glu Leu Phe Asn Leu Leu Arg Lys
                85                  90                  95

agc agc gag gag aag cag ctg ccg ggc ttc agg ctg ctg aac tgg cac      336
Ser Ser Glu Glu Lys Gln Leu Pro Gly Phe Arg Leu Leu Asn Trp His
            100                 105                 110

ttc ttc ttc acc gcc atg ctg tac acc tac ggc agg ttc ctg agc agg      384
Phe Phe Phe Thr Ala Met Leu Tyr Thr Tyr Gly Arg Phe Leu Ser Arg
        115                 120                 125

cag ctg gtg aac acc gtg acc agc gac cac ctg ctg tac aag gtg gtg      432
Gln Leu Val Asn Thr Val Thr Ser Asp His Leu Leu Tyr Lys Val Val
    130                 135                 140

agc ggc ctg atc aag tac cag atg ttc atc tgc tac ttc ctg tac atc      480
Ser Gly Leu Ile Lys Tyr Gln Met Phe Ile Cys Tyr Phe Leu Tyr Ile
145                 150                 155                 160

gcc ggc ttc gtg tgg ttc atc ctg acc ctg aag aag aag acc tac aag      528
Ala Gly Phe Val Trp Phe Ile Leu Thr Leu Lys Lys Lys Thr Tyr Lys
                165                 170                 175

tac cag ttc aag cag tac gcc tgg acc cac atg atc ctg ctg acc gtg      576
Tyr Gln Phe Lys Gln Tyr Ala Trp Thr His Met Ile Leu Leu Thr Val
            180                 185                 190

ttc gcc cag agc gcc ttc acc gtg gcc aac atc ttc gag ggc atc ttc      624
Phe Ala Gln Ser Ala Phe Thr Val Ala Asn Ile Phe Glu Gly Ile Phe
        195                 200                 205

tgg ttc ctg ctg ccg gcc agc ctg atc gtg atc aac gac atc ttc gcc      672
Trp Phe Leu Leu Pro Ala Ser Leu Ile Val Ile Asn Asp Ile Phe Ala
    210                 215                 220

tac ctg ttc ggc ttc ttc ctg ggc agg acc ccg ctg atc aag ctg agc      720
Tyr Leu Phe Gly Phe Phe Leu Gly Arg Thr Pro Leu Ile Lys Leu Ser
225                 230                 235                 240

ccg aag aag acc tgg gag ggc ttc atc ggc gcc agc gtg acc acc atc      768
Pro Lys Lys Thr Trp Glu Gly Phe Ile Gly Ala Ser Val Thr Thr Ile

245                 250                 255

atc agc gcc ttc ctg ctg gcc aac gtg atg ggc agg ttc cag tgg ctg       816
Ile Ser Ala Phe Leu Leu Ala Asn Val Met Gly Arg Phe Gln Trp Leu
            260                 265                 270

acc tgc ccg agg aag gac ctg agc acc ggc tgg ctg tac tgc gac ccg       864
Thr Cys Pro Arg Lys Asp Leu Ser Thr Gly Trp Leu Tyr Cys Asp Pro
        275                 280                 285

ggc ccg atg ttc aag ccg gag cac tac agc ctg ggc gag agc gtg ccg       912
Gly Pro Met Phe Lys Pro Glu His Tyr Ser Leu Gly Glu Ser Val Pro
    290                 295                 300

cac tgg ttc ccg tgg aag gac ctg gcc atc atg ccg gtg cag tgg cac       960
His Trp Phe Pro Trp Lys Asp Leu Ala Ile Met Pro Val Gln Trp His
305                 310                 315                 320

gcc ctg gcc ctg ggc ctg ttc gcc agc atc atc gcc ccg ttc ggc ggc      1008
Ala Leu Ala Leu Gly Leu Phe Ala Ser Ile Ile Ala Pro Phe Gly Gly
                325                 330                 335

ttc ttc gcc agc ggc ttc aag agg gcc ttc aag atc aag gac ttc ggc      1056
Phe Phe Ala Ser Gly Phe Lys Arg Ala Phe Lys Ile Lys Asp Phe Gly
            340                 345                 350

gac agc atc ccg ggc cac ggc ggc atc acc gac agg atg gac tgc cag      1104
Asp Ser Ile Pro Gly His Gly Gly Ile Thr Asp Arg Met Asp Cys Gln
        355                 360                 365

atg gtg atg gcc gtg ttc gcc tac atc tac cac cag agc ttc atc gcc      1152
Met Val Met Ala Val Phe Ala Tyr Ile Tyr His Gln Ser Phe Ile Ala
    370                 375                 380

ccg cag aac ttc agc gtg gag atc atc ctg gac cag atc ctg agg aac      1200
Pro Gln Asn Phe Ser Val Glu Ile Ile Leu Asp Gln Ile Leu Arg Asn
385                 390                 395                 400

ctg acc tac gag gag cag aag tac ctg tac gag cag ctg ggc gag atg      1248
Leu Thr Tyr Glu Glu Gln Lys Tyr Leu Tyr Glu Gln Leu Gly Glu Met
                405                 410                 415

ttc cac gag agg cag ctg ggc cag agc tga                              1278
Phe His Glu Arg Gln Leu Gly Gln Ser
            420                 425

<210>  6
<211>  425
<212>  PRT
<213>  Zea mays

<400>  6

Met Gln Arg Asp Thr Ser Ser Ser Asp Val Ser Ala Ser His Val Gly
1               5                   10                  15

Arg Val Arg Arg Arg Lys His Pro Ser Glu Ala Ser Thr Asp Gly Asn
            20                  25                  30

Arg Ala Asn Gly Gln Pro Leu Leu Val Asn Asp Gln Asn Lys Tyr Lys
        35                  40                  45

Ser Met Leu Ile Arg Thr Tyr Ser Thr Val Trp Met Ile Gly Gly Phe
    50                  55                  60

Ala Phe Ile Ile Tyr Val Gly His Leu Tyr Ile Trp Ala Met Val Val
65                  70                  75                  80

Val Ile Gln Ile Tyr Met Ala Arg Glu Leu Phe Asn Leu Leu Arg Lys
                85                  90                  95

Ser Ser Glu Glu Lys Gln Leu Pro Gly Phe Arg Leu Leu Asn Trp His
            100                 105                 110

Phe Phe Phe Thr Ala Met Leu Tyr Thr Tyr Gly Arg Phe Leu Ser Arg
        115                 120                 125

Gln Leu Val Asn Thr Val Thr Ser Asp His Leu Leu Tyr Lys Val Val
    130                 135                 140

Ser Gly Leu Ile Lys Tyr Gln Met Phe Ile Cys Tyr Phe Leu Tyr Ile
145                 150                 155                 160

Ala Gly Phe Val Trp Phe Ile Leu Thr Leu Lys Lys Lys Thr Tyr Lys
                165                 170                 175

Tyr Gln Phe Lys Gln Tyr Ala Trp Thr His Met Ile Leu Leu Thr Val
            180                 185                 190

Phe Ala Gln Ser Ala Phe Thr Val Ala Asn Ile Phe Glu Gly Ile Phe
        195                 200                 205

Trp Phe Leu Leu Pro Ala Ser Leu Ile Val Ile Asn Asp Ile Phe Ala
    210                 215                 220

Tyr Leu Phe Gly Phe Phe Leu Gly Arg Thr Pro Leu Ile Lys Leu Ser
225                 230                 235                 240

Pro Lys Lys Thr Trp Glu Gly Phe Ile Gly Ala Ser Val Thr Thr Ile
                245                 250                 255

Ile Ser Ala Phe Leu Leu Ala Asn Val Met Gly Arg Phe Gln Trp Leu
            260                 265                 270

Thr Cys Pro Arg Lys Asp Leu Ser Thr Gly Trp Leu Tyr Cys Asp Pro
        275                 280                 285

Gly Pro Met Phe Lys Pro Glu His Tyr Ser Leu Gly Glu Ser Val Pro
    290                 295                 300

His Trp Phe Pro Trp Lys Asp Leu Ala Ile Met Pro Val Gln Trp His
305                 310                 315                 320

Ala Leu Ala Leu Gly Leu Phe Ala Ser Ile Ile Ala Pro Phe Gly Gly
                325                 330                 335

Phe Phe Ala Ser Gly Phe Lys Arg Ala Phe Lys Ile Lys Asp Phe Gly
            340                 345                 350

Asp Ser Ile Pro Gly His Gly Gly Ile Thr Asp Arg Met Asp Cys Gln
        355                 360                 365

Met Val Met Ala Val Phe Ala Tyr Ile Tyr His Gln Ser Phe Ile Ala
    370                 375                 380

Pro Gln Asn Phe Ser Val Glu Ile Ile Leu Asp Gln Ile Leu Arg Asn
385                 390                 395                 400

Leu Thr Tyr Glu Glu Gln Lys Tyr Leu Tyr Glu Gln Leu Gly Glu Met
                405                 410                 415

Phe His Glu Arg Gln Leu Gly Gln Ser
            420                 425

<210>  7
<211>  378
<212>  DNA
<213>  Saccharomyces cerevisiae

<220>
<221>  CDS
<222>  (1)..(378)
<223>  acyl-carrier protein (YKL192C)

<400>  7
atg ttt aga tcc gtt tgc cgc att tct tcc cgc gtg gca cct tct gcg      48
Met Phe Arg Ser Val Cys Arg Ile Ser Ser Arg Val Ala Pro Ser Ala
1               5                   10                  15

tac cgc act ata atg ggc cgt tcc gtt atg tcc aac acc ata ctc gca      96
Tyr Arg Thr Ile Met Gly Arg Ser Val Met Ser Asn Thr Ile Leu Ala
            20                  25                  30

caa aga ttt tat tct gca aac ttg agc aaa gat cag gtt tct caa agg      144
Gln Arg Phe Tyr Ser Ala Asn Leu Ser Lys Asp Gln Val Ser Gln Arg
        35                  40                  45

gtc att gat gtt atc aag gcg ttt gat aag aac tct ccc aac att gcc      192
Val Ile Asp Val Ile Lys Ala Phe Asp Lys Asn Ser Pro Asn Ile Ala
    50                  55                  60

aac aag caa atc tcc agc gat acc caa ttt cac aag gat ttg ggg ttg      240
Asn Lys Gln Ile Ser Ser Asp Thr Gln Phe His Lys Asp Leu Gly Leu
65                  70                  75                  80

gac tcc ttg gac act gtc gag ctg ctc gta gct att gaa gaa gaa ttt      288
Asp Ser Leu Asp Thr Val Glu Leu Leu Val Ala Ile Glu Glu Glu Phe
                85                  90                  95

gat att gaa atc cct gac aaa gtg gct gat gag ttg aga agt gtt ggt      336
Asp Ile Glu Ile Pro Asp Lys Val Ala Asp Glu Leu Arg Ser Val Gly
            100                 105                 110

gaa acg gtc gat tat atc gct tcc aat ccc gac gca aac taa              378
Glu Thr Val Asp Tyr Ile Ala Ser Asn Pro Asp Ala Asn
        115                 120                 125

<210>  8
<211>  125
<212>  PRT
<213>  Saccharomyces cerevisiae

<400>  8

Met Phe Arg Ser Val Cys Arg Ile Ser Ser Arg Val Ala Pro Ser Ala
1               5                   10                  15

Tyr Arg Thr Ile Met Gly Arg Ser Val Met Ser Asn Thr Ile Leu Ala
            20                  25                  30

Gln Arg Phe Tyr Ser Ala Asn Leu Ser Lys Asp Gln Val Ser Gln Arg
        35                  40                  45

Val Ile Asp Val Ile Lys Ala Phe Asp Lys Asn Ser Pro Asn Ile Ala
    50                  55                  60

Asn Lys Gln Ile Ser Ser Asp Thr Gln Phe His Lys Asp Leu Gly Leu
65                  70                  75                  80

Asp Ser Leu Asp Thr Val Glu Leu Leu Val Ala Ile Glu Glu Glu Phe
                85                  90                  95

Asp Ile Glu Ile Pro Asp Lys Val Ala Asp Glu Leu Arg Ser Val Gly
            100                 105                 110

Glu Thr Val Asp Tyr Ile Ala Ser Asn Pro Asp Ala Asn
        115                 120                 125


<210>  9
<211>  354
<212>  DNA
<213>  Brassica napus


<220>
<221>  CDS
<222>  (1)..(354)
<223>  acyl-carrier protein (BN1004MS43616414)

<400>  9
atg cac tgc atc agg agc agc atc ctg cag cac ctg agg ctg agg gtg      48
Met His Cys Ile Arg Ser Ser Ile Leu Gln His Leu Arg Leu Arg Val
1               5                   10                  15

ccg gtg agg agc gtg ctg ctg ctg gag aag gag aac gtg ctg atc agc      96
Pro Val Arg Ser Val Leu Leu Leu Glu Lys Glu Asn Val Leu Ile Ser
            20                  25                  30

aag atg aac ttc acc agc ggc ggc ggc cag gac cag gtg ctg agc aag      144
Lys Met Asn Phe Thr Ser Gly Gly Gly Gln Asp Gln Val Leu Ser Lys
        35                  40                  45

gtg atc gag ctg gtg aag aag tac gac acc acc agc gcc agc aag gtg      192
Val Ile Glu Leu Val Lys Lys Tyr Asp Thr Thr Ser Ala Ser Lys Val
    50                  55                  60

acc gag acc gcc gac ttc aag aag gac ctg agc ctg gac agc ctg gac      240
Thr Glu Thr Ala Asp Phe Lys Lys Asp Leu Ser Leu Asp Ser Leu Asp

65                  70                  75                  80

agg gtg gag atc gtg atg gcc atc gag gag gag ttc agc gtg gag atc       288
Arg Val Glu Ile Val Met Ala Ile Glu Glu Glu Phe Ser Val Glu Ile
                85                  90                  95

ccg gac gag aag gcc gac aag ctg acc tgc tgc gcc gac atc gcc agc       336
Pro Asp Glu Lys Ala Asp Lys Leu Thr Cys Cys Ala Asp Ile Ala Ser
            100                 105                 110

ttc atc gtg agc gag tga                                               354
Phe Ile Val Ser Glu
        115

<210>  10
<211>  117
<212>  PRT
<213>  Brassica napus

<400>  10

Met His Cys Ile Arg Ser Ser Ile Leu Gln His Leu Arg Leu Arg Val
1               5                   10                  15

Pro Val Arg Ser Val Leu Leu Leu Glu Lys Glu Asn Val Leu Ile Ser
            20                  25                  30

Lys Met Asn Phe Thr Ser Gly Gly Gly Gln Asp Gln Val Leu Ser Lys
        35                  40                  45

Val Ile Glu Leu Val Lys Lys Tyr Asp Thr Thr Ser Ala Ser Lys Val
    50                  55                  60

Thr Glu Thr Ala Asp Phe Lys Lys Asp Leu Ser Leu Asp Ser Leu Asp
65                  70                  75                  80

Arg Val Glu Ile Val Met Ala Ile Glu Glu Glu Phe Ser Val Glu Ile
                85                  90                  95

Pro Asp Glu Lys Ala Asp Lys Leu Thr Cys Cys Ala Asp Ile Ala Ser
            100                 105                 110

Phe Ile Val Ser Glu
        115

<210>  11
<211>  384
<212>  DNA
<213>  Glycine max

<220>
<221>  CDS
<222>  (1)..(384)
<223>  acyl-carrier protein (GM06MC07589)

<400>  11
atg caa agc ata agg aaa tct atc ttg act cgt gtg aat ttg agg aga        48
Met Gln Ser Ile Arg Lys Ser Ile Leu Thr Arg Val Asn Leu Arg Arg
1               5                   10                  15

tca acc gaa aga tgg ttt ttg acc agg gat gag gtt gtg cat atg caa          96
Ser Thr Glu Arg Trp Phe Leu Thr Arg Asp Glu Val Val His Met Gln
            20                  25                  30

ttg aga tgt tgg tgc tct tca aca gct gtc agc tct gat caa ata ctg         144
Leu Arg Cys Trp Cys Ser Ser Thr Ala Val Ser Ser Asp Gln Ile Leu
        35                  40                  45

gac cga gtg att gca ctg gcc aag aaa tat gat aaa att gat ggc tca         192
Asp Arg Val Ile Ala Leu Ala Lys Lys Tyr Asp Lys Ile Asp Gly Ser
    50                  55                  60

aag gtc act gaa aca gct gat ttt caa aaa gac ttg aac ctg gac agt         240
Lys Val Thr Glu Thr Ala Asp Phe Gln Lys Asp Leu Asn Leu Asp Ser
65                  70                  75                  80

ttg gac cga gtg gaa ctc att atg gcc ctt gaa gaa gaa ttt tct att         288
Leu Asp Arg Val Glu Leu Ile Met Ala Leu Glu Glu Glu Phe Ser Ile
                85                  90                  95

gaa atc cct gat gag aag gct gat aag ctt gct tgc tgt gct gat att         336
Glu Ile Pro Asp Glu Lys Ala Asp Lys Leu Ala Cys Cys Ala Asp Ile
            100                 105                 110

gca aaa tac ata gca gag gtt gat cag aaa aac ctg gaa aag ccc tga         384
Ala Lys Tyr Ile Ala Glu Val Asp Gln Lys Asn Leu Glu Lys Pro
        115                 120                 125


<210>  12
<211>  127
<212>  PRT
<213>  Glycine max

<400>  12

Met Gln Ser Ile Arg Lys Ser Ile Leu Thr Arg Val Asn Leu Arg Arg
1               5                   10                  15


Ser Thr Glu Arg Trp Phe Leu Thr Arg Asp Glu Val Val His Met Gln
            20                  25                  30


Leu Arg Cys Trp Cys Ser Ser Thr Ala Val Ser Ser Asp Gln Ile Leu
        35                  40                  45


Asp Arg Val Ile Ala Leu Ala Lys Lys Tyr Asp Lys Ile Asp Gly Ser
    50                  55                  60


Lys Val Thr Glu Thr Ala Asp Phe Gln Lys Asp Leu Asn Leu Asp Ser
65                  70                  75                  80


Leu Asp Arg Val Glu Leu Ile Met Ala Leu Glu Glu Glu Phe Ser Ile
                85                  90                  95


Glu Ile Pro Asp Glu Lys Ala Asp Lys Leu Ala Cys Cys Ala Asp Ile
            100                 105                 110


Ala Lys Tyr Ile Ala Glu Val Asp Gln Lys Asn Leu Glu Lys Pro
        115                 120                 125

<210>  13
<211>  360
<212>  DNA
<213>  Helianthus annuus



<220>
<221>  CDS
<222>  (1)..(360)
<223>  acyl-carrier protein (HA1004MS66693619)

<400>  13
atg gcc gcc agg aac gcc ctg ctg aag tac ctg agg gtg aac gtg acc          48
Met Ala Ala Arg Asn Ala Leu Leu Lys Tyr Leu Arg Val Asn Val Thr
1               5                   10                  15

ccg gcc ctg cag agc agc agc gcc cag aac ccg agc gcc atc ggc ggc          96
Pro Ala Leu Gln Ser Ser Ser Ala Gln Asn Pro Ser Ala Ile Gly Gly
            20                  25                  30

ggc ctg atc cag ctg ttc agg agg cac ttc agc gag gag gtg agg ggc         144
Gly Leu Ile Gln Leu Phe Arg Arg His Phe Ser Glu Glu Val Arg Gly
        35                  40                  45

agc ttc ctg gac aag agc gag gtg acc gac agg gtg gtg acc tgc gtg         192
Ser Phe Leu Asp Lys Ser Glu Val Thr Asp Arg Val Val Thr Cys Val
    50                  55                  60

aag aac ttc cag aag gtg gac ccg agc aag gtg acc ccg acc gcc cac         240
Lys Asn Phe Gln Lys Val Asp Pro Ser Lys Val Thr Pro Thr Ala His
65                  70                  75                  80

ttc cag aac gac ctg ggc ctg gac agc ctg gac acc gtg gag gtg gtg         288
Phe Gln Asn Asp Leu Gly Leu Asp Ser Leu Asp Thr Val Glu Val Val
                85                  90                  95

atg gcc ctg gag gag gag ttc ggc ttc gag atc ccg gac aac gag gcc         336
Met Ala Leu Glu Glu Glu Phe Gly Phe Glu Ile Pro Asp Asn Glu Ala
            100                 105                 110

gac aag atc agc agc atc gag tga                                         360
Asp Lys Ile Ser Ser Ile Glu
        115


<210>  14
<211>  119
<212>  PRT
<213>  Helianthus annuus

<400>  14

Met Ala Ala Arg Asn Ala Leu Leu Lys Tyr Leu Arg Val Asn Val Thr
1               5                   10                  15


Pro Ala Leu Gln Ser Ser Ser Ala Gln Asn Pro Ser Ala Ile Gly Gly
            20                  25                  30


Gly Leu Ile Gln Leu Phe Arg Arg His Phe Ser Glu Glu Val Arg Gly
        35                  40                  45


Ser Phe Leu Asp Lys Ser Glu Val Thr Asp Arg Val Val Thr Cys Val
    50                  55                  60


Lys Asn Phe Gln Lys Val Asp Pro Ser Lys Val Thr Pro Thr Ala His

65                  70                  75                  80

Phe Gln Asn Asp Leu Gly Leu Asp Ser Leu Asp Thr Val Glu Val Val
                85                  90                  95

Met Ala Leu Glu Glu Glu Phe Gly Phe Glu Ile Pro Asp Asn Glu Ala
            100                 105                 110

Asp Lys Ile Ser Ser Ile Glu
        115

<210>  15
<211>  1191
<212>  DNA
<213>  Saccharomyces cerevisiae

<220>
<221>  CDS
<222>  (1)..(1191)
<223>  acyltransferase (YDR018C)

<400>  15
atg aag cat tcc caa aaa tac cgt agg tat gga att tat gaa aag act      48
Met Lys His Ser Gln Lys Tyr Arg Arg Tyr Gly Ile Tyr Glu Lys Thr
1               5                   10                  15

ggt aat ccc ttt ata aaa ggg ttg caa agg ctg ctt atc gct tgc ttg      96
Gly Asn Pro Phe Ile Lys Gly Leu Gln Arg Leu Leu Ile Ala Cys Leu
            20                  25                  30

ttc att tca ggc tcg ctg agt att gtc gtt ttt cag atc tgt cta cag      144
Phe Ile Ser Gly Ser Leu Ser Ile Val Val Phe Gln Ile Cys Leu Gln
        35                  40                  45

gtg ctt ctc cct tgg agc aag att aga ttt caa aat ggt ata aat caa      192
Val Leu Leu Pro Trp Ser Lys Ile Arg Phe Gln Asn Gly Ile Asn Gln
    50                  55                  60

agt aag aag gct ttt atc gtt tta tta tgc atg atc ttg aac atg gtg      240
Ser Lys Lys Ala Phe Ile Val Leu Leu Cys Met Ile Leu Asn Met Val
65                  70                  75                  80

gct ccc tct tct ttg aat gtc act ttt gaa aca tcg cgg cca ttg aag      288
Ala Pro Ser Ser Leu Asn Val Thr Phe Glu Thr Ser Arg Pro Leu Lys
                85                  90                  95

aac tct tct aac gcc aag cca tgc ttt aga ttt aaa gac agg gct ata      336
Asn Ser Ser Asn Ala Lys Pro Cys Phe Arg Phe Lys Asp Arg Ala Ile
            100                 105                 110

ata att gca aat cat caa atg tat gca gac tgg att tat ctc tgg tgg      384
Ile Ile Ala Asn His Gln Met Tyr Ala Asp Trp Ile Tyr Leu Trp Trp
        115                 120                 125

ctt tcc ttt gtt tca aat ttg ggt ggt aac gtt tat atc atc ctg aag      432
Leu Ser Phe Val Ser Asn Leu Gly Gly Asn Val Tyr Ile Ile Leu Lys
    130                 135                 140

aaa gct ctg cag tac ata cca tta ctg gga ttt ggc atg cga aat ttt      480
Lys Ala Leu Gln Tyr Ile Pro Leu Leu Gly Phe Gly Met Arg Asn Phe
145                 150                 155                 160

aag ttt ata ttt tta agt agg aac tgg caa aag gat gag aaa gct tta      528
Lys Phe Ile Phe Leu Ser Arg Asn Trp Gln Lys Asp Glu Lys Ala Leu

165                 170                 175

aca aat agt ttg gtt tct atg gac tta aac gcg agg tgc aag ggg ccc      576
Thr Asn Ser Leu Val Ser Met Asp Leu Asn Ala Arg Cys Lys Gly Pro
            180                 185                 190

ctt aca aat tat aag agt tgt tat tcc aag aca aat gaa tcc att gcc      624
Leu Thr Asn Tyr Lys Ser Cys Tyr Ser Lys Thr Asn Glu Ser Ile Ala
        195                 200                 205

gct tat aat tta atc atg ttc cct gag ggt aca aat cta agc ctc aag      672
Ala Tyr Asn Leu Ile Met Phe Pro Glu Gly Thr Asn Leu Ser Leu Lys
    210                 215                 220

aca aga gaa aaa agc gag gca ttc tgt caa aga gca cat ttg gac cat      720
Thr Arg Glu Lys Ser Glu Ala Phe Cys Gln Arg Ala His Leu Asp His
225                 230                 235                 240

gtc caa tta aga cat ttg tta tta ccg cac tct aaa ggc ttg aag ttt      768
Val Gln Leu Arg His Leu Leu Leu Pro His Ser Lys Gly Leu Lys Phe
                245                 250                 255

gca gta gaa aaa cta gct cct agt tta gat gct atc tac gat gtc act      816
Ala Val Glu Lys Leu Ala Pro Ser Leu Asp Ala Ile Tyr Asp Val Thr
            260                 265                 270

att gga tat tct ccc gcc ttg aga acg gaa tac gtc ggc acc aaa ttc      864
Ile Gly Tyr Ser Pro Ala Leu Arg Thr Glu Tyr Val Gly Thr Lys Phe
        275                 280                 285

acc ttg aag aaa ata ttc tta atg ggt gtc tat ccg gag aaa gta gat      912
Thr Leu Lys Lys Ile Phe Leu Met Gly Val Tyr Pro Glu Lys Val Asp
    290                 295                 300

ttt tat att agg gaa ttt aga gtt aat gag atc cct ttg caa gat gac      960
Phe Tyr Ile Arg Glu Phe Arg Val Asn Glu Ile Pro Leu Gln Asp Asp
305                 310                 315                 320

gaa gtt ttt ttc aat tgg tta ctg ggc gtg tgg aaa gaa aaa gat caa     1008
Glu Val Phe Phe Asn Trp Leu Leu Gly Val Trp Lys Glu Lys Asp Gln
                325                 330                 335

ctg cta gaa gac tac tac aac aca ggc caa ttt aaa agt aat gct aaa     1056
Leu Leu Glu Asp Tyr Tyr Asn Thr Gly Gln Phe Lys Ser Asn Ala Lys
            340                 345                 350

aat gac aac caa tcc atc gtt gtt acg aca caa acg act gga ttt cag     1104
Asn Asp Asn Gln Ser Ile Val Val Thr Thr Gln Thr Thr Gly Phe Gln
        355                 360                 365

cac gaa aca ttg aca ccc cgt atc ctt tca tat tac ggg ttc ttc gct     1152
His Glu Thr Leu Thr Pro Arg Ile Leu Ser Tyr Tyr Gly Phe Phe Ala
    370                 375                 380

ttt ctt att ctt gta ttt gtg atg aaa aaa aat cat tga                 1191
Phe Leu Ile Leu Val Phe Val Met Lys Lys Asn His
385                 390                 395


<210>  16
<211>  396
<212>  PRT
<213>  Saccharomyces cerevisiae

<400>  16

Met Lys His Ser Gln Lys Tyr Arg Arg Tyr Gly Ile Tyr Glu Lys Thr
1               5                   10                  15

Gly Asn Pro Phe Ile Lys Gly Leu Gln Arg Leu Leu Ile Ala Cys Leu
            20                  25                  30

Phe Ile Ser Gly Ser Leu Ser Ile Val Val Phe Gln Ile Cys Leu Gln
        35                  40                  45

Val Leu Leu Pro Trp Ser Lys Ile Arg Phe Gln Asn Gly Ile Asn Gln
    50                  55                  60

Ser Lys Lys Ala Phe Ile Val Leu Leu Cys Met Ile Leu Asn Met Val
65                  70                  75                  80

Ala Pro Ser Ser Leu Asn Val Thr Phe Glu Thr Ser Arg Pro Leu Lys
                85                  90                  95

Asn Ser Ser Asn Ala Lys Pro Cys Phe Arg Phe Lys Asp Arg Ala Ile
            100                 105                 110

Ile Ile Ala Asn His Gln Met Tyr Ala Asp Trp Ile Tyr Leu Trp Trp
        115                 120                 125

Leu Ser Phe Val Ser Asn Leu Gly Gly Asn Val Tyr Ile Ile Leu Lys
    130                 135                 140

Lys Ala Leu Gln Tyr Ile Pro Leu Leu Gly Phe Gly Met Arg Asn Phe
145                 150                 155                 160

Lys Phe Ile Phe Leu Ser Arg Asn Trp Gln Lys Asp Glu Lys Ala Leu
                165                 170                 175

Thr Asn Ser Leu Val Ser Met Asp Leu Asn Ala Arg Cys Lys Gly Pro
            180                 185                 190

Leu Thr Asn Tyr Lys Ser Cys Tyr Ser Lys Thr Asn Glu Ser Ile Ala
        195                 200                 205

Ala Tyr Asn Leu Ile Met Phe Pro Glu Gly Thr Asn Leu Ser Leu Lys
    210                 215                 220

Thr Arg Glu Lys Ser Glu Ala Phe Cys Gln Arg Ala His Leu Asp His
225                 230                 235                 240

Val Gln Leu Arg His Leu Leu Leu Pro His Ser Lys Gly Leu Lys Phe
                245                 250                 255

Ala Val Glu Lys Leu Ala Pro Ser Leu Asp Ala Ile Tyr Asp Val Thr
            260                 265                 270

Ile Gly Tyr Ser Pro Ala Leu Arg Thr Glu Tyr Val Gly Thr Lys Phe
        275                 280                 285

Thr Leu Lys Lys Ile Phe Leu Met Gly Val Tyr Pro Glu Lys Val Asp
    290                 295                 300

Phe Tyr Ile Arg Glu Phe Arg Val Asn Glu Ile Pro Leu Gln Asp Asp
305                 310                 315                 320

Glu Val Phe Phe Asn Trp Leu Leu Gly Val Trp Lys Glu Lys Asp Gln
                325                 330                 335

Leu Leu Glu Asp Tyr Tyr Asn Thr Gly Gln Phe Lys Ser Asn Ala Lys
            340                 345                 350

Asn Asp Asn Gln Ser Ile Val Val Thr Thr Gln Thr Thr Gly Phe Gln
        355                 360                 365

His Glu Thr Leu Thr Pro Arg Ile Leu Ser Tyr Tyr Gly Phe Phe Ala
    370                 375                 380

Phe Leu Ile Leu Val Phe Val Met Lys Lys Asn His
385                 390                 395

<210>  17
<211>  1155
<212>  DNA
<213>  Glycine max

<220>
<221>  CDS
<222>  (1)..(1155)
<223>  acyltransferase (GM06MC27072)

<400>  17
atg acc gcc gtg gtg gtg gtg ccg ctg ggc ctg ctg ttc ttc gcc agc      48
Met Thr Ala Val Val Val Val Pro Leu Gly Leu Leu Phe Phe Ala Ser
1               5                   10                  15

ggc ctg atc gtg aac ctg atc cag gcc atc tgc tac gtg gtg gtg agg      96
Gly Leu Ile Val Asn Leu Ile Gln Ala Ile Cys Tyr Val Val Val Arg
            20                  25                  30

ccg gtg agc aag aac ctg tac agg agg atg aac agg gtg gtg gcc gag      144
Pro Val Ser Lys Asn Leu Tyr Arg Arg Met Asn Arg Val Val Ala Glu
        35                  40                  45

ctg ctg tgg ctg gag ctg gtg tgg atc atc gac tgg tgg gcc ggc gtg      192
Leu Leu Trp Leu Glu Leu Val Trp Ile Ile Asp Trp Trp Ala Gly Val
    50                  55                  60

aag gtg cag gtg ttc acc gac ccg gag acc ttc cac agc atg ggc aag      240
Lys Val Gln Val Phe Thr Asp Pro Glu Thr Phe His Ser Met Gly Lys
65                  70                  75                  80

gag cac gcc ctg gtg atc agc aac cac agg agc gac atc gac tgg ctg      288
Glu His Ala Leu Val Ile Ser Asn His Arg Ser Asp Ile Asp Trp Leu
                85                  90                  95

gtg ggc tgg gtg ctg gcc cag agg agc ggc tgc ctg ggc agc acc ctg      336
Val Gly Trp Val Leu Ala Gln Arg Ser Gly Cys Leu Gly Ser Thr Leu
            100                 105                 110

gcc gtg atg aag aag agc agc aag ttc ctg ccg gtg atc ggc tgg agc      384

Ala Val Met Lys Lys Ser Ser Lys Phe Leu Pro Val Ile Gly Trp Ser
        115                 120                 125

atg tgg ttc agc gag tac ctg ttc ctg gag agg agc tgg gcc aag gac     432
Met Trp Phe Ser Glu Tyr Leu Phe Leu Glu Arg Ser Trp Ala Lys Asp
    130                 135                 140

gag agg acc ctg aag agc ggc ctg cag cag ctg agg gac ttc ccg ctg     480
Glu Arg Thr Leu Lys Ser Gly Leu Gln Gln Leu Arg Asp Phe Pro Leu
145                 150                 155                 160

ccg ttc tgg ctg gcc ctg ttc gtg gag ggc acc agg ttc acc cag gcc     528
Pro Phe Trp Leu Ala Leu Phe Val Glu Gly Thr Arg Phe Thr Gln Ala
                165                 170                 175

aag ctg ctg gcc gcc cag gag tac gcc gcc agc gcc ggc ctg ccg gtg     576
Lys Leu Leu Ala Ala Gln Glu Tyr Ala Ala Ser Ala Gly Leu Pro Val
            180                 185                 190

ccg agg aac gtg ctg atc ccg agg acc aag ggc ttc gtg agc gcc gtg     624
Pro Arg Asn Val Leu Ile Pro Arg Thr Lys Gly Phe Val Ser Ala Val
        195                 200                 205

aac cac atg agg agc ttc gtg ccg gcc atc tac gac gtg acc gtg gcc     672
Asn His Met Arg Ser Phe Val Pro Ala Ile Tyr Asp Val Thr Val Ala
    210                 215                 220

atc ccg aag agc agc ccg gcc ccg acc atg ctg agg ctg ttc agg ggc     720
Ile Pro Lys Ser Ser Pro Ala Pro Thr Met Leu Arg Leu Phe Arg Gly
225                 230                 235                 240

aag agc agc ctg gtg cac gtg cac atc aag agg cac gcc atg aag gac     768
Lys Ser Ser Leu Val His Val His Ile Lys Arg His Ala Met Lys Asp
                245                 250                 255

ctg ccg gag gag gac gag gcc gtg gcc cag tgg tgc agg gac gtg ttc     816
Leu Pro Glu Glu Asp Glu Ala Val Ala Gln Trp Cys Arg Asp Val Phe
            260                 265                 270

gtg gcc aag gac gcc ctg ctg gac aag cac atc gcc gag gac acc ttc     864
Val Ala Lys Asp Ala Leu Leu Asp Lys His Ile Ala Glu Asp Thr Phe
        275                 280                 285

agc gac cag gag ctg cag gac acc ggc agg ccg atc aag agc ctg ctg     912
Ser Asp Gln Glu Leu Gln Asp Thr Gly Arg Pro Ile Lys Ser Leu Leu
    290                 295                 300

gtg gtg atc agc tgg gtg tgc ctg gtg gtg gcc ggc agc gtg aag ttc     960
Val Val Ile Ser Trp Val Cys Leu Val Val Ala Gly Ser Val Lys Phe
305                 310                 315                 320

ctg cag agg agc agc ctg ctg agc agc tgg aag ggc gtg gcc ttc agc    1008
Leu Gln Arg Ser Ser Leu Leu Ser Ser Trp Lys Gly Val Ala Phe Ser
                325                 330                 335

gcc ttc ggc ctg gcc gtg gtg acc gcc ctg atg cag atc ctg atc cag    1056
Ala Phe Gly Leu Ala Val Val Thr Ala Leu Met Gln Ile Leu Ile Gln
            340                 345                 350

ttc agc cag agc gag agg agc aac ccg gcc aag atc gtg ccg gcc aag    1104
Phe Ser Gln Ser Glu Arg Ser Asn Pro Ala Lys Ile Val Pro Ala Lys
        355                 360                 365

agc aag aac aag ggc ggc cag ctg gag gcc agg aac gac aag cag cag    1152
Ser Lys Asn Lys Gly Gly Gln Leu Glu Ala Arg Asn Asp Lys Gln Gln
    370                 375                 380

tga                                                                1155

<210>  18
<211>  384
<212>  PRT
<213>  Glycine max

<400>  18

Met Thr Ala Val Val Val Val Pro Leu Gly Leu Leu Phe Phe Ala Ser
1               5                   10                  15

Gly Leu Ile Val Asn Leu Ile Gln Ala Ile Cys Tyr Val Val Val Arg
            20                  25                  30

Pro Val Ser Lys Asn Leu Tyr Arg Arg Met Asn Arg Val Val Ala Glu
        35                  40                  45

Leu Leu Trp Leu Glu Leu Val Trp Ile Ile Asp Trp Trp Ala Gly Val
    50                  55                  60

Lys Val Gln Val Phe Thr Asp Pro Glu Thr Phe His Ser Met Gly Lys
65                  70                  75                  80

Glu His Ala Leu Val Ile Ser Asn His Arg Ser Asp Ile Asp Trp Leu
                85                  90                  95

Val Gly Trp Val Leu Ala Gln Arg Ser Gly Cys Leu Gly Ser Thr Leu
            100                 105                 110

Ala Val Met Lys Lys Ser Ser Lys Phe Leu Pro Val Ile Gly Trp Ser
        115                 120                 125

Met Trp Phe Ser Glu Tyr Leu Phe Leu Glu Arg Ser Trp Ala Lys Asp
    130                 135                 140

Glu Arg Thr Leu Lys Ser Gly Leu Gln Gln Leu Arg Asp Phe Pro Leu
145                 150                 155                 160

Pro Phe Trp Leu Ala Leu Phe Val Glu Gly Thr Arg Phe Thr Gln Ala
                165                 170                 175

Lys Leu Leu Ala Ala Gln Glu Tyr Ala Ala Ser Ala Gly Leu Pro Val
            180                 185                 190

Pro Arg Asn Val Leu Ile Pro Arg Thr Lys Gly Phe Val Ser Ala Val
        195                 200                 205

Asn His Met Arg Ser Phe Val Pro Ala Ile Tyr Asp Val Thr Val Ala
    210                 215                 220

Ile Pro Lys Ser Ser Pro Ala Pro Thr Met Leu Arg Leu Phe Arg Gly
225                 230                 235                 240

Lys Ser Ser Leu Val His Val His Ile Lys Arg His Ala Met Lys Asp

245                 250                 255

Leu Pro Glu Glu Asp Glu Ala Val Ala Gln Trp Cys Arg Asp Val Phe
            260                 265                 270

Val Ala Lys Asp Ala Leu Leu Asp Lys His Ile Ala Glu Asp Thr Phe
        275                 280                 285

Ser Asp Gln Glu Leu Gln Asp Thr Gly Arg Pro Ile Lys Ser Leu Leu
    290                 295                 300

Val Val Ile Ser Trp Val Cys Leu Val Val Ala Gly Ser Val Lys Phe
305                 310                 315                 320

Leu Gln Arg Ser Ser Leu Leu Ser Ser Trp Lys Gly Val Ala Phe Ser
                325                 330                 335

Ala Phe Gly Leu Ala Val Val Thr Ala Leu Met Gln Ile Leu Ile Gln
            340                 345                 350

Phe Ser Gln Ser Glu Arg Ser Asn Pro Ala Lys Ile Val Pro Ala Lys
        355                 360                 365

Ser Lys Asn Lys Gly Gly Gln Leu Glu Ala Arg Asn Asp Lys Gln Gln
    370                 375                 380

<210>  19
<211>  999
<212>  DNA
<213>  Zea mays

<220>
<221>  CDS
<222>  (1)..(999)
<223>  acyltransferase (ZM06MC04863)

<400>  19
atg gag tgc agc aac agc acc agc agc cag ggc cac cac gtg ggc aag      48
Met Glu Cys Ser Asn Ser Thr Ser Ser Gln Gly His His Val Gly Lys
1               5                   10                  15

tgg aac cag gtg cag atc atc gac ccg agc atc gtg gag atg ggc ccg      96
Trp Asn Gln Val Gln Ile Ile Asp Pro Ser Ile Val Glu Met Gly Pro
            20                  25                  30

agc cac ctg ccg ctg agc ttc atg agg agg tgc agg ggc gtg ctg tgc      144
Ser His Leu Pro Leu Ser Phe Met Arg Arg Cys Arg Gly Val Leu Cys
        35                  40                  45

ctg gtg atc atg atc ctg acc gcc ttc atg atg atg gtg tac ctg agc      192
Leu Val Ile Met Ile Leu Thr Ala Phe Met Met Met Val Tyr Leu Ser
    50                  55                  60

ccg gtg acc acc ttc ctg gtg agg ctg ttc agc gtg cac tac agc agg      240
Pro Val Thr Thr Phe Leu Val Arg Leu Phe Ser Val His Tyr Ser Arg
65                  70                  75                  80

aag agc acc tgc ttc ctg ttc ggc atg tgg ctg gcc atg tgg ccg ttc      288
Lys Ser Thr Cys Phe Leu Phe Gly Met Trp Leu Ala Met Trp Pro Phe

85                  90                  95

ctg ttc gag aag atc aac aag acc agg ttc gtg ttc agc ggc gag agc      336
Leu Phe Glu Lys Ile Asn Lys Thr Arg Phe Val Phe Ser Gly Glu Ser
            100                 105                 110

gtg ccg gcc aag gag agg gtg ctg ctg ttc gcc aac cac agg acc gag      384
Val Pro Ala Lys Glu Arg Val Leu Leu Phe Ala Asn His Arg Thr Glu
        115                 120                 125

gtg gac tgg atg tac ctg tgg gac ttc gcc ctg agg aag ggc agg ctg      432
Val Asp Trp Met Tyr Leu Trp Asp Phe Ala Leu Arg Lys Gly Arg Leu
    130                 135                 140

cag tgc atc aag tac atc ctg aag aag agc ctg atg aag ctg ccg gtg      480
Gln Cys Ile Lys Tyr Ile Leu Lys Lys Ser Leu Met Lys Leu Pro Val
145                 150                 155                 160

ttc aac tgg gcc ttc cac atc atc gag ttc atc ccg gtg gag agg aag      528
Phe Asn Trp Ala Phe His Ile Ile Glu Phe Ile Pro Val Glu Arg Lys
                165                 170                 175

tgg gag atc gac gag gcc atc atc agg agc agg ctg agc gag ttc aag      576
Trp Glu Ile Asp Glu Ala Ile Ile Arg Ser Arg Leu Ser Glu Phe Lys
            180                 185                 190

aac ccg aag gac ccg ctg tgg ctg gcc gtg ttc ccg gag ggc acc gac      624
Asn Pro Lys Asp Pro Leu Trp Leu Ala Val Phe Pro Glu Gly Thr Asp
        195                 200                 205

tac acc gag aag aag tgc atc aag agc cag gag tac gcc gcc gag cac      672
Tyr Thr Glu Lys Lys Cys Ile Lys Ser Gln Glu Tyr Ala Ala Glu His
    210                 215                 220

ggc ctg ccg gtg ctg aag aac gtg ctg ctg ccg aag acc aag ggc ttc      720
Gly Leu Pro Val Leu Lys Asn Val Leu Leu Pro Lys Thr Lys Gly Phe
225                 230                 235                 240

aac tgc tgc ctg cag gtg ctg agg agc acc atc gac gcc gtg tac gac      768
Asn Cys Cys Leu Gln Val Leu Arg Ser Thr Ile Asp Ala Val Tyr Asp
                245                 250                 255

atc acc atc gcc tac aag cac agg ccg ccg acc ttc ctg gac aac gtg      816
Ile Thr Ile Ala Tyr Lys His Arg Pro Pro Thr Phe Leu Asp Asn Val
            260                 265                 270

tac ggc gtg ggc ctg agg aag agc agc agc acc agc gcc gcc agc agg      864
Tyr Gly Val Gly Leu Arg Lys Ser Ser Ser Thr Ser Ala Ala Ser Arg
        275                 280                 285

agc ccg acc tac agg agg ccg aag acc ggc tgg ctg gcc ggc tgg tgg      912
Ser Pro Thr Tyr Arg Arg Pro Lys Thr Gly Trp Leu Ala Gly Trp Trp
    290                 295                 300

agc ggc agc ggc agc agg acc agc tgc tgc ccg gcc agc ccg agg tgg      960
Ser Gly Ser Gly Ser Arg Thr Ser Cys Cys Pro Ala Ser Pro Arg Trp
305                 310                 315                 320

gcc acc agc ccg acc aag ggc ccg ccg agg ggc acc tga                  999
Ala Thr Ser Pro Thr Lys Gly Pro Pro Arg Gly Thr
                325                 330

<210>  20
<211>  332
<212>  PRT
<213>  Zea mays

<400>  20

Met Glu Cys Ser Asn Ser Thr Ser Ser Gln Gly His His Val Gly Lys
1               5                   10                  15

Trp Asn Gln Val Gln Ile Ile Asp Pro Ser Ile Val Glu Met Gly Pro
            20                  25                  30

Ser His Leu Pro Leu Ser Phe Met Arg Arg Cys Arg Gly Val Leu Cys
        35                  40                  45

Leu Val Ile Met Ile Leu Thr Ala Phe Met Met Met Val Tyr Leu Ser
    50                  55                  60

Pro Val Thr Thr Phe Leu Val Arg Leu Phe Ser Val His Tyr Ser Arg
65                  70                  75                  80

Lys Ser Thr Cys Phe Leu Phe Gly Met Trp Leu Ala Met Trp Pro Phe
                85                  90                  95

Leu Phe Glu Lys Ile Asn Lys Thr Arg Phe Val Phe Ser Gly Glu Ser
            100                 105                 110

Val Pro Ala Lys Glu Arg Val Leu Leu Phe Ala Asn His Arg Thr Glu
        115                 120                 125

Val Asp Trp Met Tyr Leu Trp Asp Phe Ala Leu Arg Lys Gly Arg Leu
    130                 135                 140

Gln Cys Ile Lys Tyr Ile Leu Lys Lys Ser Leu Met Lys Leu Pro Val
145                 150                 155                 160

Phe Asn Trp Ala Phe His Ile Ile Glu Phe Ile Pro Val Glu Arg Lys
                165                 170                 175

Trp Glu Ile Asp Glu Ala Ile Ile Arg Ser Arg Leu Ser Glu Phe Lys
            180                 185                 190

Asn Pro Lys Asp Pro Leu Trp Leu Ala Val Phe Pro Glu Gly Thr Asp
        195                 200                 205

Tyr Thr Glu Lys Lys Cys Ile Lys Ser Gln Glu Tyr Ala Ala Glu His
    210                 215                 220

Gly Leu Pro Val Leu Lys Asn Val Leu Leu Pro Lys Thr Lys Gly Phe
225                 230                 235                 240

Asn Cys Cys Leu Gln Val Leu Arg Ser Thr Ile Asp Ala Val Tyr Asp
                245                 250                 255

Ile Thr Ile Ala Tyr Lys His Arg Pro Pro Thr Phe Leu Asp Asn Val
            260                 265                 270

Tyr Gly Val Gly Leu Arg Lys Ser Ser Ser Thr Ser Ala Ala Ser Arg
        275                 280                 285

Ser Pro Thr Tyr Arg Arg Pro Lys Thr Gly Trp Leu Ala Gly Trp Trp
    290                 295                 300

Ser Gly Ser Gly Ser Arg Thr Ser Cys Cys Pro Ala Ser Pro Arg Trp
305                 310                 315                 320

Ala Thr Ser Pro Thr Lys Gly Pro Pro Arg Gly Thr
                325                 330

<210>  21
<211>  2142
<212>  DNA
<213>  Escherichia coli

<220>
<221>  CDS
<222>  (1)..(2142)
<223>  bifunctional anaerobic fatty acid oxidation complex protein
       (B2341)

<400>  21
atg gaa atg aca tca gcg ttt acc ctt aat gtt cgt ctg gac aac att       48
Met Glu Met Thr Ser Ala Phe Thr Leu Asn Val Arg Leu Asp Asn Ile
1               5                   10                  15

gcc gtt atc acc atc gac gta ccg ggt gag aaa atg aat acc ctg aag       96
Ala Val Ile Thr Ile Asp Val Pro Gly Glu Lys Met Asn Thr Leu Lys
            20                  25                  30

gcg gag ttt gcc tcg cag gtg cgc gcc att att aag caa ctc cgt gaa      144
Ala Glu Phe Ala Ser Gln Val Arg Ala Ile Ile Lys Gln Leu Arg Glu
        35                  40                  45

aac aaa gag ttg cga ggc gtg gtg ttt gtc tcc gct aaa ccg gac aac      192
Asn Lys Glu Leu Arg Gly Val Val Phe Val Ser Ala Lys Pro Asp Asn
    50                  55                  60

ttc att gct ggc gca gac atc aac atg atc ggc aac tgc aaa acg gcg      240
Phe Ile Ala Gly Ala Asp Ile Asn Met Ile Gly Asn Cys Lys Thr Ala
65                  70                  75                  80

caa gaa gcg gaa gct ctg gcg cgg cag ggc caa cag ttg atg gcg gag      288
Gln Glu Ala Glu Ala Leu Ala Arg Gln Gly Gln Gln Leu Met Ala Glu
                85                  90                  95

att cat gct ttg ccc att cag gtt atc gcg gct att cat ggc gct tgc      336
Ile His Ala Leu Pro Ile Gln Val Ile Ala Ala Ile His Gly Ala Cys
            100                 105                 110

ctg ggt ggt ggg ctg gag ttg gcg ctg gcg tgc cac ggt cgc gtt tgt      384
Leu Gly Gly Gly Leu Glu Leu Ala Leu Ala Cys His Gly Arg Val Cys
        115                 120                 125

act gac gat cct aaa acg gtg ctc ggt ttg cct gaa gta caa ctt gga      432
Thr Asp Asp Pro Lys Thr Val Leu Gly Leu Pro Glu Val Gln Leu Gly
    130                 135                 140

ttg tta ccc ggt tca ggc ggc acc cag cgt tta ccg cgt ctg ata ggc      480
Leu Leu Pro Gly Ser Gly Gly Thr Gln Arg Leu Pro Arg Leu Ile Gly
145                 150                 155                 160

gtc agc aca gca tta gag atg atc ctc acc gga aaa caa ctt cgg gcg 528
Val Ser Thr Ala Leu Glu Met Ile Leu Thr Gly Lys Gln Leu Arg Ala
                165                 170                 175

aaa cag gca tta aag ctg ggg ctg gtg gat gac gtt gtt ccg cac tcc 576
Lys Gln Ala Leu Lys Leu Gly Leu Val Asp Asp Val Val Pro His Ser
            180                 185                 190

att ctg ctg gaa gcc gct gtt gag ctg gca aag aag gag cgc cca tct 624
Ile Leu Leu Glu Ala Ala Val Glu Leu Ala Lys Lys Glu Arg Pro Ser
        195                 200                 205

tcc cgc cct cta cct gta cgc gag cgt att ctg gcg ggg ccg tta ggt 672
Ser Arg Pro Leu Pro Val Arg Glu Arg Ile Leu Ala Gly Pro Leu Gly
    210                 215                 220

cgt gcg ctg ctg ttc aaa atg gtc ggc aag aaa aca gaa cac aaa act 720
Arg Ala Leu Leu Phe Lys Met Val Gly Lys Lys Thr Glu His Lys Thr
225                 230                 235                 240

caa ggc aat tat ccg gcg aca gaa cgc atc ctg gag gtt gtt gaa acg 768
Gln Gly Asn Tyr Pro Ala Thr Glu Arg Ile Leu Glu Val Val Glu Thr
                245                 250                 255

gga tta gcg cag ggc acc agc agc ggt tat gac gcc gaa gct cgg gcg 816
Gly Leu Ala Gln Gly Thr Ser Ser Gly Tyr Asp Ala Glu Ala Arg Ala
            260                 265                 270

ttt ggc gaa ctg gcg atg acg cca caa tcg cag gcg ctg cgt agt atc 864
Phe Gly Glu Leu Ala Met Thr Pro Gln Ser Gln Ala Leu Arg Ser Ile
        275                 280                 285

ttt ttt gcc agt acg gac gtg aag aaa gat ccc ggc agt gat gcg ccg 912
Phe Phe Ala Ser Thr Asp Val Lys Lys Asp Pro Gly Ser Asp Ala Pro
    290                 295                 300

cct gcg cca tta aac agc gtg ggg att tta ggt ggt ggc ttg atg ggc 960
Pro Ala Pro Leu Asn Ser Val Gly Ile Leu Gly Gly Gly Leu Met Gly
305                 310                 315                 320

ggc ggt att gct tat gtc act gct tgt aaa gcg ggg att ccg gtc aga 1008
Gly Gly Ile Ala Tyr Val Thr Ala Cys Lys Ala Gly Ile Pro Val Arg
                325                 330                 335

att aaa gat atc aac ccg cag ggc ata aat cat gcg ctg aag tac agt 1056
Ile Lys Asp Ile Asn Pro Gln Gly Ile Asn His Ala Leu Lys Tyr Ser
            340                 345                 350

tgg gat cag ctg gag ggc aaa gtt cgc cgt cgt cat ctc aaa gcc agc 1104
Trp Asp Gln Leu Glu Gly Lys Val Arg Arg Arg His Leu Lys Ala Ser
        355                 360                 365

gaa cgt gac aaa cag ctg gca tta atc tcc gga acg acg gac tat cgc 1152
Glu Arg Asp Lys Gln Leu Ala Leu Ile Ser Gly Thr Thr Asp Tyr Arg
    370                 375                 380

ggc ttt gcc cat cgc gat ctg att att gaa gcg gtg ttt gaa aat ctc 1200
Gly Phe Ala His Arg Asp Leu Ile Ile Glu Ala Val Phe Glu Asn Leu
385                 390                 395                 400

ttg aaa caa cag atg gtg gcg gaa gtt gag caa aat tgc gcc gct cat 1248
Leu Lys Gln Gln Met Val Ala Glu Val Glu Gln Asn Cys Ala Ala His
                405                 410                 415

acc atc ttt gct tcg aat acg tca tct tta ccg att ggt gat atc gcc 1296
Thr Ile Phe Ala Ser Asn Thr Ser Ser Leu Pro Ile Gly Asp Ile Ala
            420                 425                 430

gct cac gcc acg cga cct gag caa gtt atc ggc ctg cat ttc ttc agt 1344

Ala His Ala Thr Arg Pro Glu Gln Val Ile Gly Leu His Phe Phe Ser
        435                 440                 445

ccg gtg gaa aaa atg ccg ctg gtg gag att att cct cat gcg ggg aca        1392
Pro Val Glu Lys Met Pro Leu Val Glu Ile Ile Pro His Ala Gly Thr
    450                 455                 460

tcg gcg caa acc atc gct acc aca gta aaa ctg gcg aaa aaa cag ggt        1440
Ser Ala Gln Thr Ile Ala Thr Thr Val Lys Leu Ala Lys Lys Gln Gly
465                 470                 475                 480

aaa acg cca att gtc gtg cgt gac aaa gcc ggt ttt tac gtc aat cgc        1488
Lys Thr Pro Ile Val Val Arg Asp Lys Ala Gly Phe Tyr Val Asn Arg
                485                 490                 495

atc tta gcg cct tac att aat gaa gct atc cgc atg ttg acc caa ggt        1536
Ile Leu Ala Pro Tyr Ile Asn Glu Ala Ile Arg Met Leu Thr Gln Gly
            500                 505                 510

gaa cgg gta gag cac att gat gcc gcg cta gtg aaa ttt ggt ttt ccg        1584
Glu Arg Val Glu His Ile Asp Ala Ala Leu Val Lys Phe Gly Phe Pro
        515                 520                 525

gta ggc cca atc caa ctt ttg gat gag gta gga atc gac acc ggg act        1632
Val Gly Pro Ile Gln Leu Leu Asp Glu Val Gly Ile Asp Thr Gly Thr
    530                 535                 540

aaa att att cct gta ctg gaa gcc gct tat gga gaa cgt ttt agc gcg        1680
Lys Ile Ile Pro Val Leu Glu Ala Ala Tyr Gly Glu Arg Phe Ser Ala
545                 550                 555                 560

cct gca aat gtt gtt tct tca att ttg aac gac gat cgc aaa ggc aga        1728
Pro Ala Asn Val Val Ser Ser Ile Leu Asn Asp Asp Arg Lys Gly Arg
                565                 570                 575

aaa aat ggc cgg ggt ttc tat ctt tat ggt cag aaa ggg cgt aaa agc        1776
Lys Asn Gly Arg Gly Phe Tyr Leu Tyr Gly Gln Lys Gly Arg Lys Ser
            580                 585                 590

aaa aaa cag gtc gat ccc gcc att tac ccg ctg att ggc aca caa ggg        1824
Lys Lys Gln Val Asp Pro Ala Ile Tyr Pro Leu Ile Gly Thr Gln Gly
        595                 600                 605

cag ggg cga atc tcc gca ccg cag gtt gct gaa cgg tgt gtg atg ttg        1872
Gln Gly Arg Ile Ser Ala Pro Gln Val Ala Glu Arg Cys Val Met Leu
    610                 615                 620

atg ctg aat gaa gca gta cgt tgt gtt gat gag cag gtt atc cgt agc        1920
Met Leu Asn Glu Ala Val Arg Cys Val Asp Glu Gln Val Ile Arg Ser
625                 630                 635                 640

gtg cgt gac ggg gat att ggc gcg gta ttt ggc att ggt ttt ccg cca        1968
Val Arg Asp Gly Asp Ile Gly Ala Val Phe Gly Ile Gly Phe Pro Pro
                645                 650                 655

ttt ctc ggt gga ccg ttc cgc tat atc gat tct ctc ggc gcg ggc gaa        2016
Phe Leu Gly Gly Pro Phe Arg Tyr Ile Asp Ser Leu Gly Ala Gly Glu
            660                 665                 670

gtg gtt gca ata atg caa cga ctt gcc acg cag tat ggt tcc cgt ttt        2064
Val Val Ala Ile Met Gln Arg Leu Ala Thr Gln Tyr Gly Ser Arg Phe
        675                 680                 685

acc cct tgc gag cgt ttg gtc gag atg ggc gcg cgt ggg gaa agt ttt        2112
Thr Pro Cys Glu Arg Leu Val Glu Met Gly Ala Arg Gly Glu Ser Phe
    690                 695                 700

tgg aaa aca act gca act gac ctg caa taa                                2142
Trp Lys Thr Thr Ala Thr Asp Leu Gln

705                 710


<210>  22
<211>  713
<212>  PRT
<213>  Escherichia coli

<400>  22

Met Glu Met Thr Ser Ala Phe Thr Leu Asn Val Arg Leu Asp Asn Ile
1               5                   10                  15


Ala Val Ile Thr Ile Asp Val Pro Gly Glu Lys Met Asn Thr Leu Lys
            20                  25                  30


Ala Glu Phe Ala Ser Gln Val Arg Ala Ile Ile Lys Gln Leu Arg Glu
        35                  40                  45


Asn Lys Glu Leu Arg Gly Val Val Phe Val Ser Ala Lys Pro Asp Asn
    50                  55                  60


Phe Ile Ala Gly Ala Asp Ile Asn Met Ile Gly Asn Cys Lys Thr Ala
65                  70                  75                  80


Gln Glu Ala Glu Ala Leu Ala Arg Gln Gly Gln Gln Leu Met Ala Glu
                85                  90                  95


Ile His Ala Leu Pro Ile Gln Val Ile Ala Ala Ile His Gly Ala Cys
            100                 105                 110


Leu Gly Gly Gly Leu Glu Leu Ala Leu Ala Cys His Gly Arg Val Cys
        115                 120                 125


Thr Asp Asp Pro Lys Thr Val Leu Gly Leu Pro Glu Val Gln Leu Gly
    130                 135                 140


Leu Leu Pro Gly Ser Gly Gly Thr Gln Arg Leu Pro Arg Leu Ile Gly
145                 150                 155                 160


Val Ser Thr Ala Leu Glu Met Ile Leu Thr Gly Lys Gln Leu Arg Ala
                165                 170                 175


Lys Gln Ala Leu Lys Leu Gly Leu Val Asp Asp Val Val Pro His Ser
            180                 185                 190


Ile Leu Leu Glu Ala Ala Val Glu Leu Ala Lys Lys Glu Arg Pro Ser
        195                 200                 205


Ser Arg Pro Leu Pro Val Arg Glu Arg Ile Leu Ala Gly Pro Leu Gly
    210                 215                 220


Arg Ala Leu Leu Phe Lys Met Val Gly Lys Lys Thr Glu His Lys Thr
225                 230                 235                 240

Gln Gly Asn Tyr Pro Ala Thr Glu Arg Ile Leu Glu Val Val Glu Thr
                245                 250                 255

Gly Leu Ala Gln Gly Thr Ser Ser Gly Tyr Asp Ala Glu Ala Arg Ala
            260                 265                 270

Phe Gly Glu Leu Ala Met Thr Pro Gln Ser Gln Ala Leu Arg Ser Ile
        275                 280                 285

Phe Phe Ala Ser Thr Asp Val Lys Lys Asp Pro Gly Ser Asp Ala Pro
    290                 295                 300

Pro Ala Pro Leu Asn Ser Val Gly Ile Leu Gly Gly Gly Leu Met Gly
305                 310                 315                 320

Gly Gly Ile Ala Tyr Val Thr Ala Cys Lys Ala Gly Ile Pro Val Arg
                325                 330                 335

Ile Lys Asp Ile Asn Pro Gln Gly Ile Asn His Ala Leu Lys Tyr Ser
            340                 345                 350

Trp Asp Gln Leu Glu Gly Lys Val Arg Arg Arg His Leu Lys Ala Ser
        355                 360                 365

Glu Arg Asp Lys Gln Leu Ala Leu Ile Ser Gly Thr Thr Asp Tyr Arg
    370                 375                 380

Gly Phe Ala His Arg Asp Leu Ile Ile Glu Ala Val Phe Glu Asn Leu
385                 390                 395                 400

Leu Lys Gln Gln Met Val Ala Glu Val Glu Gln Asn Cys Ala Ala His
                405                 410                 415

Thr Ile Phe Ala Ser Asn Thr Ser Ser Leu Pro Ile Gly Asp Ile Ala
            420                 425                 430

Ala His Ala Thr Arg Pro Glu Gln Val Ile Gly Leu His Phe Phe Ser
        435                 440                 445

Pro Val Glu Lys Met Pro Leu Val Glu Ile Ile Pro His Ala Gly Thr
    450                 455                 460

Ser Ala Gln Thr Ile Ala Thr Thr Val Lys Leu Ala Lys Lys Gln Gly
465                 470                 475                 480

Lys Thr Pro Ile Val Val Arg Asp Lys Ala Gly Phe Tyr Val Asn Arg
                485                 490                 495

Ile Leu Ala Pro Tyr Ile Asn Glu Ala Ile Arg Met Leu Thr Gln Gly
            500                 505                 510

Glu Arg Val Glu His Ile Asp Ala Ala Leu Val Lys Phe Gly Phe Pro
        515                 520                 525

Val Gly Pro Ile Gln Leu Leu Asp Glu Val Gly Ile Asp Thr Gly Thr
    530                 535                 540

Lys Ile Ile Pro Val Leu Glu Ala Ala Tyr Gly Glu Arg Phe Ser Ala
545                 550                 555                 560

Pro Ala Asn Val Val Ser Ser Ile Leu Asn Asp Asp Arg Lys Gly Arg
                565                 570                 575

Lys Asn Gly Arg Gly Phe Tyr Leu Tyr Gly Gln Lys Gly Arg Lys Ser
            580                 585                 590

Lys Lys Gln Val Asp Pro Ala Ile Tyr Pro Leu Ile Gly Thr Gln Gly
        595                 600                 605

Gln Gly Arg Ile Ser Ala Pro Gln Val Ala Glu Arg Cys Val Met Leu
    610                 615                 620

Met Leu Asn Glu Ala Val Arg Cys Val Asp Glu Gln Val Ile Arg Ser
625                 630                 635                 640

Val Arg Asp Gly Asp Ile Gly Ala Val Phe Gly Ile Gly Phe Pro Pro
                645                 650                 655

Phe Leu Gly Gly Pro Phe Arg Tyr Ile Asp Ser Leu Gly Ala Gly Glu
            660                 665                 670

Val Val Ala Ile Met Gln Arg Leu Ala Thr Gln Tyr Gly Ser Arg Phe
        675                 680                 685

Thr Pro Cys Glu Arg Leu Val Glu Met Gly Ala Arg Gly Glu Ser Phe
    690                 695                 700

Trp Lys Thr Thr Ala Thr Asp Leu Gln
705                 710

<210>  23
<211>  2172
<212>  DNA
<213>  Zea mays

<220>
<221>  CDS
<222>  (1)..(2172)
<223>  bifunctional anaerobic fatty acid oxidation complex protein
       (ZM06MC04303)

<400>  23
atg gcc gcc aag gtg agg acc gag ctg gac gtg ggc gcc gac ggc gtg        48
Met Ala Ala Lys Val Arg Thr Glu Leu Asp Val Gly Ala Asp Gly Val
1               5                   10                  15

gcc gtg atc acc atc tac aac ccg ccg gtg aac agc ctg agc atc gac 96
Ala Val Ile Thr Ile Tyr Asn Pro Pro Val Asn Ser Leu Ser Ile Asp
            20                  25                  30

gtg ctg cac agc ctg aag gag agc tac gag gag gcc ctg agg agg aac 144
Val Leu His Ser Leu Lys Glu Ser Tyr Glu Glu Ala Leu Arg Arg Asn
        35                  40                  45

gac gtg aag gcc atc gtg gtg acc ggc aag ggc ggc aag ttc agc ggc 192
Asp Val Lys Ala Ile Val Val Thr Gly Lys Gly Gly Lys Phe Ser Gly
    50                  55                  60

ggc ttc gac atc agc agc ttc ggc ggc gtg cag ggc ggc cag acc atg 240
Gly Phe Asp Ile Ser Ser Phe Gly Gly Val Gln Gly Gly Gln Thr Met
65                  70                  75                  80

cag ccg aag gtg ggc tac atc gcc atc gac atc ctg acc gac acc gtg 288
Gln Pro Lys Val Gly Tyr Ile Ala Ile Asp Ile Leu Thr Asp Thr Val
                85                  90                  95

gag gcc gcc acc aag ccg agc gtg gcc gcc atc gac ggc ctg gcc ctg 336
Glu Ala Ala Thr Lys Pro Ser Val Ala Ala Ile Asp Gly Leu Ala Leu
            100                 105                 110

ggc ggc ggc ctg gag gtg gcc atg gcc tgc cac gcc agg atc gcc acc 384
Gly Gly Gly Leu Glu Val Ala Met Ala Cys His Ala Arg Ile Ala Thr
        115                 120                 125

ccg acc gcc cag ctg ggc ctg ccg gag ctg cag ctg ggc atc atc ccg 432
Pro Thr Ala Gln Leu Gly Leu Pro Glu Leu Gln Leu Gly Ile Ile Pro
    130                 135                 140

ggc ttc ggc ggc acc cag agg ctg ccg agg ctg gtg ggc ctg acc aag 480
Gly Phe Gly Gly Thr Gln Arg Leu Pro Arg Leu Val Gly Leu Thr Lys
145                 150                 155                 160

agc ctg gag atg atg ctg ctg agc aag ccg atc aag ggc ggc gag gcc 528
Ser Leu Glu Met Met Leu Leu Ser Lys Pro Ile Lys Gly Gly Glu Ala
                165                 170                 175

cac cag ctg ggc ctg gtg gac gcc ctg gtg agc ccg aac gac ctg gtg 576
His Gln Leu Gly Leu Val Asp Ala Leu Val Ser Pro Asn Asp Leu Val
            180                 185                 190

aac acc gcc agg cag tgg gcc ctg gac atc tac gag tgc agg agg ccg 624
Asn Thr Ala Arg Gln Trp Ala Leu Asp Ile Tyr Glu Cys Arg Arg Pro
        195                 200                 205

tgg atc aag agc ctg tac aag acc gac aag ctg gag ccg ctg ggc gag 672
Trp Ile Lys Ser Leu Tyr Lys Thr Asp Lys Leu Glu Pro Leu Gly Glu
    210                 215                 220

gcc agg gag atc ctg aag ttc gcc agg gcc cag gcc cag aag cag gcc 720
Ala Arg Glu Ile Leu Lys Phe Ala Arg Ala Gln Ala Gln Lys Gln Ala
225                 230                 235                 240

gcc aac ctg cac cac ccg ctg gtg tgc atc gac gtg atc gag gag ggc 768
Ala Asn Leu His His Pro Leu Val Cys Ile Asp Val Ile Glu Glu Gly
                245                 250                 255

atc gtg gcc ggc ccg agg gcc ggc ctg tgg aag gag gcc acc agc ttc 816
Ile Val Ala Gly Pro Arg Ala Gly Leu Trp Lys Glu Ala Thr Ser Phe
            260                 265                 270

cag gag ctg ctg ttc agc gac acc tgc aag agc ctg gtg cac gtg ttc 864
Gln Glu Leu Leu Phe Ser Asp Thr Cys Lys Ser Leu Val His Val Phe
        275                 280                 285

ttc agc cag agg gcc acc agc aag atc ccg ggc gcc acc gac ctg ggc 912

Phe Ser Gln Arg Ala Thr Ser Lys Ile Pro Gly Ala Thr Asp Leu Gly
    290                 295                 300

ctg atg ccg agg aag atc acc aag gtg gcc atc ctg ggc ggc ggc ctg      960
Leu Met Pro Arg Lys Ile Thr Lys Val Ala Ile Leu Gly Gly Gly Leu
305                 310                 315                 320

atg ggc agc ggc atc gcc acc gcc atg gtg ctg agc aac tac ccg gtg      1008
Met Gly Ser Gly Ile Ala Thr Ala Met Val Leu Ser Asn Tyr Pro Val
                325                 330                 335

ctg ctg aag gag gtg aac gag aag ttc ctg acc gcc ggc atc aac agg      1056
Leu Leu Lys Glu Val Asn Glu Lys Phe Leu Thr Ala Gly Ile Asn Arg
            340                 345                 350

atc cag gcc aac ctg cag agc agg gtg aag aag ggc aag atg acc gag      1104
Ile Gln Ala Asn Leu Gln Ser Arg Val Lys Lys Gly Lys Met Thr Glu
        355                 360                 365

gag agg tac gag aag gcc atg agc ctg gtg acc ggc gtg ctg gac tac      1152
Glu Arg Tyr Glu Lys Ala Met Ser Leu Val Thr Gly Val Leu Asp Tyr
    370                 375                 380

gag agg ttc aag gac gtg gac ctg gtg atc gag gcc gtg atc gag aac      1200
Glu Arg Phe Lys Asp Val Asp Leu Val Ile Glu Ala Val Ile Glu Asn
385                 390                 395                 400

gtg aag ctg aag cag cag atc ttc agc gac ctg gag aag tac tgc ccg      1248
Val Lys Leu Lys Gln Gln Ile Phe Ser Asp Leu Glu Lys Tyr Cys Pro
                405                 410                 415

agc cac tgc atc ctg gcc acc aac acc agc acc atc gac ctg aac ctg      1296
Ser His Cys Ile Leu Ala Thr Asn Thr Ser Thr Ile Asp Leu Asn Leu
            420                 425                 430

atc ggc gag aag acc aag gcc cag gac agg atc gcc ggc gcc cac ttc      1344
Ile Gly Glu Lys Thr Lys Ala Gln Asp Arg Ile Ala Gly Ala His Phe
        435                 440                 445

ttc agc ccg gcc cac gtg atg ccg ctg ctg gag atc gtg agg acc cag      1392
Phe Ser Pro Ala His Val Met Pro Leu Leu Glu Ile Val Arg Thr Gln
    450                 455                 460

cac acc agc ccg cag gtg gtg gtg gac ctg ctg gac gtg ggc aag aag      1440
His Thr Ser Pro Gln Val Val Val Asp Leu Leu Asp Val Gly Lys Lys
465                 470                 475                 480

atc aag aag acc ccg atc gtg gtg ggc aac tgc acc ggc ttc gcc gtg      1488
Ile Lys Lys Thr Pro Ile Val Val Gly Asn Cys Thr Gly Phe Ala Val
                485                 490                 495

aac agg atg ttc ttc ccg tac acc cag agc gcc ctg ttc tac gtg gac      1536
Asn Arg Met Phe Phe Pro Tyr Thr Gln Ser Ala Leu Phe Tyr Val Asp
            500                 505                 510

ctg ggc atg gac gtg tac aag atc gac agg gcc tgc acc aag ttc ggc      1584
Leu Gly Met Asp Val Tyr Lys Ile Asp Arg Ala Cys Thr Lys Phe Gly
        515                 520                 525

atg ccg atg ggc ccg ttc agg ctg gcc gac ctg gtg ggc ttc ggc gtg      1632
Met Pro Met Gly Pro Phe Arg Leu Ala Asp Leu Val Gly Phe Gly Val
    530                 535                 540

gcc gtg gcc acc ggc atg cag tac ctg gag aac ttc ccg gag agg gtg      1680
Ala Val Ala Thr Gly Met Gln Tyr Leu Glu Asn Phe Pro Glu Arg Val
545                 550                 555                 560

tac aag agc atg ctg ctg ccg ctg atg atg gag ggc aac agg gcc ggc      1728
Tyr Lys Ser Met Leu Leu Pro Leu Met Met Glu Gly Asn Arg Ala Gly

565                 570                 575

gag gcc acc cag aag ggc ttc tac aag tac gag ggc aag agg aag gcc      1776
Glu Ala Thr Gln Lys Gly Phe Tyr Lys Tyr Glu Gly Lys Arg Lys Ala
            580                 585                 590

acc ccg gac ccg gag atc atg aag tac atc gag aag agc agg agc atg      1824
Thr Pro Asp Pro Glu Ile Met Lys Tyr Ile Glu Lys Ser Arg Ser Met
        595                 600                 605

gcc ggc gtg acc ccg gac ccg gag ctg atg aag ctg agc gag aag gac      1872
Ala Gly Val Thr Pro Asp Pro Glu Leu Met Lys Leu Ser Glu Lys Asp
    610                 615                 620

atc gtg gag atg gtg ttc ttc ccg gtg atc aac gag gcc tgc agg gtg      1920
Ile Val Glu Met Val Phe Phe Pro Val Ile Asn Glu Ala Cys Arg Val
625                 630                 635                 640

ctg gac gag ggc atc gcc gtg aag gcc agc gac ctg gac atc gcc agc      1968
Leu Asp Glu Gly Ile Ala Val Lys Ala Ser Asp Leu Asp Ile Ala Ser
                645                 650                 655

atc ttc ggc atg ggc ttc ccg ccg tac agg ggc ggc gtg atg cac tgg      2016
Ile Phe Gly Met Gly Phe Pro Pro Tyr Arg Gly Gly Val Met His Trp
            660                 665                 670

gcc gac agc atc ggc gcc aag tac atc cac ggc aag ctg gag gag tgg      2064
Ala Asp Ser Ile Gly Ala Lys Tyr Ile His Gly Lys Leu Glu Glu Trp
        675                 680                 685

acc aag agg tac ggc ggc ttc ttc aag ccg tgc agc tac ctg gcc gag      2112
Thr Lys Arg Tyr Gly Gly Phe Phe Lys Pro Cys Ser Tyr Leu Ala Glu
    690                 695                 700

agg gcc gcc aag ggc atc ccg ctg agc gcc ccg acc aag aag gtg cag      2160
Arg Ala Ala Lys Gly Ile Pro Leu Ser Ala Pro Thr Lys Lys Val Gln
705                 710                 715                 720

gcc agg ctg tga                                                      2172
Ala Arg Leu

<210>  24
<211>  723
<212>  PRT
<213>  Zea mays

<400>  24

Met Ala Ala Lys Val Arg Thr Glu Leu Asp Val Gly Ala Asp Gly Val
1               5                   10                  15

Ala Val Ile Thr Ile Tyr Asn Pro Pro Val Asn Ser Leu Ser Ile Asp
            20                  25                  30

Val Leu His Ser Leu Lys Glu Ser Tyr Glu Glu Ala Leu Arg Arg Asn
        35                  40                  45

Asp Val Lys Ala Ile Val Val Thr Gly Lys Gly Gly Lys Phe Ser Gly
    50                  55                  60

Gly Phe Asp Ile Ser Ser Phe Gly Gly Val Gln Gly Gly Gln Thr Met
65                  70                  75                  80

Gln Pro Lys Val Gly Tyr Ile Ala Ile Asp Ile Leu Thr Asp Thr Val
                85                  90                  95

Glu Ala Ala Thr Lys Pro Ser Val Ala Ala Ile Asp Gly Leu Ala Leu
            100                 105                 110

Gly Gly Gly Leu Glu Val Ala Met Ala Cys His Ala Arg Ile Ala Thr
        115                 120                 125

Pro Thr Ala Gln Leu Gly Leu Pro Glu Leu Gln Leu Gly Ile Ile Pro
    130                 135                 140

Gly Phe Gly Gly Thr Gln Arg Leu Pro Arg Leu Val Gly Leu Thr Lys
145                 150                 155                 160

Ser Leu Glu Met Met Leu Leu Ser Lys Pro Ile Lys Gly Gly Glu Ala
                165                 170                 175

His Gln Leu Gly Leu Val Asp Ala Leu Val Ser Pro Asn Asp Leu Val
            180                 185                 190

Asn Thr Ala Arg Gln Trp Ala Leu Asp Ile Tyr Glu Cys Arg Arg Pro
        195                 200                 205

Trp Ile Lys Ser Leu Tyr Lys Thr Asp Lys Leu Glu Pro Leu Gly Glu
    210                 215                 220

Ala Arg Glu Ile Leu Lys Phe Ala Arg Ala Gln Ala Gln Lys Gln Ala
225                 230                 235                 240

Ala Asn Leu His His Pro Leu Val Cys Ile Asp Val Ile Glu Glu Gly
                245                 250                 255

Ile Val Ala Gly Pro Arg Ala Gly Leu Trp Lys Glu Ala Thr Ser Phe
            260                 265                 270

Gln Glu Leu Leu Phe Ser Asp Thr Cys Lys Ser Leu Val His Val Phe
        275                 280                 285

Phe Ser Gln Arg Ala Thr Ser Lys Ile Pro Gly Ala Thr Asp Leu Gly
    290                 295                 300

Leu Met Pro Arg Lys Ile Thr Lys Val Ala Ile Leu Gly Gly Gly Leu
305                 310                 315                 320

Met Gly Ser Gly Ile Ala Thr Ala Met Val Leu Ser Asn Tyr Pro Val
                325                 330                 335

Leu Leu Lys Glu Val Asn Glu Lys Phe Leu Thr Ala Gly Ile Asn Arg
            340                 345                 350

Ile Gln Ala Asn Leu Gln Ser Arg Val Lys Lys Gly Lys Met Thr Glu
        355                 360                 365

Glu Arg Tyr Glu Lys Ala Met Ser Leu Val Thr Gly Val Leu Asp Tyr
    370                 375                 380

Glu Arg Phe Lys Asp Val Asp Leu Val Ile Glu Ala Val Ile Glu Asn
385                 390                 395                 400

Val Lys Leu Lys Gln Gln Ile Phe Ser Asp Leu Glu Lys Tyr Cys Pro
                405                 410                 415

Ser His Cys Ile Leu Ala Thr Asn Thr Ser Thr Ile Asp Leu Asn Leu
            420                 425                 430

Ile Gly Glu Lys Thr Lys Ala Gln Asp Arg Ile Ala Gly Ala His Phe
        435                 440                 445

Phe Ser Pro Ala His Val Met Pro Leu Leu Glu Ile Val Arg Thr Gln
    450                 455                 460

His Thr Ser Pro Gln Val Val Val Asp Leu Leu Asp Val Gly Lys Lys
465                 470                 475                 480

Ile Lys Lys Thr Pro Ile Val Val Gly Asn Cys Thr Gly Phe Ala Val
                485                 490                 495

Asn Arg Met Phe Phe Pro Tyr Thr Gln Ser Ala Leu Phe Tyr Val Asp
            500                 505                 510

Leu Gly Met Asp Val Tyr Lys Ile Asp Arg Ala Cys Thr Lys Phe Gly
        515                 520                 525

Met Pro Met Gly Pro Phe Arg Leu Ala Asp Leu Val Gly Phe Gly Val
    530                 535                 540

Ala Val Ala Thr Gly Met Gln Tyr Leu Glu Asn Phe Pro Glu Arg Val
545                 550                 555                 560

Tyr Lys Ser Met Leu Leu Pro Leu Met Met Glu Gly Asn Arg Ala Gly
                565                 570                 575

Glu Ala Thr Gln Lys Gly Phe Tyr Lys Tyr Glu Gly Lys Arg Lys Ala
            580                 585                 590

Thr Pro Asp Pro Glu Ile Met Lys Tyr Ile Glu Lys Ser Arg Ser Met
        595                 600                 605

Ala Gly Val Thr Pro Asp Pro Glu Leu Met Lys Leu Ser Glu Lys Asp
    610                 615                 620

Ile Val Glu Met Val Phe Phe Pro Val Ile Asn Glu Ala Cys Arg Val

625                 630                 635                 640

Leu Asp Glu Gly Ile Ala Val Lys Ala Ser Asp Leu Asp Ile Ala Ser
                645                 650                 655

Ile Phe Gly Met Gly Phe Pro Pro Tyr Arg Gly Gly Val Met His Trp
            660                 665                 670

Ala Asp Ser Ile Gly Ala Lys Tyr Ile His Gly Lys Leu Glu Glu Trp
        675                 680                 685

Thr Lys Arg Tyr Gly Gly Phe Phe Lys Pro Cys Ser Tyr Leu Ala Glu
    690                 695                 700

Arg Ala Ala Lys Gly Ile Pro Leu Ser Ala Pro Thr Lys Lys Val Gln
705                 710                 715                 720

Ala Arg Leu

<210>  25
<211>  2184
<212>  DNA
<213>  Zea mays

<220>
<221>  CDS
<222>  (1)..(2184)
<223>  bifunctional anaerobic fatty acid oxidation complex protein
       (ZM06MC15742)

<400>  25
atg gcc gcc ggc agc atc agg gtg acc atg gag gtg ggc gcc gac ggc       48
Met Ala Ala Gly Ser Ile Arg Val Thr Met Glu Val Gly Ala Asp Gly
1               5                   10                  15

gtg gcc ctg atc acc atc gcc aac ccg ccg gtg aac gcc ctg cac ccg       96
Val Ala Leu Ile Thr Ile Ala Asn Pro Pro Val Asn Ala Leu His Pro
            20                  25                  30

atc atc atc gcc ggc ctg aag gac aag tac gcc gag gcc ctg agg agg      144
Ile Ile Ile Ala Gly Leu Lys Asp Lys Tyr Ala Glu Ala Leu Arg Arg
        35                  40                  45

gac gac gtg aag gcc atc gtg ctg acc ggc gcc ggc ggc aag ttc tgc      192
Asp Asp Val Lys Ala Ile Val Leu Thr Gly Ala Gly Gly Lys Phe Cys
    50                  55                  60

ggc ggc ttc gac atc aac gtg ttc acc aag gtg cac cag acc ggc gac      240
Gly Gly Phe Asp Ile Asn Val Phe Thr Lys Val His Gln Thr Gly Asp
65                  70                  75                  80

gtg agc ctg atg ccg gac gtg agc gtg gag ctg gtg agc aac atg atg      288
Val Ser Leu Met Pro Asp Val Ser Val Glu Leu Val Ser Asn Met Met
                85                  90                  95

gag gag ggc aag aag ccg agc gtg gcc gcc atc cag ggc ctg gcc ctg      336
Glu Glu Gly Lys Lys Pro Ser Val Ala Ala Ile Gln Gly Leu Ala Leu
            100                 105                 110

ggc ggc ggc ctg gag ctg acc atg ggc tgc cac gcc agg atc agc acc      384

Gly Gly Gly Leu Glu Leu Thr Met Gly Cys His Ala Arg Ile Ser Thr
        115                 120                 125

ccg gag gcc cag ctg ggc ctg ccg gag ctg acc ctg ggc atc atc ccg      432
Pro Glu Ala Gln Leu Gly Leu Pro Glu Leu Thr Leu Gly Ile Ile Pro
    130                 135                 140

ggc ttc ggc ggc acc cag agg ctg ccg agg ctg gtg ggc ctg ccg aag      480
Gly Phe Gly Gly Thr Gln Arg Leu Pro Arg Leu Val Gly Leu Pro Lys
145                 150                 155                 160

gcc atc gag atg atg ctg cag agc aag ttc atc acc gcc aag gag ggc      528
Ala Ile Glu Met Met Leu Gln Ser Lys Phe Ile Thr Ala Lys Glu Gly
                165                 170                 175

aag gag agg ggc ctg atc gac gcc ctg tgc agc ccg gac gag ctg atc      576
Lys Glu Arg Gly Leu Ile Asp Ala Leu Cys Ser Pro Asp Glu Leu Ile
            180                 185                 190

aag acc agc agg ctg tgg gcc ctg gag atc gcc aac tgc agg aag ccg      624
Lys Thr Ser Arg Leu Trp Ala Leu Glu Ile Ala Asn Cys Arg Lys Pro
        195                 200                 205

tgg atg agg agc ctg ggc agg acc gac agg ctg ggc ccg ctg agc gag      672
Trp Met Arg Ser Leu Gly Arg Thr Asp Arg Leu Gly Pro Leu Ser Glu
    210                 215                 220

gcc agg gcc gtg ctg aac gcc gcc agg cag cag gcc atg aag atc gcc      720
Ala Arg Ala Val Leu Asn Ala Ala Arg Gln Gln Ala Met Lys Ile Ala
225                 230                 235                 240

ccg aac atg ccg cag aac cag gcc tgc ctg gac gtg atg gag gag ggc      768
Pro Asn Met Pro Gln Asn Gln Ala Cys Leu Asp Val Met Glu Glu Gly
                245                 250                 255

atc ctg tgc ggc ggc cag gcc ggc gtg ctg aag gag gcc atg gtg ttc      816
Ile Leu Cys Gly Gly Gln Ala Gly Val Leu Lys Glu Ala Met Val Phe
            260                 265                 270

aag gag ctg gtg atc gcc ccg acc agc aag gcc ctg gtg cac gtg ttc      864
Lys Glu Leu Val Ile Ala Pro Thr Ser Lys Ala Leu Val His Val Phe
        275                 280                 285

ttc gcc cag agg agc acc acc aag gtg ccg ggc gtg acc gac gtg cag      912
Phe Ala Gln Arg Ser Thr Thr Lys Val Pro Gly Val Thr Asp Val Gln
    290                 295                 300

ctg aag ccg agg ccg atc agg aag gtg gcc gtg atc ggc ggc ggc ctg      960
Leu Lys Pro Arg Pro Ile Arg Lys Val Ala Val Ile Gly Gly Gly Leu
305                 310                 315                 320

atg ggc agc ggc atc gcc acc agc ctg ctg gtg agc aac atc agc gtg     1008
Met Gly Ser Gly Ile Ala Thr Ser Leu Leu Val Ser Asn Ile Ser Val
                325                 330                 335

gtg ctg aag gag gtg aac ccg cag ttc ctg cag agg ggc gag aag acc     1056
Val Leu Lys Glu Val Asn Pro Gln Phe Leu Gln Arg Gly Glu Lys Thr
            340                 345                 350

atc gcc ggc aac ctg gag ggc ctg gtg aag agg ggc agc ccg acc aag     1104
Ile Ala Gly Asn Leu Glu Gly Leu Val Lys Arg Gly Ser Pro Thr Lys
        355                 360                 365

gac agg atg cac aag gcc atg gcc ctg ctg aag ggc gcc ctg gac tac     1152
Asp Arg Met His Lys Ala Met Ala Leu Leu Lys Gly Ala Leu Asp Tyr
    370                 375                 380

agc gac ttc aag gac gtg gac atg gtg atc gag gcc gtg atc gag aag     1200
Ser Asp Phe Lys Asp Val Asp Met Val Ile Glu Ala Val Ile Glu Lys

385                 390                 395                 400

atc ccg ctg aag cag agc atc ttc gcc gac atc gag aag atc tgc ccg      1248
Ile Pro Leu Lys Gln Ser Ile Phe Ala Asp Ile Glu Lys Ile Cys Pro
                405                 410                 415

aag cac tgc atc ctg gcc acc aac acc agc acc atc gac ctg aac gtg      1296
Lys His Cys Ile Leu Ala Thr Asn Thr Ser Thr Ile Asp Leu Asn Val
            420                 425                 430

gtg ggc aag aag acc aac agc cag gac agg atc atc ggc gcc cac ttc      1344
Val Gly Lys Lys Thr Asn Ser Gln Asp Arg Ile Ile Gly Ala His Phe
        435                 440                 445

ttc agc ccg gcc cac atc atg ccg ctg ctg gag atc gtg agg acc gag      1392
Phe Ser Pro Ala His Ile Met Pro Leu Leu Glu Ile Val Arg Thr Glu
    450                 455                 460

aag acc agc ccg cag gcc atc ctg gac ctg atc acc atc ggc aag atc      1440
Lys Thr Ser Pro Gln Ala Ile Leu Asp Leu Ile Thr Ile Gly Lys Ile
465                 470                 475                 480

atc aag aag gtg ccg atc gtg gtg ggc aac tgc acc ggc ttc gcc gtg      1488
Ile Lys Lys Val Pro Ile Val Val Gly Asn Cys Thr Gly Phe Ala Val
                485                 490                 495

aac ctg acc ttc ttc ccg tac acc cag ggc agc cac ctg ctg gtg agc      1536
Asn Leu Thr Phe Phe Pro Tyr Thr Gln Gly Ser His Leu Leu Val Ser
            500                 505                 510

ctg ggc atc gac gtg ttc agg atc gac agg gtg atc agc acc ttc ggc      1584
Leu Gly Ile Asp Val Phe Arg Ile Asp Arg Val Ile Ser Thr Phe Gly
        515                 520                 525

atg ccg atg ggc ccg ttc cag ctg cag gac gtg gcc ggc tac ggc gtg      1632
Met Pro Met Gly Pro Phe Gln Leu Gln Asp Val Ala Gly Tyr Gly Val
    530                 535                 540

gcc ctg gcc gtg aag gac atc tac gcc gac gcc ttc ggc gag agg aac      1680
Ala Leu Ala Val Lys Asp Ile Tyr Ala Asp Ala Phe Gly Glu Arg Asn
545                 550                 555                 560

ctg gac agc gac ctg gtg gac ctg atg gtg aag gac ggc agg cag ggc      1728
Leu Asp Ser Asp Leu Val Asp Leu Met Val Lys Asp Gly Arg Gln Gly
                565                 570                 575

aag gtg aac ggc aag ggc tac tac atc tac gag aag ggc ggc aag ccg      1776
Lys Val Asn Gly Lys Gly Tyr Tyr Ile Tyr Glu Lys Gly Gly Lys Pro
            580                 585                 590

aag ccg gac ccg agc gtg aag cac gtg atc gag gag tac agg aag cac      1824
Lys Pro Asp Pro Ser Val Lys His Val Ile Glu Glu Tyr Arg Lys His
        595                 600                 605

gcc aac acc atg ccg ggc ggc aag ccg gtg acc ctg acc gac cag gac      1872
Ala Asn Thr Met Pro Gly Gly Lys Pro Val Thr Leu Thr Asp Gln Asp
    610                 615                 620

atc ctg gag atg atc ttc ttc ccg gtg gtg aac gag gcc tgc agg gtg      1920
Ile Leu Glu Met Ile Phe Phe Pro Val Val Asn Glu Ala Cys Arg Val
625                 630                 635                 640

atg gac gag aac gtg gtg atc agg gcc agc gac ctg gac atc gcc agc      1968
Met Asp Glu Asn Val Val Ile Arg Ala Ser Asp Leu Asp Ile Ala Ser
                645                 650                 655

gtg ctg ggc atg ggc ttc ccg aag tac agg ggc ggc ctg gtg ttc tgg      2016
Val Leu Gly Met Gly Phe Pro Lys Tyr Arg Gly Gly Leu Val Phe Trp
            660                 665                 670

gcc gac acc gtg ggc gcc ccg tac atc cac agc aag ctg agc aag tgg     2064
Ala Asp Thr Val Gly Ala Pro Tyr Ile His Ser Lys Leu Ser Lys Trp
        675                 680                 685

gcc gag atc tac ggc ccg ttc ttc aag ccg agc agc tac ctg gag cag     2112
Ala Glu Ile Tyr Gly Pro Phe Phe Lys Pro Ser Ser Tyr Leu Glu Gln
    690                 695                 700

agg gcc aag agc ggc gtg ccg ctg agc gcc ccg ggc gcc agc cag cag     2160
Arg Ala Lys Ser Gly Val Pro Leu Ser Ala Pro Gly Ala Ser Gln Gln
705                 710                 715                 720

ggc agc gcc agg agc agg atg tga                                     2184
Gly Ser Ala Arg Ser Arg Met
                725

<210>  26
<211>  727
<212>  PRT
<213>  Zea mays

<400>  26

Met Ala Ala Gly Ser Ile Arg Val Thr Met Glu Val Gly Ala Asp Gly
1               5                   10                  15

Val Ala Leu Ile Thr Ile Ala Asn Pro Pro Val Asn Ala Leu His Pro
            20                  25                  30

Ile Ile Ile Ala Gly Leu Lys Asp Lys Tyr Ala Glu Ala Leu Arg Arg
        35                  40                  45

Asp Asp Val Lys Ala Ile Val Leu Thr Gly Ala Gly Gly Lys Phe Cys
    50                  55                  60

Gly Gly Phe Asp Ile Asn Val Phe Thr Lys Val His Gln Thr Gly Asp
65                  70                  75                  80

Val Ser Leu Met Pro Asp Val Ser Val Glu Leu Val Ser Asn Met Met
                85                  90                  95

Glu Glu Gly Lys Lys Pro Ser Val Ala Ala Ile Gln Gly Leu Ala Leu
            100                 105                 110

Gly Gly Gly Leu Glu Leu Thr Met Gly Cys His Ala Arg Ile Ser Thr
        115                 120                 125

Pro Glu Ala Gln Leu Gly Leu Pro Glu Leu Thr Leu Gly Ile Ile Pro
    130                 135                 140

Gly Phe Gly Gly Thr Gln Arg Leu Pro Arg Leu Val Gly Leu Pro Lys
145                 150                 155                 160

Ala Ile Glu Met Met Leu Gln Ser Lys Phe Ile Thr Ala Lys Glu Gly
                165                 170                 175

Lys Glu Arg Gly Leu Ile Asp Ala Leu Cys Ser Pro Asp Glu Leu Ile
            180                 185                 190

Lys Thr Ser Arg Leu Trp Ala Leu Glu Ile Ala Asn Cys Arg Lys Pro
        195                 200                 205

Trp Met Arg Ser Leu Gly Arg Thr Asp Arg Leu Gly Pro Leu Ser Glu
    210                 215                 220

Ala Arg Ala Val Leu Asn Ala Ala Arg Gln Gln Ala Met Lys Ile Ala
225                 230                 235                 240

Pro Asn Met Pro Gln Asn Gln Ala Cys Leu Asp Val Met Glu Glu Gly
                245                 250                 255

Ile Leu Cys Gly Gly Gln Ala Gly Val Leu Lys Glu Ala Met Val Phe
            260                 265                 270

Lys Glu Leu Val Ile Ala Pro Thr Ser Lys Ala Leu Val His Val Phe
        275                 280                 285

Phe Ala Gln Arg Ser Thr Thr Lys Val Pro Gly Val Thr Asp Val Gln
    290                 295                 300

Leu Lys Pro Arg Pro Ile Arg Lys Val Ala Val Ile Gly Gly Gly Leu
305                 310                 315                 320

Met Gly Ser Gly Ile Ala Thr Ser Leu Leu Val Ser Asn Ile Ser Val
                325                 330                 335

Val Leu Lys Glu Val Asn Pro Gln Phe Leu Gln Arg Gly Glu Lys Thr
            340                 345                 350

Ile Ala Gly Asn Leu Glu Gly Leu Val Lys Arg Gly Ser Pro Thr Lys
        355                 360                 365

Asp Arg Met His Lys Ala Met Ala Leu Leu Lys Gly Ala Leu Asp Tyr
    370                 375                 380

Ser Asp Phe Lys Asp Val Asp Met Val Ile Glu Ala Val Ile Glu Lys
385                 390                 395                 400

Ile Pro Leu Lys Gln Ser Ile Phe Ala Asp Ile Glu Lys Ile Cys Pro
                405                 410                 415

Lys His Cys Ile Leu Ala Thr Asn Thr Ser Thr Ile Asp Leu Asn Val
            420                 425                 430

Val Gly Lys Lys Thr Asn Ser Gln Asp Arg Ile Ile Gly Ala His Phe
        435                 440                 445

Phe Ser Pro Ala His Ile Met Pro Leu Leu Glu Ile Val Arg Thr Glu

450                 455                 460

Lys Thr Ser Pro Gln Ala Ile Leu Asp Leu Ile Thr Ile Gly Lys Ile
465                 470                 475                 480

Ile Lys Lys Val Pro Ile Val Val Gly Asn Cys Thr Gly Phe Ala Val
                485                 490                 495

Asn Leu Thr Phe Phe Pro Tyr Thr Gln Gly Ser His Leu Leu Val Ser
            500                 505                 510

Leu Gly Ile Asp Val Phe Arg Ile Asp Arg Val Ile Ser Thr Phe Gly
        515                 520                 525

Met Pro Met Gly Pro Phe Gln Leu Gln Asp Val Ala Gly Tyr Gly Val
    530                 535                 540

Ala Leu Ala Val Lys Asp Ile Tyr Ala Asp Ala Phe Gly Glu Arg Asn
545                 550                 555                 560

Leu Asp Ser Asp Leu Val Asp Leu Met Val Lys Asp Gly Arg Gln Gly
                565                 570                 575

Lys Val Asn Gly Lys Gly Tyr Tyr Ile Tyr Glu Lys Gly Gly Lys Pro
            580                 585                 590

Lys Pro Asp Pro Ser Val Lys His Val Ile Glu Glu Tyr Arg Lys His
        595                 600                 605

Ala Asn Thr Met Pro Gly Gly Lys Pro Val Thr Leu Thr Asp Gln Asp
    610                 615                 620

Ile Leu Glu Met Ile Phe Phe Pro Val Val Asn Glu Ala Cys Arg Val
625                 630                 635                 640

Met Asp Glu Asn Val Val Ile Arg Ala Ser Asp Leu Asp Ile Ala Ser
                645                 650                 655

Val Leu Gly Met Gly Phe Pro Lys Tyr Arg Gly Gly Leu Val Phe Trp
            660                 665                 670

Ala Asp Thr Val Gly Ala Pro Tyr Ile His Ser Lys Leu Ser Lys Trp
        675                 680                 685

Ala Glu Ile Tyr Gly Pro Phe Phe Lys Pro Ser Ser Tyr Leu Glu Gln
    690                 695                 700

Arg Ala Lys Ser Gly Val Pro Leu Ser Ala Pro Gly Ala Ser Gln Gln
705                 710                 715                 720

Gly Ser Ala Arg Ser Arg Met
                725

<210>  27
<211>  861
<212>  DNA
<213>  Escherichia coli

<220>
<221>  CDS
<222>  (1)..(861)
<223>  Acyl-CoA thioesterase (B0452)

<400>  27
atg agt cag gcg cta aaa aat tta ctg aca ttg tta aat ctg gaa aaa      48
Met Ser Gln Ala Leu Lys Asn Leu Leu Thr Leu Leu Asn Leu Glu Lys
1               5                   10                  15

att gag gaa gga ctc ttt cgc ggc cag agt gaa gat tta ggt tta cgc      96
Ile Glu Glu Gly Leu Phe Arg Gly Gln Ser Glu Asp Leu Gly Leu Arg
            20                  25                  30

cag gtg ttt ggc ggc cag gtc gtg ggt cag gcc ttg tat gct gca aaa      144
Gln Val Phe Gly Gly Gln Val Val Gly Gln Ala Leu Tyr Ala Ala Lys
        35                  40                  45

gag acc gtc cct gaa gag cgg ctg gta cat tcg ttt cac agc tac ttt      192
Glu Thr Val Pro Glu Glu Arg Leu Val His Ser Phe His Ser Tyr Phe
    50                  55                  60

ctt cgc cct ggc gat agt aag aag ccg att att tat gat gtc gaa acg      240
Leu Arg Pro Gly Asp Ser Lys Lys Pro Ile Ile Tyr Asp Val Glu Thr
65                  70                  75                  80

ctg cgt gac ggt aac agc ttc agc gcc cgc cgg gtt gct gct att caa      288
Leu Arg Asp Gly Asn Ser Phe Ser Ala Arg Arg Val Ala Ala Ile Gln
                85                  90                  95

aac ggc aaa ccg att ttt tat atg act gcc tct ttc cag gca cca gaa      336
Asn Gly Lys Pro Ile Phe Tyr Met Thr Ala Ser Phe Gln Ala Pro Glu
            100                 105                 110

gcg ggt ttc gaa cat caa aaa aca atg ccg tcc gcg cca gcg cct gat      384
Ala Gly Phe Glu His Gln Lys Thr Met Pro Ser Ala Pro Ala Pro Asp
        115                 120                 125

ggc ctc cct tcg gaa acg caa atc gcc caa tcg ctg gcg cac ctg ctg      432
Gly Leu Pro Ser Glu Thr Gln Ile Ala Gln Ser Leu Ala His Leu Leu
    130                 135                 140

ccg cca gtg ctg aaa gat aaa ttc atc tgc gat cgt ccg ctg gaa gtc      480
Pro Pro Val Leu Lys Asp Lys Phe Ile Cys Asp Arg Pro Leu Glu Val
145                 150                 155                 160

cgt ccg gtg gag ttt cat aac cca ctg aaa ggt cac gtc gca gaa cca      528
Arg Pro Val Glu Phe His Asn Pro Leu Lys Gly His Val Ala Glu Pro
                165                 170                 175

cat cgt cag gtg tgg atc cgc gca aat ggt agc gtg ccg gat gac ctg      576
His Arg Gln Val Trp Ile Arg Ala Asn Gly Ser Val Pro Asp Asp Leu
            180                 185                 190

cgc gtt cat cag tat ctg ctc ggt tac gct tct gat ctt aac ttc ctg      624
Arg Val His Gln Tyr Leu Leu Gly Tyr Ala Ser Asp Leu Asn Phe Leu
        195                 200                 205

ccg gta gct cta cag ccg cac ggc atc ggt ttt ctc gaa ccg ggg att      672
Pro Val Ala Leu Gln Pro His Gly Ile Gly Phe Leu Glu Pro Gly Ile
    210                 215                 220

cag att gcc acc att gac cat tcc atg tgg ttc cat cgc ccg ttt aat 720
Gln Ile Ala Thr Ile Asp His Ser Met Trp Phe His Arg Pro Phe Asn
225                 230                 235                 240

ttg aat gaa tgg ctg ctg tat agc gtg gag agc acc tcg gcg tcc agc 768
Leu Asn Glu Trp Leu Leu Tyr Ser Val Glu Ser Thr Ser Ala Ser Ser
                245                 250                 255

gca cgt ggc ttt gtg cgc ggt gag ttt tat acc caa gac ggc gta ctg 816
Ala Arg Gly Phe Val Arg Gly Glu Phe Tyr Thr Gln Asp Gly Val Leu
            260                 265                 270

gtt gcc tcg acc gtt cag gaa ggg gtg atg cgt aat cac aat taa 861
Val Ala Ser Thr Val Gln Glu Gly Val Met Arg Asn His Asn
        275                 280                 285

<210> 28
<211> 286
<212> PRT
<213> Escherichia coli

<400> 28

Met Ser Gln Ala Leu Lys Asn Leu Leu Thr Leu Leu Asn Leu Glu Lys
1               5                   10                  15

Ile Glu Glu Gly Leu Phe Arg Gly Gln Ser Glu Asp Leu Gly Leu Arg
            20                  25                  30

Gln Val Phe Gly Gly Gln Val Val Gly Gln Ala Leu Tyr Ala Ala Lys
        35                  40                  45

Glu Thr Val Pro Glu Glu Arg Leu Val His Ser Phe His Ser Tyr Phe
    50                  55                  60

Leu Arg Pro Gly Asp Ser Lys Lys Pro Ile Ile Tyr Asp Val Glu Thr
65                  70                  75                  80

Leu Arg Asp Gly Asn Ser Phe Ser Ala Arg Arg Val Ala Ala Ile Gln
                85                  90                  95

Asn Gly Lys Pro Ile Phe Tyr Met Thr Ala Ser Phe Gln Ala Pro Glu
            100                 105                 110

Ala Gly Phe Glu His Gln Lys Thr Met Pro Ser Ala Pro Ala Pro Asp
        115                 120                 125

Gly Leu Pro Ser Glu Thr Gln Ile Ala Gln Ser Leu Ala His Leu Leu
    130                 135                 140

Pro Pro Val Leu Lys Asp Lys Phe Ile Cys Asp Arg Pro Leu Glu Val
145                 150                 155                 160

Arg Pro Val Glu Phe His Asn Pro Leu Lys Gly His Val Ala Glu Pro
                165                 170                 175

His Arg Gln Val Trp Ile Arg Ala Asn Gly Ser Val Pro Asp Asp Leu
            180                 185                 190

Arg Val His Gln Tyr Leu Leu Gly Tyr Ala Ser Asp Leu Asn Phe Leu
        195                 200                 205

Pro Val Ala Leu Gln Pro His Gly Ile Gly Phe Leu Glu Pro Gly Ile
    210                 215                 220

Gln Ile Ala Thr Ile Asp His Ser Met Trp Phe His Arg Pro Phe Asn
225                 230                 235                 240

Leu Asn Glu Trp Leu Leu Tyr Ser Val Glu Ser Thr Ser Ala Ser Ser
                245                 250                 255

Ala Arg Gly Phe Val Arg Gly Glu Phe Tyr Thr Gln Asp Gly Val Leu
            260                 265                 270

Val Ala Ser Thr Val Gln Glu Gly Val Met Arg Asn His Asn
        275                 280                 285

<210>  29
<211>  747
<212>  DNA
<213>  Brassica napus

<220>
<221>  CDS
<222>  (1)..(747)
<223>  Acyl-CoA thioesterase (BN42634969)

<400>  29
atg aag tta gtc cat agt tta cac gcc tat ttc ctt ctt tct gga gat      48
Met Lys Leu Val His Ser Leu His Ala Tyr Phe Leu Leu Ser Gly Asp
1               5                   10                  15

act aat att ccc atc ata tat gaa gtt agc cgc tta cgc gat ggc aac      96
Thr Asn Ile Pro Ile Ile Tyr Glu Val Ser Arg Leu Arg Asp Gly Asn
            20                  25                  30

aat ttt gcc acc cga aga gta gat gca aga cag aaa gga aaa acc ata      144
Asn Phe Ala Thr Arg Arg Val Asp Ala Arg Gln Lys Gly Lys Thr Ile
        35                  40                  45

ttc acc ttg ttc gca tca ttt cag aga gag caa caa ggt ttt gat cac      192
Phe Thr Leu Phe Ala Ser Phe Gln Arg Glu Gln Gln Gly Phe Asp His
    50                  55                  60

cag gag tcg aac atg cct cat atg cta cct cct gaa acg ctt gta tca      240
Gln Glu Ser Asn Met Pro His Met Leu Pro Pro Glu Thr Leu Val Ser
65                  70                  75                  80

agg gat gaa atg ctt caa cgg cgt atg act gac cat ctg ata cct agg      288
Arg Asp Glu Met Leu Gln Arg Arg Met Thr Asp His Leu Ile Pro Arg
                85                  90                  95

tat tac cga aat aaa gtt gca acc caa tat act gcc cca ctg cct ata      336
Tyr Tyr Arg Asn Lys Val Ala Thr Gln Tyr Thr Ala Pro Leu Pro Ile
            100                 105                 110

gat att cga ttt tgt gag cca aat tac tct aca gaa gag aga aag tct      384

Asp Ile Arg Phe Cys Glu Pro Asn Tyr Ser Thr Glu Glu Arg Lys Ser
        115                 120                 125

cct tca aga ttg aac tat tgg ttt aag gca agg gga aaa ctt tct gat     432
Pro Ser Arg Leu Asn Tyr Trp Phe Lys Ala Arg Gly Lys Leu Ser Asp
    130                 135                 140

gac caa gct tta cac cga tgt gtg gtt gca ttt gct tca gat ctg ata     480
Asp Gln Ala Leu His Arg Cys Val Val Ala Phe Ala Ser Asp Leu Ile
145                 150                 155                 160

ttt gca tgt atc ggt tta aac cct cac cgt aga aag ggc atg agt gca     528
Phe Ala Cys Ile Gly Leu Asn Pro His Arg Arg Lys Gly Met Ser Ala
                165                 170                 175

gct gct ctt agc cta gac cac tcg ttg tgg ttc cac cga cct cta aga     576
Ala Ala Leu Ser Leu Asp His Ser Leu Trp Phe His Arg Pro Leu Arg
            180                 185                 190

gct gat gac tgg ctt ctc ttt gtg atg gtg aat ccc aca tca ttc cag     624
Ala Asp Asp Trp Leu Leu Phe Val Met Val Asn Pro Thr Ser Phe Gln
        195                 200                 205

agt cgt ggt ctg acc act gga gaa atg ttc aac aga aaa gga gag cta     672
Ser Arg Gly Leu Thr Thr Gly Glu Met Phe Asn Arg Lys Gly Glu Leu
    210                 215                 220

gtg gta tca ttg acg caa gaa gca ttg cta aaa gaa gcg gtg acg att     720
Val Val Ser Leu Thr Gln Glu Ala Leu Leu Lys Glu Ala Val Thr Ile
225                 230                 235                 240

aag ccc atc ttc ggc gcc aag ctc taa                                 747
Lys Pro Ile Phe Gly Ala Lys Leu
                245


<210>  30
<211>  248
<212>  PRT
<213>  Brassica napus

<400>  30

Met Lys Leu Val His Ser Leu His Ala Tyr Phe Leu Leu Ser Gly Asp
1               5                   10                  15


Thr Asn Ile Pro Ile Ile Tyr Glu Val Ser Arg Leu Arg Asp Gly Asn
            20                  25                  30


Asn Phe Ala Thr Arg Arg Val Asp Ala Arg Gln Lys Gly Lys Thr Ile
        35                  40                  45


Phe Thr Leu Phe Ala Ser Phe Gln Arg Glu Gln Gln Gly Phe Asp His
    50                  55                  60


Gln Glu Ser Asn Met Pro His Met Leu Pro Pro Glu Thr Leu Val Ser
65                  70                  75                  80


Arg Asp Glu Met Leu Gln Arg Arg Met Thr Asp His Leu Ile Pro Arg
                85                  90                  95


Tyr Tyr Arg Asn Lys Val Ala Thr Gln Tyr Thr Ala Pro Leu Pro Ile
            100                 105                 110

Asp Ile Arg Phe Cys Glu Pro Asn Tyr Ser Thr Glu Glu Arg Lys Ser
        115                 120                 125

Pro Ser Arg Leu Asn Tyr Trp Phe Lys Ala Arg Gly Lys Leu Ser Asp
    130                 135                 140

Asp Gln Ala Leu His Arg Cys Val Val Ala Phe Ala Ser Asp Leu Ile
145                 150                 155                 160

Phe Ala Cys Ile Gly Leu Asn Pro His Arg Arg Lys Gly Met Ser Ala
                165                 170                 175

Ala Ala Leu Ser Leu Asp His Ser Leu Trp Phe His Arg Pro Leu Arg
            180                 185                 190

Ala Asp Asp Trp Leu Leu Phe Val Met Val Asn Pro Thr Ser Phe Gln
        195                 200                 205

Ser Arg Gly Leu Thr Thr Gly Glu Met Phe Asn Arg Lys Gly Glu Leu
    210                 215                 220

Val Val Ser Leu Thr Gln Glu Ala Leu Leu Lys Glu Ala Val Thr Ile
225                 230                 235                 240

Lys Pro Ile Phe Gly Ala Lys Leu
                245

<210>  31
<211>  639
<212>  DNA
<213>  Brassica napus

<220>
<221>  CDS
<222>  (1)..(639)
<223>  Acyl-CoA thioesterase (BNP5302_30)

<400>  31
atg tct cat cat tgt tct ttg ctt gac acc aac cct gtc tcc gat cca      48
Met Ser His His Cys Ser Leu Leu Asp Thr Asn Pro Val Ser Asp Pro
1               5                   10                  15

gct aaa gat ctt gag acc ccc tgt ctt gtg gaa cgc att ttg tct ctc      96
Ala Lys Asp Leu Glu Thr Pro Cys Leu Val Glu Arg Ile Leu Ser Leu
            20                  25                  30

cat cca tta tat ttg act ctt ttt cga ggt ttc act ata ccc aat gct      144
His Pro Leu Tyr Leu Thr Leu Phe Arg Gly Phe Thr Ile Pro Asn Ala
        35                  40                  45

ata acc ttt ggc aag gtt ttt gga cga caa tta gtt gga cag gca ctt      192
Ile Thr Phe Gly Lys Val Phe Gly Arg Gln Leu Val Gly Gln Ala Leu
    50                  55                  60

gcc gca ccg aca aac act gat gaa tct acc aag att gtt cat agt tta      240
Ala Ala Pro Thr Asn Thr Asp Glu Ser Thr Lys Ile Val His Ser Leu
65                  70                  75                  80

cac tcc tat ttc cta ctc gtt gga gat ata act att ccc atc cta tac 288
His Ser Tyr Phe Leu Leu Val Gly Asp Ile Thr Ile Pro Ile Leu Tyr
                85                  90                  95

gaa gtt aac cac tta cgt gat ggc aac aac ttt gcc acc cga aga gtt 336
Glu Val Asn His Leu Arg Asp Gly Asn Asn Phe Ala Thr Arg Arg Val
            100                 105                 110

gat gct aga cag aaa ggc aaa acc att ttc atc ttg ttt gct tca ttt 384
Asp Ala Arg Gln Lys Gly Lys Thr Ile Phe Ile Leu Phe Ala Ser Phe
        115                 120                 125

cag aga gat caa caa ggt ttc gat cac caa gag tcg aac atg cct ctt 432
Gln Arg Asp Gln Gln Gly Phe Asp His Gln Glu Ser Asn Met Pro Leu
    130                 135                 140

atg tca cct cct gaa acg ctt gta aca aga gag gag atg att gaa cgg 480
Met Ser Pro Pro Glu Thr Leu Val Thr Arg Glu Glu Met Ile Glu Arg
145                 150                 155                 160

cgt atg act gac cct ctg cta cct agg gat tac cga aac aaa att gca 528
Arg Met Thr Asp Pro Leu Leu Pro Arg Asp Tyr Arg Asn Lys Ile Ala
                165                 170                 175

gct gaa aaa atc ctc aca tgg cct ata gac att cga ttt tgt gag cca 576
Ala Glu Lys Ile Leu Thr Trp Pro Ile Asp Ile Arg Phe Cys Glu Pro
            180                 185                 190

agt tat tat aca gaa cat aca aag tct cct cca aga ttg aac tat tgg 624
Ser Tyr Tyr Thr Glu His Thr Lys Ser Pro Pro Arg Leu Asn Tyr Trp
        195                 200                 205

ttt aag gca agg tga 639
Phe Lys Ala Arg
    210

<210> 32
<211> 212
<212> PRT
<213> Brassica napus

<400> 32

Met Ser His His Cys Ser Leu Leu Asp Thr Asn Pro Val Ser Asp Pro
1               5                   10                  15

Ala Lys Asp Leu Glu Thr Pro Cys Leu Val Glu Arg Ile Leu Ser Leu
            20                  25                  30

His Pro Leu Tyr Leu Thr Leu Phe Arg Gly Phe Thr Ile Pro Asn Ala
        35                  40                  45

Ile Thr Phe Gly Lys Val Phe Gly Arg Gln Leu Val Gly Gln Ala Leu
    50                  55                  60

Ala Ala Pro Thr Asn Thr Asp Glu Ser Thr Lys Ile Val His Ser Leu
65                  70                  75                  80

His Ser Tyr Phe Leu Leu Val Gly Asp Ile Thr Ile Pro Ile Leu Tyr
                85                  90                  95

Glu Val Asn His Leu Arg Asp Gly Asn Asn Phe Ala Thr Arg Arg Val
            100                 105                 110

Asp Ala Arg Gln Lys Gly Lys Thr Ile Phe Ile Leu Phe Ala Ser Phe
        115                 120                 125

Gln Arg Asp Gln Gln Gly Phe Asp His Gln Glu Ser Asn Met Pro Leu
    130                 135                 140

Met Ser Pro Pro Glu Thr Leu Val Thr Arg Glu Glu Met Ile Glu Arg
145                 150                 155                 160

Arg Met Thr Asp Pro Leu Leu Pro Arg Asp Tyr Arg Asn Lys Ile Ala
                165                 170                 175

Ala Glu Lys Ile Leu Thr Trp Pro Ile Asp Ile Arg Phe Cys Glu Pro
            180                 185                 190

Ser Tyr Tyr Thr Glu His Thr Lys Ser Pro Pro Arg Leu Asn Tyr Trp
        195                 200                 205

Phe Lys Ala Arg
    210

<210>  33
<211>  594
<212>  DNA
<213>  Glycine max

<220>
<221>  CDS
<222>  (1)..(594)
<223>  Acyl-CoA thioesterase (GMsae90f11)

<400>  33
atg gct tca ttt caa aaa gaa gaa tca ggg atg gtc cac caa gag gta      48
Met Ala Ser Phe Gln Lys Glu Glu Ser Gly Met Val His Gln Glu Val
1               5                   10                  15

gct ata cca tct gtc cct gct cca gat aag ctt ctg ccg atg gaa gag      96
Ala Ile Pro Ser Val Pro Ala Pro Asp Lys Leu Leu Pro Met Glu Glu
            20                  25                  30

cta cgg gag aga cgt ctt act gac cct cgt tta cca ata acc tat cgg      144
Leu Arg Glu Arg Arg Leu Thr Asp Pro Arg Leu Pro Ile Thr Tyr Arg
        35                  40                  45

aac aaa gta gct aca tct caa ttc atc cca tgg ccc ata gag ata cga      192
Asn Lys Val Ala Thr Ser Gln Phe Ile Pro Trp Pro Ile Glu Ile Arg
    50                  55                  60

tta tgt gaa tat gaa act gca aca aat atg aca aaa tct cct ccc agt      240
Leu Cys Glu Tyr Glu Thr Ala Thr Asn Met Thr Lys Ser Pro Pro Ser
65                  70                  75                  80

ttg aga tac tgg ttt aga gcc aag gga aaa ctt tca gat gat caa gcc      288
Leu Arg Tyr Trp Phe Arg Ala Lys Gly Lys Leu Ser Asp Asp Gln Ala
                85                  90                  95

ttg cat agg tgt gtg gta gca tat aca tca gat cta atc ttc ctt caa      336

Leu His Arg Cys Val Val Ala Tyr Thr Ser Asp Leu Ile Phe Leu Gln
            100                 105                 110

gtg agt ttg aac ccc aac cgt agg aag gga agg aag gct cgt gct gtg        384
Val Ser Leu Asn Pro Asn Arg Arg Lys Gly Arg Lys Ala Arg Ala Val
        115                 120                 125

agt ctg gac cac tcc atg tgg ttt cac aga cct tta aga gct gat gat        432
Ser Leu Asp His Ser Met Trp Phe His Arg Pro Leu Arg Ala Asp Asp
    130                 135                 140

tgg ata cta ttt gtg atc ttt agt cct act gcc aat aat gcc cgc ggc        480
Trp Ile Leu Phe Val Ile Phe Ser Pro Thr Ala Asn Asn Ala Arg Gly
145                 150                 155                 160

tat gtc act ggc caa atg ttc aat cag aag gga gag cat ctt gtg tct        528
Tyr Val Thr Gly Gln Met Phe Asn Gln Lys Gly Glu His Leu Val Ser
                165                 170                 175

gtg gtt caa gaa ggt gta atg agg gaa gtt att tct gct aag tca gcc        576
Val Val Gln Glu Gly Val Met Arg Glu Val Ile Ser Ala Lys Ser Ala
            180                 185                 190

atc aaa tct aat cta tga                                                594
Ile Lys Ser Asn Leu
        195


<210>  34
<211>  197
<212>  PRT
<213>  Glycine max

<400>  34

Met Ala Ser Phe Gln Lys Glu Glu Ser Gly Met Val His Gln Glu Val
1               5                   10                  15


Ala Ile Pro Ser Val Pro Ala Pro Asp Lys Leu Leu Pro Met Glu Glu
            20                  25                  30


Leu Arg Glu Arg Arg Leu Thr Asp Pro Arg Leu Pro Ile Thr Tyr Arg
        35                  40                  45


Asn Lys Val Ala Thr Ser Gln Phe Ile Pro Trp Pro Ile Glu Ile Arg
    50                  55                  60


Leu Cys Glu Tyr Glu Thr Ala Thr Asn Met Thr Lys Ser Pro Pro Ser
65                  70                  75                  80


Leu Arg Tyr Trp Phe Arg Ala Lys Gly Lys Leu Ser Asp Asp Gln Ala
                85                  90                  95


Leu His Arg Cys Val Val Ala Tyr Thr Ser Asp Leu Ile Phe Leu Gln
            100                 105                 110


Val Ser Leu Asn Pro Asn Arg Arg Lys Gly Arg Lys Ala Arg Ala Val
        115                 120                 125


Ser Leu Asp His Ser Met Trp Phe His Arg Pro Leu Arg Ala Asp Asp
    130                 135                 140

Trp Ile Leu Phe Val Ile Phe Ser Pro Thr Ala Asn Asn Ala Arg Gly
145                 150                 155                 160

Tyr Val Thr Gly Gln Met Phe Asn Gln Lys Gly Glu His Leu Val Ser
                165                 170                 175

Val Val Gln Glu Gly Val Met Arg Glu Val Ile Ser Ala Lys Ser Ala
            180                 185                 190

Ile Lys Ser Asn Leu
        195

<210>  35
<211>  888
<212>  DNA
<213>  Saccharomyces cerevisiae

<220>
<221>  CDS
<222>  (1)..(888)
<223>  2,4-dienoyl-CoA reductase (YNL202W)

<400>  35
atg gat act atg aat aca gca aac act ttg gac ggc aaa ttc gtt act      48
Met Asp Thr Met Asn Thr Ala Asn Thr Leu Asp Gly Lys Phe Val Thr
1               5                   10                  15

gag ggt tct tgg aga cct gat tta ttt aag ggt aaa gtg gca ttt gtc      96
Glu Gly Ser Trp Arg Pro Asp Leu Phe Lys Gly Lys Val Ala Phe Val
            20                  25                  30

act ggt gga gct ggc acg ata tgt cgg gta cag aca gaa gcc ttg gtt      144
Thr Gly Gly Ala Gly Thr Ile Cys Arg Val Gln Thr Glu Ala Leu Val
        35                  40                  45

ctt ctt ggc tgt aag gca gct att gtc ggc agg gac caa gaa aga aca      192
Leu Leu Gly Cys Lys Ala Ala Ile Val Gly Arg Asp Gln Glu Arg Thr
    50                  55                  60

gaa cag gca gca aaa ggt att tcg cag tta gca aag gac aaa gat gca      240
Glu Gln Ala Ala Lys Gly Ile Ser Gln Leu Ala Lys Asp Lys Asp Ala
65                  70                  75                  80

gtc ttg gct att gca aac gtc gat gtt cgc aat ttt gaa caa gtg gag      288
Val Leu Ala Ile Ala Asn Val Asp Val Arg Asn Phe Glu Gln Val Glu
                85                  90                  95

aat gcg gtt aaa aag acc gta gag aag ttc ggt aag atc gat ttc gtc      336
Asn Ala Val Lys Lys Thr Val Glu Lys Phe Gly Lys Ile Asp Phe Val
            100                 105                 110

att gcc ggt gct gct gga aat ttt gtg tgc gat ttt gcg aac ctc tct      384
Ile Ala Gly Ala Ala Gly Asn Phe Val Cys Asp Phe Ala Asn Leu Ser
        115                 120                 125

cca aac gcc ttc aaa tct gtt gtt gac ata gat tta cta gga agt ttt      432
Pro Asn Ala Phe Lys Ser Val Val Asp Ile Asp Leu Leu Gly Ser Phe
    130                 135                 140

aat acc gcc aag gca tgc ttg aag gag tta aaa aag tca aaa ggt tcc      480
Asn Thr Ala Lys Ala Cys Leu Lys Glu Leu Lys Lys Ser Lys Gly Ser
145                 150                 155                 160

atc ctt ttt gtc tct gcc act ttc cat tat tat ggt gtg ccg ttt caa          528
Ile Leu Phe Val Ser Ala Thr Phe His Tyr Tyr Gly Val Pro Phe Gln
                165                 170                 175

gga cat gtg ggc gct gct aaa gcg gga ata gac gca ttg gca aag aat          576
Gly His Val Gly Ala Ala Lys Ala Gly Ile Asp Ala Leu Ala Lys Asn
            180                 185                 190

tta gcg gtt gag ttg ggg cct ctg ggt ata cgt tcg aat tgc att gct          624
Leu Ala Val Glu Leu Gly Pro Leu Gly Ile Arg Ser Asn Cys Ile Ala
        195                 200                 205

cca ggt gct atc gat aat acg gaa ggc ttg aag aga tta gcc ggc aaa          672
Pro Gly Ala Ile Asp Asn Thr Glu Gly Leu Lys Arg Leu Ala Gly Lys
    210                 215                 220

aaa tat aag gaa aag gcc ttg gca aag ata cca tta caa agg ctt gga          720
Lys Tyr Lys Glu Lys Ala Leu Ala Lys Ile Pro Leu Gln Arg Leu Gly
225                 230                 235                 240

tct aca cgg gat ata gcc gaa tct act gtt tat att ttt tca ccc gcc          768
Ser Thr Arg Asp Ile Ala Glu Ser Thr Val Tyr Ile Phe Ser Pro Ala
                245                 250                 255

gca tcg tat gtc acc ggt gcc gtc tta gtt gtt gac ggt ggt atg tgg          816
Ala Ser Tyr Val Thr Gly Ala Val Leu Val Val Asp Gly Gly Met Trp
            260                 265                 270

cat tta ggg acc tat ttt gga cat gag ttg tat cca gaa gcc tta ata          864
His Leu Gly Thr Tyr Phe Gly His Glu Leu Tyr Pro Glu Ala Leu Ile
        275                 280                 285

aag agt atg aca tct aaa tta taa                                          888
Lys Ser Met Thr Ser Lys Leu
    290                 295


<210>  36
<211>  295
<212>  PRT
<213>  Saccharomyces cerevisiae

<400>  36

Met Asp Thr Met Asn Thr Ala Asn Thr Leu Asp Gly Lys Phe Val Thr
1               5                   10                  15


Glu Gly Ser Trp Arg Pro Asp Leu Phe Lys Gly Lys Val Ala Phe Val
            20                  25                  30


Thr Gly Gly Ala Gly Thr Ile Cys Arg Val Gln Thr Glu Ala Leu Val
        35                  40                  45


Leu Leu Gly Cys Lys Ala Ala Ile Val Gly Arg Asp Gln Glu Arg Thr
    50                  55                  60


Glu Gln Ala Ala Lys Gly Ile Ser Gln Leu Ala Lys Asp Lys Asp Ala
65                  70                  75                  80


Val Leu Ala Ile Ala Asn Val Asp Val Arg Asn Phe Glu Gln Val Glu
                85                  90                  95

Asn Ala Val Lys Lys Thr Val Glu Lys Phe Gly Lys Ile Asp Phe Val
            100                 105                 110

Ile Ala Gly Ala Ala Gly Asn Phe Val Cys Asp Phe Ala Asn Leu Ser
        115                 120                 125

Pro Asn Ala Phe Lys Ser Val Val Asp Ile Asp Leu Leu Gly Ser Phe
    130                 135                 140

Asn Thr Ala Lys Ala Cys Leu Lys Glu Leu Lys Lys Ser Lys Gly Ser
145                 150                 155                 160

Ile Leu Phe Val Ser Ala Thr Phe His Tyr Tyr Gly Val Pro Phe Gln
                165                 170                 175

Gly His Val Gly Ala Ala Lys Ala Gly Ile Asp Ala Leu Ala Lys Asn
            180                 185                 190

Leu Ala Val Glu Leu Gly Pro Leu Gly Ile Arg Ser Asn Cys Ile Ala
        195                 200                 205

Pro Gly Ala Ile Asp Asn Thr Glu Gly Leu Lys Arg Leu Ala Gly Lys
    210                 215                 220

Lys Tyr Lys Glu Lys Ala Leu Ala Lys Ile Pro Leu Gln Arg Leu Gly
225                 230                 235                 240

Ser Thr Arg Asp Ile Ala Glu Ser Thr Val Tyr Ile Phe Ser Pro Ala
                245                 250                 255

Ala Ser Tyr Val Thr Gly Ala Val Leu Val Val Asp Gly Gly Met Trp
            260                 265                 270

His Leu Gly Thr Tyr Phe Gly His Glu Leu Tyr Pro Glu Ala Leu Ile
        275                 280                 285

Lys Ser Met Thr Ser Lys Leu
    290                 295

<210>  37
<211>  795
<212>  DNA
<213>  Helianthus annuus

<220>
<221>  CDS
<222>  (1)..(795)
<223>  2,4-dienoyl-CoA reductase (HA66688442)

<400>  37
atg gcg ctt cca tcg ctg tca tgg gac gtc gca aaa ccg tcg ttg act        48
Met Ala Leu Pro Ser Leu Ser Trp Asp Val Ala Lys Pro Ser Leu Thr
1               5                   10                  15

ctg ccg act cgt ccc tcc agt cac ttg gta ttc cgg ctg tcg gct ttt        96

Leu Pro Thr Arg Pro Ser Ser His Leu Val Phe Arg Leu Ser Ala Phe
            20                  25                  30

gcg ggt gat gtt cgc aag cag gaa gat gca aag aga gtg ata gaa tca         144
Ala Gly Asp Val Arg Lys Gln Glu Asp Ala Lys Arg Val Ile Glu Ser
        35                  40                  45

acc gtt aaa cac ttt gga aag ctc gat att ctt gtg aac tct gca gcc         192
Thr Val Lys His Phe Gly Lys Leu Asp Ile Leu Val Asn Ser Ala Ala
    50                  55                  60

ggg aat ttt ctg gtt tca ccc gaa gat cta tct cca aat ggc ttt aaa         240
Gly Asn Phe Leu Val Ser Pro Glu Asp Leu Ser Pro Asn Gly Phe Lys
65                  70                  75                  80

aca gtg atg gac atc gac tcg gtt ggt acg ttt acc atg tgc cat gaa         288
Thr Val Met Asp Ile Asp Ser Val Gly Thr Phe Thr Met Cys His Glu
                85                  90                  95

gca atc aat tat cta aag aaa gga gga ccc gga aga agc tca gat gac         336
Ala Ile Asn Tyr Leu Lys Lys Gly Gly Pro Gly Arg Ser Ser Asp Asp
            100                 105                 110

agt ggt gga ata ata ctg aat atc agt gcc acc ctg cac tac act gct         384
Ser Gly Gly Ile Ile Leu Asn Ile Ser Ala Thr Leu His Tyr Thr Ala
        115                 120                 125

act tgg tac caa att cat gta gct gct gct aag gca gca gtg gat gct         432
Thr Trp Tyr Gln Ile His Val Ala Ala Ala Lys Ala Ala Val Asp Ala
    130                 135                 140

att aca aga aat ctg gca ctg gaa tgg gga acg gat tat gat ata cgc         480
Ile Thr Arg Asn Leu Ala Leu Glu Trp Gly Thr Asp Tyr Asp Ile Arg
145                 150                 155                 160

gtc aat ggg att gca cct gga ccg ata ggc gac act gct ggc gtg agg         528
Val Asn Gly Ile Ala Pro Gly Pro Ile Gly Asp Thr Ala Gly Val Arg
                165                 170                 175

aaa ctt ggg cca gct gag ata aaa agc ggg acc agg gag ata atg ccg         576
Lys Leu Gly Pro Ala Glu Ile Lys Ser Gly Thr Arg Glu Ile Met Pro
            180                 185                 190

ctt ttt aaa ctt gga gaa aag tgg gat att gcg gtt gca gcc gtg tat         624
Leu Phe Lys Leu Gly Glu Lys Trp Asp Ile Ala Val Ala Ala Val Tyr
        195                 200                 205

ctt gca tca gat gcg ggt aaa ttt gtg aat ggg acg acg ctt gtt gtt         672
Leu Ala Ser Asp Ala Gly Lys Phe Val Asn Gly Thr Thr Leu Val Val
    210                 215                 220

gat gga gga cag tgg tta agt ggt ccg cgt cat atg ccc aag gag gaa         720
Asp Gly Gly Gln Trp Leu Ser Gly Pro Arg His Met Pro Lys Glu Glu
225                 230                 235                 240

gtg aaa atg ctt tct agg gta gta gaa aaa cgc gct cgt gtt gca cct         768
Val Lys Met Leu Ser Arg Val Val Glu Lys Arg Ala Arg Val Ala Pro
                245                 250                 255

act ggg gta cct tct agc aaa ctc tag                                     795
Thr Gly Val Pro Ser Ser Lys Leu
            260

<210>  38
<211>  264
<212>  PRT
<213>  Helianthus annuus

<400>  38

Met Ala Leu Pro Ser Leu Ser Trp Asp Val Ala Lys Pro Ser Leu Thr
1               5                   10                  15

Leu Pro Thr Arg Pro Ser Ser His Leu Val Phe Arg Leu Ser Ala Phe
            20                  25                  30

Ala Gly Asp Val Arg Lys Gln Glu Asp Ala Lys Arg Val Ile Glu Ser
        35                  40                  45

Thr Val Lys His Phe Gly Lys Leu Asp Ile Leu Val Asn Ser Ala Ala
    50                  55                  60

Gly Asn Phe Leu Val Ser Pro Glu Asp Leu Ser Pro Asn Gly Phe Lys
65                  70                  75                  80

Thr Val Met Asp Ile Asp Ser Val Gly Thr Phe Thr Met Cys His Glu
                85                  90                  95

Ala Ile Asn Tyr Leu Lys Lys Gly Gly Pro Gly Arg Ser Ser Asp Asp
            100                 105                 110

Ser Gly Gly Ile Ile Leu Asn Ile Ser Ala Thr Leu His Tyr Thr Ala
        115                 120                 125

Thr Trp Tyr Gln Ile His Val Ala Ala Ala Lys Ala Ala Val Asp Ala
    130                 135                 140

Ile Thr Arg Asn Leu Ala Leu Glu Trp Gly Thr Asp Tyr Asp Ile Arg
145                 150                 155                 160

Val Asn Gly Ile Ala Pro Gly Pro Ile Gly Asp Thr Ala Gly Val Arg
                165                 170                 175

Lys Leu Gly Pro Ala Glu Ile Lys Ser Gly Thr Arg Glu Ile Met Pro
            180                 185                 190

Leu Phe Lys Leu Gly Glu Lys Trp Asp Ile Ala Val Ala Ala Val Tyr
        195                 200                 205

Leu Ala Ser Asp Ala Gly Lys Phe Val Asn Gly Thr Thr Leu Val Val
    210                 215                 220

Asp Gly Gly Gln Trp Leu Ser Gly Pro Arg His Met Pro Lys Glu Glu
225                 230                 235                 240

Val Lys Met Leu Ser Arg Val Val Glu Lys Arg Ala Arg Val Ala Pro
                245                 250                 255

Thr Gly Val Pro Ser Ser Lys Leu
            260

<210>  39
<211>  1647
<212>  DNA
<213>  Saccharomyces cerevisiae

<220>
<221>  CDS
<222>  (1)..(1647)
<223>  sterol esterase (YKL140W)

<400>  39
atg tac ttc ccc ttt tta ggc aga tta tcg ata aca gat tac att ata        48
Met Tyr Phe Pro Phe Leu Gly Arg Leu Ser Ile Thr Asp Tyr Ile Ile
1               5                   10                  15

gtc gtc ttg gta tac ata gaa agc att att tca tca gtc ctt aaa ctt        96
Val Val Leu Val Tyr Ile Glu Ser Ile Ile Ser Ser Val Leu Lys Leu
            20                  25                  30

ata cca caa cca atg att aac ctt ttc gaa tgg cta ata aat ttc tca       144
Ile Pro Gln Pro Met Ile Asn Leu Phe Glu Trp Leu Ile Asn Phe Ser
        35                  40                  45

acg tcc tcc gat gac aat acc atc gaa gaa aag tta agg tca gct cca       192
Thr Ser Ser Asp Asp Asn Thr Ile Glu Glu Lys Leu Arg Ser Ala Pro
    50                  55                  60

act atc cat gaa atg tgt gca att ttt gat ata tct gtc gag gat cat       240
Thr Ile His Glu Met Cys Ala Ile Phe Asp Ile Ser Val Glu Asp His
65                  70                  75                  80

ttg gta aga att gaa gac aat tat atc ttg aca ctg cat aga atc cca       288
Leu Val Arg Ile Glu Asp Asn Tyr Ile Leu Thr Leu His Arg Ile Pro
                85                  90                  95

ccg att tct aaa aac aga ttt aac aat aaa gtg gta tac tta cat cac       336
Pro Ile Ser Lys Asn Arg Phe Asn Asn Lys Val Val Tyr Leu His His
            100                 105                 110

ggt ctt ttg atg tgt tct gat gtt tgg tgt tgc aat att gaa aga cat       384
Gly Leu Leu Met Cys Ser Asp Val Trp Cys Cys Asn Ile Glu Arg His
        115                 120                 125

aaa aac tta ccg ttt gta ttg cat gat tta ggt tac gac gtc tgg atg       432
Lys Asn Leu Pro Phe Val Leu His Asp Leu Gly Tyr Asp Val Trp Met
    130                 135                 140

gga aat aat aga ggt aat aaa tat tca act gcc cac ttg aac aaa cca       480
Gly Asn Asn Arg Gly Asn Lys Tyr Ser Thr Ala His Leu Asn Lys Pro
145                 150                 155                 160

cca aaa tcg aac aag ttt tgg gat ttt tct atc gac gaa ttt gcg ttc       528
Pro Lys Ser Asn Lys Phe Trp Asp Phe Ser Ile Asp Glu Phe Ala Phe
                165                 170                 175

ttt gac att cca aac tca att gaa ttc atc tta gat ata aca aaa gtg       576
Phe Asp Ile Pro Asn Ser Ile Glu Phe Ile Leu Asp Ile Thr Lys Val
            180                 185                 190

gac aag gtc atc tgc atc gga ttc tct caa ggc tct gct caa atg ttt       624
Asp Lys Val Ile Cys Ile Gly Phe Ser Gln Gly Ser Ala Gln Met Phe
        195                 200                 205

gct gca ttt tcg ttg agt gaa aaa ttg aat cga aaa gtc tcc cat ttt       672
Ala Ala Phe Ser Leu Ser Glu Lys Leu Asn Arg Lys Val Ser His Phe
    210                 215                 220

ata gcc ata gca ccc gct atg act cca aag ggg ttg cac aac aga att 720
Ile Ala Ile Ala Pro Ala Met Thr Pro Lys Gly Leu His Asn Arg Ile
225                 230                 235                 240

gtc gat acc ttg gcc aaa tca tcg ccc ggc ttt atg tat ctt ttc ttt 768
Val Asp Thr Leu Ala Lys Ser Ser Pro Gly Phe Met Tyr Leu Phe Phe
                245                 250                 255

ggt agg aaa att gtg tta cct tca gct gtc att tgg caa aga act tta 816
Gly Arg Lys Ile Val Leu Pro Ser Ala Val Ile Trp Gln Arg Thr Leu
            260                 265                 270

cat cca aca ctt ttc aat ttg tgt atc gat atc gca aac aag ata ctg 864
His Pro Thr Leu Phe Asn Leu Cys Ile Asp Ile Ala Asn Lys Ile Leu
        275                 280                 285

ttc aat tgg aag tcg ttt aac att cta ccg aga caa aag att gct tct 912
Phe Asn Trp Lys Ser Phe Asn Ile Leu Pro Arg Gln Lys Ile Ala Ser
    290                 295                 300

tac gca aaa ctt tat tca acg acc agt gta aaa tcc att gtt cac tgg 960
Tyr Ala Lys Leu Tyr Ser Thr Thr Ser Val Lys Ser Ile Val His Trp
305                 310                 315                 320

ttc caa ata tta aga tct cag aaa ttt caa atg ttt gaa gag tct gat 1008
Phe Gln Ile Leu Arg Ser Gln Lys Phe Gln Met Phe Glu Glu Ser Asp
                325                 330                 335

aac atg cta aat tcc tta act agg cct tac caa att gct aat ttt cct 1056
Asn Met Leu Asn Ser Leu Thr Arg Pro Tyr Gln Ile Ala Asn Phe Pro
            340                 345                 350

act aga aca aat atc aag att ccc att ctt tta att tat ggt ggc ata 1104
Thr Arg Thr Asn Ile Lys Ile Pro Ile Leu Leu Ile Tyr Gly Gly Ile
        355                 360                 365

gat tct tta gtt gat att gat gtg atg aaa aaa aat cta ccc ttc aac 1152
Asp Ser Leu Val Asp Ile Asp Val Met Lys Lys Asn Leu Pro Phe Asn
    370                 375                 380

tcc gtc ttt gat gtt aaa gtt gac aat tat gaa cac ctg gat ttg att 1200
Ser Val Phe Asp Val Lys Val Asp Asn Tyr Glu His Leu Asp Leu Ile
385                 390                 395                 400

tgg ggc aaa gat gcc gat acc ttg gtc att gct aaa gtc ttg agg ttt 1248
Trp Gly Lys Asp Ala Asp Thr Leu Val Ile Ala Lys Val Leu Arg Phe
                405                 410                 415

att gaa ttt ttt aac cct ggt aat gtt tca gtg aag act aac cag tta 1296
Ile Glu Phe Phe Asn Pro Gly Asn Val Ser Val Lys Thr Asn Gln Leu
            420                 425                 430

cta cca tca gca agt ctg gtt gag gaa tta cca agc acg aca tgg aag 1344
Leu Pro Ser Ala Ser Leu Val Glu Glu Leu Pro Ser Thr Thr Trp Lys
        435                 440                 445

aca act cat cca acg cat ggt ctc agt tat aga act cac tcg gca gac 1392
Thr Thr His Pro Thr His Gly Leu Ser Tyr Arg Thr His Ser Ala Asp
    450                 455                 460

cgt tct ccg ttg tct gta caa gct gat gaa gcg gat gag gtc cac aat 1440
Arg Ser Pro Leu Ser Val Gln Ala Asp Glu Ala Asp Glu Val His Asn
465                 470                 475                 480

gct gac aat tca agg ttc tta aga cga gtg ttt tct act agt gcc ata 1488
Ala Asp Asn Ser Arg Phe Leu Arg Arg Val Phe Ser Thr Ser Ala Ile
                485                 490                 495

gac gag gac aac gaa aat gag cac cag gat gat aca gaa gat caa atc 1536

Asp Glu Asp Asn Glu Asn Glu His Gln Asp Asp Thr Glu Asp Gln Ile
                500                 505                 510

cat aag gag cag caa aga cgg tta agt gtc tat ctg gaa tca tcc aaa      1584
His Lys Glu Gln Gln Arg Arg Leu Ser Val Tyr Leu Glu Ser Ser Lys
            515                 520                 525

gat tta cga caa cta gat gcc aac tct tcg aca act gcg ctg gat gct      1632
Asp Leu Arg Gln Leu Asp Ala Asn Ser Ser Thr Thr Ala Leu Asp Ala
    530                 535                 540

cta aat aaa gaa tga                                                  1647
Leu Asn Lys Glu
545

<210>  40
<211>  548
<212>  PRT
<213>  Saccharomyces cerevisiae

<400>  40

Met Tyr Phe Pro Phe Leu Gly Arg Leu Ser Ile Thr Asp Tyr Ile Ile
1               5                   10                  15

Val Val Leu Val Tyr Ile Glu Ser Ile Ile Ser Ser Val Leu Lys Leu
            20                  25                  30

Ile Pro Gln Pro Met Ile Asn Leu Phe Glu Trp Leu Ile Asn Phe Ser
        35                  40                  45

Thr Ser Ser Asp Asp Asn Thr Ile Glu Glu Lys Leu Arg Ser Ala Pro
    50                  55                  60

Thr Ile His Glu Met Cys Ala Ile Phe Asp Ile Ser Val Glu Asp His
65                  70                  75                  80

Leu Val Arg Ile Glu Asp Asn Tyr Ile Leu Thr Leu His Arg Ile Pro
                85                  90                  95

Pro Ile Ser Lys Asn Arg Phe Asn Asn Lys Val Val Tyr Leu His His
            100                 105                 110

Gly Leu Leu Met Cys Ser Asp Val Trp Cys Cys Asn Ile Glu Arg His
        115                 120                 125

Lys Asn Leu Pro Phe Val Leu His Asp Leu Gly Tyr Asp Val Trp Met
    130                 135                 140

Gly Asn Asn Arg Gly Asn Lys Tyr Ser Thr Ala His Leu Asn Lys Pro
145                 150                 155                 160

Pro Lys Ser Asn Lys Phe Trp Asp Phe Ser Ile Asp Glu Phe Ala Phe
                165                 170                 175

Phe Asp Ile Pro Asn Ser Ile Glu Phe Ile Leu Asp Ile Thr Lys Val
            180                 185                 190

Asp Lys Val Ile Cys Ile Gly Phe Ser Gln Gly Ser Ala Gln Met Phe
        195                 200                 205

Ala Ala Phe Ser Leu Ser Glu Lys Leu Asn Arg Lys Val Ser His Phe
    210                 215                 220

Ile Ala Ile Ala Pro Ala Met Thr Pro Lys Gly Leu His Asn Arg Ile
225                 230                 235                 240

Val Asp Thr Leu Ala Lys Ser Ser Pro Gly Phe Met Tyr Leu Phe Phe
                245                 250                 255

Gly Arg Lys Ile Val Leu Pro Ser Ala Val Ile Trp Gln Arg Thr Leu
            260                 265                 270

His Pro Thr Leu Phe Asn Leu Cys Ile Asp Ile Ala Asn Lys Ile Leu
        275                 280                 285

Phe Asn Trp Lys Ser Phe Asn Ile Leu Pro Arg Gln Lys Ile Ala Ser
    290                 295                 300

Tyr Ala Lys Leu Tyr Ser Thr Thr Ser Val Lys Ser Ile Val His Trp
305                 310                 315                 320

Phe Gln Ile Leu Arg Ser Gln Lys Phe Gln Met Phe Glu Glu Ser Asp
                325                 330                 335

Asn Met Leu Asn Ser Leu Thr Arg Pro Tyr Gln Ile Ala Asn Phe Pro
            340                 345                 350

Thr Arg Thr Asn Ile Lys Ile Pro Ile Leu Leu Ile Tyr Gly Gly Ile
        355                 360                 365

Asp Ser Leu Val Asp Ile Asp Val Met Lys Lys Asn Leu Pro Phe Asn
    370                 375                 380

Ser Val Phe Asp Val Lys Val Asp Asn Tyr Glu His Leu Asp Leu Ile
385                 390                 395                 400

Trp Gly Lys Asp Ala Asp Thr Leu Val Ile Ala Lys Val Leu Arg Phe
                405                 410                 415

Ile Glu Phe Phe Asn Pro Gly Asn Val Ser Val Lys Thr Asn Gln Leu
            420                 425                 430

Leu Pro Ser Ala Ser Leu Val Glu Glu Leu Pro Ser Thr Thr Trp Lys
        435                 440                 445

Thr Thr His Pro Thr His Gly Leu Ser Tyr Arg Thr His Ser Ala Asp
    450                 455                 460

Arg Ser Pro Leu Ser Val Gln Ala Asp Glu Ala Asp Glu Val His Asn
465                 470                 475                 480

Ala Asp Asn Ser Arg Phe Leu Arg Arg Val Phe Ser Thr Ser Ala Ile
                485                 490                 495

Asp Glu Asp Asn Glu Asn Glu His Gln Asp Asp Thr Glu Asp Gln Ile
            500                 505                 510

His Lys Glu Gln Gln Arg Arg Leu Ser Val Tyr Leu Glu Ser Ser Lys
        515                 520                 525

Asp Leu Arg Gln Leu Asp Ala Asn Ser Ser Thr Thr Ala Leu Asp Ala
    530                 535                 540

Leu Asn Lys Glu
545

<210>  41
<211>  1206
<212>  DNA
<213>  Synechocystis sp. pcc6803

<220>
<221>  CDS
<222>  (1)..(1206)
<223>  succinate-CoA ligase (SLL1023)

<400>  41
atg gac ttg tta gag tac cag gcc aag gaa cta ttt cag caa gtg ggc      48
Met Asp Leu Leu Glu Tyr Gln Ala Lys Glu Leu Phe Gln Gln Val Gly
1               5                   10                  15

att ccc att ttg cct tcc cag act atc caa aac acc act gcc ctc aaa      96
Ile Pro Ile Leu Pro Ser Gln Thr Ile Gln Asn Thr Thr Ala Leu Lys
            20                  25                  30

cgg ttg caa att ccc tac ccc atg gtg ctc aaa tcc cag gtg cgg gcg      144
Arg Leu Gln Ile Pro Tyr Pro Met Val Leu Lys Ser Gln Val Arg Ala
        35                  40                  45

ggg ggc cgg ggt aag gcc ggc ggg gtt agg ttt gtg gaa aat acc att      192
Gly Gly Arg Gly Lys Ala Gly Gly Val Arg Phe Val Glu Asn Thr Ile
    50                  55                  60

gat gcc att gcc gcc gct tcg gcc att ttc cat ttg ccc att gcg gat      240
Asp Ala Ile Ala Ala Ala Ser Ala Ile Phe His Leu Pro Ile Ala Asp
65                  70                  75                  80

gag tac cca gag gtg att ttg gct gag gcc cgc tac gat gcc cag tcg      288
Glu Tyr Pro Glu Val Ile Leu Ala Glu Ala Arg Tyr Asp Ala Gln Ser
                85                  90                  95

gaa tta ttt ttg gcg atc gtg ctg gac tac caa cgg cag tgc cct gtg      336
Glu Leu Phe Leu Ala Ile Val Leu Asp Tyr Gln Arg Gln Cys Pro Val
            100                 105                 110

tta atg ggt tcc agc gag ggg ggc att gat gtg gaa acc ctg ttg gag      384
Leu Met Gly Ser Ser Glu Gly Gly Ile Asp Val Glu Thr Leu Leu Glu
        115                 120                 125

caa atg cag agc gtt tcc ctg cgg act aac ttt tct ccc tat cta gcc     432
Gln Met Gln Ser Val Ser Leu Arg Thr Asn Phe Ser Pro Tyr Leu Ala
    130                 135                 140

cga cgg cta gcg gtg aaa atg ggt ttg acc ggg ccc ctg gtg act gcg     480
Arg Arg Leu Ala Val Lys Met Gly Leu Thr Gly Pro Leu Val Thr Ala
145                 150                 155                 160

gtg agc ggc atc atc ggc aaa atg tat gaa ttg ttt gtc acc tat gac     528
Val Ser Gly Ile Ile Gly Lys Met Tyr Glu Leu Phe Val Thr Tyr Asp
                165                 170                 175

ttg gac gtg att gaa att aat ccc ctg ggt att agt gct gac ggg gag     576
Leu Asp Val Ile Glu Ile Asn Pro Leu Gly Ile Ser Ala Asp Gly Glu
            180                 185                 190

gtg atg gcc ctg gac ggc aaa att aca gtc aac gat acg gcg atc aac     624
Val Met Ala Leu Asp Gly Lys Ile Thr Val Asn Asp Thr Ala Ile Asn
        195                 200                 205

cgg cat ccg gat ttg atc aat tgg cga tcg gag caa tgg acg ggg cat     672
Arg His Pro Asp Leu Ile Asn Trp Arg Ser Glu Gln Trp Thr Gly His
    210                 215                 220

agt tgg ctg ccg ggg aac ttg gcc cag gga caa att ggc cta att tgt     720
Ser Trp Leu Pro Gly Asn Leu Ala Gln Gly Gln Ile Gly Leu Ile Cys
225                 230                 235                 240

aac agt gaa ggg cta gcc ctg agt acg tgg gac ctg ctc aat tcc ttc     768
Asn Ser Glu Gly Leu Ala Leu Ser Thr Trp Asp Leu Leu Asn Ser Phe
                245                 250                 255

ggc atc acc ggc gcc tat tta ttg gat gaa ggt cgc agt gac ata acc     816
Gly Ile Thr Gly Ala Tyr Leu Leu Asp Glu Gly Arg Ser Asp Ile Thr
            260                 265                 270

ctg ggg gaa caa ctg gaa ttg gcc ttt aac cac ctc agc cag gcc ccc     864
Leu Gly Glu Gln Leu Glu Leu Ala Phe Asn His Leu Ser Gln Ala Pro
        275                 280                 285

aat tta aaa gga att ttt gtt aac ctg gcc acc aga gca acg gac acc     912
Asn Leu Lys Gly Ile Phe Val Asn Leu Ala Thr Arg Ala Thr Asp Thr
    290                 295                 300

tcg gcc ttg gcg gaa gat tta cgg tct ttt tta ccc ctg ccc ccc aac     960
Ser Ala Leu Ala Glu Asp Leu Arg Ser Phe Leu Pro Leu Pro Pro Asn
305                 310                 315                 320

ctc tcc agt gaa gac cgc agt tta cgg ggg acg ggc ccc agt ttg ccc     1008
Leu Ser Ser Glu Asp Arg Ser Leu Arg Gly Thr Gly Pro Ser Leu Pro
                325                 330                 335

cag cgc caa cgg gtt ccc caa cgg caa acc tat aca ggg gaa gtg cta     1056
Gln Arg Gln Arg Val Pro Gln Arg Gln Thr Tyr Thr Gly Glu Val Leu
            340                 345                 350

ccg gtg gtt atc cga ttt agc cag gga aat tta gat cat ctc cag cag     1104
Pro Val Val Ile Arg Phe Ser Gln Gly Asn Leu Asp His Leu Gln Gln
        355                 360                 365

atg tac gac aac agc ctt gtc cat tgg cac aat gat ttg gaa acg gcg     1152
Met Tyr Asp Asn Ser Leu Val His Trp His Asn Asp Leu Glu Thr Ala
    370                 375                 380

atc gcc aag atg ctg agt ttg atg ccc caa gag acg gca ttg gcc cag     1200
Ile Ala Lys Met Leu Ser Leu Met Pro Gln Glu Thr Ala Leu Ala Gln
385                 390                 395                 400

gga taa                                                             1206

Gly

<210>  42
<211>  401
<212>  PRT
<213>  Synechocystis sp. pcc6803

<400>  42

Met Asp Leu Leu Glu Tyr Gln Ala Lys Glu Leu Phe Gln Gln Val Gly
1               5                   10                  15

Ile Pro Ile Leu Pro Ser Gln Thr Ile Gln Asn Thr Thr Ala Leu Lys
            20                  25                  30

Arg Leu Gln Ile Pro Tyr Pro Met Val Leu Lys Ser Gln Val Arg Ala
        35                  40                  45

Gly Gly Arg Gly Lys Ala Gly Gly Val Arg Phe Val Glu Asn Thr Ile
    50                  55                  60

Asp Ala Ile Ala Ala Ala Ser Ala Ile Phe His Leu Pro Ile Ala Asp
65                  70                  75                  80

Glu Tyr Pro Glu Val Ile Leu Ala Glu Ala Arg Tyr Asp Ala Gln Ser
                85                  90                  95

Glu Leu Phe Leu Ala Ile Val Leu Asp Tyr Gln Arg Gln Cys Pro Val
            100                 105                 110

Leu Met Gly Ser Ser Glu Gly Gly Ile Asp Val Glu Thr Leu Leu Glu
        115                 120                 125

Gln Met Gln Ser Val Ser Leu Arg Thr Asn Phe Ser Pro Tyr Leu Ala
    130                 135                 140

Arg Arg Leu Ala Val Lys Met Gly Leu Thr Gly Pro Leu Val Thr Ala
145                 150                 155                 160

Val Ser Gly Ile Ile Gly Lys Met Tyr Glu Leu Phe Val Thr Tyr Asp
                165                 170                 175

Leu Asp Val Ile Glu Ile Asn Pro Leu Gly Ile Ser Ala Asp Gly Glu
            180                 185                 190

Val Met Ala Leu Asp Gly Lys Ile Thr Val Asn Asp Thr Ala Ile Asn
        195                 200                 205

Arg His Pro Asp Leu Ile Asn Trp Arg Ser Glu Gln Trp Thr Gly His
    210                 215                 220

Ser Trp Leu Pro Gly Asn Leu Ala Gln Gly Gln Ile Gly Leu Ile Cys
225                 230                 235                 240

Asn Ser Glu Gly Leu Ala Leu Ser Thr Trp Asp Leu Leu Asn Ser Phe
                245                 250                 255

Gly Ile Thr Gly Ala Tyr Leu Leu Asp Glu Gly Arg Ser Asp Ile Thr
            260                 265                 270

Leu Gly Glu Gln Leu Glu Leu Ala Phe Asn His Leu Ser Gln Ala Pro
        275                 280                 285

Asn Leu Lys Gly Ile Phe Val Asn Leu Ala Thr Arg Ala Thr Asp Thr
    290                 295                 300

Ser Ala Leu Ala Glu Asp Leu Arg Ser Phe Leu Pro Leu Pro Pro Asn
305                 310                 315                 320

Leu Ser Ser Glu Asp Arg Ser Leu Arg Gly Thr Gly Pro Ser Leu Pro
                325                 330                 335

Gln Arg Gln Arg Val Pro Gln Arg Gln Thr Tyr Thr Gly Glu Val Leu
            340                 345                 350

Pro Val Val Ile Arg Phe Ser Gln Gly Asn Leu Asp His Leu Gln Gln
        355                 360                 365

Met Tyr Asp Asn Ser Leu Val His Trp His Asn Asp Leu Glu Thr Ala
    370                 375                 380

Ile Ala Lys Met Leu Ser Leu Met Pro Gln Glu Thr Ala Leu Ala Gln
385                 390                 395                 400

Gly

<210>  43
<211>  786
<212>  DNA
<213>  Synechocystis sp.

<220>
<221>  CDS
<222>  (1)..(786)
<223>  cobalt-precorrin-6A reductase (SLR0252)

<400>  43
atg gtg gat agc ctg gca acc gtt tgg tta att ggt gga acg gtg gat      48
Met Val Asp Ser Leu Ala Thr Val Trp Leu Ile Gly Gly Thr Val Asp
1               5                   10                  15

agt cgt gcc gta gcc gaa ggg tta att gcc cag gga ata aat tgt ctg      96
Ser Arg Ala Val Ala Glu Gly Leu Ile Ala Gln Gly Ile Asn Cys Leu
            20                  25                  30

gtg aca gtc acc aca tcg gag gcc aag cat tta tat ccg att cat caa      144
Val Thr Val Thr Thr Ser Glu Ala Lys His Leu Tyr Pro Ile His Gln
        35                  40                  45

tgt ttg acg gtt cac gtt gga gct tta acc cca cag gaa ata ccg aaa 192
Cys Leu Thr Val His Val Gly Ala Leu Thr Pro Gln Glu Ile Pro Lys
    50                  55                  60

ttt cta aaa cgc cat agc att gca gtc att gtc gat gcg tcc cat ccc 240
Phe Leu Lys Arg His Ser Ile Ala Val Ile Val Asp Ala Ser His Pro
65                  70                  75                  80

ttt gcc gcc caa att act acc acg gtc acg gcg atc gcc aaa gaa caa 288
Phe Ala Ala Gln Ile Thr Thr Thr Val Thr Ala Ile Ala Lys Glu Gln
                85                  90                  95

caa att ccc tat atc cga ttt gag cgg ccg ccc ctg gct ttg gga aaa 336
Gln Ile Pro Tyr Ile Arg Phe Glu Arg Pro Pro Leu Ala Leu Gly Lys
            100                 105                 110

aat acc tta gaa gta cct gat att cag agt tta acc aga ggg aaa tat 384
Asn Thr Leu Glu Val Pro Asp Ile Gln Ser Leu Thr Arg Gly Lys Tyr
        115                 120                 125

cag cct tac ttg agg gga aaa cgg gtg ctg ttg acc gtt gga gcc cgc 432
Gln Pro Tyr Leu Arg Gly Lys Arg Val Leu Leu Thr Val Gly Ala Arg
    130                 135                 140

tgg tta agc cat ttc agc ctc cta cag gat gaa gca gtt cta ttt gcc 480
Trp Leu Ser His Phe Ser Leu Leu Gln Asp Glu Ala Val Leu Phe Ala
145                 150                 155                 160

cgt att ctg ccc tat ccc caa gcc cta gcc cag gcg atc gcc gct ggg 528
Arg Ile Leu Pro Tyr Pro Gln Ala Leu Ala Gln Ala Ile Ala Ala Gly
                165                 170                 175

ttt act tcc gac cgt atc att gct ttg cgc ccc ccc gta gcc gaa cca 576
Phe Thr Ser Asp Arg Ile Ile Ala Leu Arg Pro Pro Val Ala Glu Pro
            180                 185                 190

ttg gaa aaa gcc ctg tgg caa caa tgg caa att caa ggg gtg gta act 624
Leu Glu Lys Ala Leu Trp Gln Gln Trp Gln Ile Gln Gly Val Val Thr
        195                 200                 205

aaa gcc tcc ggt gcc cag ggg gga gaa ttg gtt aag caa aaa gtg gcg 672
Lys Ala Ser Gly Ala Gln Gly Gly Glu Leu Val Lys Gln Lys Val Ala
    210                 215                 220

gaa gcg ttg ggg gta aat ctg atc aga att gcc cgt ccc cag act att 720
Glu Ala Leu Gly Val Asn Leu Ile Arg Ile Ala Arg Pro Gln Thr Ile
225                 230                 235                 240

cca ggg caa ata act gac gat tta agc cag atc aac caa ttt tgc caa 768
Pro Gly Gln Ile Thr Asp Asp Leu Ser Gln Ile Asn Gln Phe Cys Gln
                245                 250                 255

aga cat ttg cca agc taa 786
Arg His Leu Pro Ser
            260

<210> 44
<211> 261
<212> PRT
<213> Synechocystis sp.

<400> 44

Met Val Asp Ser Leu Ala Thr Val Trp Leu Ile Gly Gly Thr Val Asp
1               5                   10                  15

Ser Arg Ala Val Ala Glu Gly Leu Ile Ala Gln Gly Ile Asn Cys Leu
            20                  25                  30

Val Thr Val Thr Thr Ser Glu Ala Lys His Leu Tyr Pro Ile His Gln
        35                  40                  45

Cys Leu Thr Val His Val Gly Ala Leu Thr Pro Gln Glu Ile Pro Lys
    50                  55                  60

Phe Leu Lys Arg His Ser Ile Ala Val Ile Val Asp Ala Ser His Pro
65                  70                  75                  80

Phe Ala Ala Gln Ile Thr Thr Thr Val Thr Ala Ile Ala Lys Glu Gln
                85                  90                  95

Gln Ile Pro Tyr Ile Arg Phe Glu Arg Pro Pro Leu Ala Leu Gly Lys
            100                 105                 110

Asn Thr Leu Glu Val Pro Asp Ile Gln Ser Leu Thr Arg Gly Lys Tyr
        115                 120                 125

Gln Pro Tyr Leu Arg Gly Lys Arg Val Leu Leu Thr Val Gly Ala Arg
    130                 135                 140

Trp Leu Ser His Phe Ser Leu Leu Gln Asp Glu Ala Val Leu Phe Ala
145                 150                 155                 160

Arg Ile Leu Pro Tyr Pro Gln Ala Leu Ala Gln Ala Ile Ala Ala Gly
                165                 170                 175

Phe Thr Ser Asp Arg Ile Ile Ala Leu Arg Pro Pro Val Ala Glu Pro
            180                 185                 190

Leu Glu Lys Ala Leu Trp Gln Gln Trp Gln Ile Gln Gly Val Val Thr
        195                 200                 205

Lys Ala Ser Gly Ala Gln Gly Gly Glu Leu Val Lys Gln Lys Val Ala
    210                 215                 220

Glu Ala Leu Gly Val Asn Leu Ile Arg Ile Ala Arg Pro Gln Thr Ile
225                 230                 235                 240

Pro Gly Gln Ile Thr Asp Asp Leu Ser Gln Ile Asn Gln Phe Cys Gln
                245                 250                 255

Arg His Leu Pro Ser
            260

<210> 45
<211> 1182
<212> DNA
<213> Escherichia coli

<220>
<221>  CDS
<222>  (1)..(1182)
<223>  uroporphyrin III C-methyltransferase (b3803)

<400>  45
atg acg gaa caa gaa aaa acc tcc gcc gtg gtt gaa gag acc agg gag        48
Met Thr Glu Gln Glu Lys Thr Ser Ala Val Val Glu Glu Thr Arg Glu
1               5                   10                  15

gcc gtg gac acc acg tca caa cct gtc gca aca gaa aaa aag agt aag        96
Ala Val Asp Thr Thr Ser Gln Pro Val Ala Thr Glu Lys Lys Ser Lys
            20                  25                  30

aac aat acc gca ttg att ctc agc gcg gtg gct atc gct att gct ctg       144
Asn Asn Thr Ala Leu Ile Leu Ser Ala Val Ala Ile Ala Ile Ala Leu
        35                  40                  45

gcg gcg ggc atc ggt ttg tat ggc tgg ggt aaa caa cag gcc gtc aat       192
Ala Ala Gly Ile Gly Leu Tyr Gly Trp Gly Lys Gln Gln Ala Val Asn
    50                  55                  60

cag acc gcc acc agc gat gcc ctg gct aac caa ctg acg gca ttg caa       240
Gln Thr Ala Thr Ser Asp Ala Leu Ala Asn Gln Leu Thr Ala Leu Gln
65                  70                  75                  80

aaa gcc cag gag agc caa aaa gcc gag ctg gaa ggc att att aag caa       288
Lys Ala Gln Glu Ser Gln Lys Ala Glu Leu Glu Gly Ile Ile Lys Gln
                85                  90                  95

caa gct gca caa ctt aag cag gcg aat cgt cag caa gaa acg ctg gca       336
Gln Ala Ala Gln Leu Lys Gln Ala Asn Arg Gln Gln Glu Thr Leu Ala
            100                 105                 110

aaa cag ttg gat gaa gtc caa caa aag gtc gcc acc att tcc ggc agc       384
Lys Gln Leu Asp Glu Val Gln Gln Lys Val Ala Thr Ile Ser Gly Ser
        115                 120                 125

gat gct aaa acc tgg ctg ctg gct cag gcc gat ttt ctg gtg aaa ctc       432
Asp Ala Lys Thr Trp Leu Leu Ala Gln Ala Asp Phe Leu Val Lys Leu
    130                 135                 140

gcc gga cgg aag ctg tgg agc gat cag gac gtc acg acc gct gca gcg       480
Ala Gly Arg Lys Leu Trp Ser Asp Gln Asp Val Thr Thr Ala Ala Ala
145                 150                 155                 160

ttg ctg aaa agt gca gac gcc agc ctg gcg gat atg aat gac ccg agt       528
Leu Leu Lys Ser Ala Asp Ala Ser Leu Ala Asp Met Asn Asp Pro Ser
                165                 170                 175

ctg att acc gtt cgt cgg gca att acc gat gat atc gcc agc ctt tct       576
Leu Ile Thr Val Arg Arg Ala Ile Thr Asp Asp Ile Ala Ser Leu Ser
            180                 185                 190

gca gta tcg cag gtg gat tat gac ggc atc atc ctt aag ctt aat cag       624
Ala Val Ser Gln Val Asp Tyr Asp Gly Ile Ile Leu Lys Leu Asn Gln
        195                 200                 205

ctt tca aat cag gta gat aac ctg cgt ctg gcc gat aat gac agc gat       672
Leu Ser Asn Gln Val Asp Asn Leu Arg Leu Ala Asp Asn Asp Ser Asp
    210                 215                 220

ggt tcg ccg atg gat tca gac ggt gaa gag ctt tcc agt tcc atc agc       720
Gly Ser Pro Met Asp Ser Asp Gly Glu Glu Leu Ser Ser Ser Ile Ser
225                 230                 235                 240

gaa tgg cgt atc aat ctg caa aaa agc tgg cag aac ttt atg gac aac       768
Glu Trp Arg Ile Asn Leu Gln Lys Ser Trp Gln Asn Phe Met Asp Asn

245                 250                 255

ttc att acg att cgc cgt cgt gat gac acc gcc gta ccg ctg tta gcg      816
Phe Ile Thr Ile Arg Arg Arg Asp Asp Thr Ala Val Pro Leu Leu Ala
            260                 265                 270

cca aat cag gat atc tat ctg cgc gaa aat att cgc tct cgc ctg ctg      864
Pro Asn Gln Asp Ile Tyr Leu Arg Glu Asn Ile Arg Ser Arg Leu Leu
        275                 280                 285

gtc gca gca caa gct gta ccg cgt cac cag gaa gag act tat cgc cag      912
Val Ala Ala Gln Ala Val Pro Arg His Gln Glu Glu Thr Tyr Arg Gln
    290                 295                 300

gcg ctg gag aac gtc tcc acc tgg gta cgt gct tac tac gat act gat      960
Ala Leu Glu Asn Val Ser Thr Trp Val Arg Ala Tyr Tyr Asp Thr Asp
305                 310                 315                 320

gat gcc acc acc aaa gcg ttc ctc gac gag gtg gac cag tta agc cag     1008
Asp Ala Thr Thr Lys Ala Phe Leu Asp Glu Val Asp Gln Leu Ser Gln
                325                 330                 335

caa aat atc tcg atg gat ctt ccg gaa acc ctg caa agc cag gcg atg     1056
Gln Asn Ile Ser Met Asp Leu Pro Glu Thr Leu Gln Ser Gln Ala Met
            340                 345                 350

ctg gaa aaa ctg atg cag act cgc gtg cgt aac ctg ctg gca caa ccg     1104
Leu Glu Lys Leu Met Gln Thr Arg Val Arg Asn Leu Leu Ala Gln Pro
        355                 360                 365

gca gcg ggg aca acg gaa gct aaa cct gca cct gca ccg caa gct gat     1152
Ala Ala Gly Thr Thr Glu Ala Lys Pro Ala Pro Ala Pro Gln Ala Asp
    370                 375                 380

act ccg gca gcc gcg ccg caa gga gaa taa                             1182
Thr Pro Ala Ala Ala Pro Gln Gly Glu
385                 390


<210>  46
<211>  393
<212>  PRT
<213>  Escherichia coli

<400>  46

Met Thr Glu Gln Glu Lys Thr Ser Ala Val Val Glu Glu Thr Arg Glu
1               5                   10                  15


Ala Val Asp Thr Thr Ser Gln Pro Val Ala Thr Glu Lys Lys Ser Lys
            20                  25                  30


Asn Asn Thr Ala Leu Ile Leu Ser Ala Val Ala Ile Ala Ile Ala Leu
        35                  40                  45


Ala Ala Gly Ile Gly Leu Tyr Gly Trp Gly Lys Gln Gln Ala Val Asn
    50                  55                  60


Gln Thr Ala Thr Ser Asp Ala Leu Ala Asn Gln Leu Thr Ala Leu Gln
65                  70                  75                  80


Lys Ala Gln Glu Ser Gln Lys Ala Glu Leu Glu Gly Ile Ile Lys Gln
                85                  90                  95

Gln Ala Ala Gln Leu Lys Gln Ala Asn Arg Gln Gln Glu Thr Leu Ala
            100                 105                 110

Lys Gln Leu Asp Glu Val Gln Gln Lys Val Ala Thr Ile Ser Gly Ser
        115                 120                 125

Asp Ala Lys Thr Trp Leu Leu Ala Gln Ala Asp Phe Leu Val Lys Leu
    130                 135                 140

Ala Gly Arg Lys Leu Trp Ser Asp Gln Asp Val Thr Thr Ala Ala Ala
145                 150                 155                 160

Leu Leu Lys Ser Ala Asp Ala Ser Leu Ala Asp Met Asn Asp Pro Ser
                165                 170                 175

Leu Ile Thr Val Arg Arg Ala Ile Thr Asp Asp Ile Ala Ser Leu Ser
            180                 185                 190

Ala Val Ser Gln Val Asp Tyr Asp Gly Ile Ile Leu Lys Leu Asn Gln
        195                 200                 205

Leu Ser Asn Gln Val Asp Asn Leu Arg Leu Ala Asp Asn Asp Ser Asp
    210                 215                 220

Gly Ser Pro Met Asp Ser Asp Gly Glu Glu Leu Ser Ser Ser Ile Ser
225                 230                 235                 240

Glu Trp Arg Ile Asn Leu Gln Lys Ser Trp Gln Asn Phe Met Asp Asn
                245                 250                 255

Phe Ile Thr Ile Arg Arg Arg Asp Asp Thr Ala Val Pro Leu Leu Ala
            260                 265                 270

Pro Asn Gln Asp Ile Tyr Leu Arg Glu Asn Ile Arg Ser Arg Leu Leu
        275                 280                 285

Val Ala Ala Gln Ala Val Pro Arg His Gln Glu Glu Thr Tyr Arg Gln
    290                 295                 300

Ala Leu Glu Asn Val Ser Thr Trp Val Arg Ala Tyr Tyr Asp Thr Asp
305                 310                 315                 320

Asp Ala Thr Thr Lys Ala Phe Leu Asp Glu Val Asp Gln Leu Ser Gln
                325                 330                 335

Gln Asn Ile Ser Met Asp Leu Pro Glu Thr Leu Gln Ser Gln Ala Met
            340                 345                 350

Leu Glu Lys Leu Met Gln Thr Arg Val Arg Asn Leu Leu Ala Gln Pro
        355                 360                 365

Ala Ala Gly Thr Thr Glu Ala Lys Pro Ala Pro Ala Pro Gln Ala Asp
    370                 375                 380

Thr Pro Ala Ala Ala Pro Gln Gly Glu
385                 390

<210>  47
<211>  1107
<212>  DNA
<213>  Brassica napus

<220>
<221>  CDS
<222>  (1)..(1107)
<223>  uroporphyrin III C-methyltransferase (BN51286476)

<400>  47
   atg gct ctt gtc cag cgg att ccc agt ttc tca tcc aat ctt cga aac      48
   Met Ala Leu Val Gln Arg Ile Pro Ser Phe Ser Ser Asn Leu Arg Asn
   1               5                   10                  15
   tgg aag agc acc aat tcg aca aac cat aag cct gtt tct tct ctc cat      96
   Trp Lys Ser Thr Asn Ser Thr Asn His Lys Pro Val Ser Ser Leu His
               20                  25                  30
   tac aaa act cag atc act gct tct tct tcc tcg ccc ttc acg gag aag      144
   Tyr Lys Thr Gln Ile Thr Ala Ser Ser Ser Ser Pro Phe Thr Glu Lys
           35                  40                  45
   cac tct gtt gag aga tac caa aga gat caa tgg ctg tac aaa gca tcc      192
   His Ser Val Glu Arg Tyr Gln Arg Asp Gln Trp Leu Tyr Lys Ala Ser
       50                  55                  60
   act cca tct cca tct cca tcg ccg ccg tcg aat cag caa gat gaa gtc      240
   Thr Pro Ser Pro Ser Pro Ser Pro Pro Ser Asn Gln Gln Asp Glu Val
   65                  70                  75                  80
   ttt gtt agg gaa aac gac atc gca tcg cag ctg cct gag ctg aag aag      288
   Phe Val Arg Glu Asn Asp Ile Ala Ser Gln Leu Pro Glu Leu Lys Lys
                   85                  90                  95
   ctc ttg gag gtt ctg aga gag aag aga gag agt gga tgc aga ggc ggt      336
   Leu Leu Glu Val Leu Arg Glu Lys Arg Glu Ser Gly Cys Arg Gly Gly
               100                 105                 110
   gat tgc gga cca gga gat gtg ttt ctg gtc ggg aca gga ccg gga gat      384
   Asp Cys Gly Pro Gly Asp Val Phe Leu Val Gly Thr Gly Pro Gly Asp
           115                 120                 125
   cct gag ctt ttg act ttg aaa gct gtc aga gtc att caa agc gct gat      432
   Pro Glu Leu Leu Thr Leu Lys Ala Val Arg Val Ile Gln Ser Ala Asp
       130                 135                 140
   ctt ctg ctt tac gac agg ctt gtc tct aat gat gtt ctg gag ttg gtt      480
   Leu Leu Leu Tyr Asp Arg Leu Val Ser Asn Asp Val Leu Glu Leu Val
   145                 150                 155                 160
   gct cct gat gct agg ctt ctc tat gtc ggc aaa act gct ggc tat cat      528
   Ala Pro Asp Ala Arg Leu Leu Tyr Val Gly Lys Thr Ala Gly Tyr His
                   165                 170                 175
   agc aga act cag gag gag ata cat gaa cta ctc ctt agt ttt gct gaa      576
   Ser Arg Thr Gln Glu Glu Ile His Glu Leu Leu Leu Ser Phe Ala Glu
               180                 185                 190
   gct ggt gct act gtt gtg agg ctt aaa ggt gga gat cct ttg gtt ttt      624
   Ala Gly Ala Thr Val Val Arg Leu Lys Gly Gly Asp Pro Leu Val Phe
           195                 200                 205
   gga cga ggt ggg gaa gag atg gac ttt ctg caa caa caa ggg att cga 672
   Gly Arg Gly Gly Glu Glu Met Asp Phe Leu Gln Gln Gln Gly Ile Arg
   210 215 220
   gtt aaa gtt atc cca ggg att aca gcg gcg tct ggg ata gca gca gag        720
   Val Lys Val Ile Pro Gly Ile Thr Ala Ala Ser Gly Ile Ala Ala Glu
   225                 230                 235                 240
   ctt ggg att ccc tta acg cat cga ggt gtt gca act agt gtg agg ttc        768
   Leu Gly Ile Pro Leu Thr His Arg Gly Val Ala Thr Ser Val Arg Phe
                   245                 250                 255
   ctc act ggt cat tcc agg aaa gga ggg acc gat cct ctc ttc gtc gca        816
   Leu Thr Gly His Ser Arg Lys Gly Gly Thr Asp Pro Leu Phe Val Ala
               260                 265                 270
   gag aat gca gct gat cct gac acg aca ctt gtt gtt tat atg ggt ttg        864
   Glu Asn Ala Ala Asp Pro Asp Thr Thr Leu Val Val Tyr Met Gly Leu
           275                 280                 285
   gga act tta cct tct ctt gcg cag aaa ctg atg gac cac ggt cta cct        912
   Gly Thr Leu Pro Ser Leu Ala Gln Lys Leu Met Asp His Gly Leu Pro
       290                 295                 300
   tgt gac aca cca gct gtt gcg gtt gaa cgt gga acc act cct ctt cag        960
   Cys Asp Thr Pro Ala Val Ala Val Glu Arg Gly Thr Thr Pro Leu Gln
   305                 310                 315                 320
   cgt aat gtt ttt gct gag ctt aaa gac ttt gcg act gag att cag gca        1008
   Arg Asn Val Phe Ala Glu Leu Lys Asp Phe Ala Thr Glu Ile Gln Ala
                   325                 330                 335
   gct gga ttg gtg tca cca acg ctc atc atc ata ggg aaa gtc gtc gag        1056
   Ala Gly Leu Val Ser Pro Thr Leu Ile Ile Ile Gly Lys Val Val Glu
               340                 345                 350
   cta tct cct tta tgg cct cat tgc acg aag gaa tac ttt ttg ctt gtt        1104
   Leu Ser Pro Leu Trp Pro His Cys Thr Lys Glu Tyr Phe Leu Leu Val
           355                 360                 365
   taa                                                                1107


<210>  48
<211>  368
<212>  PRT
<213>  Brassica napus

<400>  48

Met Ala Leu Val Gln Arg Ile Pro Ser Phe Ser Ser Asn Leu Arg Asn
1               5                   10                  15


Trp Lys Ser Thr Asn Ser Thr Asn His Lys Pro Val Ser Ser Leu His
            20                  25                  30


Tyr Lys Thr Gln Ile Thr Ala Ser Ser Ser Ser Pro Phe Thr Glu Lys
        35                  40                  45


His Ser Val Glu Arg Tyr Gln Arg Asp Gln Trp Leu Tyr Lys Ala Ser
    50                  55                  60

Thr Pro Ser Pro Ser Pro Ser Pro Pro Ser Asn Gln Gln Asp Glu Val
65                  70                  75                  80

Phe Val Arg Glu Asn Asp Ile Ala Ser Gln Leu Pro Glu Leu Lys Lys
                85                  90                  95

Leu Leu Glu Val Leu Arg Glu Lys Arg Glu Ser Gly Cys Arg Gly Gly
            100                 105                 110

Asp Cys Gly Pro Gly Asp Val Phe Leu Val Gly Thr Gly Pro Gly Asp
        115                 120                 125

Pro Glu Leu Leu Thr Leu Lys Ala Val Arg Val Ile Gln Ser Ala Asp
    130                 135                 140

Leu Leu Leu Tyr Asp Arg Leu Val Ser Asn Asp Val Leu Glu Leu Val
145                 150                 155                 160

Ala Pro Asp Ala Arg Leu Leu Tyr Val Gly Lys Thr Ala Gly Tyr His
                165                 170                 175

Ser Arg Thr Gln Glu Glu Ile His Glu Leu Leu Leu Ser Phe Ala Glu
            180                 185                 190

Ala Gly Ala Thr Val Val Arg Leu Lys Gly Gly Asp Pro Leu Val Phe
        195                 200                 205

Gly Arg Gly Gly Glu Glu Met Asp Phe Leu Gln Gln Gln Gly Ile Arg
    210                 215                 220

Val Lys Val Ile Pro Gly Ile Thr Ala Ala Ser Gly Ile Ala Ala Glu
225                 230                 235                 240

Leu Gly Ile Pro Leu Thr His Arg Gly Val Ala Thr Ser Val Arg Phe
                245                 250                 255

Leu Thr Gly His Ser Arg Lys Gly Gly Thr Asp Pro Leu Phe Val Ala
            260                 265                 270

Glu Asn Ala Ala Asp Pro Asp Thr Thr Leu Val Val Tyr Met Gly Leu
        275                 280                 285

Gly Thr Leu Pro Ser Leu Ala Gln Lys Leu Met Asp His Gly Leu Pro
    290                 295                 300

Cys Asp Thr Pro Ala Val Ala Val Glu Arg Gly Thr Thr Pro Leu Gln
305                 310                 315                 320

Arg Asn Val Phe Ala Glu Leu Lys Asp Phe Ala Thr Glu Ile Gln Ala
                325                 330                 335

Ala Gly Leu Val Ser Pro Thr Leu Ile Ile Ile Gly Lys Val Val Glu

340                 345                 350

Leu Ser Pro Leu Trp Pro His Cys Thr Lys Glu Tyr Phe Leu Leu Val
        355                 360                 365

<210>  49
<211>  1092
<212>  DNA
<213>  Glycine max

<220>
<221>  CDS
<222>  (1)..(1092)
<223>  uroporphyrin III C-methyltransferase (GM59791864)

<400>  49
atg gct ctc ctc cac aag ctc gca cct ttc acc tct ctc tac acc aat      48
Met Ala Leu Leu His Lys Leu Ala Pro Phe Thr Ser Leu Tyr Thr Asn
1               5                   10                  15

acc ccc cat tcc cca cca ccc cgc cgc cac gtc atc atc tcc ttc tcc      96
Thr Pro His Ser Pro Pro Pro Arg Arg His Val Ile Ile Ser Phe Ser
            20                  25                  30

gtc tca ccc ttc acc gag aag cac tcc aca gag cgc tac cag agg gac      144
Val Ser Pro Phe Thr Glu Lys His Ser Thr Glu Arg Tyr Gln Arg Asp
        35                  40                  45

caa tgg gtc tac cag agc acc act caa gag ggt caa cct caa acg acg      192
Gln Trp Val Tyr Gln Ser Thr Thr Gln Glu Gly Gln Pro Gln Thr Thr
    50                  55                  60

ccg ttt cct cct ctc ccg tgc gat tca gcg tcc tta aga gac gac gac      240
Pro Phe Pro Pro Leu Pro Cys Asp Ser Ala Ser Leu Arg Asp Asp Asp
65                  70                  75                  80

ata gcg ttg cag ctg cca gag ctg aag aag ctg ctt cag gtt ctg agg      288
Ile Ala Leu Gln Leu Pro Glu Leu Lys Lys Leu Leu Gln Val Leu Arg
                85                  90                  95

gag aag agg gag tgt atc aat gga gag ggt tgt gag ccg ggg aat gtg      336
Glu Lys Arg Glu Cys Ile Asn Gly Glu Gly Cys Glu Pro Gly Asn Val
            100                 105                 110

ttc ttg gtg gga acg gga cct ggg gac cct gag ctt ctc act ctc aag      384
Phe Leu Val Gly Thr Gly Pro Gly Asp Pro Glu Leu Leu Thr Leu Lys
        115                 120                 125

gct gtg aga gtc atc aag agt gct gat ctt ttg ctc tat gac agg ttg      432
Ala Val Arg Val Ile Lys Ser Ala Asp Leu Leu Leu Tyr Asp Arg Leu
    130                 135                 140

gtg tcc aat gat gtg ctg gat ttg gtt ggc ccc ggt gct agg ctt ctc      480
Val Ser Asn Asp Val Leu Asp Leu Val Gly Pro Gly Ala Arg Leu Leu
145                 150                 155                 160

tat gtg ggc aag act gct ggg tat cat agc aga aca cag gag gag ata      528
Tyr Val Gly Lys Thr Ala Gly Tyr His Ser Arg Thr Gln Glu Glu Ile
                165                 170                 175

cat gag ctt ctt ttg agt ttt gca gaa gct ggg gca act gtt gtg aga      576
His Glu Leu Leu Leu Ser Phe Ala Glu Ala Gly Ala Thr Val Val Arg
            180                 185                 190

ctt aaa ggg ggt gat cca ttg gtg ttt ggg agg ggt ggg gaa gaa atg      624
Leu Lys Gly Gly Asp Pro Leu Val Phe Gly Arg Gly Gly Glu Glu Met

195                 200                 205

gat ttt ttg caa cag caa ggt atc caa gtg aaa gtt att cca ggt ata      672
Asp Phe Leu Gln Gln Gln Gly Ile Gln Val Lys Val Ile Pro Gly Ile
    210                 215                 220

act gct gca tct gga ata gca gca gag ctt gga att cca tta act cac      720
Thr Ala Ala Ser Gly Ile Ala Ala Glu Leu Gly Ile Pro Leu Thr His
225                 230                 235                 240

cgt ggc att gca aat agt gtg aga ttc ctc aca ggg cat tca agg aaa      768
Arg Gly Ile Ala Asn Ser Val Arg Phe Leu Thr Gly His Ser Arg Lys
                245                 250                 255

gga gga tcc gat cct ctc ttt gta tcg gaa aat gct gct gat ccg gat      816
Gly Gly Ser Asp Pro Leu Phe Val Ser Glu Asn Ala Ala Asp Pro Asp
            260                 265                 270

tct acc ttg gtg gtt tac atg ggc ttg gcg acc ttc cct tca ctt gca      864
Ser Thr Leu Val Val Tyr Met Gly Leu Ala Thr Phe Pro Ser Leu Ala
        275                 280                 285

cag aag cta atg cat cat ggt ctg tcc cct cag acc cca gct gca gcc      912
Gln Lys Leu Met His His Gly Leu Ser Pro Gln Thr Pro Ala Ala Ala
    290                 295                 300

att gag cga gga acc aca ctt cat cag cgc acg gtg ttt gct gaa cta      960
Ile Glu Arg Gly Thr Thr Leu His Gln Arg Thr Val Phe Ala Glu Leu
305                 310                 315                 320

aag gac ctt cac gag aaa att aca tct gct cag ctg gtg tca cca acg     1008
Lys Asp Leu His Glu Lys Ile Thr Ser Ala Gln Leu Val Ser Pro Thr
                325                 330                 335

ctg att att ata gga aaa gtt gtt gaa cta tca cca ttt tgg cca att     1056
Leu Ile Ile Ile Gly Lys Val Val Glu Leu Ser Pro Phe Trp Pro Ile
            340                 345                 350

cct aca aaa gaa gaa tca tgt tta atg cag gca tga                     1092
Pro Thr Lys Glu Glu Ser Cys Leu Met Gln Ala
        355                 360


<210>  50
<211>  363
<212>  PRT
<213>  Glycine max

<400>  50

Met Ala Leu Leu His Lys Leu Ala Pro Phe Thr Ser Leu Tyr Thr Asn
1               5                   10                  15


Thr Pro His Ser Pro Pro Pro Arg Arg His Val Ile Ile Ser Phe Ser
            20                  25                  30


Val Ser Pro Phe Thr Glu Lys His Ser Thr Glu Arg Tyr Gln Arg Asp
        35                  40                  45


Gln Trp Val Tyr Gln Ser Thr Thr Gln Glu Gly Gln Pro Gln Thr Thr
    50                  55                  60


Pro Phe Pro Pro Leu Pro Cys Asp Ser Ala Ser Leu Arg Asp Asp Asp
65                  70                  75                  80

Ile Ala Leu Gln Leu Pro Glu Leu Lys Lys Leu Leu Gln Val Leu Arg
                85                  90                  95

Glu Lys Arg Glu Cys Ile Asn Gly Glu Gly Cys Glu Pro Gly Asn Val
            100                 105                 110

Phe Leu Val Gly Thr Gly Pro Gly Asp Pro Glu Leu Leu Thr Leu Lys
        115                 120                 125

Ala Val Arg Val Ile Lys Ser Ala Asp Leu Leu Leu Tyr Asp Arg Leu
    130                 135                 140

Val Ser Asn Asp Val Leu Asp Leu Val Gly Pro Gly Ala Arg Leu Leu
145                 150                 155                 160

Tyr Val Gly Lys Thr Ala Gly Tyr His Ser Arg Thr Gln Glu Glu Ile
                165                 170                 175

His Glu Leu Leu Leu Ser Phe Ala Glu Ala Gly Ala Thr Val Val Arg
            180                 185                 190

Leu Lys Gly Gly Asp Pro Leu Val Phe Gly Arg Gly Gly Glu Glu Met
        195                 200                 205

Asp Phe Leu Gln Gln Gln Gly Ile Gln Val Lys Val Ile Pro Gly Ile
    210                 215                 220

Thr Ala Ala Ser Gly Ile Ala Ala Glu Leu Gly Ile Pro Leu Thr His
225                 230                 235                 240

Arg Gly Ile Ala Asn Ser Val Arg Phe Leu Thr Gly His Ser Arg Lys
                245                 250                 255

Gly Gly Ser Asp Pro Leu Phe Val Ser Glu Asn Ala Ala Asp Pro Asp
            260                 265                 270

Ser Thr Leu Val Val Tyr Met Gly Leu Ala Thr Phe Pro Ser Leu Ala
        275                 280                 285

Gln Lys Leu Met His His Gly Leu Ser Pro Gln Thr Pro Ala Ala Ala
    290                 295                 300

Ile Glu Arg Gly Thr Thr Leu His Gln Arg Thr Val Phe Ala Glu Leu
305                 310                 315                 320

Lys Asp Leu His Glu Lys Ile Thr Ser Ala Gln Leu Val Ser Pro Thr
                325                 330                 335

Leu Ile Ile Ile Gly Lys Val Val Glu Leu Ser Pro Phe Trp Pro Ile
            340                 345                 350

Pro Thr Lys Glu Glu Ser Cys Leu Met Gln Ala
        355                 360

<210>  51
<211>  1140
<212>  DNA
<213>  Zea mays

<220>
<221>  CDS
<222>  (1)..(1140)
<223>  uroporphyrin III C-methyltransferase (ZMBFb0243J04)

<400>  51
atg gct ctc gcc gtc cgc acg cct cgg ttc cag cct cga ccg gcc tcc      48
Met Ala Leu Ala Val Arg Thr Pro Arg Phe Gln Pro Arg Pro Ala Ser
1               5                   10                  15

att tct gcg ccc aca aca ccc gca tcg acc tcc ctc gcc gcc ggg aac      96
Ile Ser Ala Pro Thr Thr Pro Ala Ser Thr Ser Leu Ala Ala Gly Asn
            20                  25                  30

gcg agg ccc cgc ccc tgc gcc ggc gcc gcc gtt cgc gcc tcg ccc ttc     144
Ala Arg Pro Arg Pro Cys Ala Gly Ala Ala Val Arg Ala Ser Pro Phe
        35                  40                  45

acc gag gcc acc tcc tcc tcg cgc tac cgc cgc gac gcc tgg tca tac     192
Thr Glu Ala Thr Ser Ser Ser Arg Tyr Arg Arg Asp Ala Trp Ser Tyr
    50                  55                  60

gcc gcc gat ggc tcc tcc aac tcc gca tcc ccg tcc tcc gct gcc gct     240
Ala Ala Asp Gly Ser Ser Asn Ser Ala Ser Pro Ser Ser Ala Ala Ala
65                  70                  75                  80

gct gcc gcg ggc cgt cgg gat gac gag atc gcg ctg cag ctg ccg gaa     288
Ala Ala Ala Gly Arg Arg Asp Asp Glu Ile Ala Leu Gln Leu Pro Glu
                85                  90                  95

ctg cgg agg ctc ctg gac gtg ctg agg gcg tcc agg ggg agg ggc gcc     336
Leu Arg Arg Leu Leu Asp Val Leu Arg Ala Ser Arg Gly Arg Gly Ala
            100                 105                 110

gag gag ggc ggc gga ccc ggg agg gtg gca ctg gtg ggc aca ggg ccc     384
Glu Glu Gly Gly Gly Pro Gly Arg Val Ala Leu Val Gly Thr Gly Pro
        115                 120                 125

ggg gac ccc gag ctg ctc acg ctc aag gcg gtc cgg gcc atc gag gcg     432
Gly Asp Pro Glu Leu Leu Thr Leu Lys Ala Val Arg Ala Ile Glu Ala
    130                 135                 140

gcg gac ctg gtg ctc tac gac agg ctg gtg tct aac gac gtg ttg gac     480
Ala Asp Leu Val Leu Tyr Asp Arg Leu Val Ser Asn Asp Val Leu Asp
145                 150                 155                 160

ttg gtc ggg gag ggc gcc agg ctg ctc tat gtc ggc aag acc gcg gga     528
Leu Val Gly Glu Gly Ala Arg Leu Leu Tyr Val Gly Lys Thr Ala Gly
                165                 170                 175

tac cac agc cgc acc cag gag gag att cac gag ctg ctg ctg agc ttt     576
Tyr His Ser Arg Thr Gln Glu Glu Ile His Glu Leu Leu Leu Ser Phe
            180                 185                 190

gcg gag gcc ggt gcc aat gtt gtg cgc ttg aaa ggt ggc gat cct ctg     624
Ala Glu Ala Gly Ala Asn Val Val Arg Leu Lys Gly Gly Asp Pro Leu
        195                 200                 205

gtt ttt gga agg ggt gga gaa gag atg gat ttc tta cag caa cag gga     672

Val Phe Gly Arg Gly Gly Glu Glu Met Asp Phe Leu Gln Gln Gln Gly
    210                 215                 220

att aaa gtt gaa gtt atc cca gga att act tct gct tcg ggt att gca      720
Ile Lys Val Glu Val Ile Pro Gly Ile Thr Ser Ala Ser Gly Ile Ala
225                 230                 235                 240

gca gag cta ggc att ccg tta acc cat aga ggt gtt gct acc agt gtc      768
Ala Glu Leu Gly Ile Pro Leu Thr His Arg Gly Val Ala Thr Ser Val
                245                 250                 255

aga ttt tta act ggc cac tcc aga aat ggt ggg aca gat cca cta tat      816
Arg Phe Leu Thr Gly His Ser Arg Asn Gly Gly Thr Asp Pro Leu Tyr
            260                 265                 270

gta gct gga aat gcg gct gat cca gac act act ttg gtt gtg tat atg      864
Val Ala Gly Asn Ala Ala Asp Pro Asp Thr Thr Leu Val Val Tyr Met
        275                 280                 285

ggt ttg tca aca ctt cca tca ctt gct cca aag tta atg aag cac ggc      912
Gly Leu Ser Thr Leu Pro Ser Leu Ala Pro Lys Leu Met Lys His Gly
    290                 295                 300

ctg ccg cca gat acc cct gcc gtt gca gta gag cgc ggg act aca cct      960
Leu Pro Pro Asp Thr Pro Ala Val Ala Val Glu Arg Gly Thr Thr Pro
305                 310                 315                 320

caa caa cga acg gtg ttc gcc tcg ttg aag gat ctt gtg gat gaa gtc      1008
Gln Gln Arg Thr Val Phe Ala Ser Leu Lys Asp Leu Val Asp Glu Val
                325                 330                 335

aag tca gcc gat cta gtt tct cca act cta ata att att ggg aaa gtg      1056
Lys Ser Ala Asp Leu Val Ser Pro Thr Leu Ile Ile Ile Gly Lys Val
            340                 345                 350

gta tct ttg tca ccc ttc tgg gtt gag tca tct gaa cat gat gca ctg      1104
Val Ser Leu Ser Pro Phe Trp Val Glu Ser Ser Glu His Asp Ala Leu
        355                 360                 365

aaa gtt cag agc tcg tat gca aat gag gcc aga tga                      1140
Lys Val Gln Ser Ser Tyr Ala Asn Glu Ala Arg
    370                 375


<210>  52
<211>  379
<212>  PRT
<213>  Zea mays

<400>  52

Met Ala Leu Ala Val Arg Thr Pro Arg Phe Gln Pro Arg Pro Ala Ser
1               5                   10                  15


Ile Ser Ala Pro Thr Thr Pro Ala Ser Thr Ser Leu Ala Ala Gly Asn
            20                  25                  30


Ala Arg Pro Arg Pro Cys Ala Gly Ala Ala Val Arg Ala Ser Pro Phe
        35                  40                  45


Thr Glu Ala Thr Ser Ser Ser Arg Tyr Arg Arg Asp Ala Trp Ser Tyr
    50                  55                  60


Ala Ala Asp Gly Ser Ser Asn Ser Ala Ser Pro Ser Ser Ala Ala Ala
65                  70                  75                  80

Ala Ala Ala Gly Arg Arg Asp Asp Glu Ile Ala Leu Gln Leu Pro Glu
                85                  90                  95

Leu Arg Arg Leu Leu Asp Val Leu Arg Ala Ser Arg Gly Arg Gly Ala
            100                 105                 110

Glu Glu Gly Gly Gly Pro Gly Arg Val Ala Leu Val Gly Thr Gly Pro
        115                 120                 125

Gly Asp Pro Glu Leu Leu Thr Leu Lys Ala Val Arg Ala Ile Glu Ala
    130                 135                 140

Ala Asp Leu Val Leu Tyr Asp Arg Leu Val Ser Asn Asp Val Leu Asp
145                 150                 155                 160

Leu Val Gly Glu Gly Ala Arg Leu Leu Tyr Val Gly Lys Thr Ala Gly
                165                 170                 175

Tyr His Ser Arg Thr Gln Glu Glu Ile His Glu Leu Leu Leu Ser Phe
            180                 185                 190

Ala Glu Ala Gly Ala Asn Val Val Arg Leu Lys Gly Gly Asp Pro Leu
        195                 200                 205

Val Phe Gly Arg Gly Gly Glu Glu Met Asp Phe Leu Gln Gln Gln Gly
    210                 215                 220

Ile Lys Val Glu Val Ile Pro Gly Ile Thr Ser Ala Ser Gly Ile Ala
225                 230                 235                 240

Ala Glu Leu Gly Ile Pro Leu Thr His Arg Gly Val Ala Thr Ser Val
                245                 250                 255

Arg Phe Leu Thr Gly His Ser Arg Asn Gly Gly Thr Asp Pro Leu Tyr
            260                 265                 270

Val Ala Gly Asn Ala Ala Asp Pro Asp Thr Thr Leu Val Val Tyr Met
        275                 280                 285

Gly Leu Ser Thr Leu Pro Ser Leu Ala Pro Lys Leu Met Lys His Gly
    290                 295                 300

Leu Pro Pro Asp Thr Pro Ala Val Ala Val Glu Arg Gly Thr Thr Pro
305                 310                 315                 320

Gln Gln Arg Thr Val Phe Ala Ser Leu Lys Asp Leu Val Asp Glu Val
                325                 330                 335

Lys Ser Ala Asp Leu Val Ser Pro Thr Leu Ile Ile Ile Gly Lys Val
            340                 345                 350

Val Ser Leu Ser Pro Phe Trp Val Glu Ser Ser Glu His Asp Ala Leu
        355                 360                 365

Lys Val Gln Ser Ser Tyr Ala Asn Glu Ala Arg
    370                 375

<210>  53
<211>  663
<212>  DNA
<213>  Escherichia coli

<220>
<221>  CDS
<222>  (1)..(663)
<223>  isoprenoid biosynthesis protein (b3209)

<400>  53
atg atc aca atg aag aaa att ggc gta att ctg agc gga tgc ggc gtc      48
Met Ile Thr Met Lys Lys Ile Gly Val Ile Leu Ser Gly Cys Gly Val
1               5                   10                  15

tat gac ggt tct gaa att cat gaa gcg gtg ttg acg ttg tta gct att      96
Tyr Asp Gly Ser Glu Ile His Glu Ala Val Leu Thr Leu Leu Ala Ile
            20                  25                  30

tca cgc agc ggt gcg cag gcg gtc tgc ttt gca ccg gat aag cag cag      144
Ser Arg Ser Gly Ala Gln Ala Val Cys Phe Ala Pro Asp Lys Gln Gln
        35                  40                  45

gtt gat gtt atc aac cat tta act ggc gaa gcg atg acg gaa acg cgc      192
Val Asp Val Ile Asn His Leu Thr Gly Glu Ala Met Thr Glu Thr Arg
    50                  55                  60

aat gtg ctg att gaa gcg gca cga ata acg cgc ggt gaa atc cgt cct      240
Asn Val Leu Ile Glu Ala Ala Arg Ile Thr Arg Gly Glu Ile Arg Pro
65                  70                  75                  80

ctg gcc cag gcc gat gcc gct gaa ctg gat gcg ttg att gtg ccg ggg      288
Leu Ala Gln Ala Asp Ala Ala Glu Leu Asp Ala Leu Ile Val Pro Gly
                85                  90                  95

ggg ttt ggc gcg gcg aag aat tta agc aat ttt gcc agt ctt ggt agc      336
Gly Phe Gly Ala Ala Lys Asn Leu Ser Asn Phe Ala Ser Leu Gly Ser
            100                 105                 110

gaa tgc acc gtt gac cgt gaa tta aag gcg ctg gca caa gcg atg cat      384
Glu Cys Thr Val Asp Arg Glu Leu Lys Ala Leu Ala Gln Ala Met His
        115                 120                 125

cag gcc gga aaa ccg ctt ggt ttt atg tgt att gcc ccg gcg atg ctg      432
Gln Ala Gly Lys Pro Leu Gly Phe Met Cys Ile Ala Pro Ala Met Leu
    130                 135                 140

ccg aaa att ttc gat ttc ccg ctg cgt ttg acc atc ggt act gat atc      480
Pro Lys Ile Phe Asp Phe Pro Leu Arg Leu Thr Ile Gly Thr Asp Ile
145                 150                 155                 160

gat acc gca gaa gtg ctg gaa gag atg ggc gcg gag cat gtg ccg tgt      528
Asp Thr Ala Glu Val Leu Glu Glu Met Gly Ala Glu His Val Pro Cys
                165                 170                 175

cct gtc gat gat atc gtg gtt gat gaa gac aat aag att gtc acc acc      576
Pro Val Asp Asp Ile Val Val Asp Glu Asp Asn Lys Ile Val Thr Thr
            180                 185                 190

cca gca tat atg ctg gcg cag aac att gca gaa gcg gcg agc ggc att          624
Pro Ala Tyr Met Leu Ala Gln Asn Ile Ala Glu Ala Ala Ser Gly Ile
        195                 200                 205

gat aag ctg gtt tcc cgc gtg ctg gtt ctt gct gaa tga                      663
Asp Lys Leu Val Ser Arg Val Leu Val Leu Ala Glu
    210                 215                 220

<210>  54
<211>  220
<212>  PRT
<213>  Escherichia coli

<400>  54

Met Ile Thr Met Lys Lys Ile Gly Val Ile Leu Ser Gly Cys Gly Val
1               5                   10                  15

Tyr Asp Gly Ser Glu Ile His Glu Ala Val Leu Thr Leu Leu Ala Ile
            20                  25                  30

Ser Arg Ser Gly Ala Gln Ala Val Cys Phe Ala Pro Asp Lys Gln Gln
        35                  40                  45

Val Asp Val Ile Asn His Leu Thr Gly Glu Ala Met Thr Glu Thr Arg
    50                  55                  60

Asn Val Leu Ile Glu Ala Ala Arg Ile Thr Arg Gly Glu Ile Arg Pro
65                  70                  75                  80

Leu Ala Gln Ala Asp Ala Ala Glu Leu Asp Ala Leu Ile Val Pro Gly
                85                  90                  95

Gly Phe Gly Ala Ala Lys Asn Leu Ser Asn Phe Ala Ser Leu Gly Ser
            100                 105                 110

Glu Cys Thr Val Asp Arg Glu Leu Lys Ala Leu Ala Gln Ala Met His
        115                 120                 125

Gln Ala Gly Lys Pro Leu Gly Phe Met Cys Ile Ala Pro Ala Met Leu
    130                 135                 140

Pro Lys Ile Phe Asp Phe Pro Leu Arg Leu Thr Ile Gly Thr Asp Ile
145                 150                 155                 160

Asp Thr Ala Glu Val Leu Glu Glu Met Gly Ala Glu His Val Pro Cys
                165                 170                 175

Pro Val Asp Asp Ile Val Val Asp Glu Asp Asn Lys Ile Val Thr Thr
            180                 185                 190

Pro Ala Tyr Met Leu Ala Gln Asn Ile Ala Glu Ala Ala Ser Gly Ile
        195                 200                 205

Asp Lys Leu Val Ser Arg Val Leu Val Leu Ala Glu

210                 215                 220


<210>  55
<211>  693
<212>  DNA
<213>  Glycine max


<220>
<221>  CDS
<222>  (1)..(693)
<223>  isoprenoid biosynthesis protein (GMss34d01)

<400>  55
atg gct ccg aag aag gtt ctt ctc ctt tgc ggg gac ttc atg gaa gac      48
Met Ala Pro Lys Lys Val Leu Leu Leu Cys Gly Asp Phe Met Glu Asp
1               5                   10                  15

tat gaa gcc atg gtt cca ttt cag gca ttg caa gcc ttc ggt ctc gcc      96
Tyr Glu Ala Met Val Pro Phe Gln Ala Leu Gln Ala Phe Gly Leu Ala
            20                  25                  30

gtc gac gcc gtc tgt ccc gga aag aaa tcc ggc gat gtc tgc cgc acc      144
Val Asp Ala Val Cys Pro Gly Lys Lys Ser Gly Asp Val Cys Arg Thr
        35                  40                  45

gct gtc cat gta ctt gcc ggc gcc cag act tat agt gag acg gtt ggt      192
Ala Val His Val Leu Ala Gly Ala Gln Thr Tyr Ser Glu Thr Val Gly
    50                  55                  60

cac aat ttt tca ctc aat gca acg ttt gat gaa gtt gat gct gca agc      240
His Asn Phe Ser Leu Asn Ala Thr Phe Asp Glu Val Asp Ala Ala Ser
65                  70                  75                  80

tat gat ggg ttg tgg gtg cca ggt ggg agg gct cca gag tat ctt gct      288
Tyr Asp Gly Leu Trp Val Pro Gly Gly Arg Ala Pro Glu Tyr Leu Ala
                85                  90                  95

cat gtt cct ggt gtt gtg gaa ctg gtg acc aag ttt gtc agt ttg gga      336
His Val Pro Gly Val Val Glu Leu Val Thr Lys Phe Val Ser Leu Gly
            100                 105                 110

aaa caa att gct agc att tgt cat gga cag ttg att ctg gct gct gct      384
Lys Gln Ile Ala Ser Ile Cys His Gly Gln Leu Ile Leu Ala Ala Ala
        115                 120                 125

gga gtg gtt aaa ggc cgc acg tgc acg gct ttt cct cct gtt aaa ccc      432
Gly Val Val Lys Gly Arg Thr Cys Thr Ala Phe Pro Pro Val Lys Pro
    130                 135                 140

gtg cta gtt gct gct ggt gct cat tgg gtt gaa cct gac act gag gca      480
Val Leu Val Ala Ala Gly Ala His Trp Val Glu Pro Asp Thr Glu Ala
145                 150                 155                 160

gca aca gtg gta gat ggt gat ctc att act gca gct act tat gag ggg      528
Ala Thr Val Val Asp Gly Asp Leu Ile Thr Ala Ala Thr Tyr Glu Gly
                165                 170                 175

cac ccg gag ctc att cgc cat ttc gtg aag gca tta gga ggc aaa ata      576
His Pro Glu Leu Ile Arg His Phe Val Lys Ala Leu Gly Gly Lys Ile
            180                 185                 190

agt ggt ttt gat aag aaa atc ctc ttt att tgt ggg gat tac atg gaa      624
Ser Gly Phe Asp Lys Lys Ile Leu Phe Ile Cys Gly Asp Tyr Met Glu
        195                 200                 205

gat tat gag gtc aag gtt cct ttt cag tcc ctt cag gct tta gga tgc      672
Asp Tyr Glu Val Lys Val Pro Phe Gln Ser Leu Gln Ala Leu Gly Cys

210                 215                 220

cat gtt gat gct gtt tgc ccc                                         693
His Val Asp Ala Val Cys Pro
225                 230


<210>  56
<211>  231
<212>  PRT
<213>  Glycine max

<400>  56

Met Ala Pro Lys Lys Val Leu Leu Leu Cys Gly Asp Phe Met Glu Asp
1               5                   10                  15


Tyr Glu Ala Met Val Pro Phe Gln Ala Leu Gln Ala Phe Gly Leu Ala
            20                  25                  30


Val Asp Ala Val Cys Pro Gly Lys Lys Ser Gly Asp Val Cys Arg Thr
        35                  40                  45


Ala Val His Val Leu Ala Gly Ala Gln Thr Tyr Ser Glu Thr Val Gly
    50                  55                  60


His Asn Phe Ser Leu Asn Ala Thr Phe Asp Glu Val Asp Ala Ala Ser
65                  70                  75                  80


Tyr Asp Gly Leu Trp Val Pro Gly Gly Arg Ala Pro Glu Tyr Leu Ala
                85                  90                  95


His Val Pro Gly Val Val Glu Leu Val Thr Lys Phe Val Ser Leu Gly
            100                 105                 110


Lys Gln Ile Ala Ser Ile Cys His Gly Gln Leu Ile Leu Ala Ala Ala
        115                 120                 125


Gly Val Val Lys Gly Arg Thr Cys Thr Ala Phe Pro Pro Val Lys Pro
    130                 135                 140


Val Leu Val Ala Ala Gly Ala His Trp Val Glu Pro Asp Thr Glu Ala
145                 150                 155                 160


Ala Thr Val Val Asp Gly Asp Leu Ile Thr Ala Ala Thr Tyr Glu Gly
                165                 170                 175


His Pro Glu Leu Ile Arg His Phe Val Lys Ala Leu Gly Gly Lys Ile
            180                 185                 190


Ser Gly Phe Asp Lys Lys Ile Leu Phe Ile Cys Gly Asp Tyr Met Glu
        195                 200                 205


Asp Tyr Glu Val Lys Val Pro Phe Gln Ser Leu Gln Ala Leu Gly Cys
    210                 215                 220

His Val Asp Ala Val Cys Pro
225                 230


<210>  57
<211>  486
<212>  DNA
<213>  Helianthus annuus


<220>
<221>  CDS
<222>  (1)..(486)
<223>  isoprenoid biosynthesis protein (HA03MC1392)

<400>  57
atg ata tgt ggc gat tac atg gaa gac tct gag gcg atg gtt ccg ttt      48
Met Ile Cys Gly Asp Tyr Met Glu Asp Ser Glu Ala Met Val Pro Phe
1               5                   10                  15

cag gca ttg caa gct tac gga tta tcc gtc gat gct gtt tgc cct ggt      96
Gln Ala Leu Gln Ala Tyr Gly Leu Ser Val Asp Ala Val Cys Pro Gly
            20                  25                  30

aaa aag tcc ggc gat att tgt cgc act gct att cat cag ggt tct cct      144
Lys Lys Ser Gly Asp Ile Cys Arg Thr Ala Ile His Gln Gly Ser Pro
        35                  40                  45

cac cag aca tat tcc gag tcg cgt ggt cac aac ttc act ctc aat gcc      192
His Gln Thr Tyr Ser Glu Ser Arg Gly His Asn Phe Thr Leu Asn Ala
    50                  55                  60

acg ttt gat gaa att gac gca aac aca tat gac gga ttg gtt cta cca      240
Thr Phe Asp Glu Ile Asp Ala Asn Thr Tyr Asp Gly Leu Val Leu Pro
65                  70                  75                  80

gga gga cgt gct cca gaa tat ctc gct atg aat gaa tca gtt ctt aac      288
Gly Gly Arg Ala Pro Glu Tyr Leu Ala Met Asn Glu Ser Val Leu Asn
                85                  90                  95

ctg gtc aca cat ttt gca aaa tct gga aaa ccc att gcc tcc att tgt      336
Leu Val Thr His Phe Ala Lys Ser Gly Lys Pro Ile Ala Ser Ile Cys
            100                 105                 110

cat ggg caa ctt ata ttg gcg gct gct gct gct aaa gct gat gta ata      384
His Gly Gln Leu Ile Leu Ala Ala Ala Ala Ala Lys Ala Asp Val Ile
        115                 120                 125

aaa ggt cga aaa gtt aca gca tat gcg act gtg gga ccc gta gct tgc      432
Lys Gly Arg Lys Val Thr Ala Tyr Ala Thr Val Gly Pro Val Ala Cys
    130                 135                 140

tgc tgc tgg agc tca ttg ggt aca ccc cga gac att ggc atc atg tac      480
Cys Cys Trp Ser Ser Leu Gly Thr Pro Arg Asp Ile Gly Ile Met Tyr
145                 150                 155                 160

cgg tga                                                              486
Arg



<210>  58
<211>  161
<212>  PRT
<213>  Helianthus annuus

<400>  58
   Met Ile Cys Gly Asp Tyr Met Glu Asp Ser Glu Ala Met Val Pro Phe
   1               5                   10                  15
   Gln Ala Leu Gln Ala Tyr Gly Leu Ser Val Asp Ala Val Cys Pro Gly
               20                  25                  30
   Lys Lys Ser Gly Asp Ile Cys Arg Thr Ala Ile His Gln Gly Ser Pro
           35                  40                  45
   His Gln Thr Tyr Ser Glu Ser Arg Gly His Asn Phe Thr Leu Asn Ala
       50                  55                  60
   Thr Phe Asp Glu Ile Asp Ala Asn Thr Tyr Asp Gly Leu Val Leu Pro
   65                  70                  75                  80
   Gly Gly Arg Ala Pro Glu Tyr Leu Ala Met Asn Glu Ser Val Leu Asn
                   85                  90                  95
   Leu Val Thr His Phe Ala Lys Ser Gly Lys Pro Ile Ala Ser Ile Cys
               100                 105                 110
   His Gly Gln Leu Ile Leu Ala Ala Ala Ala Ala Lys Ala Asp Val Ile
           115                 120                 125
   Lys Gly Arg Lys Val Thr Ala Tyr Ala Thr Val Gly Pro Val Ala Cys
       130                 135                 140
   Cys Cys Trp Ser Ser Leu Gly Thr Pro Arg Asp Ile Gly Ile Met Tyr
   145                 150                 155                 160
   Arg

<210>  59
<211>  588
<212>  DNA
<213>  Escherichia coli

<220>
<221>  CDS
<222>  (1)..(588)
<223>  LysE-type translocator (b2578)

<400>  59
atg aca ccg acc ctt tta agt gct ttt tgg act tac acc ctg att acc      48
Met Thr Pro Thr Leu Leu Ser Ala Phe Trp Thr Tyr Thr Leu Ile Thr
1               5                   10                  15

gct atg acg cca gga ccg aac aat att ctc gcc ctt agc tct gct acg      96
Ala Met Thr Pro Gly Pro Asn Asn Ile Leu Ala Leu Ser Ser Ala Thr
            20                  25                  30

tcg cat gga ttt cgt caa agt acc cgc gtg ctg gca ggg atg agt ctg      144
Ser His Gly Phe Arg Gln Ser Thr Arg Val Leu Ala Gly Met Ser Leu
        35                  40                  45

gga ttt ttg att gtg atg tta ctg tgt gcg ggc att tca ttt tca ctg      192

Gly Phe Leu Ile Val Met Leu Leu Cys Ala Gly Ile Ser Phe Ser Leu
    50                  55                  60

gca gtg att gac ccg gca gcg gta cac ctt ttg agt tgg gcg ggg gcg      240
Ala Val Ile Asp Pro Ala Ala Val His Leu Leu Ser Trp Ala Gly Ala
65                  70                  75                  80

gca tat att gtc tgg ctg gcg tgg aaa atc gcc acc agc cca aca aag      288
Ala Tyr Ile Val Trp Leu Ala Trp Lys Ile Ala Thr Ser Pro Thr Lys
                85                  90                  95

gaa gac gga ctt cag gca aaa cca atc agc ttt tgg gcc agc ttt gct      336
Glu Asp Gly Leu Gln Ala Lys Pro Ile Ser Phe Trp Ala Ser Phe Ala
            100                 105                 110

ttg cag ttt gtg aac gtc aaa atc att ttg tac ggt gtt acg gca ctg      384
Leu Gln Phe Val Asn Val Lys Ile Ile Leu Tyr Gly Val Thr Ala Leu
        115                 120                 125

tcg acg ttt gtt ctg ccg caa aca cag gcg tta agc tgg gta gtt ggc      432
Ser Thr Phe Val Leu Pro Gln Thr Gln Ala Leu Ser Trp Val Val Gly
    130                 135                 140

gtc agc gtt ttg ctg gcg atg att ggg acg ttt ggc aat gtg tgc tgg      480
Val Ser Val Leu Leu Ala Met Ile Gly Thr Phe Gly Asn Val Cys Trp
145                 150                 155                 160

gcg ctg gcg ggg cat ctg ttt cag cga ttg ttt cgc cag tat ggt cgc      528
Ala Leu Ala Gly His Leu Phe Gln Arg Leu Phe Arg Gln Tyr Gly Arg
                165                 170                 175

cag tta aat atc gtg ctt gcc ctg ttg ctg gtc tat tgc gcg gta cgc      576
Gln Leu Asn Ile Val Leu Ala Leu Leu Leu Val Tyr Cys Ala Val Arg
            180                 185                 190

att ttc tat taa                                                      588
Ile Phe Tyr
        195


<210>  60
<211>  195
<212>  PRT
<213>  Escherichia coli

<400>  60

Met Thr Pro Thr Leu Leu Ser Ala Phe Trp Thr Tyr Thr Leu Ile Thr
1               5                   10                  15


Ala Met Thr Pro Gly Pro Asn Asn Ile Leu Ala Leu Ser Ser Ala Thr
            20                  25                  30


Ser His Gly Phe Arg Gln Ser Thr Arg Val Leu Ala Gly Met Ser Leu
        35                  40                  45


Gly Phe Leu Ile Val Met Leu Leu Cys Ala Gly Ile Ser Phe Ser Leu
    50                  55                  60


Ala Val Ile Asp Pro Ala Ala Val His Leu Leu Ser Trp Ala Gly Ala
65                  70                  75                  80


Ala Tyr Ile Val Trp Leu Ala Trp Lys Ile Ala Thr Ser Pro Thr Lys
                85                  90                  95

Glu Asp Gly Leu Gln Ala Lys Pro Ile Ser Phe Trp Ala Ser Phe Ala
            100                 105                 110

Leu Gln Phe Val Asn Val Lys Ile Ile Leu Tyr Gly Val Thr Ala Leu
        115                 120                 125

Ser Thr Phe Val Leu Pro Gln Thr Gln Ala Leu Ser Trp Val Val Gly
    130                 135                 140

Val Ser Val Leu Leu Ala Met Ile Gly Thr Phe Gly Asn Val Cys Trp
145                 150                 155                 160

Ala Leu Ala Gly His Leu Phe Gln Arg Leu Phe Arg Gln Tyr Gly Arg
                165                 170                 175

Gln Leu Asn Ile Val Leu Ala Leu Leu Leu Val Tyr Cys Ala Val Arg
            180                 185                 190

Ile Phe Tyr
        195

<210>  61
<211>  738
<212>  DNA
<213>  Escherichia coli

<220>
<221>  CDS
<222>  (1)..(738)
<223>  LIV-E family branched-chain amino acid transporter (b2382)

<400>  61
atg gaa agc cct act cca cag cct gct cct ggt tcg gcg acc ttc atg      48
Met Glu Ser Pro Thr Pro Gln Pro Ala Pro Gly Ser Ala Thr Phe Met
1               5                   10                  15

gaa gga tgc aaa gac agt tta ccg att gtt att agt tat att ccg gtg      96
Glu Gly Cys Lys Asp Ser Leu Pro Ile Val Ile Ser Tyr Ile Pro Val
            20                  25                  30

gcc ttt gcg ttc ggt ctg aat gcg acc cgt ctg gga ttc tct cct ctc      144
Ala Phe Ala Phe Gly Leu Asn Ala Thr Arg Leu Gly Phe Ser Pro Leu
        35                  40                  45

gaa agc gtt ttt ttc tcc tgc atc att tat gca ggc gcg agc cag ttc      192
Glu Ser Val Phe Phe Ser Cys Ile Ile Tyr Ala Gly Ala Ser Gln Phe
    50                  55                  60

gtc att acc gcg atg ctg gca gcc ggg agt agt ttg tgg att gct gca      240
Val Ile Thr Ala Met Leu Ala Ala Gly Ser Ser Leu Trp Ile Ala Ala
65                  70                  75                  80

ctg acc gtc atg gca atg gat gtt cgc cat gtg ttg tat ggc ccg tca      288
Leu Thr Val Met Ala Met Asp Val Arg His Val Leu Tyr Gly Pro Ser
                85                  90                  95

ctg cgt agc cgt att att cag cgt ctg caa aaa tcg aaa acc gcc ctg      336
Leu Arg Ser Arg Ile Ile Gln Arg Leu Gln Lys Ser Lys Thr Ala Leu
            100                 105                 110

tgg gcg ttt ggc ctg acg gat gag gtt ttt gcc gcc gca acc gca aaa 384
Trp Ala Phe Gly Leu Thr Asp Glu Val Phe Ala Ala Ala Thr Ala Lys
        115                 120                 125

ctg gta cgc aat aat cgc cgc tgg agc gag aac tgg atg atc ggc att 432
Leu Val Arg Asn Asn Arg Arg Trp Ser Glu Asn Trp Met Ile Gly Ile
    130                 135                 140

gcc ttc agt tca tgg tca tcg tgg gta ttt ggt acg gta ata ggg gca 480
Ala Phe Ser Ser Trp Ser Ser Trp Val Phe Gly Thr Val Ile Gly Ala
145                 150                 155                 160

ttc tcc ggc agc ggc ttg ctg caa ggt tat ccc gcc gtt gaa gct gca 528
Phe Ser Gly Ser Gly Leu Leu Gln Gly Tyr Pro Ala Val Glu Ala Ala
                165                 170                 175

tta ggt ttt atg ctt ccg gca ctc ttt atg agt ttc ctg ctc gcc tct 576
Leu Gly Phe Met Leu Pro Ala Leu Phe Met Ser Phe Leu Leu Ala Ser
            180                 185                 190

ttc cag cgc aaa caa tct ctt tgc gtt acc gca gcg tta gtt ggt gcc 624
Phe Gln Arg Lys Gln Ser Leu Cys Val Thr Ala Ala Leu Val Gly Ala
        195                 200                 205

ctt gca ggc gta acg cta ttt tct att ccc gtc gcc att ctg gca ggc 672
Leu Ala Gly Val Thr Leu Phe Ser Ile Pro Val Ala Ile Leu Ala Gly
    210                 215                 220

att gtc tgt ggc tgc ctc act gcg tta atc cag gca ttc tgg caa gga 720
Ile Val Cys Gly Cys Leu Thr Ala Leu Ile Gln Ala Phe Trp Gln Gly
225                 230                 235                 240

gcg ccc gat gag cta taa 738
Ala Pro Asp Glu Leu
                245

<210> 62
<211> 245
<212> PRT
<213> Escherichia coli

<400> 62

Met Glu Ser Pro Thr Pro Gln Pro Ala Pro Gly Ser Ala Thr Phe Met
1               5                   10                  15

Glu Gly Cys Lys Asp Ser Leu Pro Ile Val Ile Ser Tyr Ile Pro Val
            20                  25                  30

Ala Phe Ala Phe Gly Leu Asn Ala Thr Arg Leu Gly Phe Ser Pro Leu
        35                  40                  45

Glu Ser Val Phe Phe Ser Cys Ile Ile Tyr Ala Gly Ala Ser Gln Phe
    50                  55                  60

Val Ile Thr Ala Met Leu Ala Ala Gly Ser Ser Leu Trp Ile Ala Ala
65                  70                  75                  80

Leu Thr Val Met Ala Met Asp Val Arg His Val Leu Tyr Gly Pro Ser
                85                  90                  95

Leu Arg Ser Arg Ile Ile Gln Arg Leu Gln Lys Ser Lys Thr Ala Leu
            100                 105                 110

Trp Ala Phe Gly Leu Thr Asp Glu Val Phe Ala Ala Ala Thr Ala Lys
        115                 120                 125

Leu Val Arg Asn Asn Arg Arg Trp Ser Glu Asn Trp Met Ile Gly Ile
    130                 135                 140

Ala Phe Ser Ser Trp Ser Ser Trp Val Phe Gly Thr Val Ile Gly Ala
145                 150                 155                 160

Phe Ser Gly Ser Gly Leu Leu Gln Gly Tyr Pro Ala Val Glu Ala Ala
                165                 170                 175

Leu Gly Phe Met Leu Pro Ala Leu Phe Met Ser Phe Leu Leu Ala Ser
            180                 185                 190

Phe Gln Arg Lys Gln Ser Leu Cys Val Thr Ala Ala Leu Val Gly Ala
        195                 200                 205

Leu Ala Gly Val Thr Leu Phe Ser Ile Pro Val Ala Ile Leu Ala Gly
    210                 215                 220

Ile Val Cys Gly Cys Leu Thr Ala Leu Ile Gln Ala Phe Trp Gln Gly
225                 230                 235                 240

Ala Pro Asp Glu Leu
                245

<210>  63
<211>  309
<212>  DNA
<213>  Escherichia coli

<220>
<221>  CDS
<222>  (1)..(309)
<223>  DNA-binding protein (b3285)

<400>  63
atg acg gaa ccg gaa gaa att ctg gaa aac ttg caa ttt gga ttg cac      48
Met Thr Glu Pro Glu Glu Ile Leu Glu Asn Leu Gln Phe Gly Leu His
1               5                   10                  15

tgg ttg cca gac gcc cct gaa aat tca ttt tat tca cca gat cag caa      96
Trp Leu Pro Asp Ala Pro Glu Asn Ser Phe Tyr Ser Pro Asp Gln Gln
            20                  25                  30

gac gtg gca ttg cca ttt cct gag ctc ctg gct aac gta gga gat gag      144
Asp Val Ala Leu Pro Phe Pro Glu Leu Leu Ala Asn Val Gly Asp Glu
        35                  40                  45

gta aca cct gtt gac gtc gtc gct gaa cgt gcc ggc caa cct gtg cca      192
Val Thr Pro Val Asp Val Val Ala Glu Arg Ala Gly Gln Pro Val Pro
    50                  55                  60

gag gta gtt act caa cta ctc gaa ctg gag tta gca gga tgg atc gca      240

Glu Val Val Thr Gln Leu Leu Glu Leu Glu Leu Ala Gly Trp Ile Ala
65                  70                  75                  80

gct gta ccc ggc ggc tat gtc cga ttg agg agg gca tgc cat gtt cga       288
Ala Val Pro Gly Gly Tyr Val Arg Leu Arg Arg Ala Cys His Val Arg
                85                  90                  95

cgt act aat gta ttt gtt taa                                           309
Arg Thr Asn Val Phe Val
            100

<210>  64
<211>  102
<212>  PRT
<213>  Escherichia coli

<400>  64

Met Thr Glu Pro Glu Glu Ile Leu Glu Asn Leu Gln Phe Gly Leu His
1               5                   10                  15

Trp Leu Pro Asp Ala Pro Glu Asn Ser Phe Tyr Ser Pro Asp Gln Gln
            20                  25                  30

Asp Val Ala Leu Pro Phe Pro Glu Leu Leu Ala Asn Val Gly Asp Glu
        35                  40                  45

Val Thr Pro Val Asp Val Val Ala Glu Arg Ala Gly Gln Pro Val Pro
    50                  55                  60

Glu Val Val Thr Gln Leu Leu Glu Leu Glu Leu Ala Gly Trp Ile Ala
65                  70                  75                  80

Ala Val Pro Gly Gly Tyr Val Arg Leu Arg Arg Ala Cys His Val Arg
                85                  90                  95

Arg Thr Asn Val Phe Val
            100

<210>  65
<211>  1659
<212>  DNA
<213>  Escherichia coli

<220>
<221>  CDS
<222>  (1)..(1659)
<223>  FLiF protein (b1938)

<400>  65
atg aat gcg act gca gcc cag aca aaa tct ctt gag tgg ctt aat cgc        48
Met Asn Ala Thr Ala Ala Gln Thr Lys Ser Leu Glu Trp Leu Asn Arg
1               5                   10                  15

ctg cgt gcg aat ccg aaa att cca ttg att gtt gcc ggt tcc gcg gca        96
Leu Arg Ala Asn Pro Lys Ile Pro Leu Ile Val Ala Gly Ser Ala Ala
            20                  25                  30

gtg gcg gtc atg gtc gca ctg atc ctg tgg gcg aaa gcc ccc gac tac       144
Val Ala Val Met Val Ala Leu Ile Leu Trp Ala Lys Ala Pro Asp Tyr

35                  40                  45

cgc aca tta ttc agc aat ctt tcc gat cag gat ggt ggc gca att gtc      192
Arg Thr Leu Phe Ser Asn Leu Ser Asp Gln Asp Gly Gly Ala Ile Val
    50                  55                  60

agc caa ctg acg caa atg aat att cct tac cgc ttc agc gaa gcc agc      240
Ser Gln Leu Thr Gln Met Asn Ile Pro Tyr Arg Phe Ser Glu Ala Ser
65                  70                  75                  80

ggc gct att gaa gtt ccg gca gat aaa gtt cac gaa ctg cgt ctg cgc      288
Gly Ala Ile Glu Val Pro Ala Asp Lys Val His Glu Leu Arg Leu Arg
                85                  90                  95

ctg gca caa caa ggt ttg cca aaa ggc ggc gcg gtc ggt ttc gaa ctg      336
Leu Ala Gln Gln Gly Leu Pro Lys Gly Gly Ala Val Gly Phe Glu Leu
            100                 105                 110

ctt gat cag gaa aag ttt ggt atc agc cag ttc agc gaa cag gtg aat      384
Leu Asp Gln Glu Lys Phe Gly Ile Ser Gln Phe Ser Glu Gln Val Asn
        115                 120                 125

tat cag cgg gcg ctg gaa ggc gag ctt tct cgt acc atc gaa act atc      432
Tyr Gln Arg Ala Leu Glu Gly Glu Leu Ser Arg Thr Ile Glu Thr Ile
    130                 135                 140

ggc ccg gta aaa ggg gcg cgc gta cat ctg gca atg ccg aaa ccg tct      480
Gly Pro Val Lys Gly Ala Arg Val His Leu Ala Met Pro Lys Pro Ser
145                 150                 155                 160

tta ttc gtc cgt gaa caa aaa tcc cct tct gca tcg gtg acg gta aat      528
Leu Phe Val Arg Glu Gln Lys Ser Pro Ser Ala Ser Val Thr Val Asn
                165                 170                 175

ctg tta ccc ggc cgc gca ctc gat gaa ggg caa att agc gcc att gtg      576
Leu Leu Pro Gly Arg Ala Leu Asp Glu Gly Gln Ile Ser Ala Ile Val
            180                 185                 190

cat ctg gtt tcc agc gcc gtt gct ggt ctg ccg ccg gga aac gtc acg      624
His Leu Val Ser Ser Ala Val Ala Gly Leu Pro Pro Gly Asn Val Thr
        195                 200                 205

ctg gtg gat cag ggc gga cat ctg tta acc cag tcc aat acc agc ggg      672
Leu Val Asp Gln Gly Gly His Leu Leu Thr Gln Ser Asn Thr Ser Gly
    210                 215                 220

cgc gat ctt aat gac gct cag ttg aaa tat gcc agc gat gtc gaa ggc      720
Arg Asp Leu Asn Asp Ala Gln Leu Lys Tyr Ala Ser Asp Val Glu Gly
225                 230                 235                 240

cgt att cag cgg cgt att gaa gcg atc ctg tcg cct att gtt ggt aac      768
Arg Ile Gln Arg Arg Ile Glu Ala Ile Leu Ser Pro Ile Val Gly Asn
                245                 250                 255

ggt aat att cac gcc cag gtt acg gcg cag ctg gac ttc gcc agt aaa      816
Gly Asn Ile His Ala Gln Val Thr Ala Gln Leu Asp Phe Ala Ser Lys
            260                 265                 270

gaa caa acg gaa gaa cag tat cgc cct aac ggt gat gaa tct cat gcg      864
Glu Gln Thr Glu Glu Gln Tyr Arg Pro Asn Gly Asp Glu Ser His Ala
        275                 280                 285

gcg ctt cgt tca cgc cag ctt aat gag agc gag caa agc ggt tcc ggt      912
Ala Leu Arg Ser Arg Gln Leu Asn Glu Ser Glu Gln Ser Gly Ser Gly
    290                 295                 300

tat ccg ggc ggc gta ccg ggg gcg ttg tcg aat caa ccg gca cct gcg      960
Tyr Pro Gly Gly Val Pro Gly Ala Leu Ser Asn Gln Pro Ala Pro Ala
305                 310                 315                 320

aat aac gcg cca atc agc acg cct ccg gca aat caa aat aac cgc cag     1008
Asn Asn Ala Pro Ile Ser Thr Pro Pro Ala Asn Gln Asn Asn Arg Gln
                325                 330                 335

cag cag gcg agc acc acc agc aat agt ggg ccg cgt agc aca cag cgg     1056
Gln Gln Ala Ser Thr Thr Ser Asn Ser Gly Pro Arg Ser Thr Gln Arg
            340                 345                 350

aat gaa acc agt aac tac gaa gtc gat cgc acc att cgt cat acc aaa     1104
Asn Glu Thr Ser Asn Tyr Glu Val Asp Arg Thr Ile Arg His Thr Lys
        355                 360                 365

atg aac gtg ggc gat gtg caa cgt ctg tca gtc gcg gtc gtg gtg aat     1152
Met Asn Val Gly Asp Val Gln Arg Leu Ser Val Ala Val Val Val Asn
    370                 375                 380

tac aaa acc ttg cca gat ggc aaa ccg ttg cct ctc agc aac gaa cag     1200
Tyr Lys Thr Leu Pro Asp Gly Lys Pro Leu Pro Leu Ser Asn Glu Gln
385                 390                 395                 400

atg aag caa att gaa gat ctg acc cgc gag gcg atg ggc ttt tct gaa     1248
Met Lys Gln Ile Glu Asp Leu Thr Arg Glu Ala Met Gly Phe Ser Glu
                405                 410                 415

aaa cgc ggt gac tcg ctc aat gtc gtt aac tcg ccg ttc aat agc agt     1296
Lys Arg Gly Asp Ser Leu Asn Val Val Asn Ser Pro Phe Asn Ser Ser
            420                 425                 430

gac gaa agc ggc gga gaa ctg cca ttc tgg caa cag caa gcg ttt atc     1344
Asp Glu Ser Gly Gly Glu Leu Pro Phe Trp Gln Gln Gln Ala Phe Ile
        435                 440                 445

gat cag tta ctt gct gcc ggt cgc tgg ttg ctg gta ctg ctg gtg gcg     1392
Asp Gln Leu Leu Ala Ala Gly Arg Trp Leu Leu Val Leu Leu Val Ala
    450                 455                 460

tgg ctg ctg tgg cgg aaa gcg gta cgt ccg cag cta aca cgt cgc gct     1440
Trp Leu Leu Trp Arg Lys Ala Val Arg Pro Gln Leu Thr Arg Arg Ala
465                 470                 475                 480

gag gcg atg aaa gct gta cag caa cag gcg cag gcc cgc gag gaa gtg     1488
Glu Ala Met Lys Ala Val Gln Gln Gln Ala Gln Ala Arg Glu Glu Val
                485                 490                 495

gaa gat gcg gtg gaa gtc cgc ctg agc aaa gac gaa caa cta caa caa     1536
Glu Asp Ala Val Glu Val Arg Leu Ser Lys Asp Glu Gln Leu Gln Gln
            500                 505                 510

cgg cgc gct aac caa cgt ctg ggg gca gaa gtc atg agc cag cgt atc     1584
Arg Arg Ala Asn Gln Arg Leu Gly Ala Glu Val Met Ser Gln Arg Ile
        515                 520                 525

cgt gaa atg tct gat aac gat ccg cgc gtg gtg gcg ctg gtc att cgc     1632
Arg Glu Met Ser Asp Asn Asp Pro Arg Val Val Ala Leu Val Ile Arg
    530                 535                 540

cag tgg ata aat aac gat cat gag taa                                 1659
Gln Trp Ile Asn Asn Asp His Glu
545                 550

<210>  66
<211>  552
<212>  PRT
<213>  Escherichia coli

<400>  66

Met Asn Ala Thr Ala Ala Gln Thr Lys Ser Leu Glu Trp Leu Asn Arg
1               5                   10                  15

Leu Arg Ala Asn Pro Lys Ile Pro Leu Ile Val Ala Gly Ser Ala Ala
            20                  25                  30

Val Ala Val Met Val Ala Leu Ile Leu Trp Ala Lys Ala Pro Asp Tyr
        35                  40                  45

Arg Thr Leu Phe Ser Asn Leu Ser Asp Gln Asp Gly Gly Ala Ile Val
    50                  55                  60

Ser Gln Leu Thr Gln Met Asn Ile Pro Tyr Arg Phe Ser Glu Ala Ser
65                  70                  75                  80

Gly Ala Ile Glu Val Pro Ala Asp Lys Val His Glu Leu Arg Leu Arg
                85                  90                  95

Leu Ala Gln Gln Gly Leu Pro Lys Gly Gly Ala Val Gly Phe Glu Leu
            100                 105                 110

Leu Asp Gln Glu Lys Phe Gly Ile Ser Gln Phe Ser Glu Gln Val Asn
        115                 120                 125

Tyr Gln Arg Ala Leu Glu Gly Glu Leu Ser Arg Thr Ile Glu Thr Ile
    130                 135                 140

Gly Pro Val Lys Gly Ala Arg Val His Leu Ala Met Pro Lys Pro Ser
145                 150                 155                 160

Leu Phe Val Arg Glu Gln Lys Ser Pro Ser Ala Ser Val Thr Val Asn
                165                 170                 175

Leu Leu Pro Gly Arg Ala Leu Asp Glu Gly Gln Ile Ser Ala Ile Val
            180                 185                 190

His Leu Val Ser Ser Ala Val Ala Gly Leu Pro Pro Gly Asn Val Thr
        195                 200                 205

Leu Val Asp Gln Gly Gly His Leu Leu Thr Gln Ser Asn Thr Ser Gly
    210                 215                 220

Arg Asp Leu Asn Asp Ala Gln Leu Lys Tyr Ala Ser Asp Val Glu Gly
225                 230                 235                 240

Arg Ile Gln Arg Arg Ile Glu Ala Ile Leu Ser Pro Ile Val Gly Asn
                245                 250                 255

Gly Asn Ile His Ala Gln Val Thr Ala Gln Leu Asp Phe Ala Ser Lys
            260                 265                 270

Glu Gln Thr Glu Glu Gln Tyr Arg Pro Asn Gly Asp Glu Ser His Ala

275                 280                 285

Ala Leu Arg Ser Arg Gln Leu Asn Glu Ser Glu Gln Ser Gly Ser Gly
    290                 295                 300

Tyr Pro Gly Gly Val Pro Gly Ala Leu Ser Asn Gln Pro Ala Pro Ala
305                 310                 315                 320

Asn Asn Ala Pro Ile Ser Thr Pro Pro Ala Asn Gln Asn Asn Arg Gln
                325                 330                 335

Gln Gln Ala Ser Thr Thr Ser Asn Ser Gly Pro Arg Ser Thr Gln Arg
            340                 345                 350

Asn Glu Thr Ser Asn Tyr Glu Val Asp Arg Thr Ile Arg His Thr Lys
        355                 360                 365

Met Asn Val Gly Asp Val Gln Arg Leu Ser Val Ala Val Val Val Asn
    370                 375                 380

Tyr Lys Thr Leu Pro Asp Gly Lys Pro Leu Pro Leu Ser Asn Glu Gln
385                 390                 395                 400

Met Lys Gln Ile Glu Asp Leu Thr Arg Glu Ala Met Gly Phe Ser Glu
                405                 410                 415

Lys Arg Gly Asp Ser Leu Asn Val Val Asn Ser Pro Phe Asn Ser Ser
            420                 425                 430

Asp Glu Ser Gly Gly Glu Leu Pro Phe Trp Gln Gln Gln Ala Phe Ile
        435                 440                 445

Asp Gln Leu Leu Ala Ala Gly Arg Trp Leu Leu Val Leu Leu Val Ala
    450                 455                 460

Trp Leu Leu Trp Arg Lys Ala Val Arg Pro Gln Leu Thr Arg Arg Ala
465                 470                 475                 480

Glu Ala Met Lys Ala Val Gln Gln Gln Ala Gln Ala Arg Glu Glu Val
                485                 490                 495

Glu Asp Ala Val Glu Val Arg Leu Ser Lys Asp Glu Gln Leu Gln Gln
            500                 505                 510

Arg Arg Ala Asn Gln Arg Leu Gly Ala Glu Val Met Ser Gln Arg Ile
        515                 520                 525

Arg Glu Met Ser Asp Asn Asp Pro Arg Val Val Ala Leu Val Ile Arg
    530                 535                 540

Gln Trp Ile Asn Asn Asp His Glu
545                 550

<210>  67
<211>  1671
<212>  DNA
<213>  Synechocystis sp. PCC 6803

<220>
<221>  CDS
<222>  (1)..(1671)
<223>  GTP cyclohydrolase II (SLL1894)

<400>  67
atg ttt gat gcc att gat gca gcc cta gct gac att aaa gcg ggc aag      48
Met Phe Asp Ala Ile Asp Ala Ala Leu Ala Asp Ile Lys Ala Gly Lys
1               5                   10                  15

gcc gtg gtg gtg gtg gac gac gaa aat cga gaa aat gaa ggg gat tta      96
Ala Val Val Val Val Asp Asp Glu Asn Arg Glu Asn Glu Gly Asp Leu
            20                  25                  30

atc tgt gcg gcc caa ttt gcc acc cct gct cta gtt aac ttt atg gca      144
Ile Cys Ala Ala Gln Phe Ala Thr Pro Ala Leu Val Asn Phe Met Ala
        35                  40                  45

gtg gaa gcc cgg ggc ttg att tgc cta gcc atg act ggt gat cgc ctg      192
Val Glu Ala Arg Gly Leu Ile Cys Leu Ala Met Thr Gly Asp Arg Leu
    50                  55                  60

gac gaa ttg gat ctg ccc ttg atg gtc agt aaa aac acc gac agc aac      240
Asp Glu Leu Asp Leu Pro Leu Met Val Ser Lys Asn Thr Asp Ser Asn
65                  70                  75                  80

caa acc gct ttc acc gtc agc att gat gca gcc ccc cat ttg ggc gta      288
Gln Thr Ala Phe Thr Val Ser Ile Asp Ala Ala Pro His Leu Gly Val
                85                  90                  95

acc aca ggg att tcc gcc gaa gac cgg gcc cgg acg atc cag att gcc      336
Thr Thr Gly Ile Ser Ala Glu Asp Arg Ala Arg Thr Ile Gln Ile Ala
            100                 105                 110

att aac ccc gtc acc cgc ccg gag gac ctt tcc cga cct ggc cat att      384
Ile Asn Pro Val Thr Arg Pro Glu Asp Leu Ser Arg Pro Gly His Ile
        115                 120                 125

ttc cct ctg cgg gcc aaa act ggg gga gta ctg aaa cga gcc ggc cac      432
Phe Pro Leu Arg Ala Lys Thr Gly Gly Val Leu Lys Arg Ala Gly His
    130                 135                 140

acc gag gcg gca gtc gat ctt tct cgc cta gca gga ctc tat cca gcg      480
Thr Glu Ala Ala Val Asp Leu Ser Arg Leu Ala Gly Leu Tyr Pro Ala
145                 150                 155                 160

ggg gta att tgc gaa att caa aat gcc gat ggc tcc atg gcc cgc cta      528
Gly Val Ile Cys Glu Ile Gln Asn Ala Asp Gly Ser Met Ala Arg Leu
                165                 170                 175

ccg gaa ttg gtg gag tat gcc cgc aaa tat gac ctc aaa ctg atc agc      576
Pro Glu Leu Val Glu Tyr Ala Arg Lys Tyr Asp Leu Lys Leu Ile Ser
            180                 185                 190

att gcc gat tta atc agt tac cga ctc caa cat gac cgc ttt gtg caa      624
Ile Ala Asp Leu Ile Ser Tyr Arg Leu Gln His Asp Arg Phe Val Gln
        195                 200                 205

cgg gaa acc atc tgt gaa ttt ccc agc cag ttt ggg gag ttt aaa ctc      672
Arg Glu Thr Ile Cys Glu Phe Pro Ser Gln Phe Gly Glu Phe Lys Leu
    210                 215                 220

tac gcc tac cgc aat tta ttg gat caa aca gag cac att gcc att gtg 720
Tyr Ala Tyr Arg Asn Leu Leu Asp Gln Thr Glu His Ile Ala Ile Val
225                 230                 235                 240

aag ggt gat cct agt gaa ttt ggt caa cag ccg gtg atg gtg cgg atg 768
Lys Gly Asp Pro Ser Glu Phe Gly Gln Gln Pro Val Met Val Arg Met
                245                 250                 255

cat tca gaa tgt tta acg ggg gat gcc ctg ggt tct ctc cgt tgt gat 816
His Ser Glu Cys Leu Thr Gly Asp Ala Leu Gly Ser Leu Arg Cys Asp
            260                 265                 270

tgc cgc atg caa ctc cag gcg gcc ctc aaa atg ctg gaa aac cat ggt 864
Cys Arg Met Gln Leu Gln Ala Ala Leu Lys Met Leu Glu Asn His Gly
        275                 280                 285

ttg ggg gtg gtg gtt tat ctc cgc cag gaa ggc cgg ggt att ggt ttg 912
Leu Gly Val Val Val Tyr Leu Arg Gln Glu Gly Arg Gly Ile Gly Leu
    290                 295                 300

gtc aat aag ctc aag gcc tat tcc cta cag gat ttg ggc tat gac acc 960
Val Asn Lys Leu Lys Ala Tyr Ser Leu Gln Asp Leu Gly Tyr Asp Thr
305                 310                 315                 320

gtg gaa gcg aat gaa aga tta ggt ttt ccg gcg gat ctg cgg gac tac 1008
Val Glu Ala Asn Glu Arg Leu Gly Phe Pro Ala Asp Leu Arg Asp Tyr
                325                 330                 335

ggc atg ggg gcg caa atg ctc aac gat tta ggg gtg aaa caa atc cgt 1056
Gly Met Gly Ala Gln Met Leu Asn Asp Leu Gly Val Lys Gln Ile Arg
            340                 345                 350

ttg atc acc aac aat ccc cgt aaa att gcc gga ctg aaa ggc tac ggt 1104
Leu Ile Thr Asn Asn Pro Arg Lys Ile Ala Gly Leu Lys Gly Tyr Gly
        355                 360                 365

cta gaa att gtc gaa cgg gtg ccc ctg tta att gag gcc aac gac tat 1152
Leu Glu Ile Val Glu Arg Val Pro Leu Leu Ile Glu Ala Asn Asp Tyr
    370                 375                 380

aac tcc cat tac ctg acc acc aag gca gaa aaa cta ggg cac tgg tta 1200
Asn Ser His Tyr Leu Thr Thr Lys Ala Glu Lys Leu Gly His Trp Leu
385                 390                 395                 400

gtc aaa aac tat ctg ctg gcg atc gcc att aaa ttt act ccc aac gtt 1248
Val Lys Asn Tyr Leu Leu Ala Ile Ala Ile Lys Phe Thr Pro Asn Val
                405                 410                 415

gct tcg gcc caa caa cgg tac gaa aaa ttg gaa aaa atc cgg gcc ctg 1296
Ala Ser Ala Gln Gln Arg Tyr Glu Lys Leu Glu Lys Ile Arg Ala Leu
            420                 425                 430

tta cag gga acc ccc ctc atg gtg cat gaa gac aac cgc ccc gtg gcg 1344
Leu Gln Gly Thr Pro Leu Met Val His Glu Asp Asn Arg Pro Val Ala
        435                 440                 445

atc gcc cta ttt ggc aaa aat agc cta att ttc cac gtc ggc cta gac 1392
Ile Ala Leu Phe Gly Lys Asn Ser Leu Ile Phe His Val Gly Leu Asp
    450                 455                 460

caa aac tta cca tcg ggc cag cct tgg caa aag cac gac cat tct ccc 1440
Gln Asn Leu Pro Ser Gly Gln Pro Trp Gln Lys His Asp His Ser Pro
465                 470                 475                 480

tac ccc gct ttg gta aag gat ttt ctc gta cag ctc aaa caa tgg cca 1488
Tyr Pro Ala Leu Val Lys Asp Phe Leu Val Gln Leu Lys Gln Trp Pro
                485                 490                 495

gac cta cag gct cta gca ttc ctc atc gcc caa ggg aaa gac ccc atg 1536
Asp Leu Gln Ala Leu Ala Phe Leu Ile Ala Gln Gly Lys Asp Pro Met
            500                 505                 510

gaa act ctc caa gtc agt ctg gac cgg gaa aat gta tcc ttt gcc gac 1584
Glu Thr Leu Gln Val Ser Leu Asp Arg Glu Asn Val Ser Phe Ala Asp
        515                 520                 525

ctc acc gcc gac gcc cta aac caa tgg cag ccc caa acg gtg tac acc 1632
Leu Thr Ala Asp Ala Leu Asn Gln Trp Gln Pro Gln Thr Val Tyr Thr
    530                 535                 540

tac agc aag caa ggc agc gga gaa atg act aac aga taa 1671
Tyr Ser Lys Gln Gly Ser Gly Glu Met Thr Asn Arg
545                 550                 555

<210> 68
<211> 556
<212> PRT
<213> Synechocystis sp. PCC 6803

<400> 68

Met Phe Asp Ala Ile Asp Ala Ala Leu Ala Asp Ile Lys Ala Gly Lys
1               5                   10                  15

Ala Val Val Val Val Asp Asp Glu Asn Arg Glu Asn Glu Gly Asp Leu
            20                  25                  30

Ile Cys Ala Ala Gln Phe Ala Thr Pro Ala Leu Val Asn Phe Met Ala
        35                  40                  45

Val Glu Ala Arg Gly Leu Ile Cys Leu Ala Met Thr Gly Asp Arg Leu
    50                  55                  60

Asp Glu Leu Asp Leu Pro Leu Met Val Ser Lys Asn Thr Asp Ser Asn
65                  70                  75                  80

Gln Thr Ala Phe Thr Val Ser Ile Asp Ala Ala Pro His Leu Gly Val
                85                  90                  95

Thr Thr Gly Ile Ser Ala Glu Asp Arg Ala Arg Thr Ile Gln Ile Ala
            100                 105                 110

Ile Asn Pro Val Thr Arg Pro Glu Asp Leu Ser Arg Pro Gly His Ile
        115                 120                 125

Phe Pro Leu Arg Ala Lys Thr Gly Gly Val Leu Lys Arg Ala Gly His
    130                 135                 140

Thr Glu Ala Ala Val Asp Leu Ser Arg Leu Ala Gly Leu Tyr Pro Ala
145                 150                 155                 160

Gly Val Ile Cys Glu Ile Gln Asn Ala Asp Gly Ser Met Ala Arg Leu
                165                 170                 175

Pro Glu Leu Val Glu Tyr Ala Arg Lys Tyr Asp Leu Lys Leu Ile Ser

            180                 185                 190

Ile Ala Asp Leu Ile Ser Tyr Arg Leu Gln His Asp Arg Phe Val Gln
        195                 200                 205

Arg Glu Thr Ile Cys Glu Phe Pro Ser Gln Phe Gly Glu Phe Lys Leu
    210                 215                 220

Tyr Ala Tyr Arg Asn Leu Leu Asp Gln Thr Glu His Ile Ala Ile Val
225                 230                 235                 240

Lys Gly Asp Pro Ser Glu Phe Gly Gln Gln Pro Val Met Val Arg Met
                245                 250                 255

His Ser Glu Cys Leu Thr Gly Asp Ala Leu Gly Ser Leu Arg Cys Asp
            260                 265                 270

Cys Arg Met Gln Leu Gln Ala Ala Leu Lys Met Leu Glu Asn His Gly
        275                 280                 285

Leu Gly Val Val Val Tyr Leu Arg Gln Glu Gly Arg Gly Ile Gly Leu
    290                 295                 300

Val Asn Lys Leu Lys Ala Tyr Ser Leu Gln Asp Leu Gly Tyr Asp Thr
305                 310                 315                 320

Val Glu Ala Asn Glu Arg Leu Gly Phe Pro Ala Asp Leu Arg Asp Tyr
                325                 330                 335

Gly Met Gly Ala Gln Met Leu Asn Asp Leu Gly Val Lys Gln Ile Arg
            340                 345                 350

Leu Ile Thr Asn Asn Pro Arg Lys Ile Ala Gly Leu Lys Gly Tyr Gly
        355                 360                 365

Leu Glu Ile Val Glu Arg Val Pro Leu Leu Ile Glu Ala Asn Asp Tyr
    370                 375                 380

Asn Ser His Tyr Leu Thr Thr Lys Ala Glu Lys Leu Gly His Trp Leu
385                 390                 395                 400

Val Lys Asn Tyr Leu Leu Ala Ile Ala Ile Lys Phe Thr Pro Asn Val
                405                 410                 415

Ala Ser Ala Gln Gln Arg Tyr Glu Lys Leu Glu Lys Ile Arg Ala Leu
            420                 425                 430

Leu Gln Gly Thr Pro Leu Met Val His Glu Asp Asn Arg Pro Val Ala
        435                 440                 445

Ile Ala Leu Phe Gly Lys Asn Ser Leu Ile Phe His Val Gly Leu Asp
    450                 455                 460

Gln Asn Leu Pro Ser Gly Gln Pro Trp Gln Lys His Asp His Ser Pro
465                 470                 475                 480

Tyr Pro Ala Leu Val Lys Asp Phe Leu Val Gln Leu Lys Gln Trp Pro
                485                 490                 495

Asp Leu Gln Ala Leu Ala Phe Leu Ile Ala Gln Gly Lys Asp Pro Met
            500                 505                 510

Glu Thr Leu Gln Val Ser Leu Asp Arg Glu Asn Val Ser Phe Ala Asp
        515                 520                 525

Leu Thr Ala Asp Ala Leu Asn Gln Trp Gln Pro Gln Thr Val Tyr Thr
    530                 535                 540

Tyr Ser Lys Gln Gly Ser Gly Glu Met Thr Asn Arg
545                 550                 555

<210>  69
<211>  1635
<212>  DNA
<213>  Glycine max

<220>
<221>  CDS
<222>  (1)..(1635)
<223>  GTP cyclohydrolase II (GM08000037)

<400>  69
atg gct tct ttc aat ctc tct tat tct tcc tca cca gcc ctc tca cgt      48
Met Ala Ser Phe Asn Leu Ser Tyr Ser Ser Ser Pro Ala Leu Ser Arg
1               5                   10                  15

ccc cgg gca agc aaa cac ttc aaa ttc ttc aat gat gtg cct ttt gtc      96
Pro Arg Ala Ser Lys His Phe Lys Phe Phe Asn Asp Val Pro Phe Val
            20                  25                  30

cga atg aat tct aaa ttt ccc tca agt gat aat gct ttt ggt aga atc      144
Arg Met Asn Ser Lys Phe Pro Ser Ser Asp Asn Ala Phe Gly Arg Ile
        35                  40                  45

aaa gct atg ttg atc tct gga ggg ggt gaa cta ctt tcc cat cca aat      192
Lys Ala Met Leu Ile Ser Gly Gly Gly Glu Leu Leu Ser His Pro Asn
    50                  55                  60

ggc aat gac att gtg ata ggt aca aat ctg gtt gga gat gaa tct gtt      240
Gly Asn Asp Ile Val Ile Gly Thr Asn Leu Val Gly Asp Glu Ser Val
65                  70                  75                  80

caa att gag gta cag cct aat gga aca ctg gca gat gac aca ggg cta      288
Gln Ile Glu Val Gln Pro Asn Gly Thr Leu Ala Asp Asp Thr Gly Leu
                85                  90                  95

aca agc aat gtt ttc tct atg gac aac aat gaa ttt gat tcg gac agc      336
Thr Ser Asn Val Phe Ser Met Asp Asn Asn Glu Phe Asp Ser Asp Ser
            100                 105                 110

ccc tca gca ggt ttt gct tcc att cct gag gca att gaa gac att cgc      384
Pro Ser Ala Gly Phe Ala Ser Ile Pro Glu Ala Ile Glu Asp Ile Arg
        115                 120                 125

cag gga aag atg gta gtg gtt gtg gac gat gaa gat cga gaa aat gaa 432
Gln Gly Lys Met Val Val Val Val Asp Asp Glu Asp Arg Glu Asn Glu
    130                 135                 140

gga gac ttg ata atg gca gca cag ttg gca aca ccc gac gca atg gct 480
Gly Asp Leu Ile Met Ala Ala Gln Leu Ala Thr Pro Asp Ala Met Ala
145                 150                 155                 160

ttt att gta aag cat gga act ggt ata gtt tgt gta agc atg aaa gag 528
Phe Ile Val Lys His Gly Thr Gly Ile Val Cys Val Ser Met Lys Glu
                165                 170                 175

gaa gat ctg gat aga ttg gaa ctt cct ttg atg gtg aac agt aag gac 576
Glu Asp Leu Asp Arg Leu Glu Leu Pro Leu Met Val Asn Ser Lys Asp
            180                 185                 190

aat gat gag aaa ctc tgt acg gca ttc act gtg aca gtg gat gct aaa 624
Asn Asp Glu Lys Leu Cys Thr Ala Phe Thr Val Thr Val Asp Ala Lys
        195                 200                 205

cat ggt aca acc act ggg gtg tca gct cac gat agg gca aca aca gtc 672
His Gly Thr Thr Thr Gly Val Ser Ala His Asp Arg Ala Thr Thr Val
    210                 215                 220

ttg gcc ctt gcg tct aga gat tct act cca ggc gat ttc aat cgc ccg 720
Leu Ala Leu Ala Ser Arg Asp Ser Thr Pro Gly Asp Phe Asn Arg Pro
225                 230                 235                 240

ggc cat att ttc cca cta aaa tac agg gaa ggt ggt gtc ttg aag aga 768
Gly His Ile Phe Pro Leu Lys Tyr Arg Glu Gly Gly Val Leu Lys Arg
                245                 250                 255

gcc gga cat aca gaa gct tct gtt gat ctt gct gta ctt gct ggt ttg 816
Ala Gly His Thr Glu Ala Ser Val Asp Leu Ala Val Leu Ala Gly Leu
            260                 265                 270

gat cca gtg gca gtt ctg tgt gag att gta gat gat gat ggt tcc atg 864
Asp Pro Val Ala Val Leu Cys Glu Ile Val Asp Asp Asp Gly Ser Met
        275                 280                 285

gct aga ctg cca aag ctt cgc cag ttt gca gag cat gaa aat ttg aaa 912
Ala Arg Leu Pro Lys Leu Arg Gln Phe Ala Glu His Glu Asn Leu Lys
    290                 295                 300

att gta tct att gct gac tta ata aga tat aga aga aaa agg gat aag 960
Ile Val Ser Ile Ala Asp Leu Ile Arg Tyr Arg Arg Lys Arg Asp Lys
305                 310                 315                 320

tta gtg gat cgc tct tct gct gca cag ata cct aca atg tgg gga cca 1008
Leu Val Asp Arg Ser Ser Ala Ala Gln Ile Pro Thr Met Trp Gly Pro
                325                 330                 335

ttc aca gct tac tgt tac aga tca ctt tta gac ggg att gag cat att 1056
Phe Thr Ala Tyr Cys Tyr Arg Ser Leu Leu Asp Gly Ile Glu His Ile
            340                 345                 350

gca atg gtt aag ggt gaa att gga gat gga caa gat gtt ctt gtg aga 1104
Ala Met Val Lys Gly Glu Ile Gly Asp Gly Gln Asp Val Leu Val Arg
        355                 360                 365

gta cac tca gag tgt ctg aca gga gac ata ttt gga tct gcc aga tgt 1152
Val His Ser Glu Cys Leu Thr Gly Asp Ile Phe Gly Ser Ala Arg Cys
    370                 375                 380

gac tgc gga aat cag ctt gca ctt gca atg caa cag att gag gct gct 1200
Asp Cys Gly Asn Gln Leu Ala Leu Ala Met Gln Gln Ile Glu Ala Ala
385                 390                 395                 400

ggt agg ggt gtg ctg gta tat ctc cgt gga cac gaa gga agg ggt att 1248
Gly Arg Gly Val Leu Val Tyr Leu Arg Gly His Glu Gly Arg Gly Ile
                405                 410                 415

gga ttg ggc cac aag ctt cgt gct tat aac cta cag gat gat gga cga 1296
Gly Leu Gly His Lys Leu Arg Ala Tyr Asn Leu Gln Asp Asp Gly Arg
            420                 425                 430

gat act gtt gaa gcc aat gag gaa ttg gga ttg cca gtt gac tcc agg 1344
Asp Thr Val Glu Ala Asn Glu Glu Leu Gly Leu Pro Val Asp Ser Arg
        435                 440                 445

gag tat ggc att ggt gca cag atg ttg agg gac ttg ggt gta cga tct 1392
Glu Tyr Gly Ile Gly Ala Gln Met Leu Arg Asp Leu Gly Val Arg Ser
    450                 455                 460

atg aag ctg atg aca aac aat ccg gca aaa tat att ggg ctc aaa ggt 1440
Met Lys Leu Met Thr Asn Asn Pro Ala Lys Tyr Ile Gly Leu Lys Gly
465                 470                 475                 480

tat ggt ttg aca gtt tcg ggt agg atc cca ttg tta act ctt atc act 1488
Tyr Gly Leu Thr Val Ser Gly Arg Ile Pro Leu Leu Thr Leu Ile Thr
                485                 490                 495

tcg gag aac aag aga tat tta gag acc aaa cgt gtg aaa atg ggt cac 1536
Ser Glu Asn Lys Arg Tyr Leu Glu Thr Lys Arg Val Lys Met Gly His
            500                 505                 510

ata tat ggc acg gaa ttt aac ggc cga ttg agc act cat gac agt ggt 1584
Ile Tyr Gly Thr Glu Phe Asn Gly Arg Leu Ser Thr His Asp Ser Gly
        515                 520                 525

aat ggt aat gcc acc agg att gag gac tct aat gct gtt act ggc tta 1632
Asn Gly Asn Ala Thr Arg Ile Glu Asp Ser Asn Ala Val Thr Gly Leu
    530                 535                 540

taa 1635


<210> 70
<211> 544
<212> PRT
<213> Glycine max

<400> 70

Met Ala Ser Phe Asn Leu Ser Tyr Ser Ser Ser Pro Ala Leu Ser Arg
1               5                   10                  15


Pro Arg Ala Ser Lys His Phe Lys Phe Phe Asn Asp Val Pro Phe Val
            20                  25                  30


Arg Met Asn Ser Lys Phe Pro Ser Ser Asp Asn Ala Phe Gly Arg Ile
        35                  40                  45


Lys Ala Met Leu Ile Ser Gly Gly Gly Glu Leu Leu Ser His Pro Asn
    50                  55                  60


Gly Asn Asp Ile Val Ile Gly Thr Asn Leu Val Gly Asp Glu Ser Val
65                  70                  75                  80


Gln Ile Glu Val Gln Pro Asn Gly Thr Leu Ala Asp Asp Thr Gly Leu
                85                  90                  95

Thr Ser Asn Val Phe Ser Met Asp Asn Asn Glu Phe Asp Ser Asp Ser
            100                 105                 110

Pro Ser Ala Gly Phe Ala Ser Ile Pro Glu Ala Ile Glu Asp Ile Arg
        115                 120                 125

Gln Gly Lys Met Val Val Val Val Asp Asp Glu Asp Arg Glu Asn Glu
    130                 135                 140

Gly Asp Leu Ile Met Ala Ala Gln Leu Ala Thr Pro Asp Ala Met Ala
145                 150                 155                 160

Phe Ile Val Lys His Gly Thr Gly Ile Val Cys Val Ser Met Lys Glu
                165                 170                 175

Glu Asp Leu Asp Arg Leu Glu Leu Pro Leu Met Val Asn Ser Lys Asp
            180                 185                 190

Asn Asp Glu Lys Leu Cys Thr Ala Phe Thr Val Thr Val Asp Ala Lys
        195                 200                 205

His Gly Thr Thr Thr Gly Val Ser Ala His Asp Arg Ala Thr Thr Val
    210                 215                 220

Leu Ala Leu Ala Ser Arg Asp Ser Thr Pro Gly Asp Phe Asn Arg Pro
225                 230                 235                 240

Gly His Ile Phe Pro Leu Lys Tyr Arg Glu Gly Gly Val Leu Lys Arg
                245                 250                 255

Ala Gly His Thr Glu Ala Ser Val Asp Leu Ala Val Leu Ala Gly Leu
            260                 265                 270

Asp Pro Val Ala Val Leu Cys Glu Ile Val Asp Asp Asp Gly Ser Met
        275                 280                 285

Ala Arg Leu Pro Lys Leu Arg Gln Phe Ala Glu His Glu Asn Leu Lys
    290                 295                 300

Ile Val Ser Ile Ala Asp Leu Ile Arg Tyr Arg Arg Lys Arg Asp Lys
305                 310                 315                 320

Leu Val Asp Arg Ser Ser Ala Ala Gln Ile Pro Thr Met Trp Gly Pro
                325                 330                 335

Phe Thr Ala Tyr Cys Tyr Arg Ser Leu Leu Asp Gly Ile Glu His Ile
            340                 345                 350

Ala Met Val Lys Gly Glu Ile Gly Asp Gly Gln Asp Val Leu Val Arg
        355                 360                 365

Val His Ser Glu Cys Leu Thr Gly Asp Ile Phe Gly Ser Ala Arg Cys
    370                 375                 380

Asp Cys Gly Asn Gln Leu Ala Leu Ala Met Gln Gln Ile Glu Ala Ala
385                 390                 395                 400

Gly Arg Gly Val Leu Val Tyr Leu Arg Gly His Glu Gly Arg Gly Ile
                405                 410                 415

Gly Leu Gly His Lys Leu Arg Ala Tyr Asn Leu Gln Asp Asp Gly Arg
            420                 425                 430

Asp Thr Val Glu Ala Asn Glu Glu Leu Gly Leu Pro Val Asp Ser Arg
        435                 440                 445

Glu Tyr Gly Ile Gly Ala Gln Met Leu Arg Asp Leu Gly Val Arg Ser
    450                 455                 460

Met Lys Leu Met Thr Asn Asn Pro Ala Lys Tyr Ile Gly Leu Lys Gly
465                 470                 475                 480

Tyr Gly Leu Thr Val Ser Gly Arg Ile Pro Leu Leu Thr Leu Ile Thr
                485                 490                 495

Ser Glu Asn Lys Arg Tyr Leu Glu Thr Lys Arg Val Lys Met Gly His
            500                 505                 510

Ile Tyr Gly Thr Glu Phe Asn Gly Arg Leu Ser Thr His Asp Ser Gly
        515                 520                 525

Asn Gly Asn Ala Thr Arg Ile Glu Asp Ser Asn Ala Val Thr Gly Leu
    530                 535                 540

<210>  71
<211>  495
<212>  DNA
<213>  Synechocystis sp. PCC 6803

<220>
<221>  CDS
<222>  (1)..(495)
<223>  lumazine synthase (SLL1282)

<400>  71
atg aca gtt tat gaa ggg tcg ttt act cct ccg gcc cgg cca ttt cgt      48
Met Thr Val Tyr Glu Gly Ser Phe Thr Pro Pro Ala Arg Pro Phe Arg
1               5                   10                  15

ttt gcc ctt gta att gcc cgt ttc aat gat tta gta acg gaa aag cta      96
Phe Ala Leu Val Ile Ala Arg Phe Asn Asp Leu Val Thr Glu Lys Leu
            20                  25                  30

ctt tct ggt tgc caa gat tgc ctc aaa cgt cat ggc att gat gtg gac      144
Leu Ser Gly Cys Gln Asp Cys Leu Lys Arg His Gly Ile Asp Val Asp
        35                  40                  45

ccc gcc ggt acc cag gtg gac tat att tgg gtt ccc ggt agc ttt gag      192

Pro Ala Gly Thr Gln Val Asp Tyr Ile Trp Val Pro Gly Ser Phe Glu
    50                  55                  60

gtg cca ttg gta acc cgt aag ttg gca gtg tcg ggg cag tat gat gcc      240
Val Pro Leu Val Thr Arg Lys Leu Ala Val Ser Gly Gln Tyr Asp Ala
65                  70                  75                  80

att atc tgc cta ggg gcg gtg att cgg gga caa acc ccc cat ttt gat      288
Ile Ile Cys Leu Gly Ala Val Ile Arg Gly Gln Thr Pro His Phe Asp
                85                  90                  95

ttt gtg gcc gga gaa gcg gcc aag ggc att gcg gcg atc gcc agc cag      336
Phe Val Ala Gly Glu Ala Ala Lys Gly Ile Ala Ala Ile Ala Ser Gln
            100                 105                 110

act ggg gta ccg gta att ttt ggc att tta acc acg gat acc atg caa      384
Thr Gly Val Pro Val Ile Phe Gly Ile Leu Thr Thr Asp Thr Met Gln
        115                 120                 125

cag gcc cta gaa agg gcg ggg att aag agt aac cac ggc tgg ggc tac      432
Gln Ala Leu Glu Arg Ala Gly Ile Lys Ser Asn His Gly Trp Gly Tyr
    130                 135                 140

gcc atg aat gca ttg gaa atg gct agt ttg atg cgg gcc atg gct cct      480
Ala Met Asn Ala Leu Glu Met Ala Ser Leu Met Arg Ala Met Ala Pro
145                 150                 155                 160

ttg acg gaa ggt taa                                                  495
Leu Thr Glu Gly

<210>  72
<211>  164
<212>  PRT
<213>  Synechocystis sp. PCC 6803

<400>  72

Met Thr Val Tyr Glu Gly Ser Phe Thr Pro Pro Ala Arg Pro Phe Arg
1               5                   10                  15

Phe Ala Leu Val Ile Ala Arg Phe Asn Asp Leu Val Thr Glu Lys Leu
            20                  25                  30

Leu Ser Gly Cys Gln Asp Cys Leu Lys Arg His Gly Ile Asp Val Asp
        35                  40                  45

Pro Ala Gly Thr Gln Val Asp Tyr Ile Trp Val Pro Gly Ser Phe Glu
    50                  55                  60

Val Pro Leu Val Thr Arg Lys Leu Ala Val Ser Gly Gln Tyr Asp Ala
65                  70                  75                  80

Ile Ile Cys Leu Gly Ala Val Ile Arg Gly Gln Thr Pro His Phe Asp
                85                  90                  95

Phe Val Ala Gly Glu Ala Ala Lys Gly Ile Ala Ala Ile Ala Ser Gln
            100                 105                 110

Thr Gly Val Pro Val Ile Phe Gly Ile Leu Thr Thr Asp Thr Met Gln
        115                 120                 125

Gln Ala Leu Glu Arg Ala Gly Ile Lys Ser Asn His Gly Trp Gly Tyr
    130                 135                 140

Ala Met Asn Ala Leu Glu Met Ala Ser Leu Met Arg Ala Met Ala Pro
145                 150                 155                 160

Leu Thr Glu Gly

<210>  73
<211>  687
<212>  DNA
<213>  Glycine max

<220>
<221>  CDS
<222>  (1)..(687)
<223>  lumazine synthase (GM06MC29296)

<400>  73
atg gct tca ttt gtt tct acc cac tgt atc ctt cct agc ggg gtt gga      48
Met Ala Ser Phe Val Ser Thr His Cys Ile Leu Pro Ser Gly Val Gly
1               5                   10                  15

ttt gtg cat gac caa gat cgt tct tgt ggt aga act aac act ttg cat      96
Phe Val His Asp Gln Asp Arg Ser Cys Gly Arg Thr Asn Thr Leu His
            20                  25                  30

ctt caa aat gct cct aaa agc cct agc tct ctc tcc ttt ttg ctc tcc      144
Leu Gln Asn Ala Pro Lys Ser Pro Ser Ser Leu Ser Phe Leu Leu Ser
        35                  40                  45

gtg cga gaa agt aga tca gat gcc gtt gtt gtt cag agc aag ggt cga      192
Val Arg Glu Ser Arg Ser Asp Ala Val Val Val Gln Ser Lys Gly Arg
    50                  55                  60

tcg tct ttt tct caa aca gct gct gtg agg cat ctt aca ggt tct gtt      240
Ser Ser Phe Ser Gln Thr Ala Ala Val Arg His Leu Thr Gly Ser Val
65                  70                  75                  80

acc aga gcc cat gga ctt cgt ttt gct gtg gtt gtt gct aga ttt aat      288
Thr Arg Ala His Gly Leu Arg Phe Ala Val Val Val Ala Arg Phe Asn
                85                  90                  95

gaa att gtt acc aaa ccg ctc ttg gaa ggt gct ttg ggt act ttc aat      336
Glu Ile Val Thr Lys Pro Leu Leu Glu Gly Ala Leu Gly Thr Phe Asn
            100                 105                 110

aat tat tca gtt caa gat gag gat atc gat gtt gtg tgg gta cct ggt      384
Asn Tyr Ser Val Gln Asp Glu Asp Ile Asp Val Val Trp Val Pro Gly
        115                 120                 125

agt ttt gaa att ggt gtt gtt gca gaa agg ctt gga aaa tca ggc aaa      432
Ser Phe Glu Ile Gly Val Val Ala Glu Arg Leu Gly Lys Ser Gly Lys
    130                 135                 140

tat gat gca atc gta tgc ata gga gct gtg gta cga gga gat aca acc      480
Tyr Asp Ala Ile Val Cys Ile Gly Ala Val Val Arg Gly Asp Thr Thr
145                 150                 155                 160

cac tat gat gca gtt gct aat tca gca gca tca gga gtt ctt tca gcc      528
His Tyr Asp Ala Val Ala Asn Ser Ala Ala Ser Gly Val Leu Ser Ala
                165                 170                 175

agt cta aac tca ggt gtt cca tgc ata ttt ggt gtc cta aca tgt gat 576
Ser Leu Asn Ser Gly Val Pro Cys Ile Phe Gly Val Leu Thr Cys Asp
            180                 185                 190

gat atg gat cag gct cta aat aga gct ggt gga aag tct ggg aat aag 624
Asp Met Asp Gln Ala Leu Asn Arg Ala Gly Gly Lys Ser Gly Asn Lys
        195                 200                 205

ggt gct gag gct gca ttg act gct att gag atg gca tct ttg ttc gag 672
Gly Ala Glu Ala Ala Leu Thr Ala Ile Glu Met Ala Ser Leu Phe Glu
    210                 215                 220

cac cat ctg aag tag 687
His His Leu Lys
225

<210> 74
<211> 228
<212> PRT
<213> Glycine max

<400> 74

Met Ala Ser Phe Val Ser Thr His Cys Ile Leu Pro Ser Gly Val Gly
1               5                   10                  15

Phe Val His Asp Gln Asp Arg Ser Cys Gly Arg Thr Asn Thr Leu His
            20                  25                  30

Leu Gln Asn Ala Pro Lys Ser Pro Ser Ser Leu Ser Phe Leu Leu Ser
        35                  40                  45

Val Arg Glu Ser Arg Ser Asp Ala Val Val Val Gln Ser Lys Gly Arg
    50                  55                  60

Ser Ser Phe Ser Gln Thr Ala Ala Val Arg His Leu Thr Gly Ser Val
65                  70                  75                  80

Thr Arg Ala His Gly Leu Arg Phe Ala Val Val Val Ala Arg Phe Asn
                85                  90                  95

Glu Ile Val Thr Lys Pro Leu Leu Glu Gly Ala Leu Gly Thr Phe Asn
            100                 105                 110

Asn Tyr Ser Val Gln Asp Glu Asp Ile Asp Val Val Trp Val Pro Gly
        115                 120                 125

Ser Phe Glu Ile Gly Val Val Ala Glu Arg Leu Gly Lys Ser Gly Lys
    130                 135                 140

Tyr Asp Ala Ile Val Cys Ile Gly Ala Val Val Arg Gly Asp Thr Thr
145                 150                 155                 160

His Tyr Asp Ala Val Ala Asn Ser Ala Ala Ser Gly Val Leu Ser Ala
                165                 170                 175

Ser Leu Asn Ser Gly Val Pro Cys Ile Phe Gly Val Leu Thr Cys Asp
            180                 185                 190

Asp Met Asp Gln Ala Leu Asn Arg Ala Gly Gly Lys Ser Gly Asn Lys
        195                 200                 205

Gly Ala Glu Ala Ala Leu Thr Ala Ile Glu Met Ala Ser Leu Phe Glu
    210                 215                 220

His His Leu Lys
225

<210>  75
<211>  648
<212>  DNA
<213>  Zea mays

<220>
<221>  CDS
<222>  (1)..(648)
<223>  lumazine synthase (ZM07MC00430)

<400>  75
atg gca act gct cct gtg aca tcc tcc atg gct acg aac tcc acc tgt        48
Met Ala Thr Ala Pro Val Thr Ser Ser Met Ala Thr Asn Ser Thr Cys
1               5                   10                  15

gtg cgg ctg cct agc atg ccc ctg agg cgt gca ccc gcc atc gtc tcc        96
Val Arg Leu Pro Ser Met Pro Leu Arg Arg Ala Pro Ala Ile Val Ser
            20                  25                  30

ttt gcc tct cgt cca tgg tca acc gca atg acc tca tgt act gga ttg       144
Phe Ala Ser Arg Pro Trp Ser Thr Ala Met Thr Ser Cys Thr Gly Leu
        35                  40                  45

tcg cgg cac tct gat gtg gtt gcc gcc gcg ggg cac cag aag ttg atg       192
Ser Arg His Ser Asp Val Val Ala Ala Ala Gly His Gln Lys Leu Met
    50                  55                  60

ggg tct ttg aac aac aac gag gga ctc atg ttt ggg gtg gtt gtg gca       240
Gly Ser Leu Asn Asn Asn Glu Gly Leu Met Phe Gly Val Val Val Ala
65                  70                  75                  80

cgg ttc aac gag gtc gtg aca aat ttg tta cta cag ggt gcc ctg gaa       288
Arg Phe Asn Glu Val Val Thr Asn Leu Leu Leu Gln Gly Ala Leu Glu
                85                  90                  95

act ttc gag cga tac tct gtt aaa gca gaa aat ata aca gtt gtt agt       336
Thr Phe Glu Arg Tyr Ser Val Lys Ala Glu Asn Ile Thr Val Val Ser
            100                 105                 110

gtt cct gga agc ttt gaa gtt cct ata acg gca caa aag ctt ggg aaa       384
Val Pro Gly Ser Phe Glu Val Pro Ile Thr Ala Gln Lys Leu Gly Lys
        115                 120                 125

tct gga aaa ttt gat gca att ctg tgc att gga gct gtg att aga ggt       432
Ser Gly Lys Phe Asp Ala Ile Leu Cys Ile Gly Ala Val Ile Arg Gly
    130                 135                 140

gac aca acc cac tat gat gcc gtt gca aac tca gct gca tca ggt gta       480
Asp Thr Thr His Tyr Asp Ala Val Ala Asn Ser Ala Ala Ser Gly Val
145                 150                 155                 160

ctc aat gct gga tta tct gct ggt gtt cct tgt gta ttt ggt gtt cta       528

Leu Asn Ala Gly Leu Ser Ala Gly Val Pro Cys Val Phe Gly Val Leu
                165                 170                 175

acc tgt gaa gac atg gat cag gca ctg aac cgt gct ggt ggc aag gct       576
Thr Cys Glu Asp Met Asp Gln Ala Leu Asn Arg Ala Gly Gly Lys Ala
            180                 185                 190

gga aac aaa gga gct gaa gct gct ctt act gcc att gag atg gcc tcg       624
Gly Asn Lys Gly Ala Glu Ala Ala Leu Thr Ala Ile Glu Met Ala Ser
        195                 200                 205

ctg ttc cgg cat cac tta ggc tga                                       648
Leu Phe Arg His His Leu Gly
    210                 215


<210>  76
<211>  215
<212>  PRT
<213>  Zea mays

<400>  76

Met Ala Thr Ala Pro Val Thr Ser Ser Met Ala Thr Asn Ser Thr Cys
1               5                   10                  15

Val Arg Leu Pro Ser Met Pro Leu Arg Arg Ala Pro Ala Ile Val Ser
            20                  25                  30

Phe Ala Ser Arg Pro Trp Ser Thr Ala Met Thr Ser Cys Thr Gly Leu
        35                  40                  45

Ser Arg His Ser Asp Val Val Ala Ala Ala Gly His Gln Lys Leu Met
    50                  55                  60

Gly Ser Leu Asn Asn Asn Glu Gly Leu Met Phe Gly Val Val Val Ala
65                  70                  75                  80

Arg Phe Asn Glu Val Val Thr Asn Leu Leu Leu Gln Gly Ala Leu Glu
                85                  90                  95

Thr Phe Glu Arg Tyr Ser Val Lys Ala Glu Asn Ile Thr Val Val Ser
            100                 105                 110

Val Pro Gly Ser Phe Glu Val Pro Ile Thr Ala Gln Lys Leu Gly Lys
        115                 120                 125

Ser Gly Lys Phe Asp Ala Ile Leu Cys Ile Gly Ala Val Ile Arg Gly
    130                 135                 140

Asp Thr Thr His Tyr Asp Ala Val Ala Asn Ser Ala Ala Ser Gly Val
145                 150                 155                 160

Leu Asn Ala Gly Leu Ser Ala Gly Val Pro Cys Val Phe Gly Val Leu
                165                 170                 175

Thr Cys Glu Asp Met Asp Gln Ala Leu Asn Arg Ala Gly Gly Lys Ala
            180                 185                 190

Gly Asn Lys Gly Ala Glu Ala Ala Leu Thr Ala Ile Glu Met Ala Ser
        195                 200                 205

Leu Phe Arg His His Leu Gly
    210                 215

<210>  77
<211>  654
<212>  DNA
<213>  Zea mays

<220>
<221>  CDS
<222>  (1)..(654)
<223>  lumazine synthase (ZM07MC23187)

<400>  77
atg ccg gcg atg gca act gct cct gtg aaa ttc tct gtg gct acg aac      48
Met Pro Ala Met Ala Thr Ala Pro Val Lys Phe Ser Val Ala Thr Asn
1               5                   10                  15

tcc cca tgt gtc cag cct agc ttg ccc ctg agg cgt aca cct gcc gcc      96
Ser Pro Cys Val Gln Pro Ser Leu Pro Leu Arg Arg Thr Pro Ala Ala
            20                  25                  30

atc tcc ttc cct tgt cgt cca tgg tca gcc gca atg aac tca tgt act      144
Ile Ser Phe Pro Cys Arg Pro Trp Ser Ala Ala Met Asn Ser Cys Thr
        35                  40                  45

gga ttg ttg agg cgc tct gac gtg gtt gca gcc gcg ggg cac cag aag      192
Gly Leu Leu Arg Arg Ser Asp Val Val Ala Ala Ala Gly His Gln Lys
    50                  55                  60

ttg atg gga tct ttg acc act aac gag gga ctc agg ttt ggg gtg gtt      240
Leu Met Gly Ser Leu Thr Thr Asn Glu Gly Leu Arg Phe Gly Val Val
65                  70                  75                  80

gtg gca cgg ttc aac gag gtc gtg aca aat ttg tta cta cag ggt gcc      288
Val Ala Arg Phe Asn Glu Val Val Thr Asn Leu Leu Leu Gln Gly Ala
                85                  90                  95

ctg gaa act ttc gag cga tac tct gtt aaa gaa gaa aat ata aca gtt      336
Leu Glu Thr Phe Glu Arg Tyr Ser Val Lys Glu Glu Asn Ile Thr Val
            100                 105                 110

gtt agt gtt ccc gga agc ttt gag gtt cct ata aca gca caa aag ctt      384
Val Ser Val Pro Gly Ser Phe Glu Val Pro Ile Thr Ala Gln Lys Leu
        115                 120                 125

ggg aaa tct ggg aaa ttt gat gca att ctg tgc att gga gct gtg att      432
Gly Lys Ser Gly Lys Phe Asp Ala Ile Leu Cys Ile Gly Ala Val Ile
    130                 135                 140

aga ggt gac aca acc cac tat gat gct gtt gca aac tca gct gca tca      480
Arg Gly Asp Thr Thr His Tyr Asp Ala Val Ala Asn Ser Ala Ala Ser
145                 150                 155                 160

ggt gta ctc aat gct gga tta tct gct ggt gtt cct tgt gta ttt ggt      528
Gly Val Leu Asn Ala Gly Leu Ser Ala Gly Val Pro Cys Val Phe Gly
                165                 170                 175

gtt cta acc tgt gaa gac atg gat cag gcg cta aac cgc gct ggt ggc      576
Val Leu Thr Cys Glu Asp Met Asp Gln Ala Leu Asn Arg Ala Gly Gly
            180                 185                 190

aag gct gga aac aag ggt gct gaa gct gct ctt act gcc att gag atg 624
Lys Ala Gly Asn Lys Gly Ala Glu Ala Ala Leu Thr Ala Ile Glu Met
        195                 200                 205

gcc tct ctg ttc cgg cat cac tta ggc tga 654
Ala Ser Leu Phe Arg His His Leu Gly
    210                 215

<210> 78
<211> 217
<212> PRT
<213> Zea mays

<400> 78

Met Pro Ala Met Ala Thr Ala Pro Val Lys Phe Ser Val Ala Thr Asn
1               5                   10                  15

Ser Pro Cys Val Gln Pro Ser Leu Pro Leu Arg Arg Thr Pro Ala Ala
            20                  25                  30

Ile Ser Phe Pro Cys Arg Pro Trp Ser Ala Ala Met Asn Ser Cys Thr
        35                  40                  45

Gly Leu Leu Arg Arg Ser Asp Val Val Ala Ala Ala Gly His Gln Lys
    50                  55                  60

Leu Met Gly Ser Leu Thr Thr Asn Glu Gly Leu Arg Phe Gly Val Val
65                  70                  75                  80

Val Ala Arg Phe Asn Glu Val Val Thr Asn Leu Leu Leu Gln Gly Ala
                85                  90                  95

Leu Glu Thr Phe Glu Arg Tyr Ser Val Lys Glu Glu Asn Ile Thr Val
            100                 105                 110

Val Ser Val Pro Gly Ser Phe Glu Val Pro Ile Thr Ala Gln Lys Leu
        115                 120                 125

Gly Lys Ser Gly Lys Phe Asp Ala Ile Leu Cys Ile Gly Ala Val Ile
    130                 135                 140

Arg Gly Asp Thr Thr His Tyr Asp Ala Val Ala Asn Ser Ala Ala Ser
145                 150                 155                 160

Gly Val Leu Asn Ala Gly Leu Ser Ala Gly Val Pro Cys Val Phe Gly
                165                 170                 175

Val Leu Thr Cys Glu Asp Met Asp Gln Ala Leu Asn Arg Ala Gly Gly
            180                 185                 190

Lys Ala Gly Asn Lys Gly Ala Glu Ala Ala Leu Thr Ala Ile Glu Met
        195                 200                 205

Ala Ser Leu Phe Arg His His Leu Gly
    210                 215

<210>  79
<211>  864
<212>  DNA
<213>  Escherichia coli

<220>
<221>  CDS
<222>  (1)..(864)
<223>  pdxY (b1636)

<400>  79
atg atg aaa aat att ctc gct atc cag tct cac gtt gtt tat ggt cat      48
Met Met Lys Asn Ile Leu Ala Ile Gln Ser His Val Val Tyr Gly His
1               5                   10                  15

gcg ggt aac agt gcg gca gag ttt ccg atg cgc cgc ctg ggc gcg aac      96
Ala Gly Asn Ser Ala Ala Glu Phe Pro Met Arg Arg Leu Gly Ala Asn
            20                  25                  30

gtc tgg ccg ctg aac acc gtt caa ttt tct aat cac acc caa tac ggc      144
Val Trp Pro Leu Asn Thr Val Gln Phe Ser Asn His Thr Gln Tyr Gly
        35                  40                  45

aaa tgg act ggc tgc gtg atg ccg ccc agc cat tta acc gaa att gtg      192
Lys Trp Thr Gly Cys Val Met Pro Pro Ser His Leu Thr Glu Ile Val
    50                  55                  60

caa ggc att gcc gcc att gat aaa tta cac acc tgt gat gcc gta tta      240
Gln Gly Ile Ala Ala Ile Asp Lys Leu His Thr Cys Asp Ala Val Leu
65                  70                  75                  80

agt ggc tat ctg gga tcg gcg gag cag ggt gaa cat atc ctc ggt atc      288
Ser Gly Tyr Leu Gly Ser Ala Glu Gln Gly Glu His Ile Leu Gly Ile
                85                  90                  95

gtc cgt cag gtg aaa gcc gcg aat ccg cag gcg aaa tat ttt tgc gat      336
Val Arg Gln Val Lys Ala Ala Asn Pro Gln Ala Lys Tyr Phe Cys Asp
            100                 105                 110

ccg gta atg ggt cat ccg gaa aaa ggc tgt atc gtt gca ccg ggt gtc      384
Pro Val Met Gly His Pro Glu Lys Gly Cys Ile Val Ala Pro Gly Val
        115                 120                 125

gca gag ttt cat gtg cgg cac ggt ttg cct gcc agc gat atc att gcg      432
Ala Glu Phe His Val Arg His Gly Leu Pro Ala Ser Asp Ile Ile Ala
    130                 135                 140

cca aat ctg gtt gag ctg gaa ata ctc tgt gag cat gcg gta aat aac      480
Pro Asn Leu Val Glu Leu Glu Ile Leu Cys Glu His Ala Val Asn Asn
145                 150                 155                 160

gtc gaa gaa gcg gtt ctg gca gcg cgc gaa ctc att gcg caa ggg cca      528
Val Glu Glu Ala Val Leu Ala Ala Arg Glu Leu Ile Ala Gln Gly Pro
                165                 170                 175

caa att gtg ttg gtt aaa cac ctg gcg cga gct ggc tac agc cgt gac      576
Gln Ile Val Leu Val Lys His Leu Ala Arg Ala Gly Tyr Ser Arg Asp
            180                 185                 190

cgt ttt gaa atg ctg ctg gtc acc gcc gat gaa gcc tgg cat atc agc      624
Arg Phe Glu Met Leu Leu Val Thr Ala Asp Glu Ala Trp His Ile Ser
        195                 200                 205

cgt ccg ctg gtg gat ttt ggt atg cgc cag ccg gta ggt gtt ggt gat      672

Arg Pro Leu Val Asp Phe Gly Met Arg Gln Pro Val Gly Val Gly Asp
    210                 215                 220

gtg acg agc ggt tta ctg ctg gtg aaa ctg ctt cag ggg gca acg ctg         720
Val Thr Ser Gly Leu Leu Leu Val Lys Leu Leu Gln Gly Ala Thr Leu
225                 230                 235                 240

cag gag gcg ctg gaa cat gtg acc gct gca gtc tac gaa atc atg gtg         768
Gln Glu Ala Leu Glu His Val Thr Ala Ala Val Tyr Glu Ile Met Val
                245                 250                 255

acc acc aaa gca atg cag gaa tat gag ctg caa gtg gtg gct gct cag         816
Thr Thr Lys Ala Met Gln Glu Tyr Glu Leu Gln Val Val Ala Ala Gln
            260                 265                 270

gat cgt att gcc aaa cca gaa cat tac ttc agc gca aca aag ctc tga         864
Asp Arg Ile Ala Lys Pro Glu His Tyr Phe Ser Ala Thr Lys Leu
        275                 280                 285


<210>  80
<211>  287
<212>  PRT
<213>  Escherichia coli

<400>  80

Met Met Lys Asn Ile Leu Ala Ile Gln Ser His Val Val Tyr Gly His
1               5                   10                  15


Ala Gly Asn Ser Ala Ala Glu Phe Pro Met Arg Arg Leu Gly Ala Asn
            20                  25                  30


Val Trp Pro Leu Asn Thr Val Gln Phe Ser Asn His Thr Gln Tyr Gly
        35                  40                  45


Lys Trp Thr Gly Cys Val Met Pro Pro Ser His Leu Thr Glu Ile Val
    50                  55                  60


Gln Gly Ile Ala Ala Ile Asp Lys Leu His Thr Cys Asp Ala Val Leu
65                  70                  75                  80


Ser Gly Tyr Leu Gly Ser Ala Glu Gln Gly Glu His Ile Leu Gly Ile
                85                  90                  95


Val Arg Gln Val Lys Ala Ala Asn Pro Gln Ala Lys Tyr Phe Cys Asp
            100                 105                 110


Pro Val Met Gly His Pro Glu Lys Gly Cys Ile Val Ala Pro Gly Val
        115                 120                 125


Ala Glu Phe His Val Arg His Gly Leu Pro Ala Ser Asp Ile Ile Ala
    130                 135                 140


Pro Asn Leu Val Glu Leu Glu Ile Leu Cys Glu His Ala Val Asn Asn
145                 150                 155                 160


Val Glu Glu Ala Val Leu Ala Ala Arg Glu Leu Ile Ala Gln Gly Pro
                165                 170                 175

Gln Ile Val Leu Val Lys His Leu Ala Arg Ala Gly Tyr Ser Arg Asp
            180                 185                 190

Arg Phe Glu Met Leu Leu Val Thr Ala Asp Glu Ala Trp His Ile Ser
        195                 200                 205

Arg Pro Leu Val Asp Phe Gly Met Arg Gln Pro Val Gly Val Gly Asp
    210                 215                 220

Val Thr Ser Gly Leu Leu Leu Val Lys Leu Leu Gln Gly Ala Thr Leu
225                 230                 235                 240

Gln Glu Ala Leu Glu His Val Thr Ala Ala Val Tyr Glu Ile Met Val
                245                 250                 255

Thr Thr Lys Ala Met Gln Glu Tyr Glu Leu Gln Val Val Ala Ala Gln
            260                 265                 270

Asp Arg Ile Ala Lys Pro Glu His Tyr Phe Ser Ala Thr Lys Leu
        275                 280                 285

<210>  81
<211>  1143
<212>  DNA
<213>  Escherichia coli

<220>
<221>  CDS
<222>  (308)..(964)
<223>  Pyridoxal 5'-phosphate synthase (pdxH)

<400>  81
   atgcaaaccg atacgctgga ataccagtgt gatgaaaaac cgttgacggt caaactgaat      60
   aatccgcgcc aggaggtcag ttttgtttac gataatcaac tactgcatct caaacagggc     120
   atttcagcct ctggcgcgcg ttacactgac ggaatctatg ttttctggtc gaaaggcgat     180
   gaagcgactg tctataaacg cgaccgcatc gtcttgaata actgtcagtt acaaaatcca     240
   cagcgttgag atttttccag gggcggcgca caatagcgtc acccactgac aatccgtaaa     300
   gaaaacc atg tct gat aac gac gaa ttg cag caa atc gcg cat ctg cgc       349
           Met Ser Asp Asn Asp Glu Leu Gln Gln Ile Ala His Leu Arg
           1               5                   10
   cgt gaa tac acc aaa ggc ggg tta cgc cgc cgc gat ctt ccc gcc gat       397
   Arg Glu Tyr Thr Lys Gly Gly Leu Arg Arg Arg Asp Leu Pro Ala Asp
   15                  20                  25                  30
   cca tta acc ctt ttt gaa cgt tgg ctc tct cag gct tgt gaa gcc aaa       445
   Pro Leu Thr Leu Phe Glu Arg Trp Leu Ser Gln Ala Cys Glu Ala Lys
                   35                  40                  45
   ctg gcg gac cct acc gcg atg gtg gtc gct acc gtg gat gaa cat ggt       493
   Leu Ala Asp Pro Thr Ala Met Val Val Ala Thr Val Asp Glu His Gly
               50                  55                  60
   cag cct tat cag cgc atc gtt tta ctc aaa cat tac gac gaa aaa ggc       541
   Gln Pro Tyr Gln Arg Ile Val Leu Leu Lys His Tyr Asp Glu Lys Gly
           65                      70                      75
   atg gtg ttt tac acc aac ctc ggc agc cgt aaa gca cat caa atc gaa      589
   Met Val Phe Tyr Thr Asn Leu Gly Ser Arg Lys Ala His Gln Ile Glu
       80                      85                      90
   aat aat ccg cgc gtt agc ctg ctg ttc ccg tgg cat acc ctt gag cgc      637
   Asn Asn Pro Arg Val Ser Leu Leu Phe Pro Trp His Thr Leu Glu Arg
   95                      100                     105                 110
   cag gtg atg gtg atc ggt aaa gca gaa cga ctt tcg act ctc gaa gtg      685
   Gln Val Met Val Ile Gly Lys Ala Glu Arg Leu Ser Thr Leu Glu Val
                   115                     120                     125
   atg aaa tat ttt cat agc cgc ccg cgt gat agc cag att ggt gca tgg      733
   Met Lys Tyr Phe His Ser Arg Pro Arg Asp Ser Gln Ile Gly Ala Trp
               130                     135                     140
   gtt tcg aag cag tcc agt cgc att tct gcc cgc ggt atc ctt gaa agt      781
   Val Ser Lys Gln Ser Ser Arg Ile Ser Ala Arg Gly Ile Leu Glu Ser
           145                     150                     155
   aaa ttc ctg gag ctg aag cag aag ttt caa cag ggc gaa gtg cca ttg      829
   Lys Phe Leu Glu Leu Lys Gln Lys Phe Gln Gln Gly Glu Val Pro Leu
       160                     165                     170
   ccg agc ttt tgg ggc ggt ttt cgc gtc agc ctt gaa cag att gag ttc      877
   Pro Ser Phe Trp Gly Gly Phe Arg Val Ser Leu Glu Gln Ile Glu Phe
   175                     180                     185                 190
   tgg cag ggt ggt gag cat cgc ctg cat gac cgc ttt ttg tac cag cgt      925
   Trp Gln Gly Gly Glu His Arg Leu His Asp Arg Phe Leu Tyr Gln Arg
                   195                     200                     205
   gaa aat gat gcg tgg aag att gat cgt ctt gca ccc tga aaagatgcaa       974
   Glu Asn Asp Ala Trp Lys Ile Asp Arg Leu Ala Pro
               210                 215
   aaatcttgct ttaatcgctg gtactcctga ttctggcact ttattctatg tctctttcgc    1034
   atctggcgaa aagtcgtgta ccggcaaagg tgcagtcgtt atatacatgg agattttgat    1094
   ggcaagcagt aacttgatta aacaattgca agagcggggg ctggtatga                 1143


<210>  82
<211>  218
<212>  PRT
<213>  Escherichia coli

<400>  82

Met Ser Asp Asn Asp Glu Leu Gln Gln Ile Ala His Leu Arg Arg Glu
1               5                   10                  15


Tyr Thr Lys Gly Gly Leu Arg Arg Arg Asp Leu Pro Ala Asp Pro Leu
            20                  25                  30


Thr Leu Phe Glu Arg Trp Leu Ser Gln Ala Cys Glu Ala Lys Leu Ala
        35                  40                  45


Asp Pro Thr Ala Met Val Val Ala Thr Val Asp Glu His Gly Gln Pro
    50                  55                  60

Tyr Gln Arg Ile Val Leu Leu Lys His Tyr Asp Glu Lys Gly Met Val
65                  70                  75                  80

Phe Tyr Thr Asn Leu Gly Ser Arg Lys Ala His Gln Ile Glu Asn Asn
                85                  90                  95

Pro Arg Val Ser Leu Leu Phe Pro Trp His Thr Leu Glu Arg Gln Val
            100                 105                 110

Met Val Ile Gly Lys Ala Glu Arg Leu Ser Thr Leu Glu Val Met Lys
        115                 120                 125

Tyr Phe His Ser Arg Pro Arg Asp Ser Gln Ile Gly Ala Trp Val Ser
    130                 135                 140

Lys Gln Ser Ser Arg Ile Ser Ala Arg Gly Ile Leu Glu Ser Lys Phe
145                 150                 155                 160

Leu Glu Leu Lys Gln Lys Phe Gln Gln Gly Glu Val Pro Leu Pro Ser
                165                 170                 175

Phe Trp Gly Gly Phe Arg Val Ser Leu Glu Gln Ile Glu Phe Trp Gln
            180                 185                 190

Gly Gly Glu His Arg Leu His Asp Arg Phe Leu Tyr Gln Arg Glu Asn
        195                 200                 205

Asp Ala Trp Lys Ile Asp Arg Leu Ala Pro
    210                 215

<210>  83
<211>  852
<212>  DNA
<213>  Escherichia coli

<220>
<221>  CDS
<222>  (1)..(852)
<223>  pyridoxine /pyridoxal /pyridoxamine kinase  (pdxK)

<400>  83
atg agc agc ctg ctg ctg ttc aac gac aag agc agg gcc ctg cag gcc        48
Met Ser Ser Leu Leu Leu Phe Asn Asp Lys Ser Arg Ala Leu Gln Ala
1               5                   10                  15

gac atc gtg gcc gtg cag agc cag gtg gtg tac ggc agc gtg ggc aac        96
Asp Ile Val Ala Val Gln Ser Gln Val Val Tyr Gly Ser Val Gly Asn
            20                  25                  30

agc atc gcc gtg ccg gcc atc aag cag aac ggc ctg aac gtg ttc gcc       144
Ser Ile Ala Val Pro Ala Ile Lys Gln Asn Gly Leu Asn Val Phe Ala
        35                  40                  45

gtg ccg acc gtg ctg ctg agc aac acc ccg cac tac gac acc ttc tac       192
Val Pro Thr Val Leu Leu Ser Asn Thr Pro His Tyr Asp Thr Phe Tyr
    50                  55                  60

ggc ggc gcc atc ccg gac gag tgg ttc agc ggc tac ctg agg gcc ctg       240

Gly Gly Ala Ile Pro Asp Glu Trp Phe Ser Gly Tyr Leu Arg Ala Leu
65                  70                  75                  80

cag gag agg gac gcc ctg agg cag ctg agg gcc gtg acc acc ggc tac       288
Gln Glu Arg Asp Ala Leu Arg Gln Leu Arg Ala Val Thr Thr Gly Tyr
                85                  90                  95

atg ggc acc gcc agc cag atc aag atc ctg gcc gag tgg ctg acc gcc       336
Met Gly Thr Ala Ser Gln Ile Lys Ile Leu Ala Glu Trp Leu Thr Ala
            100                 105                 110

ctg agg aag gac cac ccg gac ctg ctg atc atg gtg gac ccg gtg atc       384
Leu Arg Lys Asp His Pro Asp Leu Leu Ile Met Val Asp Pro Val Ile
        115                 120                 125

ggc gac atc gac agc ggc atc tac gtg aag ccg gac ctg ccg gag gcc       432
Gly Asp Ile Asp Ser Gly Ile Tyr Val Lys Pro Asp Leu Pro Glu Ala
    130                 135                 140

tac agg cag tac ctg ctg ccg ctg gcc cag ggc atc acc ccg aac atc       480
Tyr Arg Gln Tyr Leu Leu Pro Leu Ala Gln Gly Ile Thr Pro Asn Ile
145                 150                 155                 160

ttc gag ctg gag atc ctg acc ggc aag aac tgc agg gac ctg gac agc       528
Phe Glu Leu Glu Ile Leu Thr Gly Lys Asn Cys Arg Asp Leu Asp Ser
                165                 170                 175

gcc atc gcc gcc gcc aag agc ctg ctg agc gac acc ctg aag tgg gtg       576
Ala Ile Ala Ala Ala Lys Ser Leu Leu Ser Asp Thr Leu Lys Trp Val
            180                 185                 190

gtg atc acc agc gcc agc ggc aac gag gag aac cag gag atg cag gtg       624
Val Ile Thr Ser Ala Ser Gly Asn Glu Glu Asn Gln Glu Met Gln Val
        195                 200                 205

gtg gtg gtg agc gcc gac agc gtg aac gtg atc agc cac agc agg gtg       672
Val Val Val Ser Ala Asp Ser Val Asn Val Ile Ser His Ser Arg Val
    210                 215                 220

aag acc gac ctg aag ggc acc ggc gac ctg ttc tgc gcc cag ctg atc       720
Lys Thr Asp Leu Lys Gly Thr Gly Asp Leu Phe Cys Ala Gln Leu Ile
225                 230                 235                 240

agc ggc ctg ctg aag ggc aag gcc ctg aac gac gcc gtg cac agg gcc       768
Ser Gly Leu Leu Lys Gly Lys Ala Leu Asn Asp Ala Val His Arg Ala
                245                 250                 255

ggc ctg agg gtg ctg gag gtg atg agg tac acc cag cag cac gag agc       816
Gly Leu Arg Val Leu Glu Val Met Arg Tyr Thr Gln Gln His Glu Ser
            260                 265                 270

gac gag ctg atc ctg ccg ccg ctg gcc gag gcc tga                       852
Asp Glu Leu Ile Leu Pro Pro Leu Ala Glu Ala
        275                 280


<210>  84
<211>  283
<212>  PRT
<213>  Escherichia coli

<400>  84

Met Ser Ser Leu Leu Leu Phe Asn Asp Lys Ser Arg Ala Leu Gln Ala
1               5                   10                  15


Asp Ile Val Ala Val Gln Ser Gln Val Val Tyr Gly Ser Val Gly Asn
            20                  25                  30

Ser Ile Ala Val Pro Ala Ile Lys Gln Asn Gly Leu Asn Val Phe Ala
        35                  40                  45

Val Pro Thr Val Leu Leu Ser Asn Thr Pro His Tyr Asp Thr Phe Tyr
    50                  55                  60

Gly Gly Ala Ile Pro Asp Glu Trp Phe Ser Gly Tyr Leu Arg Ala Leu
65                  70                  75                  80

Gln Glu Arg Asp Ala Leu Arg Gln Leu Arg Ala Val Thr Thr Gly Tyr
                85                  90                  95

Met Gly Thr Ala Ser Gln Ile Lys Ile Leu Ala Glu Trp Leu Thr Ala
            100                 105                 110

Leu Arg Lys Asp His Pro Asp Leu Leu Ile Met Val Asp Pro Val Ile
        115                 120                 125

Gly Asp Ile Asp Ser Gly Ile Tyr Val Lys Pro Asp Leu Pro Glu Ala
    130                 135                 140

Tyr Arg Gln Tyr Leu Leu Pro Leu Ala Gln Gly Ile Thr Pro Asn Ile
145                 150                 155                 160

Phe Glu Leu Glu Ile Leu Thr Gly Lys Asn Cys Arg Asp Leu Asp Ser
                165                 170                 175

Ala Ile Ala Ala Ala Lys Ser Leu Leu Ser Asp Thr Leu Lys Trp Val
            180                 185                 190

Val Ile Thr Ser Ala Ser Gly Asn Glu Glu Asn Gln Glu Met Gln Val
        195                 200                 205

Val Val Val Ser Ala Asp Ser Val Asn Val Ile Ser His Ser Arg Val
    210                 215                 220

Lys Thr Asp Leu Lys Gly Thr Gly Asp Leu Phe Cys Ala Gln Leu Ile
225                 230                 235                 240

Ser Gly Leu Leu Lys Gly Lys Ala Leu Asn Asp Ala Val His Arg Ala
                245                 250                 255

Gly Leu Arg Val Leu Glu Val Met Arg Tyr Thr Gln Gln His Glu Ser
            260                 265                 270

Asp Glu Leu Ile Leu Pro Pro Leu Ala Glu Ala
        275                 280

<210>  85
<211>  903
<212>  DNA

<213>  Trypanosoma brucei

<220>
<221>  CDS
<222>  (1)..(903)
<223>  pyridoxine /pyridoxal /pyridoxamine kinase  (pdxK)

<400>  85
atg agc gag aag acc gtg ctg agc atc cag agc ttc gtg acc cac ggc      48
Met Ser Glu Lys Thr Val Leu Ser Ile Gln Ser Phe Val Thr His Gly
1               5                   10                  15

tac gtg ggc aac aag gcc gcc acc ttc ccg ctg cag ctg cac ggc ttc      96
Tyr Val Gly Asn Lys Ala Ala Thr Phe Pro Leu Gln Leu His Gly Phe
            20                  25                  30

gac gtg gac ggc atc aac acc gtg tgc ctg agc aac cac agc ggc tac      144
Asp Val Asp Gly Ile Asn Thr Val Cys Leu Ser Asn His Ser Gly Tyr
        35                  40                  45

ccg gtg atc agg ggc cac agg atg agc ctg cag gag tac gac gag ctg      192
Pro Val Ile Arg Gly His Arg Met Ser Leu Gln Glu Tyr Asp Glu Leu
    50                  55                  60

atg gag ggc gtg agg gcc aac aac ttc ctg agc aac tac agg tac atc      240
Met Glu Gly Val Arg Ala Asn Asn Phe Leu Ser Asn Tyr Arg Tyr Ile
65                  70                  75                  80

ctg acc ggc tac atc aac aac gtg gac atc atc ggc agg atc agg gac      288
Leu Thr Gly Tyr Ile Asn Asn Val Asp Ile Ile Gly Arg Ile Arg Asp
                85                  90                  95

acc ctg aag gag gtg agg gag ctg agg gag aag gag gac aag aag ctg      336
Thr Leu Lys Glu Val Arg Glu Leu Arg Glu Lys Glu Asp Lys Lys Leu
            100                 105                 110

acc ttc atc tgc gac ccg gtg atg ggc gac gac ggc atc atg tac tgc      384
Thr Phe Ile Cys Asp Pro Val Met Gly Asp Asp Gly Ile Met Tyr Cys
        115                 120                 125

aag aag gag gtg ctg gac gcc tac agg gag ctg gtg ccg ctg gcc gac      432
Lys Lys Glu Val Leu Asp Ala Tyr Arg Glu Leu Val Pro Leu Ala Asp
    130                 135                 140

atc gtg acc ccg aac tac ttc gag gcc agc ctg ctg agc ggc gtg acc      480
Ile Val Thr Pro Asn Tyr Phe Glu Ala Ser Leu Leu Ser Gly Val Thr
145                 150                 155                 160

gtg aac gac ctg agc agc gcc atc ctg gcc gcc gac tgg ttc cac aac      528
Val Asn Asp Leu Ser Ser Ala Ile Leu Ala Ala Asp Trp Phe His Asn
                165                 170                 175

tgc ggc gtg gcc cac gtg atc atc aag agc ttc agg gag cag gag aac      576
Cys Gly Val Ala His Val Ile Ile Lys Ser Phe Arg Glu Gln Glu Asn
            180                 185                 190

ccg acc cac ctg agg ttc ctg tac agc gtg aag gag ggc agc gag gcc      624
Pro Thr His Leu Arg Phe Leu Tyr Ser Val Lys Glu Gly Ser Glu Ala
        195                 200                 205

gcc gtg agg agg ttc agc ggc gtg gtg ccg tac cac gag ggc agg tac      672
Ala Val Arg Arg Phe Ser Gly Val Val Pro Tyr His Glu Gly Arg Tyr
    210                 215                 220

acc ggc acc ggc gac gtg ttc gcc gcc tgc ctg ctg gcc ttc agc cac      720
Thr Gly Thr Gly Asp Val Phe Ala Ala Cys Leu Leu Ala Phe Ser His
225                 230                 235                 240

agc cac ccg atg gac gtg gcc atc ggc aag agc atg gcc gtg ctg cag 768
Ser His Pro Met Asp Val Ala Ile Gly Lys Ser Met Ala Val Leu Gln
                245                 250                 255

gag ctg atc atc gcc acc agg aag gag ggc ggc gac ggc aag agc agc 816
Glu Leu Ile Ile Ala Thr Arg Lys Glu Gly Gly Asp Gly Lys Ser Ser
            260                 265                 270

ctg aag agc agg gag ctg agg gtg gtg gcc agc ccg cag gtg gtg ctg 864
Leu Lys Ser Arg Glu Leu Arg Val Val Ala Ser Pro Gln Val Val Leu
        275                 280                 285

cag ccg agc acc gtg gtg gac gtg aag ccg atc agc tga 903
Gln Pro Ser Thr Val Val Asp Val Lys Pro Ile Ser
    290                 295                 300

<210> 86
<211> 300
<212> PRT
<213> Trypanosoma brucei

<400> 86

Met Ser Glu Lys Thr Val Leu Ser Ile Gln Ser Phe Val Thr His Gly
1               5                   10                  15

Tyr Val Gly Asn Lys Ala Ala Thr Phe Pro Leu Gln Leu His Gly Phe
            20                  25                  30

Asp Val Asp Gly Ile Asn Thr Val Cys Leu Ser Asn His Ser Gly Tyr
        35                  40                  45

Pro Val Ile Arg Gly His Arg Met Ser Leu Gln Glu Tyr Asp Glu Leu
    50                  55                  60

Met Glu Gly Val Arg Ala Asn Asn Phe Leu Ser Asn Tyr Arg Tyr Ile
65                  70                  75                  80

Leu Thr Gly Tyr Ile Asn Asn Val Asp Ile Ile Gly Arg Ile Arg Asp
                85                  90                  95

Thr Leu Lys Glu Val Arg Glu Leu Arg Glu Lys Glu Asp Lys Lys Leu
            100                 105                 110

Thr Phe Ile Cys Asp Pro Val Met Gly Asp Asp Gly Ile Met Tyr Cys
        115                 120                 125

Lys Lys Glu Val Leu Asp Ala Tyr Arg Glu Leu Val Pro Leu Ala Asp
    130                 135                 140

Ile Val Thr Pro Asn Tyr Phe Glu Ala Ser Leu Leu Ser Gly Val Thr
145                 150                 155                 160

Val Asn Asp Leu Ser Ser Ala Ile Leu Ala Ala Asp Trp Phe His Asn
                165                 170                 175

Cys Gly Val Ala His Val Ile Ile Lys Ser Phe Arg Glu Gln Glu Asn

180                 185                 190

Pro Thr His Leu Arg Phe Leu Tyr Ser Val Lys Glu Gly Ser Glu Ala
        195                 200                 205

Ala Val Arg Arg Phe Ser Gly Val Val Pro Tyr His Glu Gly Arg Tyr
    210                 215                 220

Thr Gly Thr Gly Asp Val Phe Ala Ala Cys Leu Leu Ala Phe Ser His
225                 230                 235                 240

Ser His Pro Met Asp Val Ala Ile Gly Lys Ser Met Ala Val Leu Gln
                245                 250                 255

Glu Leu Ile Ile Ala Thr Arg Lys Glu Gly Gly Asp Gly Lys Ser Ser
            260                 265                 270

Leu Lys Ser Arg Glu Leu Arg Val Val Ala Ser Pro Gln Val Val Leu
        275                 280                 285

Gln Pro Ser Thr Val Val Asp Val Lys Pro Ile Ser
    290                 295                 300

<210>  87
<211>  966
<212>  DNA
<213>  Caenorhabditis elegans

<220>
<221>  CDS
<222>  (1)..(966)
<223>  pyridoxine /pyridoxal /pyridoxamine kinase  (pdxK)

<400>  87
atg agc agc agc gag ctg atc gcc gag ctg gag agg gag agg gac agg       48
Met Ser Ser Ser Glu Leu Ile Ala Glu Leu Glu Arg Glu Arg Asp Arg
1               5                   10                  15

agg gtg ctg agc atc cag agc cac gtg gtg cac ggc tac gcc ggc aac       96
Arg Val Leu Ser Ile Gln Ser His Val Val His Gly Tyr Ala Gly Asn
            20                  25                  30

aag tgc agc gtg ttc ccg ctg cag ctg cac ggc ttc gag gtg gac ttc      144
Lys Cys Ser Val Phe Pro Leu Gln Leu His Gly Phe Glu Val Asp Phe
        35                  40                  45

atc aac agc gtg cag ttc agc aac cac gcc ggc aac atc gag tac ctg      192
Ile Asn Ser Val Gln Phe Ser Asn His Ala Gly Asn Ile Glu Tyr Leu
    50                  55                  60

acc ctg ccg acc agg tac gag cac gtg aag ggc cag aag ctg acc gag      240
Thr Leu Pro Thr Arg Tyr Glu His Val Lys Gly Gln Lys Leu Thr Glu
65                  70                  75                  80

aag gag ctg gag gag ctg tac gag ggc ctg acc ctg aac aac atc aac      288
Lys Glu Leu Glu Glu Leu Tyr Glu Gly Leu Thr Leu Asn Asn Ile Asn
                85                  90                  95

aac tac acc cac gtg ctg acc ggc tac tgc ggc aac gtg acc ttc ctg      336
Asn Tyr Thr His Val Leu Thr Gly Tyr Cys Gly Asn Val Thr Phe Leu

100                 105                 110

cag aag atc gcc gac gtg gtg aag gac ctg aag aag aag aac ggc aac       384
Gln Lys Ile Ala Asp Val Val Lys Asp Leu Lys Lys Lys Asn Gly Asn
        115                 120                 125

acc acc ttc gtg tgc gac ccg gtg atg ggc gac aac ggc agg tac tac       432
Thr Thr Phe Val Cys Asp Pro Val Met Gly Asp Asn Gly Arg Tyr Tyr
    130                 135                 140

acc ccg aag gag ctg atg ccg gtg tac agg gac ctg atc atc ccg ctg       480
Thr Pro Lys Glu Leu Met Pro Val Tyr Arg Asp Leu Ile Ile Pro Leu
145                 150                 155                 160

gcc gac gtg ctg acc ccg aac gcc ttc gag ctg ggc gag ctg acc ggc       528
Ala Asp Val Leu Thr Pro Asn Ala Phe Glu Leu Gly Glu Leu Thr Gly
                165                 170                 175

agc ccg atc gag acc gag gag gac tgc ctg agg gcc gtg aac gag ctg       576
Ser Pro Ile Glu Thr Glu Glu Asp Cys Leu Arg Ala Val Asn Glu Leu
            180                 185                 190

cac gcc aag ggc gtg aag acc gtg gtg gtg acc agc ggc gtg acc ggc       624
His Ala Lys Gly Val Lys Thr Val Val Val Thr Ser Gly Val Thr Gly
        195                 200                 205

gcc cag acc aac gag agc ctg agg tgc tac gcc agc gtg aag ggc agc       672
Ala Gln Thr Asn Glu Ser Leu Arg Cys Tyr Ala Ser Val Lys Gly Ser
    210                 215                 220

cac gtg tac agg ttc acc ttc ccg agg ctg gtg ggc cag ttc gtg ggc       720
His Val Tyr Arg Phe Thr Phe Pro Arg Leu Val Gly Gln Phe Val Gly
225                 230                 235                 240

acc ggc gac acc ttc acc agc ctg ctg gtg gtg tgg ctg gac gag ctg       768
Thr Gly Asp Thr Phe Thr Ser Leu Leu Val Val Trp Leu Asp Glu Leu
                245                 250                 255

aac ggc gac gtg agc gag gcc gtg aag agg gtg ctg gcc agc atg cag       816
Asn Gly Asp Val Ser Glu Ala Val Lys Arg Val Leu Ala Ser Met Gln
            260                 265                 270

tgc ctg atc agg aag acc agc agc tac gcc cag ctg cag gtg gac acc       864
Cys Leu Ile Arg Lys Thr Ser Ser Tyr Ala Gln Leu Gln Val Asp Thr
        275                 280                 285

aac agc agg gcc atg tgc gag ctg agg ctg atc cag agc agg aag gac       912
Asn Ser Arg Ala Met Cys Glu Leu Arg Leu Ile Gln Ser Arg Lys Asp
    290                 295                 300

ctg ctg tgg ccg ccg acc tgc gac cag atc cag gtg gag aag atc ggc       960
Leu Leu Trp Pro Pro Thr Cys Asp Gln Ile Gln Val Glu Lys Ile Gly
305                 310                 315                 320

cag tga                                                               966
Gln

<210>  88
<211>  321
<212>  PRT
<213>  Caenorhabditis elegans

<400>  88

Met Ser Ser Ser Glu Leu Ile Ala Glu Leu Glu Arg Glu Arg Asp Arg
1               5                   10                  15

Arg Val Leu Ser Ile Gln Ser His Val Val His Gly Tyr Ala Gly Asn
            20                  25                  30

Lys Cys Ser Val Phe Pro Leu Gln Leu His Gly Phe Glu Val Asp Phe
        35                  40                  45

Ile Asn Ser Val Gln Phe Ser Asn His Ala Gly Asn Ile Glu Tyr Leu
    50                  55                  60

Thr Leu Pro Thr Arg Tyr Glu His Val Lys Gly Gln Lys Leu Thr Glu
65                  70                  75                  80

Lys Glu Leu Glu Glu Leu Tyr Glu Gly Leu Thr Leu Asn Asn Ile Asn
                85                  90                  95

Asn Tyr Thr His Val Leu Thr Gly Tyr Cys Gly Asn Val Thr Phe Leu
            100                 105                 110

Gln Lys Ile Ala Asp Val Val Lys Asp Leu Lys Lys Lys Asn Gly Asn
        115                 120                 125

Thr Thr Phe Val Cys Asp Pro Val Met Gly Asp Asn Gly Arg Tyr Tyr
    130                 135                 140

Thr Pro Lys Glu Leu Met Pro Val Tyr Arg Asp Leu Ile Ile Pro Leu
145                 150                 155                 160

Ala Asp Val Leu Thr Pro Asn Ala Phe Glu Leu Gly Glu Leu Thr Gly
                165                 170                 175

Ser Pro Ile Glu Thr Glu Glu Asp Cys Leu Arg Ala Val Asn Glu Leu
            180                 185                 190

His Ala Lys Gly Val Lys Thr Val Val Val Thr Ser Gly Val Thr Gly
        195                 200                 205

Ala Gln Thr Asn Glu Ser Leu Arg Cys Tyr Ala Ser Val Lys Gly Ser
    210                 215                 220

His Val Tyr Arg Phe Thr Phe Pro Arg Leu Val Gly Gln Phe Val Gly
225                 230                 235                 240

Thr Gly Asp Thr Phe Thr Ser Leu Leu Val Val Trp Leu Asp Glu Leu
                245                 250                 255

Asn Gly Asp Val Ser Glu Ala Val Lys Arg Val Leu Ala Ser Met Gln
            260                 265                 270

Cys Leu Ile Arg Lys Thr Ser Ser Tyr Ala Gln Leu Gln Val Asp Thr
        275                 280                 285

Asn Ser Arg Ala Met Cys Glu Leu Arg Leu Ile Gln Ser Arg Lys Asp
    290                 295                 300

Leu Leu Trp Pro Pro Thr Cys Asp Gln Ile Gln Val Glu Lys Ile Gly
305                 310                 315                 320

Gln

<210>  89
<211>  867
<212>  DNA
<213>  Salmonella typhimurium

<220>
<221>  CDS
<222>  (1)..(867)
<223>  pyridoxal kinase (yfei)

<400>  89
atg ggc cag gag agc gac atc cag agc gtg ctg ttc gac gac aac cac      48
agg gcc ctg cag acc gac atc gtg gcc gtg cag agc cag gtg gtg tac      96
ggc agc gtg ggc aac agc atc gcc gtg ccg gcc atc aag gcc cag ggc      144
ctg agg gtg acc gcc gtg ccg acc gtg ctg ttc agc aac acc ccg cac      192
tac aag acc ttc tac ggc ggc atc atc ccg gcc gag tgg ttc gcc ggc      240
tac ctg acc gcc ctg aac gag agg gac gcc ctg agg gag ctg aag gcc      288
atc acc acc ggc tac atg ggc agc gcc gac cag atc gtg ctg ctg agc      336
aag tgg ctg atg gcc atc agg gcc agc cac ccg gag gtg tgc atc ctg      384
gtg gac ccg gtg atc ggc gac acc gac agc ggc atg tac gtg cag gcc      432
gag atc ccg cag gcc tac agg acc cac ctg ctg ccg cag gcc cag ggc      480
ctg acc ccg aac gtg ttc gag ctg gag atg ctg agc ggc aag ccg tgc      528
agg acc ctg gag gag gcc gtg gcc gcc gcc cag agc ctg ctg agc gac      576
acc ctg aag tgg gtg gtg atc acc agc gcc ccg ggc gag agc ctg gag      624
acc atc acc gtg gcc gtg gtg acc gcc cag gtg gtg gag gtg ttc gcc      672
cac ccg agg gtg gcc acc gag ctg aag ggc acc ggc gac ctg ttc tgc      720
gcc gag ctg gtg agc ggc atc gtg cag ggc aag aag ctg acc acc gcc      768
gcc aag gac gcc gcc cag agg gtg ctg gag gtg atg acc tgg acc cag      816
cag tgc ggc tgc gac gag ctg atc ctg ccg ccg gcc ggc gag gcc agg      864
tga                                                                  867


<210>  90
<211>  288
<212>  PRT
<213>  Salmonella typhimurium

<400>  90

<210>  91
<211>  633
<212>  DNA
<213>  Haemophilus influenzae

<220>
<221>  CDS
<222>  (1)..(633)
<223>  pdxH (yfei)

<400>  91
atg gag ctg cac aac atc agg gac gag tac acc aag agg gtg ctg agc      48
Met Glu Leu His Asn Ile Arg Asp Glu Tyr Thr Lys Arg Val Leu Ser
1               5                   10                  15

cag cac gac tgc cac aag aac ccg atc agc cag ttc gag cag tgg cag      96
Gln His Asp Cys His Lys Asn Pro Ile Ser Gln Phe Glu Gln Trp Gln
            20                  25                  30

aag gag gcc atc cac gcc cag gtg aac gag ccg acc gcc atg aac atc      144
Lys Glu Ala Ile His Ala Gln Val Asn Glu Pro Thr Ala Met Asn Ile
        35                  40                  45

gcc acc gtg gac gag cag ggc agg ccg aac agc agg atg gtg ctg ctg      192
Ala Thr Val Asp Glu Gln Gly Arg Pro Asn Ser Arg Met Val Leu Leu
    50                  55                  60

aag gag gtg aac gag cag ggc ttc gtg ttc ttc acc aac tac ctg agc      240

Lys Glu Val Asn Glu Gln Gly Phe Val Phe Phe Thr Asn Tyr Leu Ser
65                  70                  75                  80

agg aag ggc ggc tgc atc gag agg aac ccg tac gtg gcc ctg acc ttc      288
Arg Lys Gly Gly Cys Ile Glu Arg Asn Pro Tyr Val Ala Leu Thr Phe
                85                  90                  95

ttc tgg ccg gag ctg gag agg cag gtg agg atc gag ggc aag gcc gtg      336
Phe Trp Pro Glu Leu Glu Arg Gln Val Arg Ile Glu Gly Lys Ala Val
            100                 105                 110

aag atc ccg gcc gag cag agc gac aag tac ttc gcc acc agg ccg tac      384
Lys Ile Pro Ala Glu Gln Ser Asp Lys Tyr Phe Ala Thr Arg Pro Tyr
        115                 120                 125

acc agc agg atc ggc gcc tgg gcc agc gag cag agc gcc gtg atc agc      432
Thr Ser Arg Ile Gly Ala Trp Ala Ser Glu Gln Ser Ala Val Ile Ser
    130                 135                 140

aac tac aag agc ctg ctg gcc aag gcc gcc ctg gtg gcc gcc aag cac      480
Asn Tyr Lys Ser Leu Leu Ala Lys Ala Ala Leu Val Ala Ala Lys His
145                 150                 155                 160

ccg ctg aac gtg ccg agg ccg gac tac tgg ggc ggc tac ctg gtg gtg      528
Pro Leu Asn Val Pro Arg Pro Asp Tyr Trp Gly Gly Tyr Leu Val Val
                165                 170                 175

ccg gag acc gtg gag ttc tgg cag ggc agg ccg agc agg ctg cac gac      576
Pro Glu Thr Val Glu Phe Trp Gln Gly Arg Pro Ser Arg Leu His Asp
            180                 185                 190

agg atc agg tac agg aag gag agc gac aac tgg atc agg gag agg ctg      624
Arg Ile Arg Tyr Arg Lys Glu Ser Asp Asn Trp Ile Arg Glu Arg Leu
        195                 200                 205

agc ccg tga                                                          633
Ser Pro
    210


<210>  92
<211>  210
<212>  PRT
<213>  Haemophilus influenzae

<400>  92

Met Glu Leu His Asn Ile Arg Asp Glu Tyr Thr Lys Arg Val Leu Ser
1               5                   10                  15


Gln His Asp Cys His Lys Asn Pro Ile Ser Gln Phe Glu Gln Trp Gln
            20                  25                  30


Lys Glu Ala Ile His Ala Gln Val Asn Glu Pro Thr Ala Met Asn Ile
        35                  40                  45


Ala Thr Val Asp Glu Gln Gly Arg Pro Asn Ser Arg Met Val Leu Leu
    50                  55                  60


Lys Glu Val Asn Glu Gln Gly Phe Val Phe Phe Thr Asn Tyr Leu Ser
65                  70                  75                  80


Arg Lys Gly Gly Cys Ile Glu Arg Asn Pro Tyr Val Ala Leu Thr Phe
                85                  90                  95

Phe Trp Pro Glu Leu Glu Arg Gln Val Arg Ile Glu Gly Lys Ala Val
            100                 105                 110

Lys Ile Pro Ala Glu Gln Ser Asp Lys Tyr Phe Ala Thr Arg Pro Tyr
        115                 120                 125

Thr Ser Arg Ile Gly Ala Trp Ala Ser Glu Gln Ser Ala Val Ile Ser
    130                 135                 140

Asn Tyr Lys Ser Leu Leu Ala Lys Ala Ala Leu Val Ala Ala Lys His
145                 150                 155                 160

Pro Leu Asn Val Pro Arg Pro Asp Tyr Trp Gly Gly Tyr Leu Val Val
                165                 170                 175

Pro Glu Thr Val Glu Phe Trp Gln Gly Arg Pro Ser Arg Leu His Asp
            180                 185                 190

Arg Ile Arg Tyr Arg Lys Glu Ser Asp Asn Trp Ile Arg Glu Arg Leu
        195                 200                 205

Ser Pro
    210

<210>  93
<211>  939
<212>  DNA
<213>  Saccharomyces cerevisiae

<220>
<221>  CDS
<222>  (1)..(939)
<223>  (Yn8fp)

<400>  93
atg ccg agg ctg ctg gcc acc cag agc cac gtg gtg cac ggc tac gtg      48
Met Pro Arg Leu Leu Ala Thr Gln Ser His Val Val His Gly Tyr Val
1               5                   10                  15

ggc aac aag gcc gcc acc ttc ccg ctg cag tgc ctg ggc tgg gac gtg      96
Gly Asn Lys Ala Ala Thr Phe Pro Leu Gln Cys Leu Gly Trp Asp Val
            20                  25                  30

gac tgc tgc aac agc gtg cag ttc agc aac cac acc ggc tac ggc ctg      144
Asp Cys Cys Asn Ser Val Gln Phe Ser Asn His Thr Gly Tyr Gly Leu
        35                  40                  45

gac aag gtg ttc ggc acc atc acc agg gag acc gac ctg aag gag ctg      192
Asp Lys Val Phe Gly Thr Ile Thr Arg Glu Thr Asp Leu Lys Glu Leu
    50                  55                  60

ctg agc ggc ctg ttc gac aac ttc agc cag gac tac cag gcc ctg ctg      240
Leu Ser Gly Leu Phe Asp Asn Phe Ser Gln Asp Tyr Gln Ala Leu Leu
65                  70                  75                  80

agc ggc tac ctg ccg aac aag aac agc gtg agg tgc atg ggc acc tac      288
Ser Gly Tyr Leu Pro Asn Lys Asn Ser Val Arg Cys Met Gly Thr Tyr
                85                  90                  95

tac gcc aag ttc aag gag gcc aac ccg gag atg atc tgg ctg atg gac 336
Tyr Ala Lys Phe Lys Glu Ala Asn Pro Glu Met Ile Trp Leu Met Asp
            100                 105                 110

ccg gtg atg ggc gac gag ggc cag ctg tac gtg agc gag gac gtg atc 384
Pro Val Met Gly Asp Glu Gly Gln Leu Tyr Val Ser Glu Asp Val Ile
        115                 120                 125

ccg gag tac agg aag ctg gcc ctg agc ccg aag cag ctg gtg gac atc 432
Pro Glu Tyr Arg Lys Leu Ala Leu Ser Pro Lys Gln Leu Val Asp Ile
    130                 135                 140

atc acc ccg aac cag ttc gag ctg gag atc ctg tac ggc ggc gag atc 480
Ile Thr Pro Asn Gln Phe Glu Leu Glu Ile Leu Tyr Gly Gly Glu Ile
145                 150                 155                 160

aag acc aag gag cac ctg aag aag gcc ctg aag aag ctg cac cag acc 528
Lys Thr Lys Glu His Leu Lys Lys Ala Leu Lys Lys Leu His Gln Thr
                165                 170                 175

atc ccg gtg atc atc gtg acc agc tgc gac tgc aag atg ttc gac gac 576
Ile Pro Val Ile Ile Val Thr Ser Cys Asp Cys Lys Met Phe Asp Asp
            180                 185                 190

aag gac ttc atc tac tgc gtg gcc agc atg gag ggc aag acc ccg atc 624
Lys Asp Phe Ile Tyr Cys Val Ala Ser Met Glu Gly Lys Thr Pro Ile
        195                 200                 205

gtg tac agg gtg ccg ttc atc gac agc tac ttc acc ggc gtg ggc gac 672
Val Tyr Arg Val Pro Phe Ile Asp Ser Tyr Phe Thr Gly Val Gly Asp
    210                 215                 220

ctg ttc agc gcc ctg ctg ctg gac agg gtg tac aag atc ctg agc aac 720
Leu Phe Ser Ala Leu Leu Leu Asp Arg Val Tyr Lys Ile Leu Ser Asn
225                 230                 235                 240

ccg acc acc acc ctg aag ttc gag gac cag gtg aac aac gtg ctg aac 768
Pro Thr Thr Thr Leu Lys Phe Glu Asp Gln Val Asn Asn Val Leu Asn
                245                 250                 255

gtg atc cag aag gtg ctg aag atc acc agg agc tac gcc agc ggc aag 816
Val Ile Gln Lys Val Leu Lys Ile Thr Arg Ser Tyr Ala Ser Gly Lys
            260                 265                 270

atg aag gcc aag atg ggc agc gcc ctg gag atg aag gag atg gag ctg 864
Met Lys Ala Lys Met Gly Ser Ala Leu Glu Met Lys Glu Met Glu Leu
        275                 280                 285

agg ctg atc gag agc agg gac atc tac gag acc atc aac atc cac cag 912
Arg Leu Ile Glu Ser Arg Asp Ile Tyr Glu Thr Ile Asn Ile His Gln
    290                 295                 300

acc gac tac atc tac gcc agg ctg tag 939
Thr Asp Tyr Ile Tyr Ala Arg Leu
305                 310

<210> 94
<211> 312
<212> PRT
<213> Saccharomyces cerevisiae

<400> 94

Met Pro Arg Leu Leu Ala Thr Gln Ser His Val Val His Gly Tyr Val
1               5                   10                  15

Gly Asn Lys Ala Ala Thr Phe Pro Leu Gln Cys Leu Gly Trp Asp Val
            20                  25                  30

Asp Cys Cys Asn Ser Val Gln Phe Ser Asn His Thr Gly Tyr Gly Leu
        35                  40                  45

Asp Lys Val Phe Gly Thr Ile Thr Arg Glu Thr Asp Leu Lys Glu Leu
    50                  55                  60

Leu Ser Gly Leu Phe Asp Asn Phe Ser Gln Asp Tyr Gln Ala Leu Leu
65                  70                  75                  80

Ser Gly Tyr Leu Pro Asn Lys Asn Ser Val Arg Cys Met Gly Thr Tyr
                85                  90                  95

Tyr Ala Lys Phe Lys Glu Ala Asn Pro Glu Met Ile Trp Leu Met Asp
            100                 105                 110

Pro Val Met Gly Asp Glu Gly Gln Leu Tyr Val Ser Glu Asp Val Ile
        115                 120                 125

Pro Glu Tyr Arg Lys Leu Ala Leu Ser Pro Lys Gln Leu Val Asp Ile
    130                 135                 140

Ile Thr Pro Asn Gln Phe Glu Leu Glu Ile Leu Tyr Gly Gly Glu Ile
145                 150                 155                 160

Lys Thr Lys Glu His Leu Lys Lys Ala Leu Lys Lys Leu His Gln Thr
                165                 170                 175

Ile Pro Val Ile Ile Val Thr Ser Cys Asp Cys Lys Met Phe Asp Asp
            180                 185                 190

Lys Asp Phe Ile Tyr Cys Val Ala Ser Met Glu Gly Lys Thr Pro Ile
        195                 200                 205

Val Tyr Arg Val Pro Phe Ile Asp Ser Tyr Phe Thr Gly Val Gly Asp
    210                 215                 220

Leu Phe Ser Ala Leu Leu Leu Asp Arg Val Tyr Lys Ile Leu Ser Asn
225                 230                 235                 240

Pro Thr Thr Thr Leu Lys Phe Glu Asp Gln Val Asn Asn Val Leu Asn
                245                 250                 255

Val Ile Gln Lys Val Leu Lys Ile Thr Arg Ser Tyr Ala Ser Gly Lys
            260                 265                 270

Met Lys Ala Lys Met Gly Ser Ala Leu Glu Met Lys Glu Met Glu Leu
        275                 280                 285

Arg Leu Ile Glu Ser Arg Asp Ile Tyr Glu Thr Ile Asn Ile His Gln

290                 295                 300

Thr Asp Tyr Ile Tyr Ala Arg Leu
305                 310

<210>  95
<211>  666
<212>  DNA
<213>  Synechocystis sp. PCC 6803

<220>
<221>  CDS
<222>  (1)..(666)
<223>  pdxJ (SLR11779)

<400>  95
atg gag cca gac ccg atc gcc gcc gct gtg ttg gcc gaa ctg gcc ggg      48
Met Glu Pro Asp Pro Ile Ala Ala Ala Val Leu Ala Glu Leu Ala Gly
1               5                   10                  15

gcg gat ggt att aca gcc cat tta cgg gaa gac cgt cgc cac att caa      96
Ala Asp Gly Ile Thr Ala His Leu Arg Glu Asp Arg Arg His Ile Gln
            20                  25                  30

gag cgg gac gtg gaa ata ttg cga aaa acg gtg cgc acc cac tta aac      144
Glu Arg Asp Val Glu Ile Leu Arg Lys Thr Val Arg Thr His Leu Asn
        35                  40                  45

ctg gag atg gcc gcc acc aag gaa atg gtg gaa att gcc ctc aac ctt      192
Leu Glu Met Ala Ala Thr Lys Glu Met Val Glu Ile Ala Leu Asn Leu
    50                  55                  60

aaa ccg gat tac gtc acc ctg gta ccg gaa cgg cgg gaa gaa gtg acc      240
Lys Pro Asp Tyr Val Thr Leu Val Pro Glu Arg Arg Glu Glu Val Thr
65                  70                  75                  80

acg gaa ggg ggt ttg gat gtg gct ggg aac ttg aac tat ttg ctg gga      288
Thr Glu Gly Gly Leu Asp Val Ala Gly Asn Leu Asn Tyr Leu Leu Gly
                85                  90                  95

gta gtt gag cag tta caa tcc cga cat att ccc gtt agt ctt ttc atc      336
Val Val Glu Gln Leu Gln Ser Arg His Ile Pro Val Ser Leu Phe Ile
            100                 105                 110

gac ccg gat gtg gcc caa ctg aaa gcc tct gcc caa acc gga gca aaa      384
Asp Pro Asp Val Ala Gln Leu Lys Ala Ser Ala Gln Thr Gly Ala Lys
        115                 120                 125

ttc att gaa tta cat acg ggc aaa tat gcc aat gcc ccc acc gct gac      432
Phe Ile Glu Leu His Thr Gly Lys Tyr Ala Asn Ala Pro Thr Ala Asp
    130                 135                 140

gac caa gct agg gaa ttg ggg tcc ctg gcg atc gcc tgt gac att gct      480
Asp Gln Ala Arg Glu Leu Gly Ser Leu Ala Ile Ala Cys Asp Ile Ala
145                 150                 155                 160

cta gaa ctg ggg cta agg atc aac gct ggc cat ggg ttg acc tat tgg      528
Leu Glu Leu Gly Leu Arg Ile Asn Ala Gly His Gly Leu Thr Tyr Trp
                165                 170                 175

aat gtg cgc cca gtg gcg gaa tta ccg ggc atg gaa gag tta aac atc      576
Asn Val Arg Pro Val Ala Glu Leu Pro Gly Met Glu Glu Leu Asn Ile
            180                 185                 190

ggc cac agc atc atg agc cgg gcc att ctg gtg ggc atg gaa agg gcc      624
Gly His Ser Ile Met Ser Arg Ala Ile Leu Val Gly Met Glu Arg Ala

195                         200                         205

gtg cgg gaa atg aaa tta gcc atg ttg ggg ctg cca ttt taa                666
Val Arg Glu Met Lys Leu Ala Met Leu Gly Leu Pro Phe
    210                 215                 220

<210>  96
<211>  221
<212>  PRT
<213>  Synechocystis sp. PCC 6803

<400>  96

Met Glu Pro Asp Pro Ile Ala Ala Ala Val Leu Ala Glu Leu Ala Gly
1               5                   10                  15

Ala Asp Gly Ile Thr Ala His Leu Arg Glu Asp Arg Arg His Ile Gln
            20                  25                  30

Glu Arg Asp Val Glu Ile Leu Arg Lys Thr Val Arg Thr His Leu Asn
        35                  40                  45

Leu Glu Met Ala Ala Thr Lys Glu Met Val Glu Ile Ala Leu Asn Leu
    50                  55                  60

Lys Pro Asp Tyr Val Thr Leu Val Pro Glu Arg Arg Glu Glu Val Thr
65                  70                  75                  80

Thr Glu Gly Gly Leu Asp Val Ala Gly Asn Leu Asn Tyr Leu Leu Gly
                85                  90                  95

Val Val Glu Gln Leu Gln Ser Arg His Ile Pro Val Ser Leu Phe Ile
            100                 105                 110

Asp Pro Asp Val Ala Gln Leu Lys Ala Ser Ala Gln Thr Gly Ala Lys
        115                 120                 125

Phe Ile Glu Leu His Thr Gly Lys Tyr Ala Asn Ala Pro Thr Ala Asp
    130                 135                 140

Asp Gln Ala Arg Glu Leu Gly Ser Leu Ala Ile Ala Cys Asp Ile Ala
145                 150                 155                 160

Leu Glu Leu Gly Leu Arg Ile Asn Ala Gly His Gly Leu Thr Tyr Trp
                165                 170                 175

Asn Val Arg Pro Val Ala Glu Leu Pro Gly Met Glu Glu Leu Asn Ile
            180                 185                 190

Gly His Ser Ile Met Ser Arg Ala Ile Leu Val Gly Met Glu Arg Ala
        195                 200                 205

Val Arg Glu Met Lys Leu Ala Met Leu Gly Leu Pro Phe
    210                 215                 220

<210>  97
<211>  732
<212>  DNA
<213>  Escherichia coli

<220>
<221>  CDS
<222>  (1)..(732)
<223>  PDXJ

<400>  97
atg gcc gag ctg ctg ctg ggc gtg aac atc gac cac atc gcc acc ctg      48
Met Ala Glu Leu Leu Leu Gly Val Asn Ile Asp His Ile Ala Thr Leu
1               5                   10                  15

agg aac gcc agg ggc acc gcc tac ccg gac ccg gtg cag gcc gcc ttc      96
Arg Asn Ala Arg Gly Thr Ala Tyr Pro Asp Pro Val Gln Ala Ala Phe
            20                  25                  30

atc gcc gag cag gcc ggc gcc gac ggc atc acc gtg cac ctg agg gag      144
Ile Ala Glu Gln Ala Gly Ala Asp Gly Ile Thr Val His Leu Arg Glu
        35                  40                  45

gac agg agg cac atc acc gac agg gac gtg agg atc ctg agg cag acc      192
Asp Arg Arg His Ile Thr Asp Arg Asp Val Arg Ile Leu Arg Gln Thr
    50                  55                  60

ctg gac acc agg atg aac ctg gag atg gcc gtg acc gag gag atg ctg      240
Leu Asp Thr Arg Met Asn Leu Glu Met Ala Val Thr Glu Glu Met Leu
65                  70                  75                  80

gcc atc gcc gtg gag acc aag ccg cac ttc tgc tgc ctg gtg ccg gag      288
Ala Ile Ala Val Glu Thr Lys Pro His Phe Cys Cys Leu Val Pro Glu
                85                  90                  95

aag agg cag gag gtg acc acc gag ggc ggc ctg gac gtg gcc ggc cag      336
Lys Arg Gln Glu Val Thr Thr Glu Gly Gly Leu Asp Val Ala Gly Gln
            100                 105                 110

agg gac aag atg agg gac gcc tgc aag agg ctg gcc gac gcc ggc atc      384
Arg Asp Lys Met Arg Asp Ala Cys Lys Arg Leu Ala Asp Ala Gly Ile
        115                 120                 125

cag gtg agc ctg ttc atc gac gcc gac gag gag cag atc aag gcc gcc      432
Gln Val Ser Leu Phe Ile Asp Ala Asp Glu Glu Gln Ile Lys Ala Ala
    130                 135                 140

gcc gag gtg ggc gcc ccg ttc atc gag atc cac acc ggc tgc tac gcc      480
Ala Glu Val Gly Ala Pro Phe Ile Glu Ile His Thr Gly Cys Tyr Ala
145                 150                 155                 160

gac gcc aag acc gac gcc gag cag gcc cag gag ctg gcc agg atc gcc      528
Asp Ala Lys Thr Asp Ala Glu Gln Ala Gln Glu Leu Ala Arg Ile Ala
                165                 170                 175

aag gcc gcc acc ttc gcc gcc agc ctg ggc ctg aag gtg aac gcc ggc      576
Lys Ala Ala Thr Phe Ala Ala Ser Leu Gly Leu Lys Val Asn Ala Gly
            180                 185                 190

cac ggc ctg acc tac cac aac gtg aag gcc atc gcc gcc atc ccg gag      624
His Gly Leu Thr Tyr His Asn Val Lys Ala Ile Ala Ala Ile Pro Glu
        195                 200                 205

atg cac gag ctg aac atc ggc cac gcc atc atc ggc agg gcc gtg atg      672
Met His Glu Leu Asn Ile Gly His Ala Ile Ile Gly Arg Ala Val Met
    210                 215                 220

acc ggc ctg aag gac gcc gtg gcc gag atg aag agg ctg atg ctg gag 720
Thr Gly Leu Lys Asp Ala Val Ala Glu Met Lys Arg Leu Met Leu Glu
225                 230                 235                 240

gcc agg ggc tga 732
Ala Arg Gly

<210> 98
<211> 243
<212> PRT
<213> Escherichia coli

<400> 98

Met Ala Glu Leu Leu Leu Gly Val Asn Ile Asp His Ile Ala Thr Leu
1               5                   10                  15

Arg Asn Ala Arg Gly Thr Ala Tyr Pro Asp Pro Val Gln Ala Ala Phe
            20                  25                  30

Ile Ala Glu Gln Ala Gly Ala Asp Gly Ile Thr Val His Leu Arg Glu
        35                  40                  45

Asp Arg Arg His Ile Thr Asp Arg Asp Val Arg Ile Leu Arg Gln Thr
    50                  55                  60

Leu Asp Thr Arg Met Asn Leu Glu Met Ala Val Thr Glu Glu Met Leu
65                  70                  75                  80

Ala Ile Ala Val Glu Thr Lys Pro His Phe Cys Cys Leu Val Pro Glu
                85                  90                  95

Lys Arg Gln Glu Val Thr Thr Glu Gly Gly Leu Asp Val Ala Gly Gln
            100                 105                 110

Arg Asp Lys Met Arg Asp Ala Cys Lys Arg Leu Ala Asp Ala Gly Ile
        115                 120                 125

Gln Val Ser Leu Phe Ile Asp Ala Asp Glu Glu Gln Ile Lys Ala Ala
    130                 135                 140

Ala Glu Val Gly Ala Pro Phe Ile Glu Ile His Thr Gly Cys Tyr Ala
145                 150                 155                 160

Asp Ala Lys Thr Asp Ala Glu Gln Ala Gln Glu Leu Ala Arg Ile Ala
                165                 170                 175

Lys Ala Ala Thr Phe Ala Ala Ser Leu Gly Leu Lys Val Asn Ala Gly
            180                 185                 190

His Gly Leu Thr Tyr His Asn Val Lys Ala Ile Ala Ala Ile Pro Glu
        195                 200                 205

Met His Glu Leu Asn Ile Gly His Ala Ile Ile Gly Arg Ala Val Met

210                 215                 220

Thr Gly Leu Lys Asp Ala Val Ala Glu Met Lys Arg Leu Met Leu Glu
225                 230                 235                 240

Ala Arg Gly

<210>  99
<211>  930
<212>  DNA
<213>  Arabidopsis thaliana

<220>
<221>  CDS
<222>  (1)..(930)
<223>  PDX1.1

<400>  99
atg gcc ggc acc ggc gtg gtg gcc gtg tac ggc gag ggc gcc atg acc      48
Met Ala Gly Thr Gly Val Val Ala Val Tyr Gly Glu Gly Ala Met Thr
1               5                   10                  15

gag acc aag cag aag agc ccg ttc agc gtg aag gtg ggc ctg gcc cag      96
Glu Thr Lys Gln Lys Ser Pro Phe Ser Val Lys Val Gly Leu Ala Gln
            20                  25                  30

atg ctg agg ggc ggc gtg atc atg gac gtg gtg aac gcc gag cag gcc      144
Met Leu Arg Gly Gly Val Ile Met Asp Val Val Asn Ala Glu Gln Ala
        35                  40                  45

agg atc gcc gag gag gcc ggc gcc tgc gcc gtg atg gcc ctg gag agg      192
Arg Ile Ala Glu Glu Ala Gly Ala Cys Ala Val Met Ala Leu Glu Arg
    50                  55                  60

gtg ccg gcc gac atc agg gcc cag ggc ggc gtg gcc agg atg agc gac      240
Val Pro Ala Asp Ile Arg Ala Gln Gly Gly Val Ala Arg Met Ser Asp
65                  70                  75                  80

ccg gag atg atc aag gag atc aag aac gcc gtg acc atc ccg gtg atg      288
Pro Glu Met Ile Lys Glu Ile Lys Asn Ala Val Thr Ile Pro Val Met
                85                  90                  95

gcc aag gcc agg atc ggc cac ttc gtg gag gcc cag atc ctg gag gcc      336
Ala Lys Ala Arg Ile Gly His Phe Val Glu Ala Gln Ile Leu Glu Ala
            100                 105                 110

atc ggc gtg gac tac gtg gac gag agc gag gtg ctg acc ctg gcc gac      384
Ile Gly Val Asp Tyr Val Asp Glu Ser Glu Val Leu Thr Leu Ala Asp
        115                 120                 125

gag gac aac cac atc aac aag cac aac ttc aag atc ccg ttc gtg tgc      432
Glu Asp Asn His Ile Asn Lys His Asn Phe Lys Ile Pro Phe Val Cys
    130                 135                 140

ggc tgc agg aac ctg ggc gag gcc ctg agg agg atc agg gag ggc gcc      480
Gly Cys Arg Asn Leu Gly Glu Ala Leu Arg Arg Ile Arg Glu Gly Ala
145                 150                 155                 160

gcc atg atc agg acc aag ggc gag gcc ggc acc ggc aac gtg gtg gag      528
Ala Met Ile Arg Thr Lys Gly Glu Ala Gly Thr Gly Asn Val Val Glu
                165                 170                 175

gcc gtg agg cac gtg agg agc gtg aac ggc gcc atc agg ctg ctg agg      576
Ala Val Arg His Val Arg Ser Val Asn Gly Ala Ile Arg Leu Leu Arg

180                    185                    190

agc atg gac gac gac gag gtg ttc acc tac gcc aag aag atc gcc gcc        624
Ser Met Asp Asp Asp Glu Val Phe Thr Tyr Ala Lys Lys Ile Ala Ala
        195                 200                 205

ccg tac gac ctg gtg gtg cag acc aag gag ctg ggc agg ctg ccg gtg        672
Pro Tyr Asp Leu Val Val Gln Thr Lys Glu Leu Gly Arg Leu Pro Val
    210                 215                 220

gtg cag ttc gcc gcc ggc ggc gtg gcc acc ccg gcc gac gcc gcc ctg        720
Val Gln Phe Ala Ala Gly Gly Val Ala Thr Pro Ala Asp Ala Ala Leu
225                 230                 235                 240

atg atg cag ctg ggc tgc gac ggc gtg ttc gtg ggc agc ggc gtg ttc        768
Met Met Gln Leu Gly Cys Asp Gly Val Phe Val Gly Ser Gly Val Phe
                245                 250                 255

aag agc ggc gac ccg gtg aag agg gcc aag gcc atc gtg cag gcc gtg        816
Lys Ser Gly Asp Pro Val Lys Arg Ala Lys Ala Ile Val Gln Ala Val
            260                 265                 270

acc aac tac agg gac gcc gcc gtg ctg gcc gag gtg agc tgc ggc ctg        864
Thr Asn Tyr Arg Asp Ala Ala Val Leu Ala Glu Val Ser Cys Gly Leu
        275                 280                 285

ggc gag gcc atg gtg ggc ctg aac ctg gac gac aag gtg gag agg ttc        912
Gly Glu Ala Met Val Gly Leu Asn Leu Asp Asp Lys Val Glu Arg Phe
    290                 295                 300

gcc agc agg agc gag tga                                                930
Ala Ser Arg Ser Glu
305


<210>  100
<211>  309
<212>  PRT
<213>  Arabidopsis thaliana

<400>  100

Met Ala Gly Thr Gly Val Val Ala Val Tyr Gly Glu Gly Ala Met Thr
1               5                   10                  15


Glu Thr Lys Gln Lys Ser Pro Phe Ser Val Lys Val Gly Leu Ala Gln
            20                  25                  30


Met Leu Arg Gly Gly Val Ile Met Asp Val Val Asn Ala Glu Gln Ala
        35                  40                  45


Arg Ile Ala Glu Glu Ala Gly Ala Cys Ala Val Met Ala Leu Glu Arg
    50                  55                  60


Val Pro Ala Asp Ile Arg Ala Gln Gly Gly Val Ala Arg Met Ser Asp
65                  70                  75                  80


Pro Glu Met Ile Lys Glu Ile Lys Asn Ala Val Thr Ile Pro Val Met
                85                  90                  95


Ala Lys Ala Arg Ile Gly His Phe Val Glu Ala Gln Ile Leu Glu Ala
            100                 105                 110

Ile Gly Val Asp Tyr Val Asp Glu Ser Glu Val Leu Thr Leu Ala Asp
        115                 120                 125

Glu Asp Asn His Ile Asn Lys His Asn Phe Lys Ile Pro Phe Val Cys
    130                 135                 140

Gly Cys Arg Asn Leu Gly Glu Ala Leu Arg Arg Ile Arg Glu Gly Ala
145                 150                 155                 160

Ala Met Ile Arg Thr Lys Gly Glu Ala Gly Thr Gly Asn Val Val Glu
                165                 170                 175

Ala Val Arg His Val Arg Ser Val Asn Gly Ala Ile Arg Leu Leu Arg
            180                 185                 190

Ser Met Asp Asp Asp Glu Val Phe Thr Tyr Ala Lys Lys Ile Ala Ala
        195                 200                 205

Pro Tyr Asp Leu Val Val Gln Thr Lys Glu Leu Gly Arg Leu Pro Val
    210                 215                 220

Val Gln Phe Ala Ala Gly Gly Val Ala Thr Pro Ala Asp Ala Ala Leu
225                 230                 235                 240

Met Met Gln Leu Gly Cys Asp Gly Val Phe Val Gly Ser Gly Val Phe
                245                 250                 255

Lys Ser Gly Asp Pro Val Lys Arg Ala Lys Ala Ile Val Gln Ala Val
            260                 265                 270

Thr Asn Tyr Arg Asp Ala Ala Val Leu Ala Glu Val Ser Cys Gly Leu
        275                 280                 285

Gly Glu Ala Met Val Gly Leu Asn Leu Asp Asp Lys Val Glu Arg Phe
    290                 295                 300

Ala Ser Arg Ser Glu
305

<210>  101
<211>  930
<212>  DNA
<213>  Arabidopsis thaliana

<220>
<221>  CDS
<222>  (1)..(930)
<223>  PDX1.3

<400>  101
atg gag ggc acc ggc gtg gtg gcc gtg tac ggc aac ggc gcc atc acc      48
Met Glu Gly Thr Gly Val Val Ala Val Tyr Gly Asn Gly Ala Ile Thr
1               5                   10                  15

gag gcc aag aag agc ccg ttc agc gtg aag gtg ggc ctg gcc cag atg 96
Glu Ala Lys Lys Ser Pro Phe Ser Val Lys Val Gly Leu Ala Gln Met
            20                  25                  30

ctg agg ggc ggc gtg atc atg gac gtg gtg aac gcc gag cag gcc agg 144
Leu Arg Gly Gly Val Ile Met Asp Val Val Asn Ala Glu Gln Ala Arg
        35                  40                  45

atc gcc gag gag gcc ggc gcc tgc gcc gtg atg gcc ctg gag agg gtg 192
Ile Ala Glu Glu Ala Gly Ala Cys Ala Val Met Ala Leu Glu Arg Val
    50                  55                  60

ccg gcc gac atc agg gcc cag ggc ggc gtg gcc agg atg agc gac ccg 240
Pro Ala Asp Ile Arg Ala Gln Gly Gly Val Ala Arg Met Ser Asp Pro
65                  70                  75                  80

cag atg atc aag gag atc aag cag gcc gtg acc atc ccg gtg atg gcc 288
Gln Met Ile Lys Glu Ile Lys Gln Ala Val Thr Ile Pro Val Met Ala
                85                  90                  95

aag gcc agg atc ggc cac ttc gtg gag gcc cag atc ctg gag gcc atc 336
Lys Ala Arg Ile Gly His Phe Val Glu Ala Gln Ile Leu Glu Ala Ile
            100                 105                 110

ggc atc gac tac atc gac gag agc gag gtg ctg acc ctg gcc gac gag 384
Gly Ile Asp Tyr Ile Asp Glu Ser Glu Val Leu Thr Leu Ala Asp Glu
        115                 120                 125

gac cac cac atc aac aag cac aac ttc agg atc ccg ttc gtg tgc ggc 432
Asp His His Ile Asn Lys His Asn Phe Arg Ile Pro Phe Val Cys Gly
    130                 135                 140

tgc agg aac ctg ggc gag gcc ctg agg agg atc agg gag ggc gcc gcc 480
Cys Arg Asn Leu Gly Glu Ala Leu Arg Arg Ile Arg Glu Gly Ala Ala
145                 150                 155                 160

atg atc agg acc aag ggc gag gcc ggc acc ggc aac atc atc gag gcc 528
Met Ile Arg Thr Lys Gly Glu Ala Gly Thr Gly Asn Ile Ile Glu Ala
                165                 170                 175

gtg agg cac gtg agg agc gtg aac ggc gac atc agg gtg ctg agg aac 576
Val Arg His Val Arg Ser Val Asn Gly Asp Ile Arg Val Leu Arg Asn
            180                 185                 190

atg gac gac gac gag gtg ttc acc ttc gcc aag aag ctg gcc gcc ccg 624
Met Asp Asp Asp Glu Val Phe Thr Phe Ala Lys Lys Leu Ala Ala Pro
        195                 200                 205

tac gac ctg gtg atg cag acc aag cag ctg ggc agg ctg ccg gtg gtg 672
Tyr Asp Leu Val Met Gln Thr Lys Gln Leu Gly Arg Leu Pro Val Val
    210                 215                 220

cag ttc gcc gcc ggc ggc gtg gcc acc ccg gcc gac gcc gcc ctg atg 720
Gln Phe Ala Ala Gly Gly Val Ala Thr Pro Ala Asp Ala Ala Leu Met
225                 230                 235                 240

atg cag ctg ggc tgc gac ggc gtg ttc gtg ggc agc ggc atc ttc aag 768
Met Gln Leu Gly Cys Asp Gly Val Phe Val Gly Ser Gly Ile Phe Lys
                245                 250                 255

agc ggc gac ccg gcc agg agg gcc agg gcc atc gtg cag gcc gtg acc 816
Ser Gly Asp Pro Ala Arg Arg Ala Arg Ala Ile Val Gln Ala Val Thr
            260                 265                 270

cac tac agc gac ccg gag atg ctg gtg gag gtg agc tgc ggc ctg ggc 864
His Tyr Ser Asp Pro Glu Met Leu Val Glu Val Ser Cys Gly Leu Gly
        275                 280                 285

gag gcc atg gtg ggc atc aac ctg aac gac gag aag gtg gag agg ttc 912

Glu Ala Met Val Gly Ile Asn Leu Asn Asp Glu Lys Val Glu Arg Phe
    290                 295                 300

gcc aac agg agc gag tga                                           930
Ala Asn Arg Ser Glu
305

<210>  102
<211>  309
<212>  PRT
<213>  Arabidopsis thaliana

<400>  102

Met Glu Gly Thr Gly Val Val Ala Val Tyr Gly Asn Gly Ala Ile Thr
1               5                   10                  15

Glu Ala Lys Lys Ser Pro Phe Ser Val Lys Val Gly Leu Ala Gln Met
            20                  25                  30

Leu Arg Gly Gly Val Ile Met Asp Val Val Asn Ala Glu Gln Ala Arg
        35                  40                  45

Ile Ala Glu Glu Ala Gly Ala Cys Ala Val Met Ala Leu Glu Arg Val
    50                  55                  60

Pro Ala Asp Ile Arg Ala Gln Gly Gly Val Ala Arg Met Ser Asp Pro
65                  70                  75                  80

Gln Met Ile Lys Glu Ile Lys Gln Ala Val Thr Ile Pro Val Met Ala
                85                  90                  95

Lys Ala Arg Ile Gly His Phe Val Glu Ala Gln Ile Leu Glu Ala Ile
            100                 105                 110

Gly Ile Asp Tyr Ile Asp Glu Ser Glu Val Leu Thr Leu Ala Asp Glu
        115                 120                 125

Asp His His Ile Asn Lys His Asn Phe Arg Ile Pro Phe Val Cys Gly
    130                 135                 140

Cys Arg Asn Leu Gly Glu Ala Leu Arg Arg Ile Arg Glu Gly Ala Ala
145                 150                 155                 160

Met Ile Arg Thr Lys Gly Glu Ala Gly Thr Gly Asn Ile Ile Glu Ala
                165                 170                 175

Val Arg His Val Arg Ser Val Asn Gly Asp Ile Arg Val Leu Arg Asn
            180                 185                 190

Met Asp Asp Asp Glu Val Phe Thr Phe Ala Lys Lys Leu Ala Ala Pro
        195                 200                 205

Tyr Asp Leu Val Met Gln Thr Lys Gln Leu Gly Arg Leu Pro Val Val
    210                 215                 220

Gln Phe Ala Ala Gly Gly Val Ala Thr Pro Ala Asp Ala Ala Leu Met
225                 230                 235                 240

Met Gln Leu Gly Cys Asp Gly Val Phe Val Gly Ser Gly Ile Phe Lys
                245                 250                 255

Ser Gly Asp Pro Ala Arg Arg Ala Arg Ala Ile Val Gln Ala Val Thr
            260                 265                 270

His Tyr Ser Asp Pro Glu Met Leu Val Glu Val Ser Cys Gly Leu Gly
        275                 280                 285

Glu Ala Met Val Gly Ile Asn Leu Asn Asp Glu Lys Val Glu Arg Phe
    290                 295                 300

Ala Asn Arg Ser Glu
305

<210>  103
<211>  1032
<212>  DNA
<213>  Cercospora nicotianae

<220>
<221>  CDS
<222>  (1)..(1032)
<223>  PDX1

<400>  103
atg gcc agc aac ggc acc agc gtg agc ccg ttc agg agc cag aag aac      48
Met Ala Ser Asn Gly Thr Ser Val Ser Pro Phe Arg Ser Gln Lys Asn
1               5                   10                  15

gcc gcc atg gcc gtg aac gac acc ccg gcc aac ggc cac gcc gag ccg      96
Ala Ala Met Ala Val Asn Asp Thr Pro Ala Asn Gly His Ala Glu Pro
            20                  25                  30

agc acc atc acc gcc gcc agc aag acc aac acc acc aag atc acc agc      144
Ser Thr Ile Thr Ala Ala Ser Lys Thr Asn Thr Thr Lys Ile Thr Ser
        35                  40                  45

cag aac gac ccg cag agc agc ttc gcc gtg aag gtg ggc ctg gcc cag      192
Gln Asn Asp Pro Gln Ser Ser Phe Ala Val Lys Val Gly Leu Ala Gln
    50                  55                  60

atg ctg aag ggc ggc gtg atc atg gac gtg gtg aac gcc gag cag gcc      240
Met Leu Lys Gly Gly Val Ile Met Asp Val Val Asn Ala Glu Gln Ala
65                  70                  75                  80

agg atc gcc gag gag gcc ggc gcc tgc gcc gtg atg gcc ctg gag agg      288
Arg Ile Ala Glu Glu Ala Gly Ala Cys Ala Val Met Ala Leu Glu Arg
                85                  90                  95

gtg ccg gcc gac atc agg aag gac ggc ggc gtg gcc agg atg agc gac      336
Val Pro Ala Asp Ile Arg Lys Asp Gly Gly Val Ala Arg Met Ser Asp
            100                 105                 110

ccg cag atg atc aag gac atc atg aac gcc gtg acc atc ccg gtg atg      384
Pro Gln Met Ile Lys Asp Ile Met Asn Ala Val Thr Ile Pro Val Met
        115                 120                 125

gcc aag agc agg atc ggc cac ttc gtg gag tgc cag atc ctg cag gcc 432
Ala Lys Ser Arg Ile Gly His Phe Val Glu Cys Gln Ile Leu Gln Ala
    130                 135                 140

atc ggc gtg gac tac atc gac gag agc gag gtg ctg acc ccg gcc gac 480
Ile Gly Val Asp Tyr Ile Asp Glu Ser Glu Val Leu Thr Pro Ala Asp
145                 150                 155                 160

ccg gtg aac cac atc gac aag agc gtg tac aac gtg ccg ttc gtg tgc 528
Pro Val Asn His Ile Asp Lys Ser Val Tyr Asn Val Pro Phe Val Cys
                165                 170                 175

ggc tgc aag aac ctg ggc gag gcc ctg agg agg atc agc gag ggc gcc 576
Gly Cys Lys Asn Leu Gly Glu Ala Leu Arg Arg Ile Ser Glu Gly Ala
            180                 185                 190

gcc atg atc agg acc aag ggc gag gcc ggc acc ggc gac gtg gtg gag 624
Ala Met Ile Arg Thr Lys Gly Glu Ala Gly Thr Gly Asp Val Val Glu
        195                 200                 205

gcc gtg agg cac atg cag acc gtg aac gcc gag atc gcc aag gcc agc 672
Ala Val Arg His Met Gln Thr Val Asn Ala Glu Ile Ala Lys Ala Ser
    210                 215                 220

agc gcc agc gac gcc gac ctg agg atg atg gcc agg gag ctg cag tgc 720
Ser Ala Ser Asp Ala Asp Leu Arg Met Met Ala Arg Glu Leu Gln Cys
225                 230                 235                 240

gac tac aac ctg ctg aag cag acc gcc cag ctg aag agg ctg ccg gtg 768
Asp Tyr Asn Leu Leu Lys Gln Thr Ala Gln Leu Lys Arg Leu Pro Val
                245                 250                 255

gtg aac ttc gcc gcc ggc ggc atc gcc acc ccg gcc gac gcc gcc ctg 816
Val Asn Phe Ala Ala Gly Gly Ile Ala Thr Pro Ala Asp Ala Ala Leu
            260                 265                 270

atg atg cag atg ggc tgc gac ggc gtg ttc gtg ggc agc ggc atc ttc 864
Met Met Gln Met Gly Cys Asp Gly Val Phe Val Gly Ser Gly Ile Phe
        275                 280                 285

aag agc ggc gac gcc gcc aag agg gcc aag gcc atc gtg cag gcc acc 912
Lys Ser Gly Asp Ala Ala Lys Arg Ala Lys Ala Ile Val Gln Ala Thr
    290                 295                 300

acc cac tac aac gac ccg aag gtg ctg gcc gag gtg agc agc ggc ctg 960
Thr His Tyr Asn Asp Pro Lys Val Leu Ala Glu Val Ser Ser Gly Leu
305                 310                 315                 320

ggc gag gcc atg gtg ggc atc aac tgc gac aag ctg ccg gag acc cag 1008
Gly Glu Ala Met Val Gly Ile Asn Cys Asp Lys Leu Pro Glu Thr Gln
                325                 330                 335

aag ctg gcc acc agg ggc tgg tga 1032
Lys Leu Ala Thr Arg Gly Trp
            340

<210> 104
<211> 343
<212> PRT
<213> Cercospora nicotianae

<400> 104

Met Ala Ser Asn Gly Thr Ser Val Ser Pro Phe Arg Ser Gln Lys Asn
1               5                   10                  15

Ala Ala Met Ala Val Asn Asp Thr Pro Ala Asn Gly His Ala Glu Pro
            20                  25                  30

Ser Thr Ile Thr Ala Ala Ser Lys Thr Asn Thr Thr Lys Ile Thr Ser
        35                  40                  45

Gln Asn Asp Pro Gln Ser Ser Phe Ala Val Lys Val Gly Leu Ala Gln
    50                  55                  60

Met Leu Lys Gly Gly Val Ile Met Asp Val Val Asn Ala Glu Gln Ala
65                  70                  75                  80

Arg Ile Ala Glu Glu Ala Gly Ala Cys Ala Val Met Ala Leu Glu Arg
                85                  90                  95

Val Pro Ala Asp Ile Arg Lys Asp Gly Gly Val Ala Arg Met Ser Asp
            100                 105                 110

Pro Gln Met Ile Lys Asp Ile Met Asn Ala Val Thr Ile Pro Val Met
        115                 120                 125

Ala Lys Ser Arg Ile Gly His Phe Val Glu Cys Gln Ile Leu Gln Ala
    130                 135                 140

Ile Gly Val Asp Tyr Ile Asp Glu Ser Glu Val Leu Thr Pro Ala Asp
145                 150                 155                 160

Pro Val Asn His Ile Asp Lys Ser Val Tyr Asn Val Pro Phe Val Cys
                165                 170                 175

Gly Cys Lys Asn Leu Gly Glu Ala Leu Arg Arg Ile Ser Glu Gly Ala
            180                 185                 190

Ala Met Ile Arg Thr Lys Gly Glu Ala Gly Thr Gly Asp Val Val Glu
        195                 200                 205

Ala Val Arg His Met Gln Thr Val Asn Ala Glu Ile Ala Lys Ala Ser
    210                 215                 220

Ser Ala Ser Asp Ala Asp Leu Arg Met Met Ala Arg Glu Leu Gln Cys
225                 230                 235                 240

Asp Tyr Asn Leu Leu Lys Gln Thr Ala Gln Leu Lys Arg Leu Pro Val
                245                 250                 255

Val Asn Phe Ala Ala Gly Gly Ile Ala Thr Pro Ala Asp Ala Ala Leu
            260                 265                 270

Met Met Gln Met Gly Cys Asp Gly Val Phe Val Gly Ser Gly Ile Phe
        275                 280                 285

Lys Ser Gly Asp Ala Ala Lys Arg Ala Lys Ala Ile Val Gln Ala Thr

290                 295                 300

Thr His Tyr Asn Asp Pro Lys Val Leu Ala Glu Val Ser Ser Gly Leu
305                 310                 315                 320

Gly Glu Ala Met Val Gly Ile Asn Cys Asp Lys Leu Pro Glu Thr Gln
                325                 330                 335

Lys Leu Ala Thr Arg Gly Trp
            340

<210>  105
<211>  1233
<212>  DNA
<213>  Saccharomyces cerevisiae

<220>
<221>  CDS
<222>  (1)..(1233)
<223>  PDX1

<400>  105
atg ctg agc gcc atc agc aag gtg agc acc ctg aag agc tgc acc agg      48
Met Leu Ser Ala Ile Ser Lys Val Ser Thr Leu Lys Ser Cys Thr Arg
1               5                   10                  15

tac ctg acc aag tgc aac tac cac gcc agc gcc aag ctg ctg gcc gtg      96
Tyr Leu Thr Lys Cys Asn Tyr His Ala Ser Ala Lys Leu Leu Ala Val
            20                  25                  30

aag acc ttc agc atg ccg gcc atg agc ccg acc atg gag aag ggc ggc      144
Lys Thr Phe Ser Met Pro Ala Met Ser Pro Thr Met Glu Lys Gly Gly
        35                  40                  45

atc gtg agc tgg aag tac aag gtg ggc gag ccg ttc agc gcc ggc gac      192
Ile Val Ser Trp Lys Tyr Lys Val Gly Glu Pro Phe Ser Ala Gly Asp
    50                  55                  60

gtg atc ctg gag gtg gag acc gac aag agc cag atc gac gtg gag gcc      240
Val Ile Leu Glu Val Glu Thr Asp Lys Ser Gln Ile Asp Val Glu Ala
65                  70                  75                  80

ctg gac gac ggc aag ctg gcc aag atc ctg aag gac gag ggc agc aag      288
Leu Asp Asp Gly Lys Leu Ala Lys Ile Leu Lys Asp Glu Gly Ser Lys
                85                  90                  95

gac gtg gac gtg ggc gag ccg atc gcc tac atc gcc gac gtg gac gac      336
Asp Val Asp Val Gly Glu Pro Ile Ala Tyr Ile Ala Asp Val Asp Asp
            100                 105                 110

gac ctg gcc acc atc aag ctg ccg cag gag gcc aac acc gcc aac gcc      384
Asp Leu Ala Thr Ile Lys Leu Pro Gln Glu Ala Asn Thr Ala Asn Ala
        115                 120                 125

aag agc atc gag atc aag aag ccg agc gcc gac agc acc gag gcc acc      432
Lys Ser Ile Glu Ile Lys Lys Pro Ser Ala Asp Ser Thr Glu Ala Thr
    130                 135                 140

cag cag cac ctg aag aag gcc acc gtg acc ccg atc aag acc gtg gac      480
Gln Gln His Leu Lys Lys Ala Thr Val Thr Pro Ile Lys Thr Val Asp
145                 150                 155                 160

ggc agc cag gcc aac ctg gag cag acc ctg ctg ccg agc gtg agc ctg      528
Gly Ser Gln Ala Asn Leu Glu Gln Thr Leu Leu Pro Ser Val Ser Leu

165                 170                 175

ctg ctg gcc gag aac aac atc agc aag cag aag gcc ctg aag gag atc      576
Leu Leu Ala Glu Asn Asn Ile Ser Lys Gln Lys Ala Leu Lys Glu Ile
            180                 185                 190

gcc ccg agc ggc agc aac ggc agg ctg ctg aag ggc gac gtg ctg gcc      624
Ala Pro Ser Gly Ser Asn Gly Arg Leu Leu Lys Gly Asp Val Leu Ala
        195                 200                 205

tac ctg ggc aag atc ccg cag gac agc gtg aac aag gtg acc gag ttc      672
Tyr Leu Gly Lys Ile Pro Gln Asp Ser Val Asn Lys Val Thr Glu Phe
    210                 215                 220

atc aag aag aac gag agg ctg gac ctg agc aac atc aag ccg atc cag      720
Ile Lys Lys Asn Glu Arg Leu Asp Leu Ser Asn Ile Lys Pro Ile Gln
225                 230                 235                 240

ctg aag ccg aag atc gcc gag cag gcc cag acc aag gcc gcc gac aag      768
Leu Lys Pro Lys Ile Ala Glu Gln Ala Gln Thr Lys Ala Ala Asp Lys
                245                 250                 255

ccg aag atc acc ccg gtg gag ttc gag gag cag ctg gtg ttc cac gcc      816
Pro Lys Ile Thr Pro Val Glu Phe Glu Glu Gln Leu Val Phe His Ala
            260                 265                 270

ccg gcc agc atc ccg ttc gac aag ctg agc gag agc ctg aac agc ttc      864
Pro Ala Ser Ile Pro Phe Asp Lys Leu Ser Glu Ser Leu Asn Ser Phe
        275                 280                 285

atg aag gag gcc tac cag ttc agc cac ggc acc ccg ctg atg gac acc      912
Met Lys Glu Ala Tyr Gln Phe Ser His Gly Thr Pro Leu Met Asp Thr
    290                 295                 300

aac agc aag tac ttc gac ccg atc ttc gag gac ctg gtg acc ctg agc      960
Asn Ser Lys Tyr Phe Asp Pro Ile Phe Glu Asp Leu Val Thr Leu Ser
305                 310                 315                 320

ccg agg gag ccg agg ttc aag ttc agc tac gac ctg atg cag atc ccg      1008
Pro Arg Glu Pro Arg Phe Lys Phe Ser Tyr Asp Leu Met Gln Ile Pro
                325                 330                 335

aag gcc aac aac atg cag gac acc tac ggc cag gag gac atc ttc gac      1056
Lys Ala Asn Asn Met Gln Asp Thr Tyr Gly Gln Glu Asp Ile Phe Asp
            340                 345                 350

ctg ctg acc ggc agc gac gcc acc gcc agc agc gtg agg ccg gtg gag      1104
Leu Leu Thr Gly Ser Asp Ala Thr Ala Ser Ser Val Arg Pro Val Glu
        355                 360                 365

aag aac ctg ccg gag aag aac gag tac atc ctg gcc ctg aac gtg agc      1152
Lys Asn Leu Pro Glu Lys Asn Glu Tyr Ile Leu Ala Leu Asn Val Ser
    370                 375                 380

gtg aac aac aag aag ttc aac gac gcc gag gcc aag gcc aag agg ttc      1200
Val Asn Asn Lys Lys Phe Asn Asp Ala Glu Ala Lys Ala Lys Arg Phe
385                 390                 395                 400

ctg gac tac gtg agg gag ctg gag agc ttc tga                          1233
Leu Asp Tyr Val Arg Glu Leu Glu Ser Phe
                405                 410

<210>  106
<211>  410
<212>  PRT
<213>  Saccharomyces cerevisiae

<400>  106

Met Leu Ser Ala Ile Ser Lys Val Ser Thr Leu Lys Ser Cys Thr Arg
1               5                   10                  15

Tyr Leu Thr Lys Cys Asn Tyr His Ala Ser Ala Lys Leu Leu Ala Val
            20                  25                  30

Lys Thr Phe Ser Met Pro Ala Met Ser Pro Thr Met Glu Lys Gly Gly
        35                  40                  45

Ile Val Ser Trp Lys Tyr Lys Val Gly Glu Pro Phe Ser Ala Gly Asp
    50                  55                  60

Val Ile Leu Glu Val Glu Thr Asp Lys Ser Gln Ile Asp Val Glu Ala
65                  70                  75                  80

Leu Asp Asp Gly Lys Leu Ala Lys Ile Leu Lys Asp Glu Gly Ser Lys
                85                  90                  95

Asp Val Asp Val Gly Glu Pro Ile Ala Tyr Ile Ala Asp Val Asp Asp
            100                 105                 110

Asp Leu Ala Thr Ile Lys Leu Pro Gln Glu Ala Asn Thr Ala Asn Ala
        115                 120                 125

Lys Ser Ile Glu Ile Lys Lys Pro Ser Ala Asp Ser Thr Glu Ala Thr
    130                 135                 140

Gln Gln His Leu Lys Lys Ala Thr Val Thr Pro Ile Lys Thr Val Asp
145                 150                 155                 160

Gly Ser Gln Ala Asn Leu Glu Gln Thr Leu Leu Pro Ser Val Ser Leu
                165                 170                 175

Leu Leu Ala Glu Asn Asn Ile Ser Lys Gln Lys Ala Leu Lys Glu Ile
            180                 185                 190

Ala Pro Ser Gly Ser Asn Gly Arg Leu Leu Lys Gly Asp Val Leu Ala
        195                 200                 205

Tyr Leu Gly Lys Ile Pro Gln Asp Ser Val Asn Lys Val Thr Glu Phe
    210                 215                 220

Ile Lys Lys Asn Glu Arg Leu Asp Leu Ser Asn Ile Lys Pro Ile Gln
225                 230                 235                 240

Leu Lys Pro Lys Ile Ala Glu Gln Ala Gln Thr Lys Ala Ala Asp Lys
                245                 250                 255

Pro Lys Ile Thr Pro Val Glu Phe Glu Glu Gln Leu Val Phe His Ala
            260                 265                 270

Pro Ala Ser Ile Pro Phe Asp Lys Leu Ser Glu Ser Leu Asn Ser Phe
        275                 280                 285

Met Lys Glu Ala Tyr Gln Phe Ser His Gly Thr Pro Leu Met Asp Thr
    290                 295                 300

Asn Ser Lys Tyr Phe Asp Pro Ile Phe Glu Asp Leu Val Thr Leu Ser
305                 310                 315                 320

Pro Arg Glu Pro Arg Phe Lys Phe Ser Tyr Asp Leu Met Gln Ile Pro
                325                 330                 335

Lys Ala Asn Asn Met Gln Asp Thr Tyr Gly Gln Glu Asp Ile Phe Asp
            340                 345                 350

Leu Leu Thr Gly Ser Asp Ala Thr Ala Ser Ser Val Arg Pro Val Glu
        355                 360                 365

Lys Asn Leu Pro Glu Lys Asn Glu Tyr Ile Leu Ala Leu Asn Val Ser
    370                 375                 380

Val Asn Asn Lys Lys Phe Asn Asp Ala Glu Ala Lys Ala Lys Arg Phe
385                 390                 395                 400

Leu Asp Tyr Val Arg Glu Leu Glu Ser Phe
                405                 410

<210>  107
<211>  918
<212>  DNA
<213>  Bacillus subtilis

<220>
<221>  CDS
<222>  (1)..(918)
<223>  PDX1

<400>  107
atg gcc agc atg gcc cag acc ggc acc gag agg gtg aag agg ggc atg      48
Met Ala Ser Met Ala Gln Thr Gly Thr Glu Arg Val Lys Arg Gly Met
1               5                   10                  15

gcc gag atg cag aag ggc ggc gtg atc atg gac gtg atc aac gcc gag      96
Ala Glu Met Gln Lys Gly Gly Val Ile Met Asp Val Ile Asn Ala Glu
            20                  25                  30

cag gcc aag atc gcc gag gag gcc ggc gcc gtg gcc gtg atg gcc ctg      144
Gln Ala Lys Ile Ala Glu Glu Ala Gly Ala Val Ala Val Met Ala Leu
        35                  40                  45

gag agg gtg ccg gcc gac atc agg gcc gcc ggc ggc gtg gcc agg atg      192
Glu Arg Val Pro Ala Asp Ile Arg Ala Ala Gly Gly Val Ala Arg Met
    50                  55                  60

gcc gac ccg acc atc gtg gag gag gtg atg aac gcc gtg agc atc ccg      240
Ala Asp Pro Thr Ile Val Glu Glu Val Met Asn Ala Val Ser Ile Pro
65                  70                  75                  80

gtg atg gcc aag gcc agg atc ggc cac atc gtg gag gcc agg gtg ctg      288

Val Met Ala Lys Ala Arg Ile Gly His Ile Val Glu Ala Arg Val Leu
                85                  90                  95

gag gcc atg ggc gtg gac tac atc gac gag agc gag gtg ctg acc ccg      336
Glu Ala Met Gly Val Asp Tyr Ile Asp Glu Ser Glu Val Leu Thr Pro
            100                 105                 110

gcc gac gag gag ttc cac ctg aac aag aac gag tac acc gtg ccg ttc      384
Ala Asp Glu Glu Phe His Leu Asn Lys Asn Glu Tyr Thr Val Pro Phe
        115                 120                 125

gtg tgc ggc tgc agg gac ctg ggc gag gcc acc agg agg atc gcc gag      432
Val Cys Gly Cys Arg Asp Leu Gly Glu Ala Thr Arg Arg Ile Ala Glu
    130                 135                 140

ggc gcc agc atg ctg agg acc aag ggc gag ccg ggc acc ggc aac atc      480
Gly Ala Ser Met Leu Arg Thr Lys Gly Glu Pro Gly Thr Gly Asn Ile
145                 150                 155                 160

gtg gag gcc gtg agg cac atg agg aag gtg aac gcc cag gtg agg aag      528
Val Glu Ala Val Arg His Met Arg Lys Val Asn Ala Gln Val Arg Lys
                165                 170                 175

gtg gtg gcc atg agc gag gac gag ctg atg acc gag gcc aag aac ctg      576
Val Val Ala Met Ser Glu Asp Glu Leu Met Thr Glu Ala Lys Asn Leu
            180                 185                 190

ggc gcc ccg tac gag ctg ctg ctg cag atc aag aag gac ggc aag ctg      624
Gly Ala Pro Tyr Glu Leu Leu Leu Gln Ile Lys Lys Asp Gly Lys Leu
        195                 200                 205

ccg gtg gtg aac ttc gcc gcc ggc ggc gtg gcc acc ccg gcc gac gcc      672
Pro Val Val Asn Phe Ala Ala Gly Gly Val Ala Thr Pro Ala Asp Ala
    210                 215                 220

gcc ctg atg atg cag ctg ggc gcc gac ggc gtg ttc gtg ggc agc ggc      720
Ala Leu Met Met Gln Leu Gly Ala Asp Gly Val Phe Val Gly Ser Gly
225                 230                 235                 240

atc ttc aag agc gac aac ccg gcc aag ttc gcc aag gcc atc gtg gag      768
Ile Phe Lys Ser Asp Asn Pro Ala Lys Phe Ala Lys Ala Ile Val Glu
                245                 250                 255

gcc acc acc cac ttc acc gac tac aag ctg atc gcc gag ctg agc aag      816
Ala Thr Thr His Phe Thr Asp Tyr Lys Leu Ile Ala Glu Leu Ser Lys
            260                 265                 270

gag ctg ggc acc gcc atg aag ggc atc gag atc agc aac ctg ctg ccg      864
Glu Leu Gly Thr Ala Met Lys Gly Ile Glu Ile Ser Asn Leu Leu Pro
        275                 280                 285

gag cag agg atg cag gag agg ggc tgg ctg gag cac cac cac cac cac      912
Glu Gln Arg Met Gln Glu Arg Gly Trp Leu Glu His His His His His
    290                 295                 300

cac tga                                                              918
His
305

<210>  108
<211>  305
<212>  PRT
<213>  Bacillus subtilis

<400>  108

Met Ala Ser Met Ala Gln Thr Gly Thr Glu Arg Val Lys Arg Gly Met
1               5                   10                  15

Ala Glu Met Gln Lys Gly Gly Val Ile Met Asp Val Ile Asn Ala Glu
            20                  25                  30

Gln Ala Lys Ile Ala Glu Glu Ala Gly Ala Val Ala Val Met Ala Leu
        35                  40                  45

Glu Arg Val Pro Ala Asp Ile Arg Ala Ala Gly Gly Val Ala Arg Met
    50                  55                  60

Ala Asp Pro Thr Ile Val Glu Glu Val Met Asn Ala Val Ser Ile Pro
65                  70                  75                  80

Val Met Ala Lys Ala Arg Ile Gly His Ile Val Glu Ala Arg Val Leu
                85                  90                  95

Glu Ala Met Gly Val Asp Tyr Ile Asp Glu Ser Glu Val Leu Thr Pro
            100                 105                 110

Ala Asp Glu Glu Phe His Leu Asn Lys Asn Glu Tyr Thr Val Pro Phe
        115                 120                 125

Val Cys Gly Cys Arg Asp Leu Gly Glu Ala Thr Arg Arg Ile Ala Glu
    130                 135                 140

Gly Ala Ser Met Leu Arg Thr Lys Gly Glu Pro Gly Thr Gly Asn Ile
145                 150                 155                 160

Val Glu Ala Val Arg His Met Arg Lys Val Asn Ala Gln Val Arg Lys
                165                 170                 175

Val Val Ala Met Ser Glu Asp Glu Leu Met Thr Glu Ala Lys Asn Leu
            180                 185                 190

Gly Ala Pro Tyr Glu Leu Leu Leu Gln Ile Lys Lys Asp Gly Lys Leu
        195                 200                 205

Pro Val Val Asn Phe Ala Ala Gly Gly Val Ala Thr Pro Ala Asp Ala
    210                 215                 220

Ala Leu Met Met Gln Leu Gly Ala Asp Gly Val Phe Val Gly Ser Gly
225                 230                 235                 240

Ile Phe Lys Ser Asp Asn Pro Ala Lys Phe Ala Lys Ala Ile Val Glu
                245                 250                 255

Ala Thr Thr His Phe Thr Asp Tyr Lys Leu Ile Ala Glu Leu Ser Lys
            260                 265                 270

Glu Leu Gly Thr Ala Met Lys Gly Ile Glu Ile Ser Asn Leu Leu Pro
        275                 280                 285

Glu Gln Arg Met Gln Glu Arg Gly Trp Leu Glu His His His His His
    290                 295                 300

His
305

<210>  109
<211>  957
<212>  DNA
<213>  Oryza sativa

<220>
<221>  CDS
<222>  (1)..(957)
<223>  PDX1.1

<400>  109
atg gcc acc gac ggc acc ggc gtg gtg acc gtg tac ggc agc ggc acc      48
Met Ala Thr Asp Gly Thr Gly Val Val Thr Val Tyr Gly Ser Gly Thr
1               5                   10                  15

aac ggc gcc gcc ctg ctg gag ccg agc aac cac aag agc gcc acc ttc      96
Asn Gly Ala Ala Leu Leu Glu Pro Ser Asn His Lys Ser Ala Thr Phe
            20                  25                  30

agc gtg aag gtg ggc ctg gcc cag atg ctg agg ggc ggc gtg atc atg      144
Ser Val Lys Val Gly Leu Ala Gln Met Leu Arg Gly Gly Val Ile Met
        35                  40                  45

gac gtg gtg acc ccg gag cag gcc agg atc gcc gag gag gcc ggc gcc      192
Asp Val Val Thr Pro Glu Gln Ala Arg Ile Ala Glu Glu Ala Gly Ala
    50                  55                  60

tgc gcc gtg atg gcc ctg gag agg gtg ccg gcc gac atc agg gcc cag      240
Cys Ala Val Met Ala Leu Glu Arg Val Pro Ala Asp Ile Arg Ala Gln
65                  70                  75                  80

ggc ggc gtg gcc agg atg agc gac ccg ggc ctg atc agg gac atc aag      288
Gly Gly Val Ala Arg Met Ser Asp Pro Gly Leu Ile Arg Asp Ile Lys
                85                  90                  95

agg gcc gtg acc atc ccg gtg atg gcc aag gcc agg atc ggc cac ttc      336
Arg Ala Val Thr Ile Pro Val Met Ala Lys Ala Arg Ile Gly His Phe
            100                 105                 110

gtg gag gcc cag atc ctg gag gcc atc ggc gtg gac tac gtg gac gag      384
Val Glu Ala Gln Ile Leu Glu Ala Ile Gly Val Asp Tyr Val Asp Glu
        115                 120                 125

agc gag gtg ctg acc ctg gcc gac gac gcc cac cac atc aac aag cac      432
Ser Glu Val Leu Thr Leu Ala Asp Asp Ala His His Ile Asn Lys His
    130                 135                 140

aac ttc agg gtg ccg ttc gtg tgc ggc tgc agg gac ctg ggc gag gcc      480
Asn Phe Arg Val Pro Phe Val Cys Gly Cys Arg Asp Leu Gly Glu Ala
145                 150                 155                 160

ctg agg agg atc agg gag ggc gcc gcc atg atc agg acc aag ggc gag      528
Leu Arg Arg Ile Arg Glu Gly Ala Ala Met Ile Arg Thr Lys Gly Glu
                165                 170                 175

gcc ggc acc ggc aac gtg gtg gag gcc gtg agg cac gtg agg agc gtg      576
Ala Gly Thr Gly Asn Val Val Glu Ala Val Arg His Val Arg Ser Val
            180                 185                 190

atg ggc gac atc agg gcc ctg agg aac atg gac gac gac gag gtg ttc 624
Met Gly Asp Ile Arg Ala Leu Arg Asn Met Asp Asp Asp Glu Val Phe
        195                 200                 205

agc tac gcc aag agg atc gcc gcc ccg tac gac ctg gtg atg cag acc 672
Ser Tyr Ala Lys Arg Ile Ala Ala Pro Tyr Asp Leu Val Met Gln Thr
    210                 215                 220

aag cag ctg ggc agg ctg ccg gtg gtg cag ttc gcc gcc ggc ggc gtg 720
Lys Gln Leu Gly Arg Leu Pro Val Val Gln Phe Ala Ala Gly Gly Val
225                 230                 235                 240

gcc acc ccg gcc gac gcc gcc ctg atg atg cag ctg ggc tgc gac ggc 768
Ala Thr Pro Ala Asp Ala Ala Leu Met Met Gln Leu Gly Cys Asp Gly
                245                 250                 255

gtg ttc gtg ggc agc ggc atc ttc aag agc ggc gac ccg gcc agg agg 816
Val Phe Val Gly Ser Gly Ile Phe Lys Ser Gly Asp Pro Ala Arg Arg
            260                 265                 270

gcc agg gcc atc gtg cag gcc gtg acc cac tac agc gac ccg aag atc 864
Ala Arg Ala Ile Val Gln Ala Val Thr His Tyr Ser Asp Pro Lys Ile
        275                 280                 285

ctg gcc gag gtg agc agc ggc ctg ggc gag gcc atg gtg ggc atc aac 912
Leu Ala Glu Val Ser Ser Gly Leu Gly Glu Ala Met Val Gly Ile Asn
    290                 295                 300

ctg agc gac ccg aag gtg gag agg ttc gcc gcc agg agc gag tga 957
Leu Ser Asp Pro Lys Val Glu Arg Phe Ala Ala Arg Ser Glu
305                 310                 315

<210> 110
<211> 318
<212> PRT
<213> Oryza sativa

<400> 110

Met Ala Thr Asp Gly Thr Gly Val Val Thr Val Tyr Gly Ser Gly Thr
1               5                   10                  15

Asn Gly Ala Ala Leu Leu Glu Pro Ser Asn His Lys Ser Ala Thr Phe
            20                  25                  30

Ser Val Lys Val Gly Leu Ala Gln Met Leu Arg Gly Gly Val Ile Met
        35                  40                  45

Asp Val Val Thr Pro Glu Gln Ala Arg Ile Ala Glu Glu Ala Gly Ala
    50                  55                  60

Cys Ala Val Met Ala Leu Glu Arg Val Pro Ala Asp Ile Arg Ala Gln
65                  70                  75                  80

Gly Gly Val Ala Arg Met Ser Asp Pro Gly Leu Ile Arg Asp Ile Lys
                85                  90                  95

Arg Ala Val Thr Ile Pro Val Met Ala Lys Ala Arg Ile Gly His Phe
            100                 105                 110

Val Glu Ala Gln Ile Leu Glu Ala Ile Gly Val Asp Tyr Val Asp Glu

115                 120                 125

Ser Glu Val Leu Thr Leu Ala Asp Asp Ala His His Ile Asn Lys His
    130                 135                 140

Asn Phe Arg Val Pro Phe Val Cys Gly Cys Arg Asp Leu Gly Glu Ala
145                 150                 155                 160

Leu Arg Arg Ile Arg Glu Gly Ala Ala Met Ile Arg Thr Lys Gly Glu
                165                 170                 175

Ala Gly Thr Gly Asn Val Val Glu Ala Val Arg His Val Arg Ser Val
            180                 185                 190

Met Gly Asp Ile Arg Ala Leu Arg Asn Met Asp Asp Asp Glu Val Phe
        195                 200                 205

Ser Tyr Ala Lys Arg Ile Ala Ala Pro Tyr Asp Leu Val Met Gln Thr
    210                 215                 220

Lys Gln Leu Gly Arg Leu Pro Val Val Gln Phe Ala Ala Gly Gly Val
225                 230                 235                 240

Ala Thr Pro Ala Asp Ala Ala Leu Met Met Gln Leu Gly Cys Asp Gly
                245                 250                 255

Val Phe Val Gly Ser Gly Ile Phe Lys Ser Gly Asp Pro Ala Arg Arg
            260                 265                 270

Ala Arg Ala Ile Val Gln Ala Val Thr His Tyr Ser Asp Pro Lys Ile
        275                 280                 285

Leu Ala Glu Val Ser Ser Gly Leu Gly Glu Ala Met Val Gly Ile Asn
    290                 295                 300

Leu Ser Asp Pro Lys Val Glu Arg Phe Ala Ala Arg Ser Glu
305                 310                 315

<210>  111
<211>  930
<212>  DNA
<213>  Hevea brasilensis

<220>
<221>  CDS
<222>  (1)..(930)
<223>  PDX1

<400>  111
atg gcc ggc acc ggc gtg gtg gcc gtg tac ggc aac ggc gcc atc acc        48
Met Ala Gly Thr Gly Val Val Ala Val Tyr Gly Asn Gly Ala Ile Thr
1               5                   10                  15

gag acc aag aag agc ccg ttc agc gtg aag gtg ggc ctg gcc cag atg        96
Glu Thr Lys Lys Ser Pro Phe Ser Val Lys Val Gly Leu Ala Gln Met

20                  25                  30

ctg agg ggc ggc gtg atc atg gac gtg gtg aac ccg gag cag gcc agg      144
Leu Arg Gly Gly Val Ile Met Asp Val Val Asn Pro Glu Gln Ala Arg
        35                  40                  45

atc gcc gag gag gcc ggc gcc tgc gcc gtg atg gcc ctg gag agg gtg      192
Ile Ala Glu Glu Ala Gly Ala Cys Ala Val Met Ala Leu Glu Arg Val
    50                  55                  60

ccg gcc gac atc agg gcc cag ggc ggc gtg gcc agg atg agc gac ccg      240
Pro Ala Asp Ile Arg Ala Gln Gly Gly Val Ala Arg Met Ser Asp Pro
65                  70                  75                  80

cag ctg atc aag gag atc aag cag agc gtg acc atc ccg gtg atg gcc      288
Gln Leu Ile Lys Glu Ile Lys Gln Ser Val Thr Ile Pro Val Met Ala
                85                  90                  95

aag gcc agg atc ggc cac ttc gtg gag gcc cag atc ctg gag gcc atc      336
Lys Ala Arg Ile Gly His Phe Val Glu Ala Gln Ile Leu Glu Ala Ile
            100                 105                 110

ggc atc gac tac gtg gac gag agc gag gtg ctg acc ccg gcc gac gag      384
Gly Ile Asp Tyr Val Asp Glu Ser Glu Val Leu Thr Pro Ala Asp Glu
        115                 120                 125

gag aac cac atc aac aag cac aac ttc agg atc ccg ttc gtg tgc ggc      432
Glu Asn His Ile Asn Lys His Asn Phe Arg Ile Pro Phe Val Cys Gly
    130                 135                 140

tgc agg aac ctg ggc gag gcc ctg agg agg atc agg gag ggc gcc gcc      480
Cys Arg Asn Leu Gly Glu Ala Leu Arg Arg Ile Arg Glu Gly Ala Ala
145                 150                 155                 160

atg atc agg acc aag ggc gag gcc ggc acc ggc aac gtg atc gag gcc      528
Met Ile Arg Thr Lys Gly Glu Ala Gly Thr Gly Asn Val Ile Glu Ala
                165                 170                 175

gtg agg cac gtg agg agc gtg atg ggc gac atc agg ctg ctg agg aac      576
Val Arg His Val Arg Ser Val Met Gly Asp Ile Arg Leu Leu Arg Asn
            180                 185                 190

atg gac gac gac gag gtg ttc acc ttc gcc aag aag atc gcc gcc ccg      624
Met Asp Asp Asp Glu Val Phe Thr Phe Ala Lys Lys Ile Ala Ala Pro
        195                 200                 205

tac gac ctg gtg atg cag acc aag cag ctg ggc agg ctg ccg gtg gtg      672
Tyr Asp Leu Val Met Gln Thr Lys Gln Leu Gly Arg Leu Pro Val Val
    210                 215                 220

cag ttc gcc gcc ggc ggc gtg gcc acc ccg gcc gac gcc gcc ctg atg      720
Gln Phe Ala Ala Gly Gly Val Ala Thr Pro Ala Asp Ala Ala Leu Met
225                 230                 235                 240

atg cag ctg ggc tgc gac ggc gtg ttc gtg ggc agc ggc gtg ttc aag      768
Met Gln Leu Gly Cys Asp Gly Val Phe Val Gly Ser Gly Val Phe Lys
                245                 250                 255

agc ggc gac ccg gcc agg agg gcc agg gcc atc gtg cag gcc gtg acc      816
Ser Gly Asp Pro Ala Arg Arg Ala Arg Ala Ile Val Gln Ala Val Thr
            260                 265                 270

cac tac agc gac ccg gac atg ctg gcc gag gtg agc tgc ggc ctg ggc      864
His Tyr Ser Asp Pro Asp Met Leu Ala Glu Val Ser Cys Gly Leu Gly
        275                 280                 285

gag gcc atg gtg ggc atc aac ctg aac gac aag aag gtg gag agg ttc      912
Glu Ala Met Val Gly Ile Asn Leu Asn Asp Lys Lys Val Glu Arg Phe
    290                 295                 300

gcc aac agg agc gag tga                                                    930
Ala Asn Arg Ser Glu
305

<210>  112
<211>  309
<212>  PRT
<213>  Hevea brasilensis

<400>  112

Met Ala Gly Thr Gly Val Val Ala Val Tyr Gly Asn Gly Ala Ile Thr
1               5                   10                  15

Glu Thr Lys Lys Ser Pro Phe Ser Val Lys Val Gly Leu Ala Gln Met
            20                  25                  30

Leu Arg Gly Gly Val Ile Met Asp Val Val Asn Pro Glu Gln Ala Arg
        35                  40                  45

Ile Ala Glu Glu Ala Gly Ala Cys Ala Val Met Ala Leu Glu Arg Val
    50                  55                  60

Pro Ala Asp Ile Arg Ala Gln Gly Gly Val Ala Arg Met Ser Asp Pro
65                  70                  75                  80

Gln Leu Ile Lys Glu Ile Lys Gln Ser Val Thr Ile Pro Val Met Ala
                85                  90                  95

Lys Ala Arg Ile Gly His Phe Val Glu Ala Gln Ile Leu Glu Ala Ile
            100                 105                 110

Gly Ile Asp Tyr Val Asp Glu Ser Glu Val Leu Thr Pro Ala Asp Glu
        115                 120                 125

Glu Asn His Ile Asn Lys His Asn Phe Arg Ile Pro Phe Val Cys Gly
    130                 135                 140

Cys Arg Asn Leu Gly Glu Ala Leu Arg Arg Ile Arg Glu Gly Ala Ala
145                 150                 155                 160

Met Ile Arg Thr Lys Gly Glu Ala Gly Thr Gly Asn Val Ile Glu Ala
                165                 170                 175

Val Arg His Val Arg Ser Val Met Gly Asp Ile Arg Leu Leu Arg Asn
            180                 185                 190

Met Asp Asp Asp Glu Val Phe Thr Phe Ala Lys Lys Ile Ala Ala Pro
        195                 200                 205

Tyr Asp Leu Val Met Gln Thr Lys Gln Leu Gly Arg Leu Pro Val Val
    210                 215                 220

Gln Phe Ala Ala Gly Gly Val Ala Thr Pro Ala Asp Ala Ala Leu Met
225                 230                 235                 240

Met Gln Leu Gly Cys Asp Gly Val Phe Val Gly Ser Gly Val Phe Lys
                245                 250                 255

Ser Gly Asp Pro Ala Arg Arg Ala Arg Ala Ile Val Gln Ala Val Thr
            260                 265                 270

His Tyr Ser Asp Pro Asp Met Leu Ala Glu Val Ser Cys Gly Leu Gly
        275                 280                 285

Glu Ala Met Val Gly Ile Asn Leu Asn Asp Lys Lys Val Glu Arg Phe
    290                 295                 300

Ala Asn Arg Ser Glu
305

<210>  113
<211>  669
<212>  DNA
<213>  Stellaria longipes

<220>
<221>  CDS
<222>  (1)..(669)
<223>  PDX1

<400>  113
atg gcc aag gcc agg atc ggc cac ttc gtg gag gcc cag atc ctg gag      48
Met Ala Lys Ala Arg Ile Gly His Phe Val Glu Ala Gln Ile Leu Glu
1               5                   10                  15

agc atc ggc gtg gac tac gtg gac gag agc gag gtg ctg acc ccg gcc      96
Ser Ile Gly Val Asp Tyr Val Asp Glu Ser Glu Val Leu Thr Pro Ala
            20                  25                  30

gac gag gac cac cac atc aac aag cac aac ttc cag atc ccg ttc gtg      144
Asp Glu Asp His His Ile Asn Lys His Asn Phe Gln Ile Pro Phe Val
        35                  40                  45

tgc ggc ctg agc atc ccg agg ggc gcc ccg ccg ccg gcc tac agg agg      192
Cys Gly Leu Ser Ile Pro Arg Gly Ala Pro Pro Pro Ala Tyr Arg Arg
    50                  55                  60

ggc tac gcc ggc cac gac acc ggc ccg agg gtg agg aag ccg agc acc      240
Gly Tyr Ala Gly His Asp Thr Gly Pro Arg Val Arg Lys Pro Ser Thr
65                  70                  75                  80

ggc aac gtg gtg gag gcc gtg agg cac atc agg agc gtg atg ggc gag      288
Gly Asn Val Val Glu Ala Val Arg His Ile Arg Ser Val Met Gly Glu
                85                  90                  95

atc agg ctg ctg agg aac atg gac gac gac gag gtg ttc gcc tac gcc      336
Ile Arg Leu Leu Arg Asn Met Asp Asp Asp Glu Val Phe Ala Tyr Ala
            100                 105                 110

aag aag atc agc gcc gcc tac gac ctg gtg atg cag acc aag cag ctg      384
Lys Lys Ile Ser Ala Ala Tyr Asp Leu Val Met Gln Thr Lys Gln Leu
        115                 120                 125

ggc agg ctg ccg gtg gtg aac ttc gcc gcc ggc ggc gtg gcc acc ccg      432

Gly Arg Leu Pro Val Val Asn Phe Ala Ala Gly Gly Val Ala Thr Pro
    130                 135                 140

gcc gac gcc gcc ctg atg atg cag ctg ggc tgc gac ggc gtg ttc gtg         480
Ala Asp Ala Ala Leu Met Met Gln Leu Gly Cys Asp Gly Val Phe Val
145                 150                 155                 160

ggc agc ggc gtg ttc aag agc ggc gac ccg gcc aag agg gcc agg gcc         528
Gly Ser Gly Val Phe Lys Ser Gly Asp Pro Ala Lys Arg Ala Arg Ala
                165                 170                 175

atc gtg cag gcc gtg acc cac tac agc gac ccg gac ctg ctg ggc agg         576
Ile Val Gln Ala Val Thr His Tyr Ser Asp Pro Asp Leu Leu Gly Arg
            180                 185                 190

ggc gag ttc ggc ctg ggc gag gcc atg gtg ggc atc atc gtg agg atg         624
Gly Glu Phe Gly Leu Gly Glu Ala Met Val Gly Ile Ile Val Arg Met
        195                 200                 205

agg agg ctg agg ggc acc ccg atc gtg ctg aac gag gtg atc tga             669
Arg Arg Leu Arg Gly Thr Pro Ile Val Leu Asn Glu Val Ile
    210                 215                 220


<210>  114
<211>  222
<212>  PRT
<213>  Stellaria longipes

<400>  114

Met Ala Lys Ala Arg Ile Gly His Phe Val Glu Ala Gln Ile Leu Glu
1               5                   10                  15


Ser Ile Gly Val Asp Tyr Val Asp Glu Ser Glu Val Leu Thr Pro Ala
            20                  25                  30


Asp Glu Asp His His Ile Asn Lys His Asn Phe Gln Ile Pro Phe Val
        35                  40                  45


Cys Gly Leu Ser Ile Pro Arg Gly Ala Pro Pro Pro Ala Tyr Arg Arg
    50                  55                  60


Gly Tyr Ala Gly His Asp Thr Gly Pro Arg Val Arg Lys Pro Ser Thr
65                  70                  75                  80


Gly Asn Val Val Glu Ala Val Arg His Ile Arg Ser Val Met Gly Glu
                85                  90                  95


Ile Arg Leu Leu Arg Asn Met Asp Asp Asp Glu Val Phe Ala Tyr Ala
            100                 105                 110


Lys Lys Ile Ser Ala Ala Tyr Asp Leu Val Met Gln Thr Lys Gln Leu
        115                 120                 125


Gly Arg Leu Pro Val Val Asn Phe Ala Ala Gly Gly Val Ala Thr Pro
    130                 135                 140


Ala Asp Ala Ala Leu Met Met Gln Leu Gly Cys Asp Gly Val Phe Val
145                 150                 155                 160

Gly Ser Gly Val Phe Lys Ser Gly Asp Pro Ala Lys Arg Ala Arg Ala
                165                 170                 175

Ile Val Gln Ala Val Thr His Tyr Ser Asp Pro Asp Leu Leu Gly Arg
            180                 185                 190

Gly Glu Phe Gly Leu Gly Glu Ala Met Val Gly Ile Ile Val Arg Met
        195                 200                 205

Arg Arg Leu Arg Gly Thr Pro Ile Val Leu Asn Glu Val Ile
    210                 215                 220

<210>  115
<211>  933
<212>  DNA
<213>  Brassica napus

<220>
<221>  CDS
<222>  (1)..(933)
<223>  PDX1

<400>  115
atg gcg gaa tca gcc gga gtg gtg gcg gtt tac ggc gaa gga tcc atg      48
Met Ala Glu Ser Ala Gly Val Val Ala Val Tyr Gly Glu Gly Ser Met
1               5                   10                  15

acc gag acg aag cag caa tct ccc ttc tcc gtc aag gtc ggc ctc gct      96
Thr Glu Thr Lys Gln Gln Ser Pro Phe Ser Val Lys Val Gly Leu Ala
            20                  25                  30

cag atg ctc cgc ggc ggc gtg atc atg gac gtc gtc aac gcc gag caa      144
Gln Met Leu Arg Gly Gly Val Ile Met Asp Val Val Asn Ala Glu Gln
        35                  40                  45

gcg cga atc gcc gaa gaa gcc ggc gcc tgc gcc gtc atg gct ctc gag      192
Ala Arg Ile Ala Glu Glu Ala Gly Ala Cys Ala Val Met Ala Leu Glu
    50                  55                  60

cgc gtt ccc gcc gat atc aga gcg caa ggt ggc gtc gcg agg atg agc      240
Arg Val Pro Ala Asp Ile Arg Ala Gln Gly Gly Val Ala Arg Met Ser
65                  70                  75                  80

gat ccg gag atg atc aaa gag atc aaa cag gcg gtt acg att ccg gtc      288
Asp Pro Glu Met Ile Lys Glu Ile Lys Gln Ala Val Thr Ile Pro Val
                85                  90                  95

atg gcc aaa gct cga atc ggc cac ttc gtc gag gcg cag atc ctc gag      336
Met Ala Lys Ala Arg Ile Gly His Phe Val Glu Ala Gln Ile Leu Glu
            100                 105                 110

tcc atc gga gtc gat tac gtc gac gag agt gaa gtc ctc act ctc gcc      384
Ser Ile Gly Val Asp Tyr Val Asp Glu Ser Glu Val Leu Thr Leu Ala
        115                 120                 125

gac gag gac aac cac atc aac aag cac aac ttc aag ata ccg ttc gtt      432
Asp Glu Asp Asn His Ile Asn Lys His Asn Phe Lys Ile Pro Phe Val
    130                 135                 140

tgt gga tgc agg aac ctc ggg gaa gcc ttg cgg cgg atc cgc gaa gga      480
Cys Gly Cys Arg Asn Leu Gly Glu Ala Leu Arg Arg Ile Arg Glu Gly
145                 150                 155                 160

gcc gcg atg ata agg acc aaa gga gag gcc ggg acg ggg aac gtc gtg     528
Ala Ala Met Ile Arg Thr Lys Gly Glu Ala Gly Thr Gly Asn Val Val
                165                 170                 175

gag gcc gtt agg cac gtg agg agc gtg atg gga gcg ata agg ttg ctg     576
Glu Ala Val Arg His Val Arg Ser Val Met Gly Ala Ile Arg Leu Leu
            180                 185                 190

aga ggc atg gat gac gac gag gtg ttc gct tac gcg aag aag atc gct     624
Arg Gly Met Asp Asp Asp Glu Val Phe Ala Tyr Ala Lys Lys Ile Ala
        195                 200                 205

gcg cct tat gag ttg gtt atg cag acg aag cag ctc ggg agg tta ccg     672
Ala Pro Tyr Glu Leu Val Met Gln Thr Lys Gln Leu Gly Arg Leu Pro
    210                 215                 220

gtg gtt cag ttc gcc gcc gga ggg gtc gcg acg ccg gcg gac gcg gcg     720
Val Val Gln Phe Ala Ala Gly Gly Val Ala Thr Pro Ala Asp Ala Ala
225                 230                 235                 240

ctg atg atg cag ctg ggg tgt gat gga gtg ttc gtt ggg tcg ggt att     768
Leu Met Met Gln Leu Gly Cys Asp Gly Val Phe Val Gly Ser Gly Ile
                245                 250                 255

ttc aag agt gga gat ccg gcg aag cgt gcg aag gcg att gtg cag gcg     816
Phe Lys Ser Gly Asp Pro Ala Lys Arg Ala Lys Ala Ile Val Gln Ala
            260                 265                 270

gtt acg aat tat aga gac gcg gcg gtg ttg gcg gag gtg agc tgt ggg     864
Val Thr Asn Tyr Arg Asp Ala Ala Val Leu Ala Glu Val Ser Cys Gly
        275                 280                 285

ttg ggt gaa gct atg gtt ggt atc aat ttg gat gat aag gtt gag agg     912
Leu Gly Glu Ala Met Val Gly Ile Asn Leu Asp Asp Lys Val Glu Arg
    290                 295                 300

ttc gcg aac cgg tct gag tga                                         933
Phe Ala Asn Arg Ser Glu
305                 310


<210>  116
<211>  310
<212>  PRT
<213>  Brassica napus

<400>  116

Met Ala Glu Ser Ala Gly Val Val Ala Val Tyr Gly Glu Gly Ser Met
1               5                   10                  15


Thr Glu Thr Lys Gln Gln Ser Pro Phe Ser Val Lys Val Gly Leu Ala
            20                  25                  30


Gln Met Leu Arg Gly Gly Val Ile Met Asp Val Val Asn Ala Glu Gln
        35                  40                  45


Ala Arg Ile Ala Glu Glu Ala Gly Ala Cys Ala Val Met Ala Leu Glu
    50                  55                  60


Arg Val Pro Ala Asp Ile Arg Ala Gln Gly Gly Val Ala Arg Met Ser
65                  70                  75                  80

Asp Pro Glu Met Ile Lys Glu Ile Lys Gln Ala Val Thr Ile Pro Val
                85                  90                  95

Met Ala Lys Ala Arg Ile Gly His Phe Val Glu Ala Gln Ile Leu Glu
            100                 105                 110

Ser Ile Gly Val Asp Tyr Val Asp Glu Ser Glu Val Leu Thr Leu Ala
        115                 120                 125

Asp Glu Asp Asn His Ile Asn Lys His Asn Phe Lys Ile Pro Phe Val
    130                 135                 140

Cys Gly Cys Arg Asn Leu Gly Glu Ala Leu Arg Arg Ile Arg Glu Gly
145                 150                 155                 160

Ala Ala Met Ile Arg Thr Lys Gly Glu Ala Gly Thr Gly Asn Val Val
                165                 170                 175

Glu Ala Val Arg His Val Arg Ser Val Met Gly Ala Ile Arg Leu Leu
            180                 185                 190

Arg Gly Met Asp Asp Asp Glu Val Phe Ala Tyr Ala Lys Lys Ile Ala
        195                 200                 205

Ala Pro Tyr Glu Leu Val Met Gln Thr Lys Gln Leu Gly Arg Leu Pro
    210                 215                 220

Val Val Gln Phe Ala Ala Gly Gly Val Ala Thr Pro Ala Asp Ala Ala
225                 230                 235                 240

Leu Met Met Gln Leu Gly Cys Asp Gly Val Phe Val Gly Ser Gly Ile
                245                 250                 255

Phe Lys Ser Gly Asp Pro Ala Lys Arg Ala Lys Ala Ile Val Gln Ala
            260                 265                 270

Val Thr Asn Tyr Arg Asp Ala Ala Val Leu Ala Glu Val Ser Cys Gly
        275                 280                 285

Leu Gly Glu Ala Met Val Gly Ile Asn Leu Asp Asp Lys Val Glu Arg
    290                 295                 300

Phe Ala Asn Arg Ser Glu
305                 310

<210>  117
<211>  942
<212>  DNA
<213>  Physcomitrella patens

<220>
<221>  CDS
<222>  (1)..(942)

<223>  PDX1

<400>  117
atg gag gag aac agg gtg gtg gcc gtg ctg aac aac ggc agc ggc ctg      48
Met Glu Glu Asn Arg Val Val Ala Val Leu Asn Asn Gly Ser Gly Leu
1               5                   10                  15

agc gag aac cag aac aag aag acc gtg agc tac gcc gtg aag gtg ggc      96
Ser Glu Asn Gln Asn Lys Lys Thr Val Ser Tyr Ala Val Lys Val Gly
            20                  25                  30

ctg gcc cag atg ctg agg ggc ggc gtg atc atg gac gtg gtg gac gtg      144
Leu Ala Gln Met Leu Arg Gly Gly Val Ile Met Asp Val Val Asp Val
        35                  40                  45

gcc cag gcc agg atc gcc gag gag gcc ggc gcc gtg gcc gtg atg gcc      192
Ala Gln Ala Arg Ile Ala Glu Glu Ala Gly Ala Val Ala Val Met Ala
    50                  55                  60

ctg gag agg gtg ccg gcc gac atc agg gcc gag ggc ggc gtg gcc agg      240
Leu Glu Arg Val Pro Ala Asp Ile Arg Ala Glu Gly Gly Val Ala Arg
65                  70                  75                  80

atg agc gac ccg agc atg atc aag gag atc aag aag gcc gtg acc atc      288
Met Ser Asp Pro Ser Met Ile Lys Glu Ile Lys Lys Ala Val Thr Ile
                85                  90                  95

ccg gtg atg gcc aag gcc agg atc ggc cac ttc gtg gag gcc cag atc      336
Pro Val Met Ala Lys Ala Arg Ile Gly His Phe Val Glu Ala Gln Ile
            100                 105                 110

ctg gag gcc atc ggc gtg gac tac atc gac gag agc gag gtg ctg acc      384
Leu Glu Ala Ile Gly Val Asp Tyr Ile Asp Glu Ser Glu Val Leu Thr
        115                 120                 125

ccg gcc gac gac gtg cac cac atc aac aag cac aac tac agg atc ccg      432
Pro Ala Asp Asp Val His His Ile Asn Lys His Asn Tyr Arg Ile Pro
    130                 135                 140

ttc gtg tgc ggc tgc agg aac ctg ggc gag gcc ctg agg agg atc gcc      480
Phe Val Cys Gly Cys Arg Asn Leu Gly Glu Ala Leu Arg Arg Ile Ala
145                 150                 155                 160

gag ggc gcc gcc atg atc agg acc aag ggc gag gcc ggc acc ggc aac      528
Glu Gly Ala Ala Met Ile Arg Thr Lys Gly Glu Ala Gly Thr Gly Asn
                165                 170                 175

gtg gtg gag gcc gtg agg cac acc agg agc gtg ctg ggc gac atc agg      576
Val Val Glu Ala Val Arg His Thr Arg Ser Val Leu Gly Asp Ile Arg
            180                 185                 190

agg ctg cag agc ctg gac gac gac gag gtg ttc acc tac gcc aag gag      624
Arg Leu Gln Ser Leu Asp Asp Asp Glu Val Phe Thr Tyr Ala Lys Glu
        195                 200                 205

atc cag gcc ccg tac gag ctg gtg agg cag acc aag cag ctg ggc agg      672
Ile Gln Ala Pro Tyr Glu Leu Val Arg Gln Thr Lys Gln Leu Gly Arg
    210                 215                 220

ctg ccg gtg gtg aac ttc gcc gcc ggc ggc gtg gcc acc ccg gcc gac      720
Leu Pro Val Val Asn Phe Ala Ala Gly Gly Val Ala Thr Pro Ala Asp
225                 230                 235                 240

gcc gcc ctg atg atg cag ctg ggc tgc gac ggc gtg ttc gtg ggc agc      768
Ala Ala Leu Met Met Gln Leu Gly Cys Asp Gly Val Phe Val Gly Ser
                245                 250                 255

ggc atc ttc aag agc ggc gac ccg gcc aag agg gcc agg gcc atc gtg      816
Gly Ile Phe Lys Ser Gly Asp Pro Ala Lys Arg Ala Arg Ala Ile Val

260                 265                 270

gag gcc gtg acc cac tac aac gac gcc agg gtg ctg gcc gag gtg agc       864
Glu Ala Val Thr His Tyr Asn Asp Ala Arg Val Leu Ala Glu Val Ser
        275                 280                 285

gag aac ctg ggc gag gcc atg gtg ggc atc aac ctg agc gac aag aag       912
Glu Asn Leu Gly Glu Ala Met Val Gly Ile Asn Leu Ser Asp Lys Lys
    290                 295                 300

gtg gag agg ttc gcc gcc agg agc gag tga                               942
Val Glu Arg Phe Ala Ala Arg Ser Glu
305                 310

<210>  118
<211>  313
<212>  PRT
<213>  Physcomitrella patens

<400>  118

Met Glu Glu Asn Arg Val Val Ala Val Leu Asn Asn Gly Ser Gly Leu
1               5                   10                  15

Ser Glu Asn Gln Asn Lys Lys Thr Val Ser Tyr Ala Val Lys Val Gly
            20                  25                  30

Leu Ala Gln Met Leu Arg Gly Gly Val Ile Met Asp Val Val Asp Val
        35                  40                  45

Ala Gln Ala Arg Ile Ala Glu Glu Ala Gly Ala Val Ala Val Met Ala
    50                  55                  60

Leu Glu Arg Val Pro Ala Asp Ile Arg Ala Glu Gly Gly Val Ala Arg
65                  70                  75                  80

Met Ser Asp Pro Ser Met Ile Lys Glu Ile Lys Lys Ala Val Thr Ile
                85                  90                  95

Pro Val Met Ala Lys Ala Arg Ile Gly His Phe Val Glu Ala Gln Ile
            100                 105                 110

Leu Glu Ala Ile Gly Val Asp Tyr Ile Asp Glu Ser Glu Val Leu Thr
        115                 120                 125

Pro Ala Asp Asp Val His His Ile Asn Lys His Asn Tyr Arg Ile Pro
    130                 135                 140

Phe Val Cys Gly Cys Arg Asn Leu Gly Glu Ala Leu Arg Arg Ile Ala
145                 150                 155                 160

Glu Gly Ala Ala Met Ile Arg Thr Lys Gly Glu Ala Gly Thr Gly Asn
                165                 170                 175

Val Val Glu Ala Val Arg His Thr Arg Ser Val Leu Gly Asp Ile Arg
            180                 185                 190

Arg Leu Gln Ser Leu Asp Asp Asp Glu Val Phe Thr Tyr Ala Lys Glu
        195                 200                 205

Ile Gln Ala Pro Tyr Glu Leu Val Arg Gln Thr Lys Gln Leu Gly Arg
    210                 215                 220

Leu Pro Val Val Asn Phe Ala Ala Gly Gly Val Ala Thr Pro Ala Asp
225                 230                 235                 240

Ala Ala Leu Met Met Gln Leu Gly Cys Asp Gly Val Phe Val Gly Ser
                245                 250                 255

Gly Ile Phe Lys Ser Gly Asp Pro Ala Lys Arg Ala Arg Ala Ile Val
            260                 265                 270

Glu Ala Val Thr His Tyr Asn Asp Ala Arg Val Leu Ala Glu Val Ser
        275                 280                 285

Glu Asn Leu Gly Glu Ala Met Val Gly Ile Asn Leu Ser Asp Lys Lys
    290                 295                 300

Val Glu Arg Phe Ala Ala Arg Ser Glu
305                 310

<210>  119
<211>  891
<212>  DNA
<213>  Schizosaccharomyces pombe

<220>
<221>  CDS
<222>  (1)..(891)
<223>  PDX1

<400>  119
atg agc gcc gag atc aag ggc agc acc cag gtg aag gcc ggc ctg gcc      48
Met Ser Ala Glu Ile Lys Gly Ser Thr Gln Val Lys Ala Gly Leu Ala
1               5                   10                  15

cag atg ctg aag ggc ggc gtg atc atg gac gtg gtg aac gcc gag cag      96
Gln Met Leu Lys Gly Gly Val Ile Met Asp Val Val Asn Ala Glu Gln
            20                  25                  30

gcc agg atc gcc gag gcc gcc ggc gcc tgc gcc gtg atg gcc ctg gag      144
Ala Arg Ile Ala Glu Ala Ala Gly Ala Cys Ala Val Met Ala Leu Glu
        35                  40                  45

agg gtg ccg gcc gac atc agg gcc cag ggc ggc gtg gcc agg atg agc      192
Arg Val Pro Ala Asp Ile Arg Ala Gln Gly Gly Val Ala Arg Met Ser
    50                  55                  60

gac ccg agc atg atc aag gag atc cag gcc gcc gtg agc atc ccg gtg      240
Asp Pro Ser Met Ile Lys Glu Ile Gln Ala Ala Val Ser Ile Pro Val
65                  70                  75                  80

atg gcc aag gtg agg atc ggc cac ttc gtg gag gcc cag atc ctg gag      288
Met Ala Lys Val Arg Ile Gly His Phe Val Glu Ala Gln Ile Leu Glu
                85                  90                  95

agc atc ggc gtg gac tac atc gac gag agc gag gtg ctg acc ccg gcc 336
Ser Ile Gly Val Asp Tyr Ile Asp Glu Ser Glu Val Leu Thr Pro Ala
            100                 105                 110

gac gac atc aac cac atc gag aag agc aag ttc acc gtg ccg ttc gtg 384
Asp Asp Ile Asn His Ile Glu Lys Ser Lys Phe Thr Val Pro Phe Val
        115                 120                 125

tgc ggc agc agg aac ctg ggc gag gcc ctg agg agg atc agc gag ggc 432
Cys Gly Ser Arg Asn Leu Gly Glu Ala Leu Arg Arg Ile Ser Glu Gly
    130                 135                 140

gcc gcc atg atc agg acc aag ggc gag gcc ggc acc ggc gac gtg gtg 480
Ala Ala Met Ile Arg Thr Lys Gly Glu Ala Gly Thr Gly Asp Val Val
145                 150                 155                 160

gag gcc gtg agg cac acc agg cag atg cag ggc gag ctg agg agg gtg 528
Glu Ala Val Arg His Thr Arg Gln Met Gln Gly Glu Leu Arg Arg Val
                165                 170                 175

aag agc atg agc ccg gac gag ctg tac acc tac gcc aag gag atc gcc 576
Lys Ser Met Ser Pro Asp Glu Leu Tyr Thr Tyr Ala Lys Glu Ile Ala
            180                 185                 190

gcc ccg atc gac ctg gtg aag gag tgc gcc cag ctg ggc agg ctg ccg 624
Ala Pro Ile Asp Leu Val Lys Glu Cys Ala Gln Leu Gly Arg Leu Pro
        195                 200                 205

gtg gtg aac ttc gcc gcc ggc ggc gtg gcc acc ccg gcc gac gcc gcc 672
Val Val Asn Phe Ala Ala Gly Gly Val Ala Thr Pro Ala Asp Ala Ala
    210                 215                 220

ctg atg atg cag ctg ggc tgc gac ggc gtg ttc gtg ggc agc ggc atc 720
Leu Met Met Gln Leu Gly Cys Asp Gly Val Phe Val Gly Ser Gly Ile
225                 230                 235                 240

ttc ctg agc ggc gac ccg gcc aag agg gcc agg gcc atc gtg agg gcc 768
Phe Leu Ser Gly Asp Pro Ala Lys Arg Ala Arg Ala Ile Val Arg Ala
                245                 250                 255

gtg acc cac tac aac gac ccg aag atc ctg gcc gag gtg agc gag aac 816
Val Thr His Tyr Asn Asp Pro Lys Ile Leu Ala Glu Val Ser Glu Asn
            260                 265                 270

ctg ggc gcc gcc atg gtg ggc agg agc gtg agc agc ctg gag gag aag 864
Leu Gly Ala Ala Met Val Gly Arg Ser Val Ser Ser Leu Glu Glu Lys
        275                 280                 285

gag aag ctg gcc acc agg ggc tgg tga 891
Glu Lys Leu Ala Thr Arg Gly Trp
    290                 295

<210> 120
<211> 296
<212> PRT
<213> Schizosaccharomyces pombe

<400> 120

Met Ser Ala Glu Ile Lys Gly Ser Thr Gln Val Lys Ala Gly Leu Ala
1               5                   10                  15

Gln Met Leu Lys Gly Gly Val Ile Met Asp Val Val Asn Ala Glu Gln
            20                  25                  30

Ala Arg Ile Ala Glu Ala Ala Gly Ala Cys Ala Val Met Ala Leu Glu

35                  40                  45

Arg Val Pro Ala Asp Ile Arg Ala Gln Gly Gly Val Ala Arg Met Ser
    50                  55                  60

Asp Pro Ser Met Ile Lys Glu Ile Gln Ala Ala Val Ser Ile Pro Val
65                  70                  75                  80

Met Ala Lys Val Arg Ile Gly His Phe Val Glu Ala Gln Ile Leu Glu
                85                  90                  95

Ser Ile Gly Val Asp Tyr Ile Asp Glu Ser Glu Val Leu Thr Pro Ala
            100                 105                 110

Asp Asp Ile Asn His Ile Glu Lys Ser Lys Phe Thr Val Pro Phe Val
        115                 120                 125

Cys Gly Ser Arg Asn Leu Gly Glu Ala Leu Arg Arg Ile Ser Glu Gly
    130                 135                 140

Ala Ala Met Ile Arg Thr Lys Gly Glu Ala Gly Thr Gly Asp Val Val
145                 150                 155                 160

Glu Ala Val Arg His Thr Arg Gln Met Gln Gly Glu Leu Arg Arg Val
                165                 170                 175

Lys Ser Met Ser Pro Asp Glu Leu Tyr Thr Tyr Ala Lys Glu Ile Ala
            180                 185                 190

Ala Pro Ile Asp Leu Val Lys Glu Cys Ala Gln Leu Gly Arg Leu Pro
        195                 200                 205

Val Val Asn Phe Ala Ala Gly Gly Val Ala Thr Pro Ala Asp Ala Ala
    210                 215                 220

Leu Met Met Gln Leu Gly Cys Asp Gly Val Phe Val Gly Ser Gly Ile
225                 230                 235                 240

Phe Leu Ser Gly Asp Pro Ala Lys Arg Ala Arg Ala Ile Val Arg Ala
                245                 250                 255

Val Thr His Tyr Asn Asp Pro Lys Ile Leu Ala Glu Val Ser Glu Asn
            260                 265                 270

Leu Gly Ala Ala Met Val Gly Arg Ser Val Ser Ser Leu Glu Glu Lys
        275                 280                 285

Glu Lys Leu Ala Thr Arg Gly Trp
    290                 295

<210>  121
<211>  986

<212>  DNA
<213>  Petroselinum crispum


<220>
<221>  promoter
<222>  (1)..(986)
<223>  Ubiquitin promoter

<400>  121
ctagaattcg aatccaaaaa ttacggatat gaatataggc atatccgtat ccgaattatc      60

cgtttgacag ctagcaacga ttgtacaatt gcttctttaa aaaaggaaga aagaaagaaa     120

gaaaagaatc aacatcagcg ttaacaaacg gccccgttac ggcccaaacg gtcatataga     180

gtaacggcgt taagcgttga aagactccta tcgaaatacg taaccgcaaa cgtgtcatag     240

tcagatcccc tcttccttca ccgcctcaaa cacaaaaata atcttctaca gcctatatat     300

acaacccccc cttctatctc tcctttctca caattcatca tctttctttc tctaccccca     360

attttaagaa atcctctctt ctcctcttca ttttcaaggt aaatctctct ctctctctct     420

ctctctgtta ttccttgttt taattaggta tgtattattg ctagtttgtt aatctgctta     480

tcttatgtat gccttatgtg aatatcttta tcttgttcat ctcatccgtt tagaagctat     540

aaatttgttg atttgactgt gtatctacac gtggttatgt ttatatctaa tcagatatga     600

atttcttcat attgttgcgt ttgtgtgtac caatccgaaa tcgttgattt ttttcattta     660

atcgtgtagc taattgtacg tatacatatg gatctacgta tcaattgttc atctgtttgt     720

gtttgtatgt atacagatct gaaaacatca cttctctcat ctgattgtgt tgttacatac     780

atagatatag atctgttata tcattttttt tattaattgt gtatatatat atgtgcatag     840

atctggatta catgattgtg attatttaca tgattttgtt atttacgtat gtatatatgt     900

agatctggac tttttggagt tgttgacttg attgtatttg tgtgtgtata tgtgtgttct     960

gatcttgata tgttatgtat gtgcag                                          986


<210>  122
<211>  1112
<212>  DNA
<213>  Artificial

<220>
<223>  synthetic sequence


<220>
<221>  promoter
<222>  (1)..(1112)
<223>  Super promoter

<400>  122
ggatccctga aagcgacgtt ggatgttaac atctacaaat tgccttttct tatcgaccat      60

gtacgtaagc gcttacgttt ttggtggacc cttgaggaaa ctggtagctg ttgtgggcct     120

gtggtctcaa gatggatcat taatttccac cttcacctac gatggggggc atcgcaccgg     180

tgagtaatat tgtacggcta agagcgaatt tggcctgtag gatccctgaa agcgacgttg     240

gatgttaaca tctacaaatt gccttttctt atcgaccatg tacgtaagcg cttacgtttt     300

tggtggaccc ttgaggaaac tggtagctgt tgtgggcctg tggtctcaag atggatcatt 360

aatttccacc ttcacctacg atggggggca tcgcaccggt gagtaatatt gtacggctaa 420

gagcgaattt ggcctgtagg atccctgaaa gcgacgttgg atgttaacat ctacaaattg 480

ccttttctta tcgaccatgt acgtaagcgc ttacgttttt ggtggaccct tgaggaaact 540

ggtagctgtt gtgggcctgt ggtctcaaga tggatcatta atttccacct tcacctacga 600

tgggggcat cgcaccggtg agtaatattg tacggctaag agcgaatttg gcctgtagga 660

tccgcgagct ggtcaatccc attgcttttg aagcagctca acattgatct ctttctcgat 720

cgagggagat ttttcaaatc agtgcgcaag acgtgacgta agtatccgag tcagttttta 780

tttttctact aatttggtcg tttatttcgg cgtgtaggac atggcaaccg ggcctgaatt 840

tcgcgggtat tctgtttcta ttccaacttt ttcttgatcc gcagccatta acgacttttg 900

aatagatacg ctgacacgcc aagcctcgct agtcaaaagt gtaccaaaca acgctttaca 960

gcaagaacgg aatgcgcgtg acgctcgcgg tgacgccatt tcgccttttc agaaatggat 1020

aaatagcctt gcttcctatt atatcttccc aaattaccaa tacattacac tagcatctga 1080

atttcataac caatctcgat acaccaaatc ga 1112

<210> 123
<211> 695
<212> DNA
<213> Vicia faba

<220>
<221> promoter
<222> (1)..(695)
<223> USP promoter variant used for expression of yeast genes in plants

<400> 123
ctagactgca gcaaatttac acattgccac taaacgtcta aacccttgta atttgttttt 60

gttttactat gtgtgttatg tatttgattt gcgataaatt tttatatttg gtactaaatt 120

tataacacct tttatgctaa cgtttgccaa cacttagcaa tttgcaagtt gattaattga 180

ttctaaatta tttttgtctt ctaaatacat atactaatca actggaaatg taaatatttg 240

ctaatatttc tactatagga gaattaaagt gagtgaatat ggtaccacaa ggtttggaga 300

tttaattgtt gcaatgctgc atggatggca tatacaccaa acattcaata attcttgagg 360

ataataatgg taccacacaa gatttgaggt gcatgaacgt cacgtggaca aaaggtttag 420

taatttttca agacaacaat gttaccacac acaagttttg aggtgcatgc atggatgccc 480

tgtggaaagt ttaaaaatat tttggaaatg atttgcatgg aagccatgtg taaaaccatg 540

acatccactt ggaggatgca ataatgaaga aaactacaaa tttacatgca actagttatg 600

catgtagtct atataatgag gattttgcaa tactttcatt catacacact cactaagttt 660

tacacgatta taatttcttc ataccattaa ttaag 695

<210> 124
<211> 674

<212>  DNA
<213>  Vicia faba

<220>
<221>  promoter
<222>  (1)..(674)
<223>  USP promoter used for expression of bacterial genes in plants

<400>  124
caaatttaca cattgccact aaacgtctaa acccttgtaa tttgtttttg ttttactatg      60

tgtgttatgt atttgatttg cgataaattt ttatatttgg tactaaattt ataacacctt     120

ttatgctaac gtttgccaac acttagcaat ttgcaagttg attaattgat tctaaattat     180

ttttgtcttc taaatacata tactaatcaa ctggaaatgt aaatatttgc taatatttct     240

actataggag aattaaagtg agtgaatatg gtaccacaag gtttggagat ttaattgttg     300

caatgctgca tggatggcat atacaccaaa cattcaataa ttcttgagga taataatggt     360

accacacaag atttgaggtg catgaacgtc acgtggacaa aaggtttagt aatttttcaa     420

gacaacaatg ttaccacaca caagttttga ggtgcatgca tggatgccct gtggaaagtt     480

taaaaatatt ttggaaatga tttgcatgga agccatgtgt aaaaccatga catccacttg     540

gaggatgcaa taatgaagaa aactacaaat ttacatgcaa ctagttatgc atgtagtcta     600

tataatgagg attttgcaat actttcattc atacacactc actaagtttt acacgattat     660

aatttcttca tagc                                                       674

<210>  125
<211>  102
<212>  DNA
<213>  Arabidopsis thaliana

<220>
<221>  CDS
<222>  (1)..(102)

<220>
<221>  transit_peptide
<222>  (1)..(81)
<223>  for targeting translational fusions to the mitochondria

<220>
<221>  misc_feature
<222>  (82)..(102)
<223>  linker sequence used in translational fusions with bacterial
       genes

<400>  125
atg cag agg ttt ttc tcc gcc aga tcg att ctc ggt tac gcc gtc aag       48
Met Gln Arg Phe Phe Ser Ala Arg Ser Ile Leu Gly Tyr Ala Val Lys
1               5                   10                  15

acg cgg agg agg tct ttc tct tct cgt tct tcg tct ctc ctt tgc tct       96
Thr Arg Arg Arg Ser Phe Ser Ser Arg Ser Ser Ser Leu Leu Cys Ser
            20                  25                  30

tcc atg                                                               102
Ser Met

<210>  126
<211>  34
<212>  PRT
<213>  Arabidopsis thaliana

<400>  126

Met Gln Arg Phe Phe Ser Ala Arg Ser Ile Leu Gly Tyr Ala Val Lys
1               5                   10                  15


Thr Arg Arg Arg Ser Phe Ser Ser Arg Ser Ser Ser Leu Leu Cys Ser
            20                  25                  30


Ser Met




<210>  127
<211>  102
<212>  DNA
<213>  Arabidopsis thaliana



<220>
<221>  CDS
<222>  (1)..(102)

<220>
<221>  transit_peptide
<222>  (1)..(81)
<223>  for targeting translational fusions to the mitochondria

<220>
<221>  misc_feature
<222>  (82)..(102)
<223>  linker sequence used in translational fusions with yeast genes

<400>  127
atg cag agg ttt ttc tcc gcc aga tcg att ctc ggt tac gcc gtc aag       48
Met Gln Arg Phe Phe Ser Ala Arg Ser Ile Leu Gly Tyr Ala Val Lys
1               5                   10                  15

acg cgg agg agg tct ttc tct tct cgt tct tcg gaa ttc cag ctg acc       96
Thr Arg Arg Arg Ser Phe Ser Ser Arg Ser Ser Glu Phe Gln Leu Thr
            20                  25                  30

acc atg                                                               102
Thr Met



<210>  128
<211>  34
<212>  PRT
<213>  Arabidopsis thaliana

<400>  128

Met Gln Arg Phe Phe Ser Ala Arg Ser Ile Leu Gly Tyr Ala Val Lys
1               5                   10                  15


Thr Arg Arg Arg Ser Phe Ser Ser Arg Ser Ser Glu Phe Gln Leu Thr
            20                  25                  30

Thr Met

<210> 129
<211> 419
<212> DNA
<213> Spinacia oleracea

<220>
<221> 5'UTR
<222> (1)..(165)
<223> 5' untranslated region

<220>
<221> CDS
<222> (166)..(273)
<223> first exon of mature chloroplast transit peptide

<220>
<221> Intron
<222> (274)..(350)
<223> intron

<220>
<221> CDS
<222> (351)..(419)
<223> second exon of mature chloroplast transit peptide

<400> 129
gcataaactt atcttcatag ttgccactcc aatttgctcc ttgaatctcc tccacccaat 60

acataatcca ctcctccatc acccacttca ctactaaatc aaacttaact ctgtttttct 120

ctctcctcct ttcatttctt attcttccaa tcatcgtact ccgcc atg acc acc gct 177
Met Thr Thr Ala
1

gtc acc gcc gct gtt tct ttc ccc tct acc aaa acc acc tct ctc tcc 225
Val Thr Ala Ala Val Ser Phe Pro Ser Thr Lys Thr Thr Ser Leu Ser
5                   10                  15                  20

gcc cga agc tcc tcc gtc att tcc cct gac aaa atc agc tac aaa aag 273
Ala Arg Ser Ser Ser Val Ile Ser Pro Asp Lys Ile Ser Tyr Lys Lys
                25                  30                  35

gtgattccca atttcactgt gtttttattt aataatttgt tattttgatg atgagatgat 333

taatttgggt gctgcag gtt cct ttg tac tac agg aat gta tct gca act 383
Val Pro Leu Tyr Tyr Arg Asn Val Ser Ala Thr
            40                  45

ggg aaa atg gga ccc atc agg gcc cag atc gcc tct 419
Gly Lys Met Gly Pro Ile Arg Ala Gln Ile Ala Ser
        50                  55

<210> 130
<211> 59
<212> PRT
<213> Spinacia oleracea

<400> 130

Met Thr Thr Ala Val Thr Ala Ala Val Ser Phe Pro Ser Thr Lys Thr
1               5                   10                  15

Thr Ser Leu Ser Ala Arg Ser Ser Ser Val Ile Ser Pro Asp Lys Ile
            20                  25                  30

Ser Tyr Lys Lys Val Pro Leu Tyr Tyr Arg Asn Val Ser Ala Thr Gly
        35                  40                  45

Lys Met Gly Pro Ile Arg Ala Gln Ile Ala Ser
    50                  55